# EUROPEAN PATENT SPECIFICATION

(11) **EP 4 444 708 B1**
(45) Date of publication and mention of the grant of the patent: **30.07.2025**
(21) Application number: 22818912.2
(22) Date of filing: 05.12.2022
(51) Int. Cl.: C07D 401/14, C07D 471/04, A61P 1/08, A61P 3/04, A61P 19/00, A61P 21/00, A61P 29/00, C07D 413/14, C07D 498/04, A61K 31/4439, A61K 31/444, A61K 31/506, A61K 31/437

(54) **MELANOCORTIN 4 RECEPTOR ANTAGONISTS AND USES THEREOF**
MELANOCORTIN-4-REZEPTORANTAGONISTEN UND VERWENDUNGEN DAVON
ANTAGONISTES DU RÉCEPTEUR 4 DE LA MÉLANOCORTINE ET LEURS UTILISATIONS

(30) Priority: 06.12.2021 US 202163286385 P; 15.11.2022 US 202263383780 P
(43) Date of publication of application: 16.10.2024
(73) Proprietor: Pfizer Inc., New York, NY 10001-2192 (US)
(72) Inventor: GARNSEY, Michelle Renee, Cambridge, Massachusetts 02139 (US); GRIFFITH, David Andrew, Cambridge, Massachusetts 02139 (US); HELAL, Christopher John, Somerville, Massachusetts 02144 (US); KUNG, Daniel Wei-Shung, Groton, Connecticut 06340 (US); LIAN, Yajing, Groton, Connecticut 06340 (US); OGILVIE, Kevin Alexander, Groton, Connecticut 06340 (US); POLIVKOVA, Jana, Groton, Connecticut 06340 (US); RAYMER, Brian, Holliston, Massachusetts 01746 (US); SAMMONS, Matthew Forrest, Cambridge, Massachusetts 02139 (US); SMITH, Aaron Christopher, Golden, Colorado 80403 (US); YANG, Qingyi, Cambridge, Massachusetts 02139 (US)
(74) Representative: Pfizer
(86) International application number: PCT/IB2022/061778
(87) International publication number: WO 2023/105387

(56) References cited:
- EP-A1- 2 003 131
- WO-A1-2008/077089
- WO-A1-2010/052256
- WO-A1-2010/081666
- WO-A1-2019/243974

## Description

### FIELD OF THE INVENTION

The present invention relates to new pharmaceutical compounds, pharmaceutical compositions containing the compounds, and use of the compounds as melanocortin receptor 4 (MC4R) antagonists. The invention is set out in the appended set of claims. In addition, any reference to methods of treatment in the subsequent paragraphs of this description is to be interpreted as references to the compounds, pharmaceutical compositions and medicaments of the present invention for use in a method for treatment of the human or animal body by therapy.

### BACKGROUND OF THE INVENTION

Melanocortins are peptides derived from pro-opiomelanocortins (POMC) that bind to and activate G-protein coupled receptors (GPCR's) of the melanocortin receptor family. Melanocortins regulate a diverse number of physiological processes including sexual function and sexual behaviour, food intake and metabolism. To date, five melanocortin receptors (MCRs) have been identified in mammals, MC1R, MC2R, MC3R, MC4R, and MC5R, which are expressed in various tissues. MC1R is specifically expressed in melanocytes and melanoma cells, MC2R is the ACTH receptor and is expressed in adrenal tissue, MC3R is predominantly expressed in the brain and limbic system, MC4R is widely expressed in the brain and spinal cord, and MC5R is expressed in the brain and many peripheral tissues including skin, adipose tissue, skeletal muscle, and lymphoid tissue. *See e.g.,* US Patent No 8,138,188 and Saleh et al., Front. Pharmacol., 2018, 9:560.

MC4R is a G-protein-coupled seven-transmembrane receptor primarily expressed in the hypothalamus, hippocampus, and thalamus (Gantz et al. 1993 J. Biol. Chem. 268:15174-15179). The receptor is implicated in the central regulation of body weight: MC4R is activated by α-melanocyte-stimulating hormone (MSH), which is derived from pro-opiomelanocortin and is inactivated by agouti gene-related protein (AGRP). α-MSH induces weight loss, whereas the ectopic expression of agouti protein results in obesity in the agouti mice (Fan et al. 1993 Nature 385:165-168; Lu et al. 1994 Nature 371:799-802). Additional evidence for the role of MC4R in weight regulation stems from both a knockout model in mice (Huszar et al. 1997 Cell 88:131-141) and haploinsufficiency mutations in humans (Vaisse et al. 1998 Nat. Genet. 20:113-114; Yeo et al. 1998 Nat. Genet. 20:111-112; Hinney et al. 1999 J. Clin. Endocrinol. Metab. 84:1483-1486). In *MC4R*-knockout mice, an increased body weight was discernible by age 5 wk. By age 15 wk, homozygous mutant females were, on average, twice as heavy as their wild-type littermates, whereas homozygous mutant males were ~50% heavier than wild-type controls. Mice heterozygous for the MC4R knockout showed a weight gain intermediate to that seen in wild-type and homozygous mutant littermates, thus demonstrating a gene dosage effect of MC4R ablation on body-weight regulation. The food intake of homozygous mutants was increased by ~50% in comparison to that in wild-type sibs (Huszar et al. 1997 Cell 88:131-141). [From Am. J. Hum. Genet, 65:1501-1507,1999]. MC4R activation has been shown to induce penile erection in rodents and MC4R inactivation has been shown to cause obesity (reviewed in Hadley, 1999, Ann. NY Acad. Sci., 885:1-21; Wikberg et al. 2000, Pharmacol. Res., 42(5), 393-420; and Saleh et al., Front. Pharmacol., 2018, 9:560).

In recent years, several kinds of small-molecule MC4R antagonists have been reported in the literature and patent applications [see e.g., EP2003131 WO2010052256; WO2010081666; US Patent 8,044,068; Chaki et al., Current Topics in Medicinal Chemistry, 2007, 7, 1145-1151; Foster et al., Current Topics in Medicinal Chemistry, 2007, 7, 1131-1136; Pontillo et al., Bioorganic & Medicinal Chemistry Letters, 2005, 15, 2541-2546; Vos et al., Bioorganic & Medicinal Chemistry Letters, 2006, 16, 2302-2305; Tao, Endocrine Reviews, 2010, 31(4):506-543; and Saleh et al., Front. Pharmacol., 2018, 9: 560]. These MC4R antagonists are useful for treating and/or preventing MC4R-related conditions, diseases, or disorders, for example, cachexia [including for example, cachexia associated with a chronic illness, such as cachexia associated with cancer, cachexia associated with acquired immunodeficiency syndrome (AIDS), cachexia associated with heart failure for example cachexia associated with congestive heart failure (CHF), cachexia associated with chronic kidney disease (CKD); cachexia associated with treatment of a chronic illness, such as cachexia associated with treatment of cancer or cachexia associated with treatment of heart failure (e.g., CHF)]; anorexia or anorexia nervosa (e.g., geriatric anorexia, anorexia associated with chemotherapy and/or radiotherapy); nausea; emesis; weight loss (e.g., involuntary weight loss); failure to thrive; sarcopenia; muscle wasting; muscle weakness; frailty; osteoporosis; bone disorders (e.g., bone loss); pain; neuropathic pain; anxiety (e.g., posttraumatic stress disorder, or PTSD); depression; hypertension; malnutrition; obesity (e.g., sarcopenia resulting from chronic obesity); sexual dysfunction; and inflammatory disease (e.g., an inflammatory disease associated with anorexia or cachexia or sarcopenia or muscle wasting).

There continues to be a need for alternative MC4R antagonists, for example, for developing new and/or improved pharmaceuticals (e.g., more effective, more selective, less toxic, and/or having improved biopharmaceutical properties such as physical stability; solubility; oral bioavailability; appropriate metabolic stability; clearance; half-life) to treat or prevent MC4R-related conditions, diseases, or disorders, such as those described herein. The present invention is directed to these and other important ends.

### SUMMARY OF THE INVENTION

In one embodiment, the present invention provides a compound of Formula I: 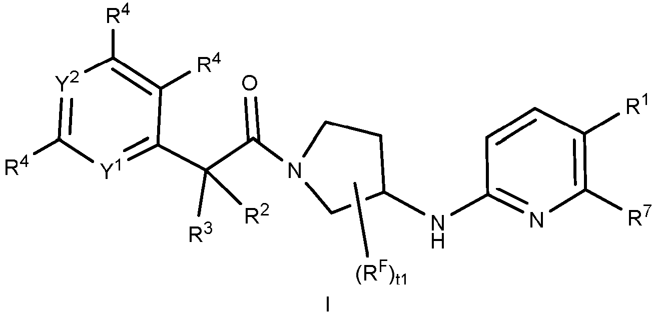 or a pharmaceutically acceptable salt thereof, wherein:
R¹ is 1,2,4-triazolyl, tetrazolyl, 1,2,4-oxadiazolyl, imidazolyl, 6,7-dihydro-[1,2,4]triazolo[5,1-b][1,3]oxazinyl, [1,2,4]triazolo[1,5-*a*]pyridinyl-, pyrazolyl, or pyrimidinyl, each of which is optionally substituted with 1, 2, or 3 substituents each independently selected from halogen, C₁₋₄ alkyl, C₁₋₄ haloalkyl, and C₃₋₄ cycloalkyl;
each of R² and R³ is independently H, halogen, C₁₋₄ alkyl, or C₁₋₄ haloalkyl;
or R² and R³ together with the carbon atom to which they are attached form a C₃₋₄ cycloalkyl optionally substituted with 1, 2, 3, 4, or 5 substituents each independently selected from halogen;
each of Y¹ and Y² is independently CR⁴ or N;
each R⁴ is independently H, halogen, C₁₋₄ alkyl, C₁₋₄ haloalkyl, C₁₋₄ alkoxy, or C₁₋₄ haloalkoxy;
each R^{F} is independently halogen, -OH, C₁₋₄ alkyl, C₁₋₄ haloalkyl, C₁₋₄ alkoxy, C₁₋₄ haloalkoxy, C₃₋₄ cycloalkyl, or (C₃₋₄ cycloalkyl)-C₁₋₄ alkyl-;
or when two R^{F} groups are substituted on the same carbon atom of the pyrrolidine ring of Formula I, they, optionally, together with the carbon atom to which they are attached form a C₃₋₆ cycloalkyl optionally substituted with 1, 2, 3, 4, or 5 substituents each independently selected from halogen, -OH, C₁₋₄ alkyl, C₁₋₄ haloalkyl, C₁₋₄ alkoxy, and C₁₋₄ haloalkoxy;
R⁷ is methyl or ethyl; and
t1 is 0, 1, 2, 3, or 4.

In one embodiment, the present invention provides a compound of Formula I: 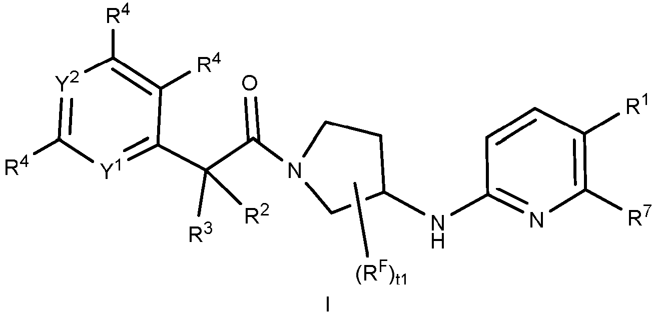 or a pharmaceutically acceptable salt thereof, wherein:
R¹ is 1,2,4-triazolyl, tetrazolyl, 1,2,4-oxadiazolyl, imidazolyl, 6,7-dihydro-[1,2,4]triazolo[5,1-b][1,3]oxazinyl, [1,2,4]triazolo[1,5-*a*]pyridinyl-, pyrazolyl, or pyrimidinyl, each of which is optionally substituted with 1, 2, or 3 substituents each independently selected from halogen, C₁₋₄ alkyl, C₁₋₄ haloalkyl, and C₃₋₄ cycloalkyl;
each of R² and R³ is independently H, halogen, C₁₋₄ alkyl, or C₁₋₄ haloalkyl;
or R² and R³ together with the carbon atom to which they are attached form a C₃₋₄ cycloalkyl optionally substituted with 1, 2, 3, 4, or 5 substituents each independently selected from halogen;
each of Y¹ and Y² is independently CR⁴ or N;
each R⁴ is independently H, halogen, C₁₋₄ alkyl, C₁₋₄ haloalkyl, C₁₋₄ alkoxy, or C₁₋₄ haloalkoxy;
each R^{F} is independently halogen, -OH, C₁₋₄ alkyl, C₁₋₄ haloalkyl, C₁₋₄ alkoxy, C₁₋₄ haloalkoxy, C₃₋₄ cycloalkyl, or (C₃₋₄ cycloalkyl)-C₁₋₄ alkyl-;
or when two R^{F} groups are substituted on the same carbon atom of the pyrrolidine ring of Formula I, they together with the carbon atom to which they are attached form a C₃₋₆ cycloalkyl optionally substituted with 1, 2, 3, 4, or 5 substituents each independently selected from halogen, -OH, C₁₋₄ alkyl, C₁₋₄ haloalkyl, C₁₋₄ alkoxy, and C₁₋₄ haloalkoxy;
R⁷ is methyl or ethyl; and
t1 is 0, 1, 2, 3, or 4.

The present invention also provides a pharmaceutical composition having a therapeutically effective amount of the compound of Formula I or a pharmaceutically acceptable salt of the compound and a pharmaceutically acceptable carrier.

The present invention also provides a method for treating an MC4R-related condition, disease, or disorder in a mammal (e.g., a human) in need of such treatment, which method includes administering to the mammal (e.g., the human) the compound of Formula I or a pharmaceutically acceptable salt of the compound.

The present invention also provides the compound of Formula I or a pharmaceutically acceptable salt of the compound for use in treating an MC4R-related condition, disease, or disorder.

The MC4R-related condition, disease, or disorder includes one selected from cachexia [including for example, cachexia associated with a chronic illness, such as cachexia associated with cancer, cachexia associated with acquired immunodeficiency syndrome (AIDS), cachexia associated with heart failure for example cachexia associated with congestive heart failure (CHF), cachexia associated with chronic kidney disease (CKD); cachexia associated with treatment of a chronic illness, such as cachexia associated with treatment of cancer or cachexia associated with treatment of heart failure (e.g., CHF)]; anorexia or anorexia nervosa (e.g., geriatric anorexia, anorexia associated with chemotherapy and/or radiotherapy); nausea; emesis; weight loss (e.g., involuntary weight loss); failure to thrive; sarcopenia; muscle wasting; muscle weakness [e.g., muscle weakness associated with chronic obstructive pulmonary disease (COPD)]; frailty; osteoporosis; bone disorders (e.g., bone loss); pain; neuropathic pain; anxiety (e.g., posttraumatic stress disorder, or PTSD); depression; hypertension; malnutrition; obesity (e.g. sarcopenia resulting from chronic obesity); sexual dysfunction; and inflammatory disease (e.g., an inflammatory disease associated with anorexia or cachexia or sarcopenia or muscle wasting).

The present invention also provides a method for treating a condition, disease, or disorder in a mammal (e.g., a human) in need of such treatment, which method includes administering to the mammal (e.g., the human) the compound of Formula I or a pharmaceutically acceptable salt of the compound, wherein the condition, disease, or disorder is selected from cachexia [including for example, cachexia associated with a chronic illness, such as cachexia associated with cancer, cachexia associated with acquired immunodeficiency syndrome (AIDS), cachexia associated with heart failure for example cachexia associated with congestive heart failure (CHF), cachexia associated with chronic kidney disease (CKD); cachexia associated with treatment of a chronic illness, such as, cachexia associated with treatment of cancer or cachexia associated with treatment of heart failure (e.g. CHF)]; anorexia or anorexia nervosa (e.g., geriatric anorexia, anorexia associated with chemotherapy and/or radiotherapy); nausea; emesis; weight loss (e.g., involuntary weight loss); failure to thrive; sarcopenia; muscle wasting; muscle weakness; frailty; osteoporosis; bone disorders (e.g., bone loss); pain; neuropathic pain; anxiety (e.g., posttraumatic stress disorder, or PTSD); depression; hypertension; malnutrition; obesity (e.g. sarcopenia resulting from chronic obesity); sexual dysfunction; and inflammatory disease (e.g. an inflammatory disease associated with anorexia or cachexia or sarcopenia or muscle wasting).

The present invention also provides a method for antagonizing a melanocortin-4 receptor (MC4R), which method includes contacting the MC4R with the compound of Formula I or a pharmaceutically acceptable salt of the compound.

It is to be understood that both the foregoing general description and the following detailed description are exemplary and explanatory only and are not restrictive of the invention, as claimed.

### BRIEF DESCRIPTION OF THE DRAWINGS

FIG. 1 shows an illustrative single crystal structure of compound Example 3A.
FIG. 2 shows an illustrative single crystal structure of compound Example 6.
FIG. 3 shows an illustrative single crystal structure of compound Example 7.

### DETAILED DESCRIPTION OF THE INVENTION

The present invention may be understood more readily by reference to the following detailed description of exemplary embodiments of the invention and the examples included therein.

It is to be understood that this invention is not limited to specific synthetic methods of making that may of course vary. It is also to be understood that the terminology used herein is for the purpose of describing particular embodiments only and is not intended to be limiting. In this specification and in the claims that follow, reference will be made to a number of terms that shall be defined to have the following meanings:
As used herein in the specification, "a" or "an" may mean one or more. As used herein in the claim(s), when used in conjunction with the word "comprising", the words "a" or "an" may mean one or more than one. As used herein "another" may mean at least a second or more.

The term "about" refers to a relative term denoting an approximation of plus or minus 10% of the nominal value to which it refers, in one embodiment, to plus or minus 5%, in another embodiment, to plus or minus 2%. For the field of this disclosure, this level of approximation is appropriate unless the value is specifically stated to require a tighter range.

The term "and/or" means one or more stated expressions/possibilities recited in connection with this term. For example, "X and/or Y" shall be understood to mean either "X and Y" or "X or Y" and shall be taken to provide explicit support for both meanings or for either meaning. Similarly, when more than 2 expressions/possibilities are listed, such as in "X, Y and/or Z", it shall be understood to mean either i) "X and Y", "X, Y and Z", "X and Z", or "Y and Z", or ii) "X or Y", "X or Z", "Y or Z", or "X or Y or Z" and shall be taken to provide explicit support for all meanings.

"Compound" when used herein includes any pharmaceutically acceptable conformational isomers (e.g., cis and trans isomers) and all optical isomers (e.g., enantiomers and diastereomers), racemic, diastereomeric and other mixtures of such isomers, as well as solvates, hydrates, isomorphs, polymorphs, tautomers, and salt forms. The expression "prodrug" refers to compounds that are drug precursors which following administration, release the drug *in vivo* via some chemical or physiological process (e.g., a prodrug on being brought to the physiological pH or through enzyme action is converted to the desired drug form).

The term "alkyl" means an acyclic, saturated aliphatic hydrocarbon group which may be straight/linear or branched. Examples of such groups include, but are not limited to, methyl, ethyl, *n*-propyl, isopropyl, butyl, *sec*-butyl, isobutyl and *tert*-butyl. The carbon atom content of alkyl and various other hydrocarbon-containing moieties is indicated by a prefix designating a lower and upper number of carbon atoms in the moiety, that is, the prefix Cᵢ₋ⱼ indicates a moiety of the integer "i" to the integer "j" carbon atoms, inclusive. Thus, for example, C₁₋₄ alkyl refers to alkyl of one to four carbon atoms, inclusive. Representative examples of C₁₋₄ alkyl include methyl, ethyl, *n*-propyl, isopropyl, *n*-butyl, *sec*-butyl, isobutyl, and *tert*-butyl. For another example, C₁₋₄ alkyl refers to alkyl of one to two carbon atoms, inclusive (i.e., methyl or ethyl). The alkyl group optionally can be substituted by 1 or more (e.g., 1 to 5) suitable substituents, when so specified.

At various places in the present specification, substituents of compounds of the invention are disclosed in groups or in ranges. It is specifically intended that the invention include each and every individual sub-combination of the members of such groups and ranges. For example, the term "C₁₋₄ alkyl" is specifically intended to include C₁ alkyl (methyl), C₂ alkyl (ethyl), C₃ alkyl, and C₄ alkyl. For another example, the term "4- to 7-membered heterocycloalkyl" is specifically intended to include any 4-, 5-, 6-, or 7-membered heterocycloalkyl group.

As used herein, the term "n-membered", where n is an integer, typically describes the number of ring-forming atoms in a moiety where the number of ring-forming atoms is n. For example, pyridinyl is an example of a 6-membered heteroaryl ring and pyrazolyl is an example of a 5-membered heteroaryl group.

As used herein, the term "alkoxy" or "alkyloxy" refers to an -O-alkyl group. For example, the term "C₁₋₄ alkoxy" or "C₁₋₄ alkyloxy" refers to an -O-(C₁₋₄ alkyl) group; For another example, the term "C₁₋₂ alkoxy" or "C₁₋₂ alkyloxy" refers to an -O-(C₁₋₂ alkyl) group. Examples of alkoxy include methoxy, ethoxy, propoxy (e.g., *n*-propoxy and isopropoxy), *tert*-butoxy, and the like. The alkoxy or alkyloxy group optionally can be substituted by 1 or more (e.g., 1 to 5) suitable substituents when so specified.

The term "halo" or "halogen" as used herein, means -F, -Cl, -Br, or -I.

As used herein, the term "haloalkyl" refers to an alkyl group having one or more halogen substituents (up to perhaloalkyl, i.e., every hydrogen atom of the alkyl group has been replaced by a halogen atom). For example, the term "C₁₋₄ haloalkyl" refers to a C₁₋₄ alkyl group having one or more halogen substituents (up to perhaloalkyl, i.e., every hydrogen atom of the alkyl group has been replaced by a halogen atom); and the term "C₁₋₂ haloalkyl" refers to a C₁₋₂ alkyl group (i.e., methyl or ethyl) having one or more halogen substituents (up to perhaloalkyl, i.e., every hydrogen atom of the alkyl group has been replaced by a halogen atom). Examples of haloalkyl groups include -CF₃, -CHF₂, -CH₂F, -CH₂CF₃, -C₂F₅, -CH₂Cl and the like.

"Fluoroalkyl" means an alkyl as defined herein substituted with one or more fluoro (-F) substituents (up to perfluoroalkyl, i.e., every hydrogen atom of the alkyl group has been replaced by a fluorine atom). The term "C₁₋₂ fluoroalkyl" refers to a C₁₋₂ alkyl group (i.e., methyl or ethyl) having one or more fluorine substituents (up to perfluoroalkyl, i.e., every hydrogen atom of the alkyl group has been replaced by a fluorine atom); and the term "C₁ fluoroalkyl" refers to methyl having 1, 2, or 3 fluorine substituents. Examples of C₁ fluoroalkyl include fluoromethyl, difluoromethyl and trifluoromethyl; some examples of C₂ fluoroalkyl include 1-fluoroethyl, 2-fluoroethyl, 2,2-difluoroethyl, 1,2-difluoroethyl, 2,2,2-trifluoroethyl, 1,1,2-trifluoroethyl, and the like.

As used here, the term "haloalkoxy" refers to an -O-haloalkyl group. For example, the term "C₁₋₄ haloalkoxy" refers to an -O-(C₁₋₄ haloalkyl) group; and the term "C₁₋₂ haloalkoxy" refers to an -O-(C₁₂ haloalkyl) group. For yet another example, the term "C₁ haloalkoxy" refers to a methoxy group having one, two, or three halogen substituents. An example of haloalkoxy is -OCF₃ or -OCHF₂.

As used here, the term "fluoroalkoxy" refers to an -O-fluoroalkyl group. For example, the term "C₁₋₂ fluoroalkoxy" refers to an -O-(C₁₋₂ fluoroalkyl) group; and the term "C₁ fluoroalkoxy" refers to an -O-(C₁ fluoroalkyl) group. Examples of C₁ fluoroalkoxy include -O-CH₂F, -O-CHF₂, and -O-CF₃. Some examples of C₂ fluoroalkoxy include -O-CH₂CHF₂, -O-CH₂-CHF₂, -O-CH₂CF₃, -O-CF₂CH₃, and -O-CF₂CF₃.

As used herein, the term "hydroxylalkyl" or "hydroxyalkyl" refers to an alkyl group having one or more (e.g., 1, 2, or 3) OH substituents. The term "C₁₋₄ hydroxylalkyl" or "C₁₋₄ hydroxyalkyl" refers to a C₁₋₄ alkyl group having one or more (e.g., 1, 2, or 3) OH substituents; and the term "C₁₋₂ hydroxylalkyl" or "C₁₋₂ hydroxyalkyl" refers to a C₁₋₂ alkyl group having one or more (e.g., 1, 2, or 3) OH substituents. An example of hydroxylalkyl is -CH₂OH or - CH₂CH₂OH.

As used herein, the term "cycloalkyl" refers to saturated or unsaturated, non-aromatic, monocyclic or polycyclic (such as bicyclic) hydrocarbon rings (e.g., monocyclics such as cyclopropyl, cyclobutyl, cyclopentyl, cyclohexyl, cycloheptyl, cyclooctyl, cyclononyl, or bicyclics including spiro, fused, or bridged systems (such as bicyclo[1.1.1]pentanyl, bicyclo[2.2.1]heptanyl, bicyclo[3.2.1]octanyl or bicyclo[5.2.0]nonanyl, decahydronaphthalenyl, etc.). The cycloalkyl group has 3 to 15 (e.g., 3 to 14, 3 to 10, 3 to 6, 3 to 4, or 4 to 6) carbon atoms. In some embodiments the cycloalkyl may optionally contain one, two, or more non-cumulative non-aromatic double or triple bonds and/or one to three oxo groups. In some embodiments, the bicycloalkyl group has 6 to 14 carbon atoms. The term "C₃₋₄ cycloalkyl" as used herein, means a saturated cyclic hydrocarbon group containing from 3 to 4 carbons. Examples of C₃₋₄ cycloalkyl include cyclopropyl and cyclobutyl. Also included in the definition of cycloalkyl are moieties that have one or more aromatic rings (including aryl and heteroaryl) fused to the cycloalkyl ring, for example, benzo or pyridinyl derivatives of cyclopentane (a 5-membered cycloalkyl), cyclopentene, cyclohexane (a 6-membered cycloalkyl), and the like, for example, 6,7-dihydro-5*H*-cyclopenta[b]pyridinyl, 5,6,7,8-tetrahydroquinolinyl, or 1 5,6,7,8-tetrahydroisoquinolinyl, each of which includes a 5-membered or 6-membered cycloalkyl moiety that is fused to a heteroaryl ring (i.e., the pyridinyl ring). The cycloalkyl or C₃₋₄ cycloalkyl group optionally can be substituted by 1 or more (e.g., 1 to 5) suitable substituents when so specified.

The term " C₃₋₄ cycloalkyl-C₁₋₄ alkyl-" as used herein, means a C₃₋₄ cycloalkyl as defined herein, appended to the parent molecular moiety through a C₁₋₄ alkyl group, as defined herein. Some examples of C₃₋₄ cycloalkyl-C₁₋₄ alkyl- include cyclopropylmethyl, 2-cyclopropylethyl, 2-cyclopropylpropyl, 3-cyclopropylpropyl, cyclobutylmethyl, 2-cyclobutylethyl, 2-cyclobutylpropyl, and 3-cyclobutylpropyl.

As used herein, the term "heterocycloalkyl" refers to a monocyclic or polycyclic [including 2 or more rings that are fused together, including spiro, fused, or bridged systems, for example, a bicyclic ring system], saturated or unsaturated, non-aromatic 4- to 15-membered ring system (such as a 4- to 14-membered ring system, 4- to 12-membered ring system, 5- to 10-membered ring system, 4- to 7-membered ring system, 4- to 6-membered ring system, or 5- to 6-membered ring system), including 1 to 14 ring-forming carbon atoms and 1 to 10 ring-forming heteroatoms each independently selected from O, S and N (and optionally P or B when present). The heterocycloalkyl group can also optionally contain one or more oxo (i.e., =O) or thiono (i.e., =S) groups. For example, the term "4- to 7-membered heterocycloalkyl" refers to a monocyclic or polycyclic, saturated or unsaturated, non-aromatic 4- to 7-membered ring system that comprises one or more ring-forming heteroatoms each independently selected from O, S and N. For another example, the term "5- or 6-membered heterocycloalkyl" refers to a monocyclic or polycyclic, saturated or unsaturated, non-aromatic 5- or 6-membered ring system that comprises one or more ring-forming heteroatoms each independently selected from O, S and N. The heterocycloalkyl group optionally can be substituted by 1 or more (e.g., 1 to 5) suitable substituents, when so specified.

Some examples of 4- to 7-membered heterocycloalkyl include azetidinyl, oxetanyl, tetrahydrofuranyl, imidazolidinyl, pyrrolidinyl, piperidinyl, piperazinyl, oxazolidinyl, thiazolidinyl, pyrazolidinyl, thiomorpholinyl, tetrahydrothiazinyl, tetrahydrothiadiazinyl, morpholinyl, 1,3-oxazinanyl, tetrahydrodiazinyl, and tetrahydropyranyl (also known as oxanyl). Some further examples of 4- to 7-heterocycloalkyl include tetrahydrofuran-2-yl, tetrahydrofuran-3-yl, tetrahydropyranyl (e.g., tetrahydro-2*H*-pyran-4-yl), imidazolidin-1-yl, imidazolidin-2-yl, imidazolidin-4-yl, pyrrolidin-1-yl, pyrrolidin-2-yl, pyrrolidin-3-yl, piperidin-1-yl, piperidin-2-yl, piperidin-3-yl, piperidin-4-yl, piperazin-1-yl, piperazin-2-yl, 1,3-oxazolidin-3-yl, 1,4-oxazepan-2-yl, isothiazolidinyl, 1,3-thiazolidin-3-yl, 1,2-pyrazolidin-2-yl, 1,2-tetrahydrothiazin-2-yl, 1,3-thiazinan-3-yl, 1,2-tetrahydrodiazin-2-yl, 1,3-tetrahydrodiazin-1-yl, 1,3-oxazinan-2-yl (also known as tetrahydro-1,3-oxazin-2-yl), 1,4-oxazin-4-yl, oxazolidinonyl, 2-oxo-piperidinyl (e.g., 2-oxo-piperidin-1-yl), 2-oxoazepan-3-yl, and the like.

As used herein, the term "heteroaryl" refers to monocyclic or fused-ring polycyclic aromatic heterocyclic groups with one or more heteroatom ring members (ring-forming atoms) each independently selected from O, S and N in at least one ring. The heteroaryl group has 5 to 14 ring-forming atoms, including 1 to 13 carbon atoms, and 1 to 8 heteroatoms selected from O, S, and N. In some embodiments, the heteroaryl group has 5 to 10 ring-forming atoms including one to four heteroatoms. The heteroaryl group can also contain one to three oxo or thiono (i.e., =S) groups. In some embodiments, the heteroaryl group has 5 to 8 ring-forming atoms including one, two or three heteroatoms. For example, the term "5-membered heteroaryl" refers to a monocyclic heteroaryl group as defined above with 5 ring-forming atoms in the monocyclic heteroaryl ring; the term "6-membered heteroaryl" refers to a monocyclic heteroaryl group as defined above with 6 ring-forming atoms in the monocyclic heteroaryl ring; and the term "5- or 6-membered heteroaryl" refers to a monocyclic heteroaryl group as defined above with 5 or 6 ring-forming atoms in the monocyclic heteroaryl ring. A heteroaryl group optionally can be substituted by 1 or more (e.g., 1 to 5) suitable substituents, when so specified. Examples of monocyclic heteroaryls include those with 5 ring-forming atoms including one to three heteroatoms or those with 6 ring-forming atoms including one, two or three nitrogen heteroatoms. Examples of fused bicyclic heteroaryls include two fused 5- and/or 6-membered monocyclic rings including one to four heteroatoms.

Some examples of heteroaryl groups include pyridinyl (e.g., pyridin-2-yl, pyridin-3-yl, pyridine-4-yl), pyrazinyl, pyrimidinyl (e.g., pyrimidin-2-yl, pyrimidin-4-yl, or pyrimidin-5-yl), pyridazinyl (e.g., pyridazin-3-yl, or pyridazin-4-yl), thienyl, furyl, imidazolyl (e.g., 1*H*-imidazol-4-yl), pyrrolyl, oxazolyl (e.g., 1,3-oxazolyl, 1,2-oxazolyl), thiazolyl (e.g., 1,2-thiazolyl, 1,3-thiazolyl), pyrazolyl (e.g., pyrazol-1-yl, pyrazol-3-yl, pyrazol-4-yl), tetrazolyl (e.g., 2*H*-tetrazol-5-yl), triazolyl (e.g., 1,2,3-triazolyl, 1,2,4-triazolyl), oxadiazolyl (e.g., 1,2,3-oxadiazolyl, 1,2,4-oxadiazolyl or 1,3,4-oxadiazolyl), thiadiazolyl (e.g., 1,3,4-thiadiazolyl, or 1,2,4-thiadiazolyl), quinolyl, isoquinolyl, benzothienyl, benzofuryl, indolyl, benzothiazolyl, 1,2-benzoxazolyl, 1*H-*imidazo[4,5-*c*]pyridinyl, imidazo[1,2-*a*]pyridinyl, 1*H*-pyrrolo[3,2-*c*]pyridinyl, imidazo[1,2-a]pyrazinyl, imidazo[2,1-*c*][1,2,4]triazinyl, imidazo[1,5-*a*]pyrazinyl, imidazo[1,2-*a*]pyrimidinyl, 1*H*-indazolyl, 9*H*-purinyl, imidazo[1,2-*a*]pyrimidinyl, [1,2,4]triazolo[1,5-*a*]pyridinyl, [1,2,4]triazolo[1,5-*a*]pyrimidinyl, [1,2,4]triazolo[4,3-*b*]pyridazinyl, isoxazolo[5,4-c]pyridazinyl, isoxazolo[3,4-c]pyridazinyl, pyrazolo[1,5-*a*]pyrimidinyl, 6,7-dihydro-5*H*-pyrrolo[1,2-*b*][1,2,4]triazolyl, pyridone, pyrimidone, pyrazinone, pyrimidinone, 7*H*-imidazol-2(3*H*)-one, *1H-*pyrrole-2,5-dione, 3-oxo-2*H*-pyridazinyl, 1*H*-2-oxo-pyrimidinyl, 1*H*-2-oxo-pyridinyl, 2,4(1*H*,3*H*)-dioxo-pyrimidinyl, 1*H*-2-oxo-pyrazinyl, and the like.

As used herein, the compound of Formula I as described herein includes optional substitutions and variables. It is understood that the normal valency of each of the designated (optionally substituted) atom or moiety is not exceeded, and that any of the optional substitution results in a stable compound. It is also understood that combinations of optional substituents and/or variables are permissible only if such combinations result in a stable compound.

As used herein, unless otherwise specified, the point of attachment of a substituent can be from any suitable position of the substituent. For example, piperidinyl can be piperidin-1-yl (attached through the N atom of the piperidinyl), piperidin-2-yl (attached through the C atom at the 2-position of the piperidinyl), piperidin-3-yl (attached through the C atom at the 3-position of the piperidinyl), or piperidin-4-yl (attached through the C atom at the 4-position of the piperidinyl). For another example, pyridinyl (or pyridyl) can be 2-pyridinyl (or pyridin-2-yl), 3-pyridinyl (or pyridin-3-yl), or 4-pyridinyl (or pyridin-4-yl).

As used herein, the point of attachment of a substituent can be specified to indicate the position where the substituent is attached to another moiety. For example, "(C₃₋₄ cycloalkyl)-C₁₋₄ alkyl-" means the point of attachment occurs at the "C₁₋₄ alkyl" part of the "(C₃₋₄ cycloalkyl)-C₁₋₄ alkyl-."

When a substituted or optionally substituted moiety is described without indicating the atom via which such moiety is bonded to a substituent, then the substituent may be bonded via any appropriate atom in such moiety. For example, in a substituted "(C₃₋₄ cycloalkyl)-C₁₋₄ alkyl-", a substituent on the cycloalkylalkyl [i.e., (C₃₋₄ cycloalkyl)-C₁₋₄ alkyl-] can be bonded to any carbon atom on the alkyl part or on the cycloalkyl part of the cycloalkylalkyl. Combinations of substituents and/or variables are permissible only if such combinations result in stable compounds.

As used herein, the term "adjacent" in describing the relative positions of two substituent groups on a ring structure refers to two substituent groups that are respectively attached to two ring-forming atoms of the same ring, wherein the two ring-forming atoms are directly connected through a chemical bond. For example, in each of the following structures: 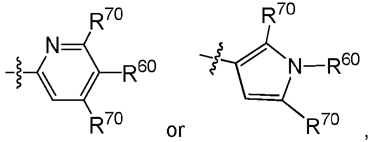 either of the two R⁷⁰ groups is an adjacent group of R⁶⁰.

As used herein, when two R^{F} are substituted on the same carbon atom in the pyrrolidine Moiety M-200 as shown below wherein the two R^{F} together with the same carbon atom they are attached form C₃₋₆ cycloalkyl (e.g., cyclobutyl); then some examples of such Moiety M-200 include Moiety M-201, M-202, or M-203.

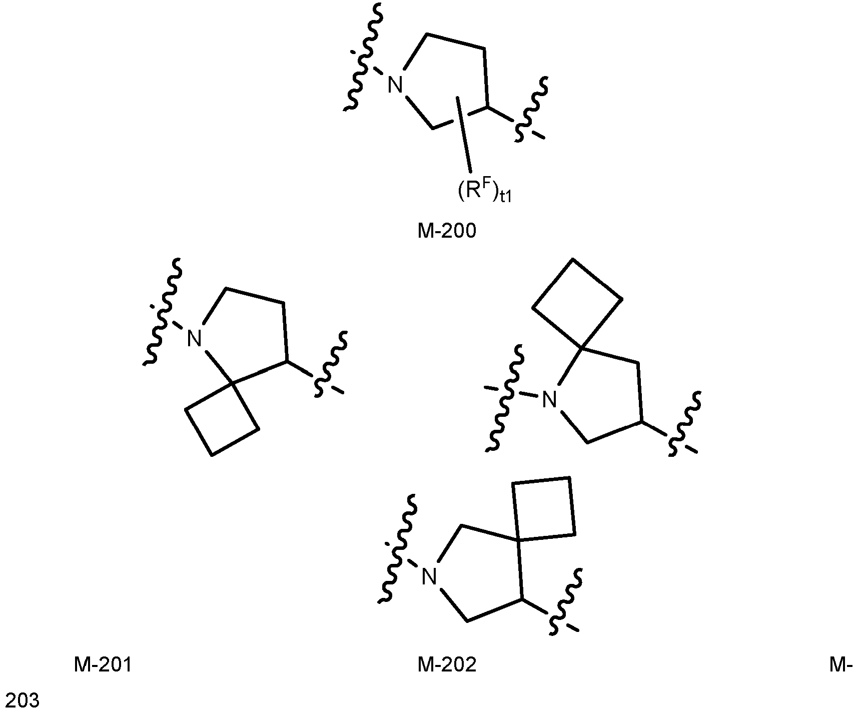

"Mammals" refers to warm-blooded vertebrate animals characterized by the secretion of milk by females for the nourishment of the young, such as guinea pigs, mice, rats, gerbils, cats, rabbits, dogs, cattle, goats, sheep, horses, monkeys, chimpanzees, and humans.

The term "pharmaceutically acceptable" means the substance (e.g., the compounds of the invention) and any salt thereof, or composition containing the substance or salt of the invention that is suitable for administration to a patient.

As used herein, the expressions "reaction-inert solvent" and "inert solvent" refer to a solvent or a mixture thereof which does not interact with starting materials, reagents, intermediates or products in a manner which adversely affects the yield of the desired product.

As used herein, the term "selectivity" or "selective" refers to a greater effect of a compound in a first assay, compared to the effect of the same compound in a second assay. For example, in "gut-selective" compounds, the first assay is for the half-life of the compound in the intestine and the second assay is for the half-life of the compound in the liver.

"Therapeutically effective amount" means an amount of a compound of the present invention that (i) treats or prevents the particular disease, condition, or disorder; (ii) attenuates, ameliorates, or eliminates one or more symptoms of the particular disease, condition, or disorder; or (iii) prevents or delays the onset of one or more symptoms of the particular disease, condition, or disorder described herein.

The term "treating", "treat", or "treatment" as used herein embraces both preventative, i.e., prophylactic, and palliative treatment, including reversing, relieving, alleviating, or slowing the progression of the disease (or disorder or condition) or any tissue damage associated with one or more symptoms of the disease (or disorder or condition).

As used herein, the term "contacting" refers to the bringing together of indicated moieties in an *in vitro* system or an *in vivo* system. For example, "contacting" MC4R with a compound of the invention includes the administration of a compound of the present invention to a mammal, such as a human, having the MC4R, as well as, for example, introducing a compound of the invention into a sample containing a cellular or purified preparation containing the MC4R.

In a first aspect, the present invention provides compound of Formula I: 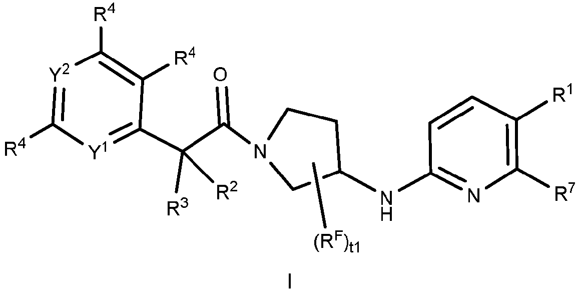 or a pharmaceutically acceptable salt thereof, wherein:
R¹ is 1,2,4-triazolyl, tetrazolyl, 1,2,4-oxadiazolyl, imidazolyl, 6,7-dihydro-[1,2,4]triazolo[5,1-b][1,3]oxazinyl, [1,2,4]triazolo[1,5-a]pyridinyl-, pyrazolyl, or pyrimidinyl, each of which is optionally substituted with 1, 2, or 3 substituents each independently selected from halogen, C₁₋₄ alkyl, C₁₋₄ haloalkyl, and C₃₋₄ cycloalkyl; or R¹ is 1,2,4-triazolyl, tetrazolyl, 1,2,4-oxadiazolyl, imidazolyl, 6,7-dihydro-[1,2,4]triazolo[5,1-b][1,3]oxazinyl, [1,2,4]triazolo[1,5-a]pyridinyl-, or pyrimidinyl, each of which is optionally substituted with 1, 2, or 3 substituents each independently selected from halogen, C₁₋₄ alkyl, C₁₋₄ haloalkyl, and C₃₋₄ cycloalkyl;
each of R² and R³ is independently H, halogen, C₁₋₄ alkyl, or C₁₋₄ haloalkyl;
or R² and R³ together with the carbon atom to which they are attached form a C₃₋₄ cycloalkyl optionally substituted with 1, 2, 3, 4, or 5 substituents each independently selected from halogen;
each of Y¹ and Y² is independently CR⁴ or N;
each R⁴ is independently H, halogen, C₁₋₄ alkyl, C₁₋₄ haloalkyl, C₁₋₄ alkoxy, or C₁₋₄ haloalkoxy;
each R^{F} is independently halogen, -OH, C₁₋₄ alkyl, C₁₋₄ haloalkyl, C₁₋₄ alkoxy, C₁₋₄ haloalkoxy, C₃₋₄ cycloalkyl, or (C₃₋₄ cycloalkyl)-C₁₋₄ alkyl-;
or when two R^{F} groups are substituted on the same carbon atom of the pyrrolidine ring of Formula I, they together with the carbon atom to which they are attached optionally form a C₃₋₆ cycloalkyl optionally substituted with 1, 2, 3, 4, or 5 substituents each independently selected from halogen, -OH, C₁₋₄ alkyl, C₁₋₄ haloalkyl, C₁₋₄ alkoxy, and C₁₋₄ haloalkoxy; or when two R^{F} groups are substituted on the same carbon atom of the pyrrolidine ring of Formula I, they together with the carbon atom to which they are attached form a C₃₋₆ cycloalkyl optionally substituted with 1, 2, 3, 4, or 5 substituents each independently selected from halogen, -OH, C₁₋₄ alkyl, C₁₋₄ haloalkyl, C₁₋₄ alkoxy, and C₁₋₄ haloalkoxy;
R⁷ is methyl or ethyl; and
t1 is 0, 1, 2, 3, or 4.

In some embodiments, the compound of Formula I or a salt thereof includes deuterated alkyls. For example, each C₁₋₄ alkyl independently can be a deuterated C₁₋₄ alkyl.

In some embodiments, the compound of Formula I or a salt thereof is a compound of Formula I-a: 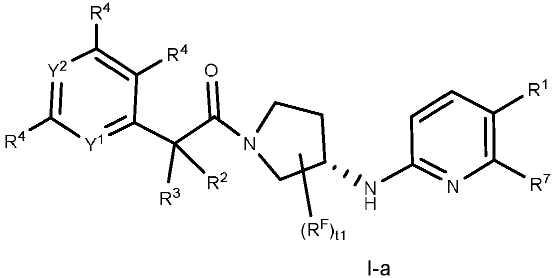 or a pharmaceutically acceptable salt, wherein each of the variables R¹, R², R³, R⁴, R⁷, R^{F}, t1, Y¹, and Y² is defined as the same as that in the compound of Formula I.

In some embodiments, the compound of Formula I or a salt thereof is a compound of Formula I-b: 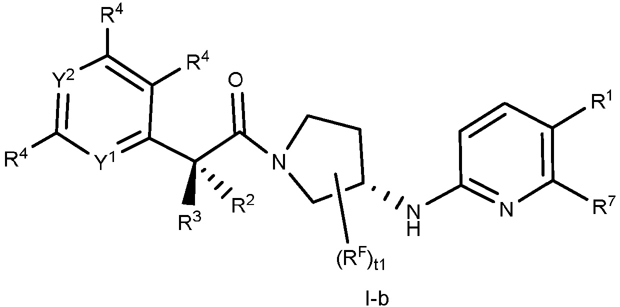 or a pharmaceutically acceptable salt, wherein each of the variables R¹, R², R³, R⁴, R⁷, R^{F}, t1, Y¹, and Y² is defined as the same as that in the compound of Formula I.

In some embodiments, the compound of Formula I or a salt thereof is a compound of Formula II: 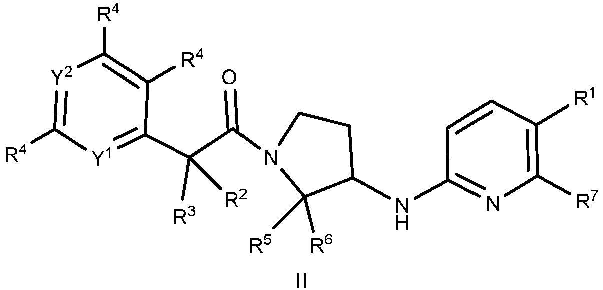 or a pharmaceutically acceptable salt wherein:
each of R⁵and R⁶ is independently H, methyl, or ethyl;
or R⁵and R⁶ together with the carbon atom to which they are attached form C₃₋₆ cycloalkyl optionally substituted with 1, 2, 3, 4, or 5 substituents each independently selected from halogen, -OH, C₁₋₄ alkyl, C₁₋₄ haloalkyl, C₁₋₄ alkoxy, and C₁₋₄ haloalkoxy;
and wherein each of the variables R¹, R², R³, R⁴, R⁷, Y¹, and Y² is defined as the same as that in the compound of Formula I.

In some further embodiments of the compound of Formula II or a salt thereof:
each of R⁵ and R⁶ is independently H, methyl, or ethyl;
or R⁵and R⁶ together with the carbon atom to which they are attached form C₃₋₅ cycloalkyl (e.g., cyclobutyl) optionally substituted with 1, 2, 3, 4, or 5 substituents each independently selected from halogen, -OH, C₁₋₄ alkyl, C₁₋₄ haloalkyl, C₁₋₄ alkoxy, and C₁₋₄ haloalkoxy.

In some further embodiments of the compound of Formula II or a salt thereof:
each of R⁵and R⁶ is independently H, methyl, or ethyl;
or R⁵ and R⁶ together with the carbon atom to which they are attached form C₄₋₆ cycloalkyl (e.g., cyclobutyl or cyclopentyl) optionally substituted with 1, 2, 3, 4, or 5 substituents each independently selected from halogen, -OH, C₁₋₄ alkyl, C₁₋₄ haloalkyl, C₁₋₄ alkoxy, and C₁₋₄ haloalkoxy.

In some embodiments, the compound of Formula II or a salt thereof is a compound of Formula II-a: 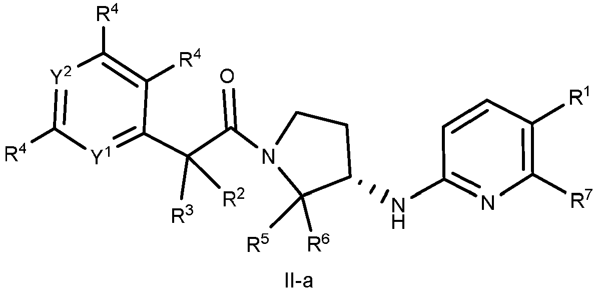 or a pharmaceutically acceptable salt thereof, wherein each of the variables R¹, R², R³, R⁴, R⁷, Y¹, and Y² is defined as the same as that in the compound of Formula I or II or a pharmaceutically acceptable salt thereof; and wherein each of the variables R⁵ and R⁶ is defined as the same as that in the compound of Formula II or a pharmaceutically acceptable salt thereof.

In some embodiments, the compound of Formula II-a or a salt thereof is a compound of Formula II-b: 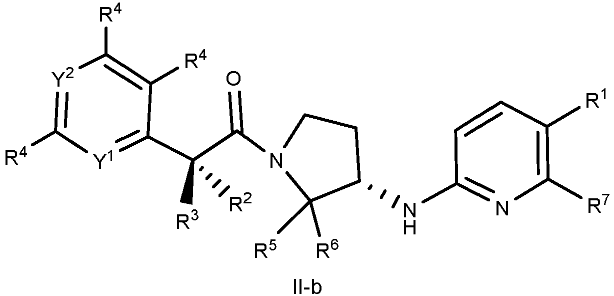 or a pharmaceutically acceptable salt thereof, wherein each of the variables R¹, R², R³, R⁴, R⁷, Y¹, and Y² is defined as the same as that in the compound of Formula I, II or IIa or a pharmaceutically acceptable salt thereof; and wherein each of the variables R⁵ and R⁶ is defined as the same as that in the compound of Formula II or IIa or a pharmaceutically acceptable salt thereof.

In some embodiments, the compound of Formula I or II, or a salt thereof, is a compound of Formula III: 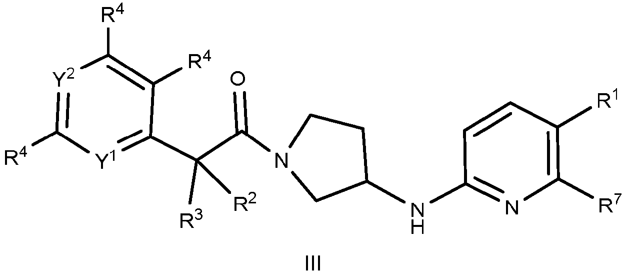 or a pharmaceutically acceptable salt thereof, wherein each of the variables R¹, R², R³, R⁴, R⁷, Y¹, and Y² is defined as the same as that in the compound of Formula I or II, or a pharmaceutically acceptable salt thereof.

In some embodiments, the compound of Formula III or a salt thereof is a compound of Formula III-a: 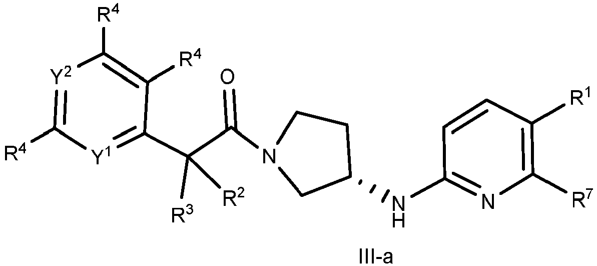 or a pharmaceutically acceptable salt thereof, wherein each of the variables R¹, R², R³, R⁴, R⁷, Y¹, and Y² is defined as the same as that in the compound of Formula I, II, or III, or a pharmaceutically acceptable salt thereof.

In some embodiments, the compound of Formula III-a or a salt thereof is a compound of Formula III-b: 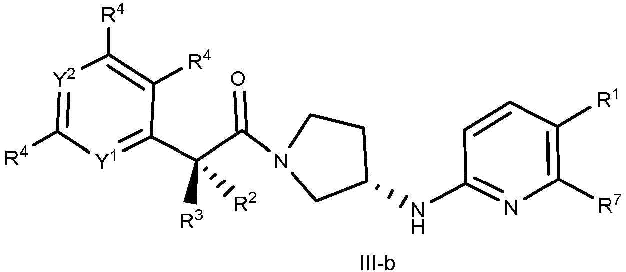 or a pharmaceutically acceptable salt thereof, wherein each of the variables R¹, R², R³, R⁴, R⁷, Y¹, and Y² is defined as the same as that in the compound of Formula III or III-a, or a pharmaceutically acceptable salt thereof.

In some embodiments of the compound of Formula I or pharmaceutically acceptable salt thereof (including, e.g., a compound of Formula I-a, I-b, II, II-a, II-b, III, III-a, or III-b, or pharmaceutically acceptable salt thereof), R¹ is 1,2,4-triazolyl, tetrazolyl, 1,2,4-oxadiazolyl,or imidazolyl, each of which is optionally substituted with 1, 2, or 3 substituents each independently selected from halogen, C₁₋₄ alkyl, C₁₋₄ haloalkyl, and C₃₋₄ cycloalkyl. In some further embodiments, R¹ is 1,2,4-triazolyl, tetrazolyl, 1,2,4-oxadiazolyl,or imidazolyl, each of which is optionally substituted with 1, 2, or 3 substituents each independently selected from halogen, C₁₋₂ alkyl, C₁₋₂ haloalkyl, and C₃₋₄ cycloalkyl.

In some embodiments of the compound of Formula I or pharmaceutically acceptable salt thereof (including, e.g., a compound of Formula I-a, I-b, II, II-a, II-b, III, III-a, or III-b, or pharmaceutically acceptable salt thereof), R¹ is 1*H*-1,2,4-triazol-3-yl, 2*H*-tetrazol-5-yl, 1,2,4-thiadiazol-5-yl, or 1*H*-imidazol-4-yl, each of which is optionally substituted with 1, 2, or 3 substituents each independently selected from halogen, C₁₋₄ alkyl, C₁₋₄ haloalkyl, and C₃₋₄ cycloalkyl. In some further embodiments, R¹ is 1*H*-1,2,4-triazol-3-yl, 2*H*-tetrazol-5-yl, 1,2,4-thiadiazol-5-yl, or 1*H*-imidazol-4-yl, each of which is optionally substituted with 1, 2, or 3 substituents each independently selected from halogen, C₁₋₂ alkyl, C₁₋₂ haloalkyl, and C₃₋₄ cycloalkyl.

In some embodiments of the compound of Formula I or pharmaceutically acceptable salt thereof (including, e.g., a compound of Formula I-a, I-b, II, II-a, II-b, III, III-a, or III-b, or pharmaceutically acceptable salt thereof), R¹ is 6,7-dihydro-[1,2,4]triazolo[5,1-b][1,3]oxazinyl or [1,2,4]triazolo[1,5-*a*]pyridinyl-, each of which is optionally substituted with 1, 2, or 3 substituents each independently selected from halogen, C₁₋₄ alkyl, C₁₋₄ haloalkyl, and C₃₋₄ cycloalkyl. In some further embodiments, R¹ is 6,7-dihydro-[1,2,4]triazolo[5,1-b][1,3]oxazinyl or [1,2,4]triazolo[1,5-*a*]pyridinyl-, each of which is optionally substituted with 1, 2, or 3 substituents each independently selected from halogen, C₁₋₂ alkyl, C₁₋₂ haloalkyl, and C₃₋₄ cycloalkyl.

In some embodiments of the compound of Formula I or pharmaceutically acceptable salt thereof (including, e.g., a compound of Formula I-a, I-b, II, II-a, II-b, III, III-a, or III-b, or pharmaceutically acceptable salt thereof), R¹ is 6,7-dihydro-[1,2,4]triazolo[5,1-b][1,3]oxazinyl optionally substituted with 1, 2, or 3 substituents each independently selected from halogen, C₁₋₄ alkyl, C₁₋₄ haloalkyl, and C₃₋₄ cycloalkyl. In some further embodiments, R¹ is 6,7-dihydro-[1,2,4]triazolo[5,1-b][1,3]oxazinyl optionally substituted with 1, 2, or 3 substituents each independently selected from halogen, C₁₋₂ alkyl, C₁₋₂ haloalkyl, and C₃₋₄ cycloalkyl. In some yet further embodiments, R¹ is 6,7-dihydro-[1,2,4]triazolo[5,1-b][1,3]oxazinyl optionally substituted with 1, 2, or 3 substituents each independently selected from halogen (e.g., F).

In some embodiments of the compound of Formula I or pharmaceutically acceptable salt thereof (including, e.g., a compound of Formula I-a, I-b, II, II-a, II-b, III, III-a, or III-b, or pharmaceutically acceptable salt thereof), R¹ is [1,2,4]triazolo[1,5-*a*]pyridinyl- optionally substituted with 1, 2, or 3 substituents each independently selected from halogen, C₁₋₂ alkyl, C₁₋₂ haloalkyl, and C₃₋₄ cycloalkyl.

In some embodiments of the compound of Formula I or pharmaceutically acceptable salt thereof (including, e.g., a compound of Formula I-a, I-b, II, II-a, II-b, III, III-a, or III-b, or pharmaceutically acceptable salt thereof), R¹ is pyrimidinyl optionally substituted with 1, 2, or 3 substituents each independently selected from halogen, C₁₋₄ alkyl, C₁₋₄ haloalkyl, and C₃₋₄ cycloalkyl. In some further embodiments, R¹ is pyrimidinyl optionally substituted with 1, 2, or 3 substituents each independently selected from halogen, C₁₋₂ alkyl, C₁₋₂ haloalkyl, and C₃₋₄ cycloalkyl.

In some embodiments of the compound of Formula I or pharmaceutically acceptable salt thereof (including, e.g., a compound of Formula I-a, I-b, II, II-a, II-b, III, III-a, or III-b, or pharmaceutically acceptable salt thereof), R¹ is pyrimidin-2-yl optionally substituted with 1, 2, or 3 substituents each independently selected from halogen, C₁₋₄ alkyl, C₁₋₄ haloalkyl, and C₃₋₄ cycloalkyl. In some further embodiments, R¹ is pyrimidin-2-yl optionally substituted with 1, 2, or 3 substituents each independently selected from halogen, C₁₋₂ alkyl, C₁₂ haloalkyl, and C₃₋₄ cycloalkyl. In some yet embodiments, R¹ is pyrimidin-2-yl.

In some embodiments of the compound of Formula I or pharmaceutically acceptable salt thereof (including, e.g., a compound of Formula I-a, I-b, II, II-a, II-b, III, III-a, or III-b, or pharmaceutically acceptable salt thereof, for example, any one of the embodiments described herein above), each of R² and R³ is independently H, F, or C₁₋₄ alkyl.

In some embodiments of the compound of Formula I or pharmaceutically acceptable salt thereof (including, e.g., a compound of Formula I-a, I-b, II, II-a, II-b, III, III-a, or III-b, or pharmaceutically acceptable salt thereof, for example, any one of the embodiments described herein above), each of R² and R³ is independently H, F, or C₁₋₂ alkyl.

In some embodiments of the compound of Formula I or pharmaceutically acceptable salt thereof (including, e.g., a compound of Formula I-a, I-b, II, II-a, II-b, III, III-a, or III-b, or pharmaceutically acceptable salt thereof, for example, any one of the embodiments described herein above), each of R² and R³ is independently H or methyl.

In some embodiments of the compound of Formula I or pharmaceutically acceptable salt thereof (including, e.g., a compound of Formula I-a, I-b, II, II-a, II-b, III, III-a, or III-b, or pharmaceutically acceptable salt thereof, for example, any one of the embodiments described herein above), R² is C₁₋₂ alkyl and R³ is H.

In some embodiments of the compound of Formula I or pharmaceutically acceptable salt thereof (including, e.g., a compound of Formula I-a, I-b, II, II-a, II-b, III, III-a, or III-b, or pharmaceutically acceptable salt thereof, for example, any one of the embodiments described herein above), R² is methyl and R³ is H.

In some embodiments of the compound of Formula II or pharmaceutically acceptable salt thereof (including, e.g., a compound of Formula II-a, or II-b, or pharmaceutically acceptable salt thereof, for example, any one of the embodiments described herein above), each of R⁵ and R⁶ is independently H or methyl.

In some embodiments of the compound of Formula II or pharmaceutically acceptable salt thereof (including, e.g., a compound of Formula II-a, or II-b, or pharmaceutically acceptable salt thereof, for example, any one of the embodiments described herein above, if applicable), R⁵ and R⁶ together with the carbon atom to which they are attached form C₃₋₅ cycloalkyl (e.g., cyclobutyl).

In some embodiments of the compound of Formula I or pharmaceutically acceptable salt thereof (including, e.g., a compound of Formula I-a, I-b, II, II-a, II-b, III, III-a, or III-b, or pharmaceutically acceptable salt thereof, for example, any one of the embodiments described herein above), R⁷ is methyl.

In some embodiments of the compound of Formula I or pharmaceutically acceptable salt thereof (including, e.g., a compound of Formula I-a, I-b, II, II-a, II-b, III, III-a, or III-b, or pharmaceutically acceptable salt thereof, for example, any one of the embodiments described herein above), each of Y¹ and Y² is independently CR⁴.

In some embodiments of the compound of Formula I or pharmaceutically acceptable salt thereof (including, e.g., a compound of Formula I-a, I-b, II, II-a, II-b, III, III-a, or III-b, or pharmaceutically acceptable salt thereof, for example, any one of the embodiments described herein above), one of Y¹ and Y² is N, and the other of Y¹ and Y² is CR⁴.

In some embodiments of the compound of Formula I or pharmaceutically acceptable salt thereof (including, e.g., a compound of Formula I-a, I-b, II, II-a, II-b, III, III-a, or III-b, or pharmaceutically acceptable salt thereof, for example, any one of the embodiments described herein above), Y² is N, and Y¹ is CR⁴.

In some embodiments of the compound of Formula I or pharmaceutically acceptable salt thereof (including, e.g., a compound of Formula I-a, I-b, II, II-a, II-b, III, III-a, or III-b, or pharmaceutically acceptable salt thereof, for example, any one of the embodiments described herein above, if applicable), Y¹ is N and Y² is N.

In some embodiments of the compound of Formula I or pharmaceutically acceptable salt thereof (including, e.g., a compound of Formula I-a, I-b, II, II-a, II-b, III, III-a, or III-b, or pharmaceutically acceptable salt thereof, for example, any one of the embodiments described herein above), each R⁴ is independently H, halogen, C₁₋₂ alkyl, C₁₋₂ haloalkyl, C₁₋₂ alkoxy, or C₁₋₂ haloalkoxy. In some further embodiments, each R⁴ is independently H, F, Cl, C₁₋₂ alkyl, C₁₋₂ fluoroalkyl, C₁₋₂ alkoxy, or C₁₋₂ fluoroalkoxy.

In some embodiments of the compound of Formula I or pharmaceutically acceptable salt thereof (including, e.g., a compound of Formula I-a, I-b, II, II-a, II-b, III, III-a, or III-b, or pharmaceutically acceptable salt thereof, for example, any one of the embodiments described herein above), each R⁴ is independently H, halogen (e.g., F or Cl), or C₁₋₂ alkoxy.

In some embodiments, the present invention provides a compound selected from:
(2*R*)-2-(5-chloro-2-methoxypyridin-4-yl)-1-[(3*S*)-3-{[6-methyl-5-(2-methyl-2*H*-tetrazol-5-yl)pyridin-2-yl]amino}pyrrolidin-1-yl]propan-1-one;
(2*R*)-2-(5-fluoro-2-methoxypyridin-4-yl)-1-[(3*S*)-3-({6-methyl-5-[1-methyl-5-(trifluoromethyl)-1*H*-1,2,4-triazol-3-yl]pyridin-2-yl}amino)pyrrolidin-1-yl]propan-1-one;
(2*R*)-1-[(3*S*)-3-({5-[5-(1,1-difluoroethyl)-1-methyl-1*H*-1,2,4-triazol-3-yl]-6-methylpyridin-2-yl}amino)pyrrolidin-1-yl]-2-(5-fluoro-2-methoxypyridin-4-yl)propan-1-one;
(2*R*)-2-(5-fluoro-2-methoxypyridin-4-yl)-1-[(3*S*)-3-{[6-methyl-5-(2-methyl-2*H*-tetrazol-5-yl)pyridin-2-yl]amino}pyrrolidin-1-yl]propan-1-one;
(2*R*)-2-(5-chloro-2-methoxypyridin-4-yl)-1-[(3*S*)-3-{[6-methyl-5-(pyrimidin-2-yl)pyridin-2-yl]amino}pyrrolidin-1-yl]propan-1-one;
(2*R*)-1-[(3*S*)-3-({5-[5-(difluoromethyl)-1-methyl-1H-1,2,4-triazol-3-yl]-6-methylpyridin-2-yl}amino)pyrrolidin-1-yl]-2-(5-fluoro-2-methoxypyridin-4-yl)propan-1-one;
(2*R*)-1-[(3*S*)-3-({5-[5-(difluoromethyl)-1-(²H₃)methyl-1*H*-1,2,4-triazol-3-yl]-6-methylpyridin-2-yl}amino)pyrrolidin-1-yl]-2-(5-fluoro-2-methoxypyridin-4-yl)propan-1-one;
2-(5-chloro-2-methoxypyridin-4-yl)-1-[(3*S*)-3-({5-[5-(difluoromethyl)-1-methyl-1*H*-1,2,4-triazol-3-yl]-6-methylpyridin-2-yl}amino)pyrrolidin-1-yl]propan-1-one, DIAST-2;
2-(5-chloro-2-methoxypyridin-4-yl)-1-[(3*S*)-3-({6-methyl-5-[1-methyl-5-(trifluoromethyl)-1*H*-1,2,4-triazol-3-yl]pyridin-2-yl}amino)pyrrolidin-1-yl]propan-1-one, DIAST-2; and
(2*R*)-2-(5-fluoro-2-methoxypyridin-4-yl)-1-[(3*S*)-3-{[6-methyl-5-([1,2,4]triazolo[1,5-*a*]pyridin-2-yl)pyridin-2-yl]amino}pyrrolidin-1-yl]propan-1-one,
or pharmaceutically acceptable salt thereof.

Every embodiment, Example, or pharmaceutically acceptable salt thereof may be claimed individually or grouped together in any combination with any number of each and every embodiment described herein.

The compound of Formula I (including, e.g., a compound of Formula la, I-b, II, II-a, IIb, III, Illa, or III-b) or pharmaceutially acceptable salt of the invention can be used in any of the pharmaceutical compositions, uses, and methods of the invention described herein.

The compound of Formula I (including, e.g., a compound of Formula la, I-b, II, II-a, IIb, III, IIIa, or III-b) or a pharmaceutically acceptable salt thereof of the present invention is an MC4R antagonist.

In some embodiments, the amount of the compound of Formula I or a pharmaceutically acceptable salt thereof used in any one of the methods (or uses) of the present invention is effective in antagonizing MC4R.

In a second aspect, the present invention provides a method for antagonizing MC4R *in vitro* only, comprising contacting (including incubating) the MC4R with the compound of Formula I or a pharmaceutically acceptable salt thereof described herein (such as any one of embodiments or Examples 1 to 44B described herein).

In a third aspect, the present invention provides a method for treating a condition, disease or disorder in a mammal (e.g. a human), which method comprises administering to the mammal (e.g. the human) a therapeutically effective amount of a compound of Formula I or a pharmaceutically acceptable salt thereof as described herein (such as any one of embodiments or Examples 1 to 44B described herein), wherein the condition, disease or disorder is an MC4R-related condition, disease, or disorder. In some embodiments, the mammal (e.g., the human) is need of such treatment.

In a fourth aspect, the present invention provides a method for treating a condition, disease or disorder in a mammal (e.g. a human), which method comprises administering to the mammal (e.g. the human) a therapeutically effective amount of a compound of Formula I or a pharmaceutically acceptable salt thereof as described herein (such as any one of embodiments or Examples 1 to 44B described herein), wherein the condition, disease or disorder is selected from cachexia [including for example, cachexia associated with a chronic illness, such as cachexia associated with cancer, cachexia associated with acquired immunodeficiency syndrome (AIDS), cachexia associated with heart failure for example cachexia associated with congestive heart failure (CHF), cachexia associated with chronic kidney disease (CKD); cachexia associated with treatment of a chronic illness, such as, cachexia associated with treatment of cancer or cachexia associated with treatment of heart failure (e.g. CHF)]; anorexia or anorexia nervosa (e.g., geriatric anorexia, anorexia associated with chemotherapy and/or radiotherapy); nausea; emesis; weight loss (e.g., involuntary weight loss); failure to thrive; sarcopenia; muscle wasting; muscle weakness; frailty; osteoporosis; bone disorders (e.g., bone loss); pain; neuropathic pain; anxiety (e.g., posttraumatic stress disorder, or PTSD); depression; hypertension; malnutrition; obesity (e.g. sarcopenia resulting from chronic obesity); sexual dysfunction; and inflammatory disease (e.g. an inflammatory disease associated with anorexia or cachexia or sarcopenia or muscle wasting). In some embodiments, the mammal (e.g., the human) is need of such treatment.

In a fifth aspect, the present invention provides a compound of Formula I or a pharmaceutically acceptable salt of the compound (such as any one of embodiments or Examples 1 to 44B described herein) for use as a medicament. In some further embodiments, the present invention provides a compound of Formula I or a pharmaceutically acceptable salt of the compound (such as any one of embodiments or Examples 1 to 44B described herein) for use in the treatment of a condition, disease, or disorder selected from cachexia [including for example, cachexia associated with a chronic illness, such as cachexia associated with cancer, cachexia associated with acquired immunodeficiency syndrome (AIDS), cachexia associated with heart failure for example cachexia associated with congestive heart failure (CHF), cachexia associated with chronic kidney disease (CKD); cachexia associated with treatment of a chronic illness, such as, cachexia associated with treatment of cancer or cachexia associated with treatment of heart failure (e.g. CHF)]; anorexia or anorexia nervosa (e.g., geriatric anorexia, anorexia associated with chemotherapy and/or radiotherapy); nausea; emesis; weight loss (e.g., involuntary weight loss); failure to thrive; sarcopenia; muscle wasting; muscle weakness; frailty; osteoporosis; bone disorders (e.g., bone loss); pain; neuropathic pain; anxiety (e.g., posttraumatic stress disorder, or PTSD); depression; hypertension; malnutrition; obesity (e.g. sarcopenia resulting from chronic obesity); sexual dysfunction; and inflammatory disease (e.g. an inflammatory disease associated with anorexia or cachexia or sarcopenia or muscle wasting).

In a sixth aspect, the present invention provides use of a compound of Formula I or a pharmaceutically acceptable salt thereof as described herein (such as any one of embodiments or Examples 1 to 44B described herein) as a medicament, particularly wherein the medicament is for use in the treatment of an MC4R-related condition, disease, or disorder, including administering to a mammal, such as a human, in need of such treatment a therapeutically effective amount.

In a seventh aspect, the present invention provides use of a compound of Formula I or a pharmaceutically acceptable salt of the compound (such as any one of embodiments or Examples 1 to 44B described herein) as a medicament, particularly wherein the medicament is for use in the treatment of a condition, disease, or disorder selected from cachexia [including for example, cachexia associated with a chronic illness, such as cachexia associated with cancer, cachexia associated with acquired immunodeficiency syndrome (AIDS), cachexia associated with heart failure for example cachexia associated with congestive heart failure (CHF), cachexia associated with chronic kidney disease (CKD); cachexia associated with treatment of a chronic illness, such as, cachexia associated with treatment of cancer or cachexia associated with treatment of heart failure (e.g. CHF)]; anorexia or anorexia nervosa (e.g., geriatric anorexia, anorexia associated with chemotherapy and/or radiotherapy); nausea; emesis; weight loss (e.g., involuntary weight loss); failure to thrive; sarcopenia; muscle wasting; muscle weakness; frailty; osteoporosis; bone disorders (e.g., bone loss); pain; neuropathic pain; anxiety (e.g., posttraumatic stress disorder, or PTSD); depression; hypertension; malnutrition; obesity (e.g. sarcopenia resulting from chronic obesity); sexual dysfunction; and inflammatory disease (e.g. an inflammatory disease associated with anorexia or cachexia or sarcopenia or muscle wasting), including administering to a mammal, such as a human, in need of such treatment a therapeutically effective amount of or a pharmaceutically acceptable salt of the compound.

In an eighth aspect, the present invention includes use of a compound of Formula I or a pharmaceutically acceptable salt of the compound (such as any one of embodiments or Examples 1 to 44B described herein) in the manufacture of a medicament. In some further embodiment, the present invention includes use of a compound of Formula I or a pharmaceutically acceptable salt of the compound (such as any one of embodiments or Examples 1 to 44B described herein) in the manufacture of a medicament for treating an MC4R-related condition, disease, or disorder, including administering to a mammal, such as a human, in need of such treatment a therapeutically effective amount.

In an nineth aspect, the present invention includes use of a compound of Formula I or a pharmaceutically acceptable salt of the compound (such as any one of embodiments or Examples 1 to 44B described herein) in the manufacture of a medicament for treating a condition, disease, or disorder selected from cachexia [including for example, cachexia associated with a chronic illness, such as cachexia associated with cancer, cachexia associated with acquired immunodeficiency syndrome (AIDS), cachexia associated with heart failure for example cachexia associated with congestive heart failure (CHF), cachexia associated with chronic kidney disease (CKD); cachexia associated with treatment of a chronic illness, such as, cachexia associated with treatment of cancer or cachexia associated with treatment of heart failure (e.g. CHF)]; anorexia or anorexia nervosa (e.g., geriatric anorexia, anorexia associated with chemotherapy and/or radiotherapy); nausea; emesis; weight loss (e.g., involuntary weight loss); failure to thrive; sarcopenia; muscle wasting; muscle weakness; frailty; osteoporosis; bone disorders (e.g., bone loss); pain; neuropathic pain; anxiety (e.g., posttraumatic stress disorder, or PTSD); depression; hypertension; malnutrition; obesity (e.g. sarcopenia resulting from chronic obesity); sexual dysfunction; and inflammatory disease (e.g. an inflammatory disease associated with anorexia or cachexia or sarcopenia or muscle wasting).

The compounds of the present invention may contain asymmetric or chiral centers, and, therefore, exist in different stereoisomeric forms. Unless specified otherwise, it is intended that all stereoisomeric forms of the compounds of the present invention as well as mixtures thereof, including racemic mixtures, form part of the present invention. In addition, the present invention embraces all geometric isomers. For example, if a compound of the present invention incorporates a double bond or a fused ring, both the *cis-* and *trans-* forms, as well as mixtures, are embraced within the scope of the invention.

Chiral compounds of the invention (and chiral precursors thereof) may be obtained in enantiomerically-enriched form using chromatography, typically high pressure liquid chromatography (HPLC) or supercritical fluid chromatography (SFC), on a resin with an asymmetric stationary phase and with a mobile phase consisting of a hydrocarbon, typically heptane or hexane, containing from 0 to 50% isopropanol, typically from 2 to 20%, and from 0 to 5% of an alkylamine, typically 0.1% diethylamine (DEA) or isopropylamine. Concentration of the eluent affords the enriched mixture. In the case where SFC is used, the mobile phase may consist of a supercritical fluid, typically carbon dioxide, containing 2-50% of an alcohol, such as methanol, ethanol or isopropanol.

Diastereomeric mixtures can be separated into their individual diastereoisomers on the basis of their physicochemical differences by methods well known to those skilled in the art, such as by chromatography and/or fractional crystallization. Enantiomers can be separated by converting the enantiomeric mixture into a diastereomeric mixture by reaction with an appropriate optically active compound (e.g., chiral auxiliary such as a chiral alcohol or Mosher's acid chloride), separating the diastereoisomers and converting (e.g., hydrolyzing) the individual diastereoisomers to the corresponding pure enantiomers. Enantiomers can also be separated by use of a chiral HPLC column. Alternatively, the specific stereoisomers may be synthesized by using an optically active starting material, by asymmetric synthesis using optically active reagents, substrates, catalysts or solvents, or by converting one stereoisomer into the other by asymmetric transformation.

In some embodiments, the compounds of the invention may have asymmetric carbon atoms. The carbon-carbon bonds of the compounds of Formula I may be depicted herein using a solid line (-), a wavy line ( 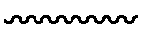 ), a solid wedge ( 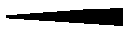 ), or a dotted wedge ( 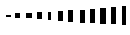 ). The use of a solid line to depict bonds to asymmetric carbon atoms is meant to indicate that all possible stereoisomers (e.g., specific enantiomers, racemic mixtures, etc.) at that carbon atom are included. The use of either a solid or dotted wedge to depict bonds to asymmetric carbon atoms is meant to indicate that only the stereoisomer shown is meant to be included. The use of a wavy line to depict bonds to asymmetric carbon atoms is meant to indicate that the stereochemistry is unknown (unless otherwise specified). It is possible that compounds of the invention may contain more than one asymmetric carbon atom. In those compounds, the use of a solid line to depict bonds to asymmetric carbon atoms is meant to indicate that all possible stereoisomers are meant to be included. For example, unless stated otherwise, it is intended that the compounds of the invention can exist as enantiomers and diastereomers or as racemates and mixtures thereof. The use of a solid line to depict bonds to one or more asymmetric carbon atoms in a compound of the invention and the use of a solid or dotted wedge to depict bonds to other asymmetric carbon atoms in the same compound is meant to indicate that a mixture of diastereomers is present.

Where the compounds of the present invention possess two or more stereogenic centers and the absolute or relative stereochemistry is given in the name, the designations R and S refer respectively to each stereogenic center in ascending numerical order (1, 2, 3, etc.) according to the conventional IUPAC number schemes for each molecule. Where the compounds of the present invention possess one or more stereogenic centers and no stereochemistry is given in the name or structure, it is understood that the name or structure is intended to encompass all forms of the compound, including the racemic form.

The compounds of this invention may contain olefin-like double bonds. When such bonds are present, the compounds of the invention exist as cis and trans configurations and as mixtures thereof. The term "cis" refers to the orientation of two substituents with reference to each other and the plane of the ring (either both "up" or both "down"). Analogously, the term "trans" refers to the orientation of two substituents with reference to each other and the plane of the ring (the substituents being on opposite sides of the ring).

It is also possible that the intermediates and compounds of the present invention may exist in different tautomeric forms, and all such forms are embraced within the scope of the invention. The term "tautomer" or "tautomeric form" refers to structural isomers of different energies which are interconvertible *via* a low energy barrier. For example, proton tautomers (also known as prototropic tautomers) include interconversions *via* migration of a proton, such as keto-enol and imine-enamine isomerizations.

Valence tautomers include interconversions by reorganization of some of the bonding electrons.

Included within the scope of the claimed compounds present invention are all stereoisomers, geometric isomers and tautomeric forms of the compounds of Formula I, including compounds exhibiting more than one type of isomerism, and mixtures of one or more thereof. Also included are acid addition or base salts wherein the counterion is optically active, for example, D-lactate or L-lysine, or racemic, for example, DL-tartrate or DL-arginine.

The present invention includes all pharmaceutically acceptable isotopically labelled compounds of Formula I wherein one or more atoms are replaced by atoms having the same atomic number, but an atomic mass or mass number different from the atomic mass or mass number usually found in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I, ¹²⁴I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S.

Certain isotopically labelled compounds of Formula I, for example, those incorporating a radioactive isotope, are useful in drug and/or substrate tissue distribution studies. The radioactive isotopes tritium, i.e., ³H, and carbon-14, i.e., ¹⁴C, are particularly useful for this purpose in view of their ease of incorporation and ready means of detection.

Substitution with heavier isotopes such as deuterium, i.e., ²H, may afford certain therapeutic advantages resulting from greater metabolic stability, for example, increased *in vivo* half-life or reduced dosage requirements, and hence may be preferred in some circumstances.

Substitution with positron emitting isotopes, such as ¹¹C, ¹⁸F, ¹⁵O and ¹³N, can be useful in Positron Emission Tomography (PET) studies for examining substrate receptor occupancy.

Isotopically labelled compounds of Formula I can generally be prepared by conventional techniques known to those skilled in the art or by processes analogous to those described in the accompanying Examples and Preparations using an appropriate isotopically labelled reagents in place of the non-labelled reagent previously employed.

The compounds of the present invention may be isolated and used *per se,* or when possible, in the form of its pharmaceutically acceptable salt. The term "salts" refers to inorganic and organic salts of a compound of the present invention. These salts can be prepared *in situ* during the final isolation and purification of a compound, or by separately treating the compound with a suitable organic or inorganic acid and isolating the salt thus formed.

Salts encompassed within the term "pharmaceutically acceptable salts" refer to the compounds of this invention which are generally prepared by reacting the free base with a suitable organic or inorganic acid to provide a salt of the compound of the invention that is suitable for administration to a patient. Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts. See e.g., Berge, et al. J. Pharm. Sci. 66, 1-19 (1977); Handbook of Pharmaceutical Salts: Properties, Selection, and Use by Stahl and Wermuth (Wiley-VCH, 2002).

The compounds of Formula I, and pharmaceutically acceptable salts thereof, may exist in unsolvated and solvated forms. The term 'solvate' is used herein to describe a molecular complex comprising the compound of Formula I, or a pharmaceutically acceptable salt thereof, and one or more pharmaceutically acceptable solvent molecules, for example, ethanol. The term 'hydrate' is employed when said solvent is water.

A currently accepted classification system for organic hydrates is one that defines isolated site, channel, or metal-ion coordinated hydrates - see Polymorphism in Pharmaceutical Solids by K. R. Morris (Ed. H. G. Brittain, Marcel Dekker, 1995). Isolated site hydrates are ones in which the water molecules are isolated from direct contact with each other by intervening organic molecules. In channel hydrates, the water molecules lie in lattice channels where they are next to other water molecules. In metal-ion coordinated hydrates, the water molecules are bonded to the metal ion.

When the solvent or water is tightly bound, the complex may have a well-defined stoichiometry independent of humidity. When, however, the solvent or water is weakly bound, as in channel solvates and hygroscopic compounds, the water/solvent content may be dependent on humidity and drying conditions. In such cases, non-stoichiometry will be the norm.

Also included within the scope of the invention are multi-component complexes (other than salts and solvates) wherein the drug and at least one other component are present in stoichiometric or non-stoichiometric amounts. Complexes of this type include clathrates (drug-host inclusion complexes) and co-crystals. The latter are typically defined as crystalline complexes of neutral molecular constituents which are bound together through non-covalent interactions, but could also be a complex of a neutral molecule with a salt. Co-crystals may be prepared by melt crystallization, by recrystallization from solvents, or by physically grinding the components together - see O. Almarsson and M. J. Zaworotko, Chem. Commun., 17, 1889-1896 (2004). For a general review of multi-component complexes, see Haleblian, J. Pharm. Sci., 64 (8), 1269-1288 (1975).

The compounds of the invention include compounds of Formula I or their pharmaceutically acceptable salts as hereinbefore defined, polymorphs, and isomers thereof (including optical, geometric and tautomeric isomers) as hereinafter defined and isotopically labelled compounds of Formula I or their pharmaceutically acceptable salts.

The compounds of the present invention may be administered as prodrugs. Thus, certain derivatives of compounds of Formula I or their pharmaceutically acceptable salts which may have little or no pharmacological activity themselves can, when administered into or onto the body, be converted into compounds of Formula I or their pharmaceutically acceptable salts having the desired activity, for example, by hydrolytic cleavage. Such derivatives are referred to as 'prodrugs'. [Further information on the use of prodrugs may be found in 'Pro-drugs as Novel Delivery Systems, Vol. 14, ACS Symposium Series (T. Higuchi and W. Stella) and 'Bioreversible Carriers in Drug Design', Pergamon Press, 1987 (ed. E. B. Roche, American Pharmaceutical Association).]

Prodrugs can, for example, be produced by replacing appropriate functionalities present in the compounds of Formula I or their pharmaceutically acceptable salts with certain moieties known to those skilled in the art as 'pro-moieties' as described, for example, in "Design of Prodrugs" by H. Bundgaard (Elsevier, 1985).

Some examples of such prodrugs include:
(i) where the compound of Formula I or its pharmaceutically acceptable salt contains an alcohol functionality (-OH), an ether thereof, for example, replacement of the hydrogen with (C₁-C₆)alkanoyloxymethyl; or a phosphate ester (-O-PO₃H₂) or sulfate ester (-O-SO₃H) or pharmaceutically acceptable salts thereof; and
(ii) an amide or carbamate of the amino functionality present in Formula (I) or (II), wherein the hydrogen of the amino NH group is replaced with (C₁-C₁₀)alkanoyl or (C₁-C₁₀)alkoxycarbonyl, respectively.

Disclosed but not falling under the scope of the present invention are active metabolites of compounds of Formula I (including prodrugs) or their pharmaceutically acceptable salts, that is, compounds formed in vivo upon administration of the drug, often by oxidation or dealkylation. Some examples of metabolites include:
(i) where the compound of Formula I or its pharmaceutically acceptable salt contains a methyl group, a hydroxymethyl derivative thereof (-CH₃ -> -CH₂OH) and
(ii) where the compound of Formula I or its pharmaceutically acceptable salt contains an alkoxy group, a hydroxy derivative thereof (-OR -> -OH).

Certain compounds of the present invention may exist in more than one crystal form (generally referred to as "polymorphs"). Polymorphs may be prepared by crystallization under various conditions, for example, using different solvents or different solvent mixtures for recrystallization; crystallization at different temperatures; and/or various modes of cooling, ranging from very fast to very slow cooling during crystallization. Polymorphs may also be obtained by heating or melting the compound of the present invention followed by gradual or fast cooling. The presence of polymorphs may be determined by solid probe NMR spectroscopy, IR spectroscopy, differential scanning calorimetry, powder X-ray diffraction or such other techniques.

In general, the compounds of this invention can be made by processes which include processes analogous to those known in the chemical arts, particularly in light of the description contained herein. Certain processes for the manufacture of the compounds of this invention are provided as further features of the invention and are illustrated by the following reaction schemes. Other processes may be described in the experimental section. Specific synthetic schemes for preparation of the compounds of Formula I and/or their pharmaceutically acceptable salts are outlined below. Note that tetrazoles are generally a high-energy functional group and care should be taken in the synthesis and handling of tetrazole-containing molecules.

The starting materials are generally available from commercial sources such as MilliporeSigma (Milwaukee, WI) or are readily prepared using methods well known to those skilled in the art [e.g., prepared by methods generally described in Louis F. Fieser and Mary Fieser, Reagents for Organic Synthesis, v. 1-19, Wiley, New York (1967-1999 ed.), or Beilsteins Handbuch der Organischen Chemie, 4, Aufl. ed. Springer-Verlag, Berlin, including supplements (also available *via* the *Beilstein* online database)]. Many of the compounds used herein are related to, or are derived from, compounds in which there is a large scientific interest and commercial need, and accordingly many such compounds are commercially available or are reported in the literature or are easily prepared from other commonly available substances by methods which are reported in the literature.

In the preparation of the compounds of Formula **I** or their salts, it is noted that some of the preparation methods described herein may require protection of remote functionality (e.g., primary amine, secondary amine, carboxyl in Formula **I** precursors). The need for such protection will vary depending on the nature of the remote functionality and the conditions of the preparation methods. The need of such protection is readily determined by one skilled in the art. The use of such protection/deprotection methods is also within the skill in the art. For a general description of protecting groups and their use, see T.W. Greene, Protective Groups in Organic Synthesis, 5th Edition, John Wiley & Sons, New York, 2014. For example, certain compounds contain primary amines or carboxylic acid functionalities which may interfere with reactions at other sites of the molecule if left unprotected. Accordingly, such functionalities may be protected by an appropriate protecting group which may be removed in a subsequent step. Suitable protecting groups for amine and carboxylic acid protection include those protecting groups commonly used in peptide synthesis (such as *N*-tert-butoxycarbonyl (Boc), benzyloxycarbonyl (Cbz), and 9-fluorenylmethoxycarbonyl (Fmoc) for amines and lower alkyl or benzyl esters for carboxylic acids) which are generally not chemically reactive under the reaction conditions described and can typically be removed without chemically altering other functionality in the compounds of Formula **I** or their salts.

Reactions can be monitored according to any suitable method known in the art. For example, product formation can be monitored by spectroscopic means, such as nuclear magnetic resonance spectroscopy (e.g., ¹H or ¹³C), infrared spectroscopy, spectrophotometry (e.g., UV-visible), mass spectrometry, or by chromatographic methods such as high-performance liquid chromatography (HPLC) or thin-layer chromatography (TLC).

Compounds of Formula **I,** salts and intermediates thereof may be prepared according to the following reaction schemes and accompanying discussion. The reaction schemes described below are intended to provide a general description of the methodology employed in the preparation of the compounds of the present invention. Some of the compounds of the present invention contain a single chiral center with stereochemical designation (R or S) and others will contain two separate chiral centers with stereochemical designation (R or S). It will be apparent to one skilled in the art that most of the synthetic transformations can be conducted in a similar manner whether the materials are enantioenriched or racemic. Moreover, the resolution to the desired optically active material may take place at any desired point in the sequence using well-known methods such as described herein and in the chemistry literature.

Unless otherwise indicated, R¹, R², R³, R⁴, R⁵, R⁶, R⁷, (R^{F})ₜ₁, Y¹, Y², and structural Formula **I** (including, e.g., **I-a, I-b,** and **II**) in the reaction schemes and discussion that follow are as defined herein or consistent with those described herein. Certain processes for the manufacture of the compounds of this invention and intermediates thereof are provided as further features of the invention and are illustrated by the following reaction schemes. Other processes are described in the experimental section. The schemes and examples provided herein (including the corresponding description) are for illustration only, and not intended to limit the scope of the present invention.

In the Reaction Schemes that follow, the variables X^{c}, X^{F}, X¹, X², X³, X⁴, X⁵, X⁶, Y⁶, M¹, R^{2c}, R¹⁰, R¹¹, R¹², R¹³, R¹⁴, R¹⁵, LG¹, LG², LG³, PG¹, PG², PG³, PG⁴, and PG⁵ are as described herein or consistent with those described in the claims of Formula **I** unless otherwise noted. For each of the variables, its meaning remains the same as initially described unless otherwise indicated in a later occurrence.

Scheme 1 refers to the synthesis of compounds of Formula **I, I-a,** and **I-b.** Acids of formula **1-1** can be reacted with amines of formula **1-2** (or salts thereof) using standard amidation conditions with coupling reagents such as 1,1'-carbonyldiimidazole, 2,4,6-tripropyl-1,3,5,2,4,6-trioxatriphosphinane 2,4,6-trioxide (T3P), *O*-(7-azabenzotriazol-1-yl)-*N,N,N',N*'-tetramethyluronium hexafluorophosphate (HATU), or others to give compounds of Formula **I.** Alternatively, acids of formula **1-1** can reacted with amines of formula **1-3** in an analogous manner to form compounds of Formula **I-a.** Alternatively, acids of type **1-4** can also be reacted with amines of formula **1-3** in an analogous manner to form compounds of Formula **I-b.** Acids of formula **1-1** and **1-4** can be purchased, synthesized as described in Org. Process Res. Dev. 1997, 1, 72, or synthesized as described herein. Acids of formula **1-1** that contain mixtures of enantiomers may be separated using supercritical fluid chromatography or reversed-phase chromatography with a chiral column when needed to separate them into individual diastereomers or enantiomers as desired to produce compounds of Formula **1-4.**

Amines of formula **1-2** can be synthesized as described herein. Compounds of Formula **I** that contain mixtures of enantiomers or diastereomers may be separated using supercritical fluid chromatography or reversed-phase chromatography with a chiral column when needed to separate them into individual diastereomers or enantiomers as desired to produce compounds of Formula **I-a** or **I-b.** When a single enantiomer of acids of formula **1-4** is used, racemization or epimerization under some general amidation conditions could occur. Instead, by employing lower temperatures, using solvents that aid in dissolution of the reactants, using additives such as imidazolium or pyridinium salts, or other methods as described in Org. Process Res. Dev. 2016, 20, 140*;* Org. Lett. 2012, 14, 1970; or Org. Process Res. Dev. 2009, 13, 106, or using the free base of amines of formula **1-3,** high enantiomeric excess could be retained throughout the reaction. Alternatively, if general conditions are used, or if epimerization or racemization occurs, the mixture of diastereomers formed may be separated using supercritical fluid or reversed-phase chromatography with a chiral column or they may be separated as a diastereomeric salt with an appropriate chiral acid under typical resolution conditions to form compounds of Formula **I-b.**

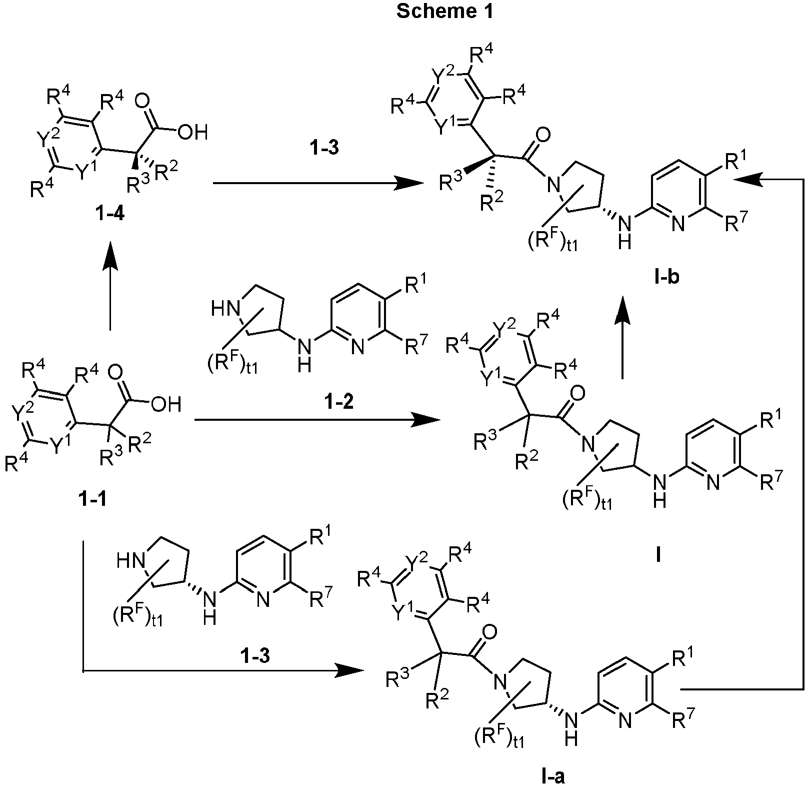

Scheme 2 describes an alternative synthesis of compounds of Formula I and I-b. Amines of formula **2-1** can be reacted with heterocycles of formula **2-2** (where X¹ is F, Cl, Br, I, OTf, SO₂Me, or others) under S_{N}Ar-type conditions (with organic or inorganic bases such as N,N-diisopropylethylamine, triethylamine; sodium, potassium, or cesium carbonate or bicarbonate; DBU, cesium fluoride, tetrabutylammonium fluoride, and the like with a variety of solvents such as dimethyl sulfoxide, ethanol, 1,4-dioxane, *N,N*-dimethylacetamide, and the like and under conventional heating or microwave conditions) or with palladium or copper catalysis with appropriate ligands under common C-N cross-coupling conditions to form compounds of Formula **I-b.** Alternatively, compounds of formula **2-3** (where X² is Cl, Br, I, OTf, or others) can be reacted with a diboron source [such as tetrahydroxydiboron, bis(neopentylglycolato)diboron (5,5,5',5'-tetramethyl-2,2'-bi-1,3,2-dioxaborinane), or bis(pinacolato)diboron (4,4,4',4',5,5,5',5'-octamethyl-2,2'-bi-1,3,2-dioxaborolane)], a di-tin source such as bis(tributyltin) or others, a strong metal-containing base such as isopropylmagnesium chloride followed by a zinc source such as zinc dichloride, or others to form compounds of formula **2-4** (where M¹ can be a boronic acid, boronate, organotin, organozinc, or other metal capable of reacting under C-C cross-coupling conditions) and isolated if stable or telescoped into another reaction if desired. Compounds of formula **2-4** can be reacted with compounds of formula **2-5** (where X³ is Cl, Br, I, OTf, or others) under C-C cross-coupling conditions such as Suzuki (M¹ = boron), Stille (M¹ = tin), Negishi (M¹ = zinc halide), Kumada (M¹ = magnesium halide) type reactions or others to form compounds of Formula **I-b.** Alternatively, compounds of formula **2-3** and **2-6** can be reacted in a similar manner as **2-4** and **2-5** with the nucleophile and electrophile reversed in a cross-coupling reaction to form compounds of Formula **I-b.** Alternatively, compounds of formula **2-7, 2-8, 2-9** can be reacted under the same conditions as their stereodefined analogous intermediates from this scheme without modification of conditions leading to the formation of compounds of Formula **I.**

Additionally, compounds of formula **2-7, 2-8, 2-9** can be separated into their analogous single enantiomers at any point in the route by using similar separation techniques as previously described for the separation of compounds of Formula **I** into **I-b.**

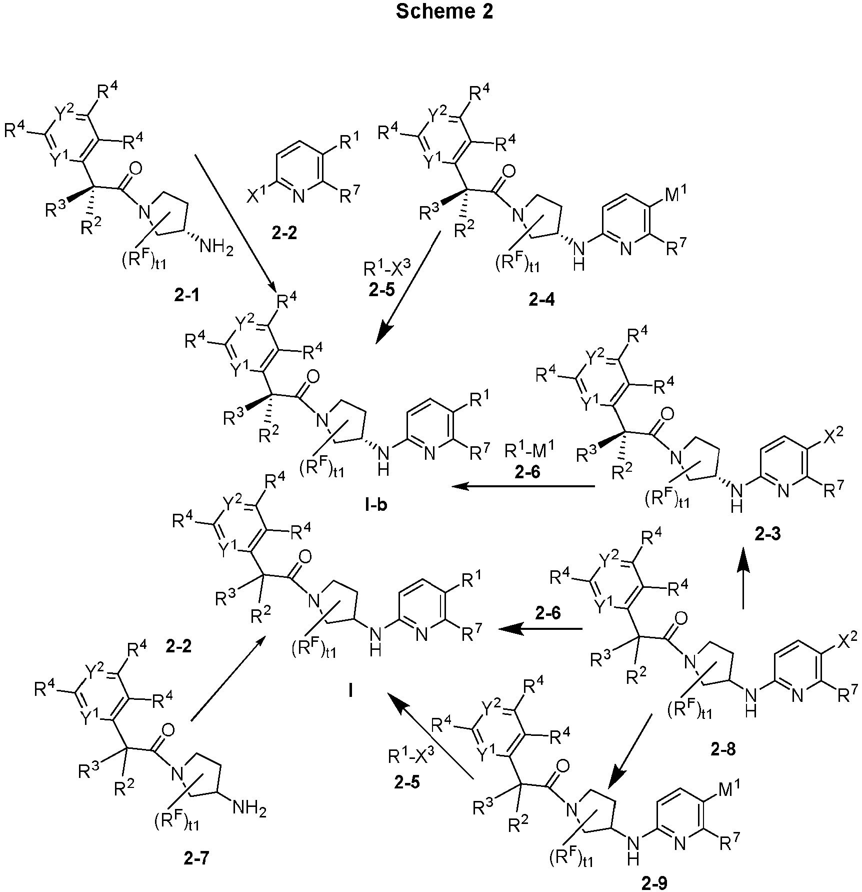

Scheme 3 describes a method to synthesize acids of formula **1-4** selectively as a single enantiomer. Acids of formula **3-1** can be purchased or synthesized using methods described in the literature or herein and reacted with a well-known chiral auxiliary (X^{c}) such as Evans-type (optically pure oxazolidinones), Myers-type (pseudoephedrine-derived), or others described in the literature to form intermediates of formula **3-2.** Treatment of compounds of formula **3-2** with a strong base such as lithium diisopropylamide, lithium bis(trimethylsilyl)amide, sodium bis(trimethylsilyl)amide, potassium bis(trimethylsilyl)amide, or the like and an alkyl halide (when R² and/or R³ is an alkyl group) or other electrophiles like *N*-fluorobenzenesulfonimide (when R² and/or R³ is a fluorine) can form compounds of formula **3-3** in high diastereomeric excess. Hydrolysis conditions of the X^{c} group in compounds of formula **3-3** depend on the individual properties but often reagents (inorganic bases) such as neat or aqueous potassium hydroxide, sodium hydroxide, lithium hydroxide with or without hydrogen peroxide and protic solvents like methanol, ethanol, or others, or aprotic solvents like tetrahydrofuran among others, can be used to form compounds of formula **1-4.**

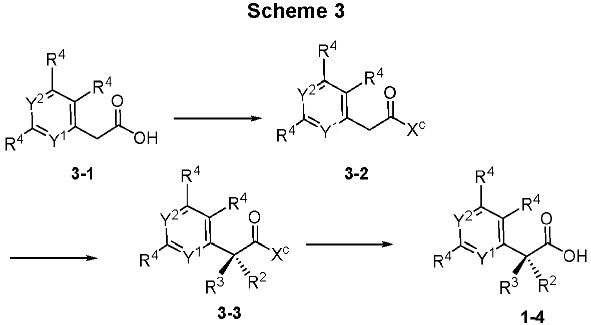

Scheme 4 describes methods that could be used to synthesize acids of formula **4-9** (a sub-type of compounds of formula **1-1**), **4-7** (a sub-type of compounds of formula **1-4**), or **4-8** (a sub-type of compounds of formula **1-4**). Aryl or heteroaryl compounds of formula **4-1** [where X¹ is as previously defined) can be reacted with diprotected malonates of formula **4-2** (where PG¹ can be methyl, ethyl, *tert*-butyl, benzyl, *p*-methoxybenzyl, or others and PG² can be an orthogonally removed protecting group selected from the same choices or could be the same protecting group) using S_{N}Ar conditions or by cross-coupling using a palladium catalyst such as palladium(II) acetate or tris(dibenzylideneacetone)dipalladium(0) [Pd₂(dba)₃] or others with a range of available phosphine ligands or a copper catalyst such as copper(I) iodide or an acidic ligand such as 2-picolinic acid as described in *Org. Lett.* **2007,** 9, 3469 to give intermediates of formula **4-3.** Compounds of formula **4-3** could be treated with an appropriate base such as sodium hydride, lithium diisopropylamide, lithium bis(trimethylsilyl)amide, sodium bis(trimethylsilyl)amide, potassium bis(trimethylsilyl)amide, potassium carbonate, cesium carbonate, or the like and subsequently alkylated with alkylating reagents such as methyl iodide, ethyl iodide, or others, fluorinating agents such as *N*-fluorobenzenesulfonimide, or other electrophiles to give compounds of formula **4-4.** Alternatively, compounds of formula **4-1** can be reacted with compounds of formula **4-6** to directly form compounds of formula **4-4** (under similar conditions as the transformation of compounds of formula **4-1** to compounds of formula **4-3**). Removal of the protecting groups of compounds of formula **4-4** can be performed using standard methods (basic or acidic hydrolysis); or when PG¹ or PG² is benzyl by employing palladium catalysts with hydrogen or reduction sources such as formate, trialkylsilanes, or others to form intermediates of formula **4-5.** Alternatively, compounds of formula **4-4** can directly form acids of formula **4-9** under similar conditions (to those used in producing compounds of formula **4-5**) or those that may require elevated temperatures. The di-acids of formula **4-5** can also be mono-decarboxylated to provide racemic acids of formula **4-9** using base, acid, copper(I) oxide, by heating, or with other suitable conditions. Acids of formula **4-9** that contain mixtures of enantiomers may be separated using supercritical fluid or reversed-phase chromatography with a chiral column or they may be separated and isolated as a diastereomeric salt with an appropriate chiral acid under classical resolution conditions such as described in Org. Process Res. Dev. 2011, 15, 53 or one enantiomer can be selectively transformed into an ester using biocatalysis as described in Adv. Synth. Catal., 2009, 351, 2333 (see also J. Org. Chem. 2003, 68, 7234) and separated to form acids of formula **4-7** or **4-8** in high enantiomeric excess. The di-acids of formula **4-5** can also be mono-decarboxylated using biocatalysis such as Aryl Malonate Decarboxylase (AMDase) enzymes to provide a single enantiomer of compounds of formula **4-7** or **4-8** in high enantiomer excess. See e.g., (a) J. Am. Chem. Soc. 1990, 112, 4077; (b) Eur. J. Biochem. 1992, 210, 475*;* (c) Appl. Environ. Microbiol. 2007, 73, 5676; (d) Appl. Microbiol. Biotechnol. 2016, 100, 8621*.*

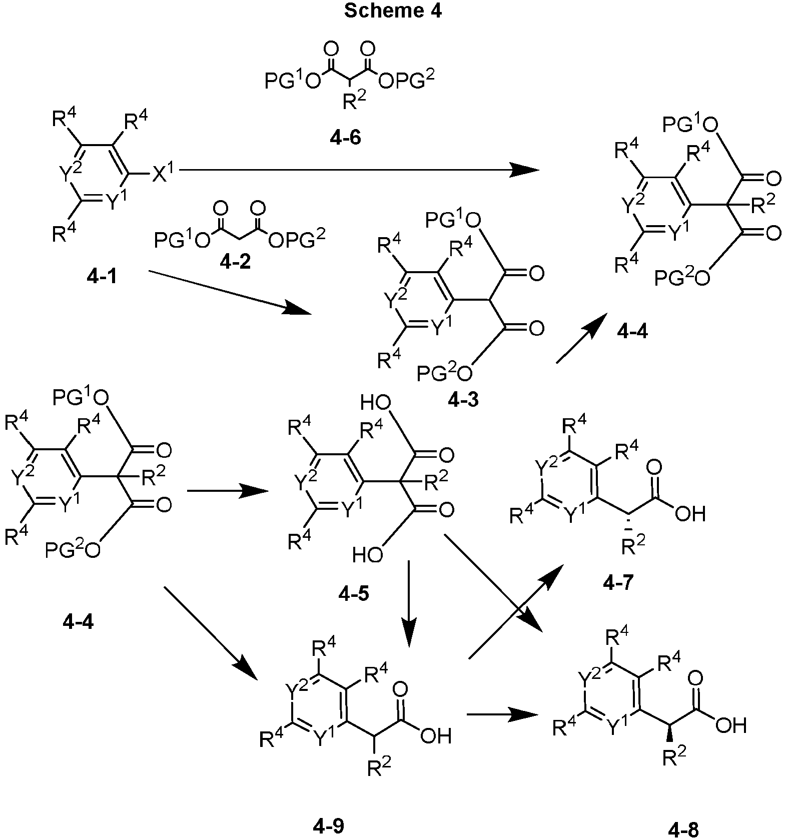

Scheme 5 refers to the synthesis of acids of formula **5-6** (a subtype of compounds of formula **1-1**), **5-7** (a subtype of compounds of formula **1-4**), and **5-8** (a subtype of compounds of formula **1-4**) wherein R^{2c} can be for example, H, alkyl, halogen, etc. (see definitions of R² or R³). Aryl or heteroaryl halides of formula **5-1** (where X⁴ is I, Br, or in some cases Cl) can be purchased or synthesized using methods familiar to those trained in the art of synthesis. Aryl or heteroaryl halides of formula **5-1** can be reacted with an appropriate reagent to perform a metal-halogen exchange such as *n*-butyllithium, isopropylmagnesium chloride or similar metal-containing bases or magnesium metal and quenched with a dicarbonyl compound of formula **5-3** to give compounds of formula **5-4.** Alternatively, arenes or heteroarenes of formula **5-2** can be directly deprotonated with a similar strong base or reagents such as lithium diisopropylamide, lithium 2,2,6,6-tetramethylpiperidide, bis(2,2,6,6-tetramethylpiperidinyl)zinc, or other variations of those and reacted with dicarbonyl compounds of formula **5-3** to form compounds of formula **5-4.** Compounds of formula **5-4** could be treated with strong acid, such as hydrochloric acid, sulfuric acid, boron trifluoride diethyl etherate or other Bronsted or Lewis acids to form compounds of formula **5-5.** Compounds of formula **5-5** could be treated with reducing agents such as silanes in the presence of acids or with hydrogen and a metal catalyst such as palladium to form compounds of formula **5-6.** Alternatively, compounds of formula **5-4** could also be treated with similar acids in the presence of a reducing agent such as silanes or with hydrogen and a metal catalyst such as palladium to form acids of formula **5-6.** Alternatively, compounds of formula **5-5** could be treated with hydrogen and metals such as ruthenium or rhodium or others and a chiral ligand or many other methods such as described in *Org. Chem. Front.* **2014,** *1*, 155 to selectively form acids of formula **5-7** or **5-8** in high enantiomeric excess. Alternatively, compounds of formula **5-5** can be transformed with a biocatalyst such as ENE-reductase (such as described in *ACS Catal.* **2018,** *8*, 3532) or other methods to selectively form compounds of formula **5-7** or **5-8** in high enantiomeric excess. Alternatively, compounds of formula **5-6** that contain mixtures of enantiomers may be separated using supercritical fluid or reversed-phase chromatography with a chiral column or they may be separated and isolated as a diastereomeric salt with an appropriate chiral acid under typical resolution conditions to form compounds of general formula **5-7** or **5-8.**

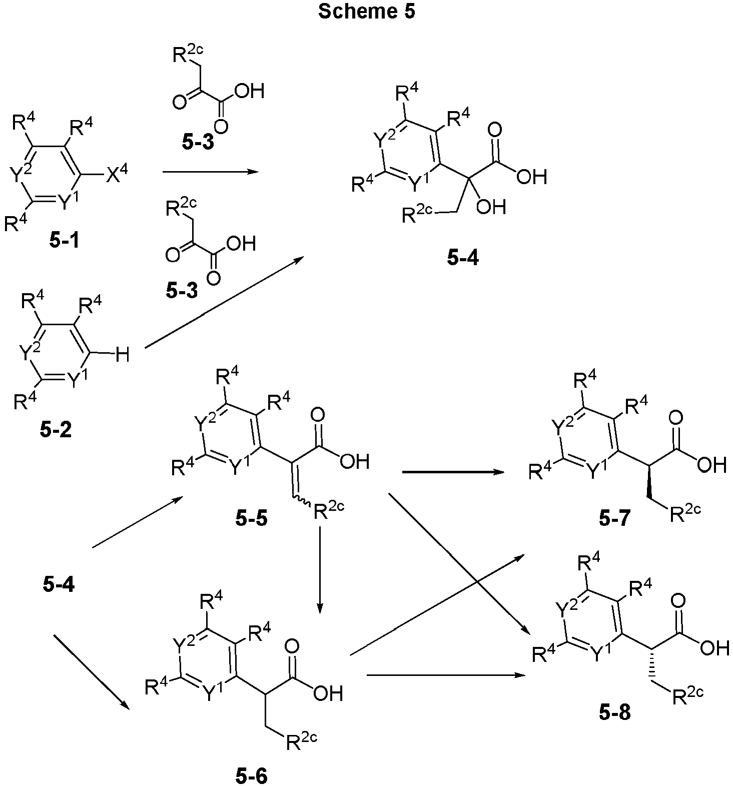

Scheme 6 describes certain other methods to synthesize acids of formulas **1-1** and **1-4.** Compounds of formula **6-1** can be deprotonated with strong bases and acylated with carbon dioxide or a carbonyl compound of formula **6-2** (wherein LG¹ is a leaving group such as a chloride or an alkoxide and PG¹ is a protecting group such as previously described) to form compounds of formula **6-3.** Compounds of formula **6-3** can be deprotonated with strong bases and treated with alkylating agents to form compounds of formula **6-4** in an analogous manner as described above for the transformation of compounds of formula **3-2** to compounds of formula **3-3.** Compounds of **6-4** can be treated under hydrolysis conditions to form compounds of formula **1-1** in a manner analogous to that described above for the transformation of compounds of formula **3-3** to compounds of formula **1-4** or with a metal catalyst such as palladium on carbon and hydrogen when PG¹ is a benzylic group or with acid when PG¹ can leave as a stable cation or be eliminated away to form compounds of formula **1-1.** Alternatively, the steps could be re-ordered such that compounds of formula **6-3** are hydrolyzed to compounds of formula **6-5** and then alkylated using similarly described conditions to form compounds of formula **1-1.** Alternatively, compounds of formula **6-4** can be treated to biocatalysis conditions such as esterase enzymes to form acids of formula **1-4** in high enantiomeric excess. Alternatively, compounds of formula **6-6** can be treated with strong acid such as hydrochloric acid or sulfuric acid in the presence of an alcohol such as methanol or ethanol to form compounds of formula **6-3.** Compounds of formula **6-6** can be alkylated to form compounds of formula **6-7** using a similar method described for the transformation of compounds of formula **6-3** to compounds of formula **6-4.** Alternatively, compounds of formula **6-7** can be directly hydrolyzed to acids of formula **1-1** using either strong acid such as hydrochloric acid or sulfuric acid or strong base such as sodium hydroxide, potassium hydroxide, or lithium hydroxide in the presence of water. Alternatively, compounds of formula **6-8** (where LG² is a leaving group such as Cl, Br, I, OMs, OTs, or others) can be treated with cyanide sources such as sodium cyanide, trimethylsilyl cyanide, or others to form compounds of formula **6-6.** Compounds of formula **6-8** could be purchased or synthesized in a variety of ways as described in the literature or when LG² is e.g., Br or Cl by reacting compounds of formula **6-1** with a halogenating electrophile like *N*-bromosuccinimide, bromine, or others with a radical initiating activator such as 2,2'-azobisisobutyronitrile, light, or other reagents. Compounds of formula **6-12** (a subtype of compounds of formula **6-8** where R² is H) can be transformed into all of the analogous intermediates and compounds derived from **6-8** using similar methods. Compounds of formula **6-14** can be treated with base and alkylating agents as described for the transformation of **6-3** to **6-4** to form compounds of formula **1-1.** Compounds of formula **6-11** may be purchased or synthesized using methods described in the literature. Compounds of formula **6-9** can be oxidized using an oxidizing reagent such as potassium permanganate to also form compounds of formula **6-11.** Compounds of formula **6-11** can be homologated using any number of methods reported in the literature such as the Arndt-Eistert reaction (using an activating reagent such as thionyl chloride, ethyl chloroformate, or others; followed by a diazomethane reagent; a silver salt such as silver benzoate, silver oxide, or others; and a nucleophile such as water or alcohol) or other methods described in the literature such as those described in J. Org. Chem. 2001, 66, 5606 to form compounds of formula **6-10.** Acids of formula **1-1** that contain mixtures of enantiomers may be separated using supercritical fluid or reversed-phase chromatography with a chiral column or they may be separated as a diastereomeric salt with an appropriate chiral acid under classical resolution conditions such as described in Org. Process Res. Dev. 2011, 15, 53 or the undesired enantiomer can be transformed into an ester using biocatalysis as described in Adv. Synth. Catal., 2009, 351, 2333 (see also J. Org. Chem. 2003, 68, 7234) and separated to form acids of formula **1-4** in high enantiomeric excess.

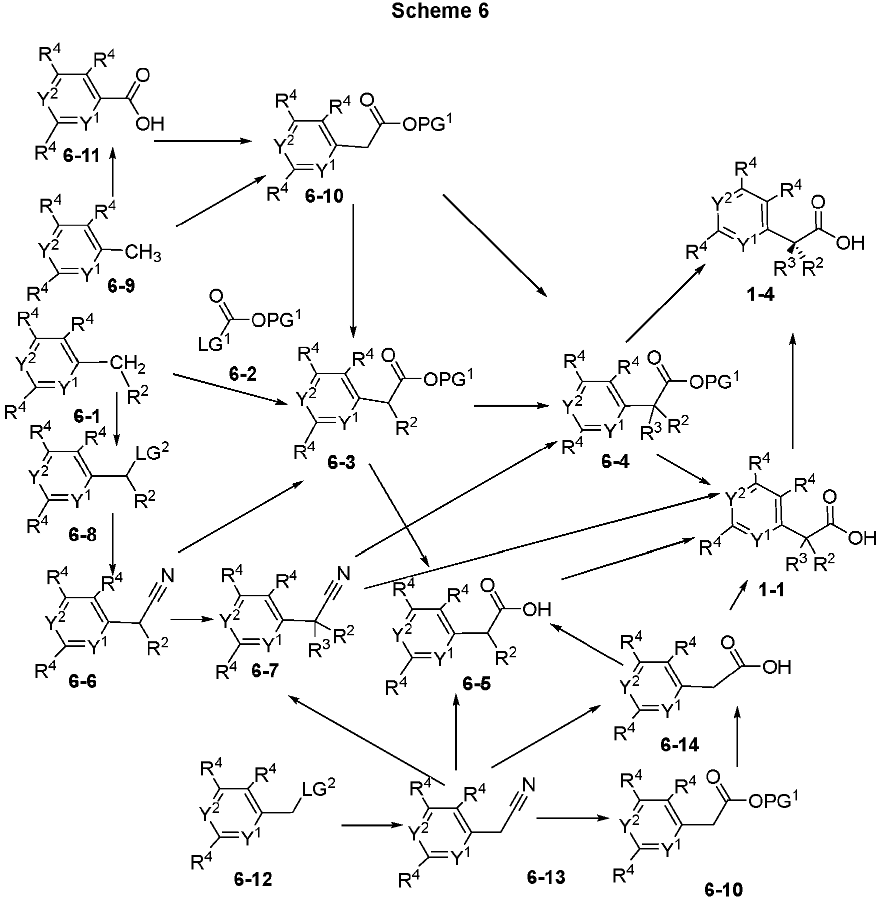

Scheme 7 describes the synthesis of compounds of formula **7-3, 7-4, 7-7, 7-8,** and **7-9** (where Q¹ can be any fragments of the compounds described in Formula **I, I-a, I-b,** or in Schemes 3-6), which can be used as any of the intermediates already described above when appropriate and these substituents can be installed at many points during the syntheses described in Schemes 3-6. Compounds of formula **7-1** (where PG¹ is already described) can be deprotected using dealkylating conditions such as trimethylsilyl iodide, sodium methanethiolate, or others, strong acids such as hydrobromic acid, boron tribromide, or when PG¹ is a benzyl group, palladium or related metals and hydrogen gas can be used to form compounds of formula **7-2.** Compounds of formula **7-2** can be reacted with difluoromethyl sources such as difluorohaloacetates or (bromodifluoromethyl)trimethylsilane to form compounds of formula **7-3.** Compounds of formula **7-2** could also be reacted with trifluoromethyl sources such as difluorohaloacetates with the addition of an electrophilic fluorine source such as Selectfluor^{™}, trifluoromethylhalides, or via an intermediate xanthate that can be treated with XtalFluor^{®} and an electrophilic fluorine source such as *N*-fluorobenzenesulfonimide or 1,3,5-trichloro-1,3,5-triazinane-2,4,6-trione (TCCA) (as described in J. Org. Chem. 2019, 84, 15776) or others to form compounds of formula **7-4.** Compounds of formula **7-5** (where LG³ can be Cl, Br, I, OTf, or others) can be treated with a nucleophilic vinyl source such as vinylboronate, vinyl stannane, or others using palladium-catalyzed cross-coupling conditions described in the literature to form compounds of formula **7-6.** Compounds of formula **7-6** can be oxidatively cleaved to an aldehyde using reagents such as ozone with triphenylphosphine or dimethyl sulfide, osmium tetroxide (or ruthenium trichloride) and sodium periodate, or others to form compounds of formula **7-7.** Alternatively, compounds of formula **7-5** can be directly converted into compound of formula **7-7** by reacting compounds of formula **7-5** under metal-halogen exchange conditions such as isopropylmagnesium chloride, *n*-butyllithium, magnesium, or others and quenching with a carbonyl source such as *N,N*-dimethylformamide, morpholine-4-carbaldehyde, or others. Compound of formula **7-7** can be reacted with nucleophilic difluoromethylation sources such as Deoxo-Fluor^{®} or XtalFluor^{®} and related reagents to form compounds of formula **7-8.** Compounds of formula **7-5** can be treated with alcohols under S_{N}Ar or cross-coupling conditions using palladium and a variety of ligands to form compounds of formula **7-9** [wherein R¹⁰ is, for example, C₁₋₄ alkyl (such as methyl) or C₁₋₄ haloalkyl (such as C₁ fluoroalkyl)]. Alternatively, compounds of formula **7-2** can be reacted with alkyl halides or fluoroalkyl halides such as methyl- (where R¹⁰ is methyl), ethyl- (where R¹⁰ is ethyl), isopropyl- (where R¹⁰ is isopropyl), or cyclopropyl iodide (where R¹⁰ is cyclopropyl) to form compounds of formula **7-9.**

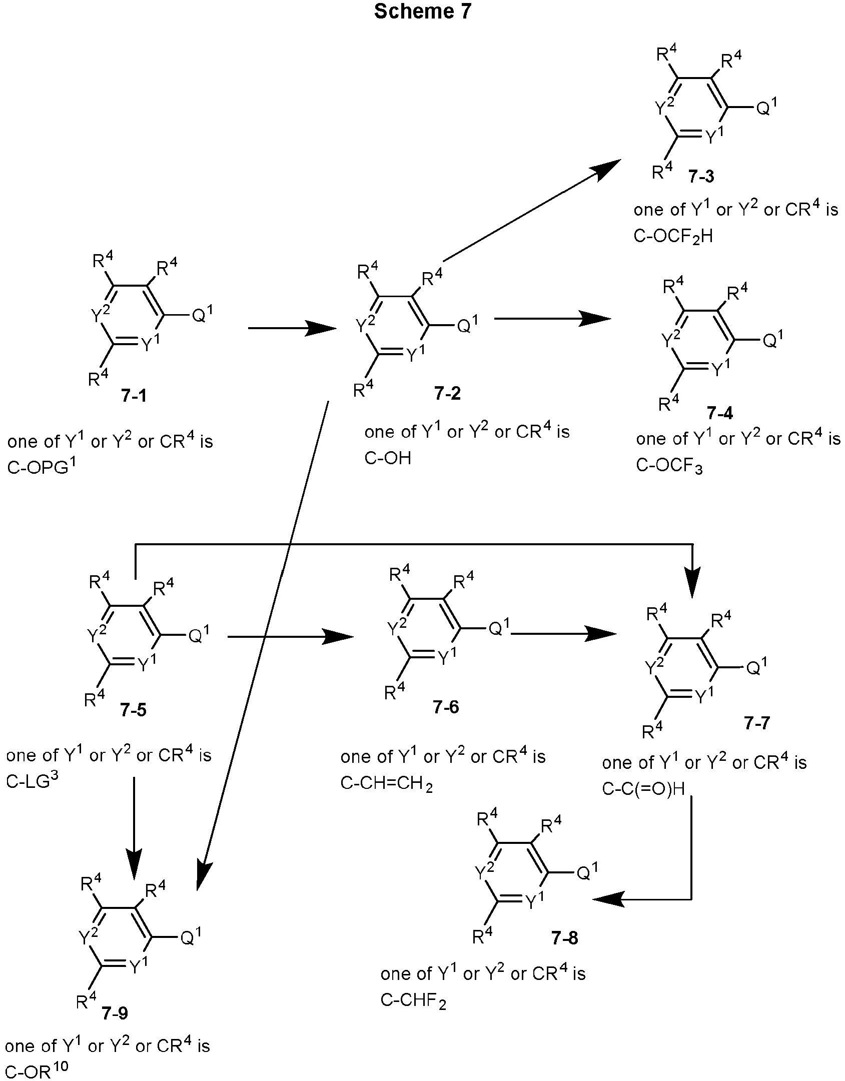

Scheme 8 describes the synthesis of compounds of formulas **1-2** and **1-3.** Compounds of formula **8-1** (wherein PG³ can be benzyl, *p*-methoxybenzyl, *tert*-butoxycarbonyl, benzyloxycarbonyl, acetyl, benzoyl, or other common nitrogen-protecting groups; PG⁴ can be the same as PG³ or can be any of the similar protecting groups that could be removed orthogonally or can be a hydrogen when a protecting group is not needed) can be halogenated by electrophilic halogenation reagents such as dibromohydantoin, *N*-bromosuccinimide, *N-*chlorosuccinimide, bromine, iodine or others to form compounds of formula **8-3** (wherein X² is previously defined). Compounds of formula **8-3** can be reacted with a diboron source [such as tetrahydroxydiboron, bis(neopentylglycolato)diboron (5,5,5',5'-tetramethyl-2,2'-bi-1,3,2-dioxaborinane), or bis(pinacolato)diboron (4,4,4',4',5,5,5',5'-octamethyl-2,2'-bi-1,3,2-dioxaborolane)], a di-tin source such as bis(tributyltin) or others, a strong metal-containing base such as isopropylmagnesium chloride followed by a zinc source such as zinc dichloride, or others to form compounds of formula **8-2** (where M¹ can be a boronic acid, boronate, organotin, organozinc, or other metal capable of reacting under C-C cross-coupling conditions) and isolated if stable or telescoped into another reaction if desired. Compounds of formula **8-2** can be reacted with compounds of formula **2-5** (where X³ is as previously described) under C-C cross-coupling conditions such as Suzuki (M¹ = boron), Stille (M¹ = tin), Negishi (M¹ = zinc halide), Kumada (M¹ = magnesium halide) type reactions or others to form compounds of formula **8-4.** Alternatively, compounds of formula **8-3** and **2-6** can be reacted in a similar manner as **8-2** and **2-5** with the nucleophile and electrophile reversed in a cross-coupling reaction to form compounds of formula **8-4.** Alternatively, in some instances, compounds of formula **8-1** could also be reacted under CH activation/direct-arylation conditions with compounds of formula **2-5** to directly form compounds of formula **8-4.** The PG³ and PG⁴ group of compounds of formula **8-4** can be removed using the appropriate deprotection conditions such as acid or hydrogenolysis or others to form compounds of formula **1-2.** Note - if compounds of formula **8-1, 8-2, 8-3, 8-4,** or **1-2** contain mixtures of stereochemistry or are racemic, they may be separated into single enantiomers using supercritical fluid or reversed-phase chromatography with a chiral column or as a diastereomeric salt with an appropriate chiral acid under classical resolution conditions and separated to form compounds of formula **8-5, 8-7, 8-6, 8-8,** or **1-3** respectively in high enantiomeric excess. Alternatively, compounds of formula **8-5, 8-7, 8-6,** and **8-8** can be reacted under the same conditions as their analogous intermediates from this scheme without modification of conditions leading to the formation of compounds of formula **1-3.**

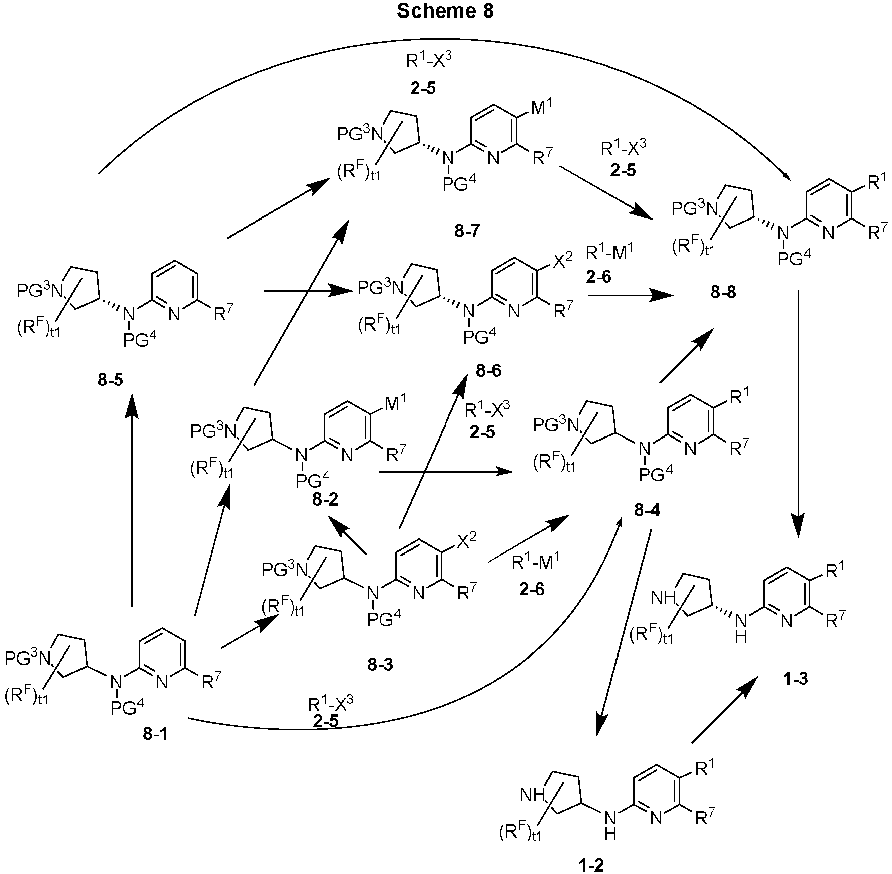

Scheme 9 describes the synthesis of compounds of formulas **2-3** and **2-8.** Compounds of formula **9-1** can be treated with halo-pyridines of formula **9-2** (where X¹ and X² are as previously described) to form compounds of formula **8-3** under S_{N}Ar or metal-catalyzed cross coupling conditions analogous to those described for the synthesis of **I-b** from **2-1** (Scheme 2). The PG³ and PG⁴ group of compounds of formula **8-3** can be removed using the appropriate deprotection conditions such as acid or hydrogenolysis or others to form compounds of formula **9-3.** Compounds of formula **9-3** can be amidated with compounds of formula **1-1** or **1-4** in analogous manner as the conversion of amines of formula **1-2** to compounds of formula **I** (Scheme 1). Note - if compounds of formula **9-1, 8-3, 9-3, or 2-8** contain mixtures of stereochemistry or are racemic, they may be separated into single enantiomers using supercritical fluid or reversed-phase chromatography with a chiral column or as a diastereomeric salt with an appropriate chiral acid under classical resolution conditions and separated to form compounds of formula **94, 8-6, 9-5,** or **2-3,** respectively in high enantiomeric excess. Alternatively, compounds of formula **9-4, 8-6,** and **9-5** can be reacted under the same conditions as their analogous intermediates from this scheme without modification of conditions leading to the formation of compounds of formula **2-3.**

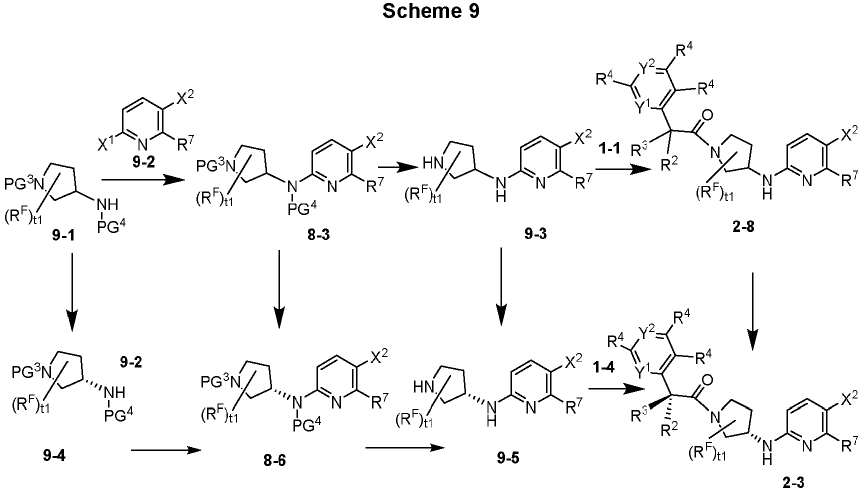

Scheme 10 describes the synthesis of compounds of formulas **2-2** and alternative syntheses of **8-4** and **8-8.** Compounds of formula **10-1** (where R¹¹ can be H, Me, Et, or cyclized with each other to form a 5- or 6-membered alkyl ring such as in a boron pinacolate or neopentylglycolate) can be reacted with compounds of formula **2-5** under Suzuki cross-coupling conditions [analogous to those previously described for the conversion of **2-4** to **I-b** (Scheme 2)] to form compounds of formula **2-2.** Alternatively, compounds of formula **9-2** (where X¹ and X² are as previously described, but X² is more reactive than X¹; for example, when X² is I, X¹ is Br, Cl, or F; or when X² is Br, X¹ is Cl or F) can be reacted with compounds of formula **2-6** in an analogous manner to the conversion of **2-3** to **I-b** (Scheme 2). Compounds of formula **9-1** can be reacted with compounds of formula **2-2** under S_{N}Ar or metal-catalyzed cross coupling conditions [analogous to those described for the synthesis of **I-b** from **2-1** (Scheme 2)] to form compounds of formula **8-4.** Compounds of formula **9-4** can also be reacted in an analogous manner with compounds of formula **2-2** to form compounds of formula **8-8.**

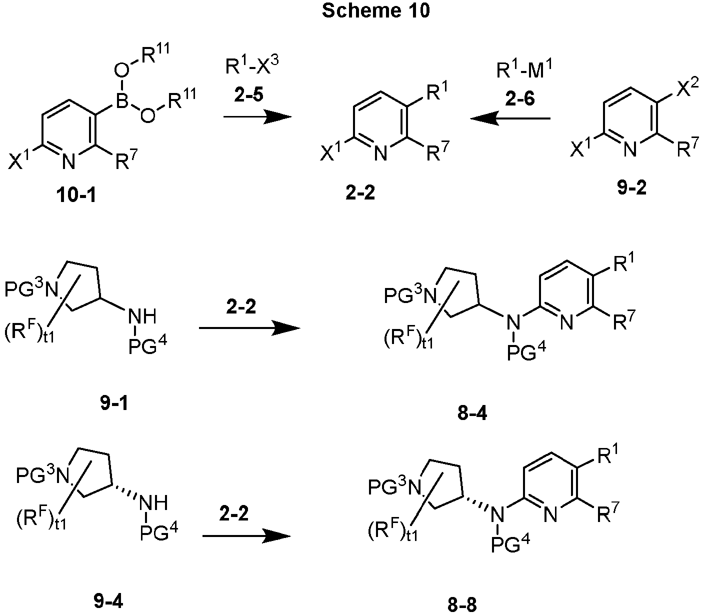

Scheme 11 describes the synthesis of compounds of formula **2-5** and **11-3** (where Y⁶ is N or CH) and **11-9** which are sub-types of **2-5** that may not be commercially available. Compounds of formula **11-1** can be reacted with an electrophilic halogen source in an analogous manner to the transformation of compounds of formulas **8-1** to **8-3** to form compounds of formula **11-2.** Compounds of formula **11-2** (where X³ is previously defined) can be reacted under analogous conditions to the transformation of compounds of formulas **7-2** to **7-3** (when R¹² is H) or compounds of formulas **7-2** to **7-4** (when R¹² is F) to produce compounds of formula **11-3.** Compounds of formula **11-4** (where X⁵ can be a halogen that is the same as X³ or can be more reactive such that X⁵ is Br when X³ is Cl, or X⁵ is I when X³ is Br or Cl) can be reacted under Chan-Lam-type conditions with boronic acids **11-5** (where R¹³ is cyclopropyl, methyl, ethyl, other alkyl groups with or without fluorine atoms, or other variations thereof that include deuterium instead of hydrogen) and a copper source such as copper(II) acetate or others and appropriate ligands such as 2,2'-bipyridine, and any number of bases such as carbonates or amines and a co-oxidant like oxygen that can be added or come from the air to produce compounds of formula **11-7.** Alternatively, compounds of formula **11-4** can be reacted with compounds of **11-6** (where R¹³ and LG³ are previously described) in nucleophilic substitution reactions that use organic bases such as *N*,*N*-diisopropylethylamine, triethylamine, or the like; inorganic bases such as cesium, potassium, or sodium carbonate, or the like; sodium or potassium hydride; lithium diisopropylamide; lithium, sodium, or potassium hexamethyldisilazide; or sodium or potassium hydroxide, or others to form compounds of formula **11-7.** Compounds of formula **11-7** (which can also be purchased such as when R¹³ is methyl or made as described herein or in the literature) can be reacted under metal-halogen exchange conditions such as isopropylmagnesium chloride, n-butyllithium, magnesium, or others and quenched with a carbonyl source such as N,N-dimethylformamide, morpholine-4-carbaldehyde, or others to produce compounds of formula **11-8.** Compounds of formula **11-8** can be reacted under analogous conditions as the transformation of compounds of formulas **7-7** to **7-8** to produce compounds of formula **11-9** wherein R¹⁴ is hydrogen. Alternatively, compounds of formula **11-10** can be treated with sodium nitrite or isoamyl nitrite with hydrobromic acid, potassium bromide, potassium iodide, or other standard Sandmeyer-type conditions to form compounds of formula **2-5.**

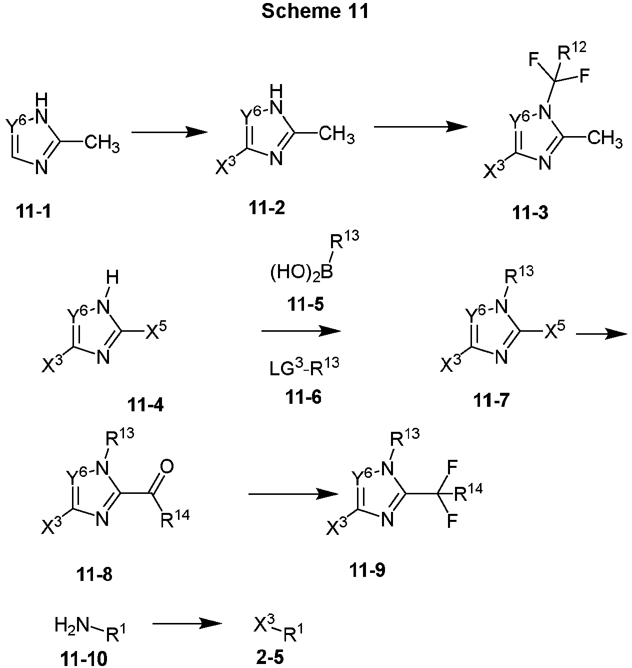

Scheme 12 describes a method of synthesis of compounds of formula **12-5** that are a sub-type of compounds of formula **8-8** where R¹ is a tetrazole substituted with R¹⁵ that can be an optionally substituted alkyl or aryl group. Compounds of formula **8-6** can be reacted through an analogous transformation as described for the syntheses of compounds of formula **7-5** to **7-6** and **7-7** (Scheme 7) to form compounds of formula **12-1** and **12-2,** respectively. Alternatively, compounds of formula **8-6** (when X² is I, Br, or sometimes Cl) can be transformed into compounds of formula **12-2** directly in an analogous manner for the transformation of compounds of formula **11-7** to compounds of formula **11-8** (Scheme 11). Compounds of formula **12-2** can be reacted with substituted hydrazines **12-3** (or their salts and where R¹⁵ is methyl, ethyl, other alkyls, fluorinated alkyls, or deuterated alkyls) under standard condensation conditions using acid or base additives (for example triethylamine or *N,N*-diisopropylethylamine) with a variety of solvents such as methanol, tetrahydrofuran, toluene, dimethyl sulfoxide, or the like to form compounds of formula **12-4.** Compounds of formula **12-4** can be reacted with azo compounds such as diethyl or di-*tert*-butyl azodicarboxylate in the presence of hypervalent iodine sources such as [bis(trifluoroacetoxy)iodo]benzene or others to produce compounds of formula **12-5.**

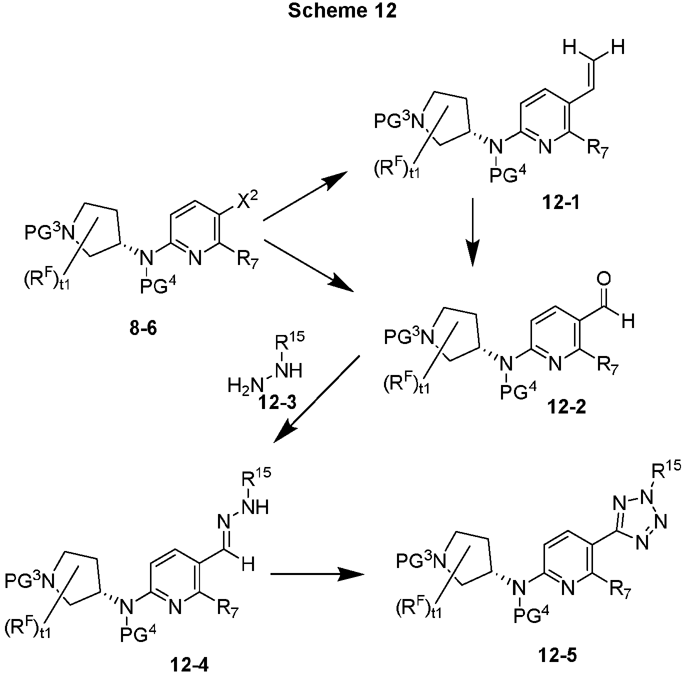

Scheme 13 describes the synthesis of compounds of formulas **13-4,** a subtype of compounds of formula **8-8** where R¹ is an oxadiazole group. Compounds of formula **9-4** can be reacted with compounds of formula **13-1** under S_{N}Ar or metal-catalyzed cross-coupling conditions (analogous to those described for the transformation of **2-1** to **I-b** in Scheme 2) to form compounds of formula **13-2.** Compounds of formula **13-2** can be reacted with hydroxylamine (as a salt or free form) to form Compounds of formula **13-3.** Compounds of formula **13-3** can be reacted with electrophilic trifluoroacetate-containing reagents such as trifluoroacetic anhydride, trifluoroacetyl halides, or others under acidic or basic conditions (such as cesium, potassium, or sodium carbonate or with organic bases such as triethylamine or *N,N-*diisopropylethylamine and the like, or with heating to cyclize to oxadiazoles of formula **13-4.**

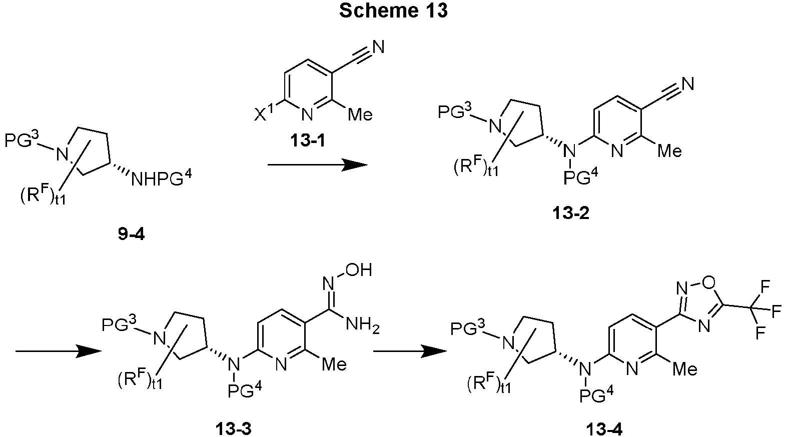

Scheme 14 describes the synthesis of compounds of formula **14-6** (a sub-type of compounds of formula **2-2** where R¹ is the substituted imidazole described herein). Carboxylic acids of formula **14-1** can be transformed into methyl ketones of formula **14-2** using any variety of homologating conditions such as treatment with methyllithium, conversion of the acid first to the Weinreb amide or other amides followed by treatment with a nucleophilic methyl source such as methyllithium, methylmagnesium halide, or other similar reagents, or other single or multi-step conditions known in the literature or to those skilled in the art. Compounds of formula **14-2** can be treated with a halogenating electrophile (like *N*-bromosuccinimide, bromine, chlorine, iodine, dibromoethane, dibromotetrachloroethane, or others) with a radical initiating activator such as (2,2'-azobisisobutyronitrile, light, or other reagents) in the presence or absence of base or in the presence or absence of acid (such as *p*-toluenesulfonic acid, methanesulfonic acid, benzenesulfonic acid, or others) to form compounds of formula **14-3** (where X⁶ is Cl, Br, or I) . Compounds of formula **14-3** can be reacted with amidines of formula **14-4** in the presence of or in the absence of bases such as triethylamine to form imidazoles of formula **14-5.** Compounds of formula **14-5** can then be reacted with compounds of formula **11-6** (where R¹³ is as previously described) in an analogous manner to the reaction of compounds of formula **11-4** and **11-6** to form compounds of formula **11-7** (Scheme 11) to form compounds of formula **14-6.**

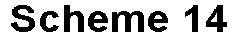

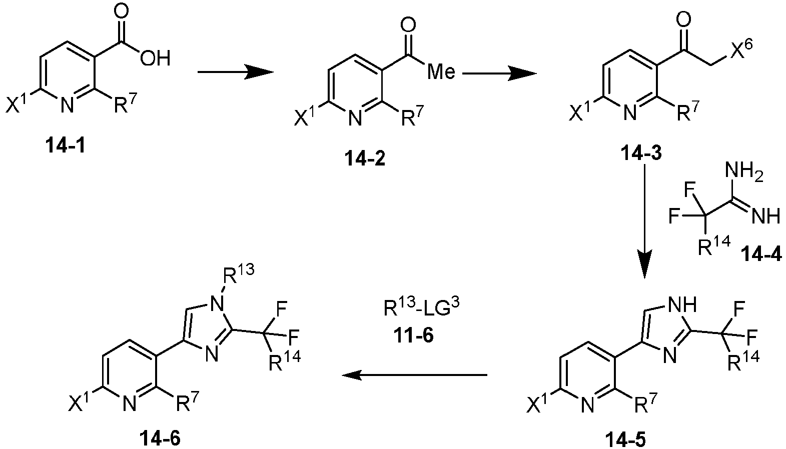

Scheme 15 describes the synthesis of compounds of formula **15-6,** a subtype of compounds of formula **2-5** where R¹ is the described bicyclic system below. Compounds of formula **11-4** can be reacted with epoxides of formula **15-1** (where PG⁵ can be any stable oxygen protecting group compatible with the reaction conditions such as *t*-butyldimethylsilyl, *t-*butyldiphenylsilyl, triisopropylsilyl, benzyl, *p*-methoxybenzyl, or many other common groups) to form the compound of formula **15-2** in an analogous manner to the reaction of compounds of formula **11-4** with compounds of formula **11-6** to form compounds of formula **11-7** (Scheme 11). Compounds of formula **15-2** can be oxidized into compounds of formula **15-3** using oxidation reagents such as Dess-Martin periodinane, under Swern-type conditions, with metal catalysis and a stoichiometric oxidant such as tetrapropylammonium perruthenate with *N-*methylmorpholine N-oxide, or any of numerous other oxidation conditions known to those skilled in the art or found in the literature. Compounds of formula **15-3** can be converted into difluoro compounds of formula **15-4** using conditions analogous to the transformation of compounds of formula **7-7** to compound of formula **7-8** (Scheme 7). Compounds of formula **15-**4 may be converted to compounds of formula **15-5** by deprotection of the PG⁵ group (such as tetrabutylammonium fluoride when PG⁵ is a silyl-protecting group; acidic conditions when PG⁵ is a *p*-methoxybenzyl group; or selective hydrogenolysis conditions when PG⁵ is a benzyl protecting group; or other appropriate deprotecting reagents for the given PG⁵ group). Compounds of formula **15-5** can be cyclized into compounds of formula **15-6** using S_{N}Ar or metal-catalyzed coupling conditions such as those described for the transformation of compounds of formulas **2-1** and **2-2** to compounds of Formula **I-b** (Scheme 2). Under certain conditions compounds of formula **15-4** can be converted directly into compounds of formula **15-6** if the deprotecting conditions used for the transformation of compounds of formula **15-4** to compounds of formula **15-5** also facilitate an intramolecular S_{N}Ar reaction such as when tetrabutylammonium fluoride is used to remove PG⁵ of t-butyldimethylsilyl.

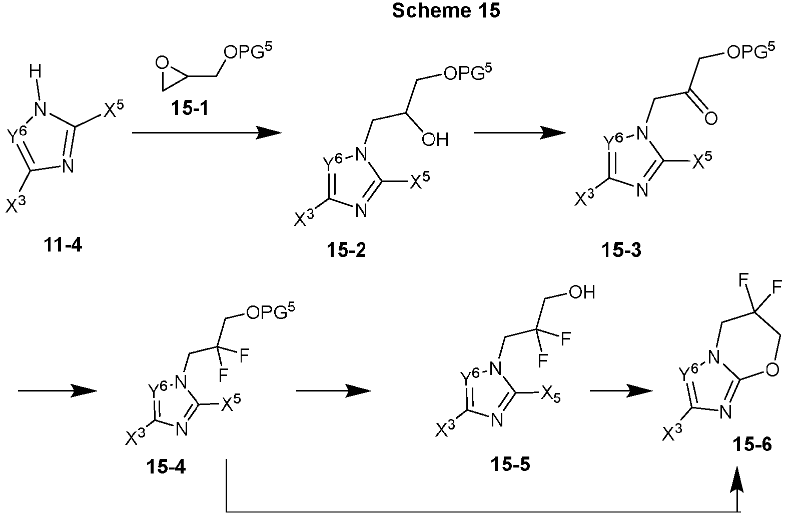

A detailed description of the individual reaction steps is provided in the Example section below. Those skilled in the art will appreciate that other synthetic routes may be used to synthesize the compounds. Although specific starting materials and reagents are discussed below, other starting materials and reagents can be easily substituted to provide a variety of derivatives and/or reaction conditions. In addition, many of the compounds prepared by the methods described below can be further modified in light of this disclosure using conventional chemistry well known to those skilled in the art.

### COMBINATION AGENTS

The compounds of the present invention can be administered alone or in combination with one or more additional therapeutic agents. By "administered in combination" or "combination therapy" it is meant that a compound of the present invention and one or more additional therapeutic agents are administered concurrently to the mammal being treated. When administered in combination, each component may be administered at the same time or sequentially in any order at different points in time. Thus, each component may be administered separately but sufficiently closely in time so as to provide the desired therapeutic effect. The phrases "concurrent administration," "co-administration," "simultaneous administration," and "administered simultaneously" mean that the compounds are administered in combination. Thus, the methods of prevention and treatment described herein include use of combination agents.

The combination agents are administered to a mammal in a therapeutically effective amount. By "therapeutically effective amount" it is meant an amount of a compound of the present invention that, when administered alone or in combination with an additional therapeutic agent to a mammal, is effective to treat the desired disease/disorder/condition (e.g., cachexia, anorexia, anorexia nervosa, nausea; emesis, failure to thrive, sarcopenia, muscle wasting, frailty, osteoporosis, bone loss, pain, anxiety, depression, or hypertension).

In some embodiments, a compound of this invention may be co-administered with one or more other agents such as Orlistat, TZDs and other insulin-sensitizing agents, FGF21 analogs, Metformin, Omega-3-acid ethyl esters (e.g., Lovaza), Fibrates, HMG CoA-reductase Inhibitors, Ezetimibe, Probucol, Ursodeoxycholic acid, TGR5 agonists, FXR agonists, Vitamin E, Betaine, Pentoxifylline, CB1 antagonists, Carnitine, N-acetylcysteine, Reduced glutathione, lorcaserin, the combination of naltrexone with buproprion, SGLT2 inhibitors (including dapagliflozin, canagliflozin, empagliflozin, tofogliflozin, ertugliflozin, ASP-1941, THR1474, TS-071, ISIS388626 and LX4211 as well as those in WO2010023594), Phentermine, Topiramate, GLP-1 receptor agonists, GIP receptor agonists, dual GLP-1 receptor/glucagon receptor agonists (e.g., OPK88003, MEDI0382, JNJ-64565111, NN9277, BI 456906), dual GLP-1 receptor/GIP receptor agonists [e.g., Tirzepatide (LY3298176), NN9423], Angiotensin-receptor blockers, an acetyl-CoA carboxylase (ACC) inhibitor, a BCKDK inhibitor, a ketohexokinase (KHK) inhibitor, ASK1 inhibitors, branched-chain alpha-keto acid dehydrogenase kinase inhibitors (BCKDK inhibitors), inhibitors of CCR2 and/or CCR5, PNPLA3 inhibitors, DGAT1 inhibitors, DGAT2 inhibitors, an FGF21 analog, FGF19 analogs, PPAR agonists, FXR agonists, AMPK activators [e.g., ETC-1002 (bempedoic acid)], SCD1 inhibitors or MPO inhibitors.

Exemplary GLP-1 receptor agonists include liraglutide, albiglutide, exenatide, albiglutide, lixisenatide, dulaglutide, semaglutide, HM15211, LY3298176, Medi-0382, NN-9924, TTP-054, TTP-273, efpeglenatide, those described in WO2018109607, those described in PCT/IB2019/054867 filed June 11, 2019, and those described in PCT/IB2019/054961 filed June 13, 2019, including the following:
2-({4-[2-(4-chloro-2-fluorophenyl)-1,3-benzodioxol-4-yl]piperidin-1-yl}methyl)-1-[(2S)-oxetan-2-ylmethyl]-1H-benzimidazole-6-carboxylic acid;
2-({4-[2-(4-chloro-2-fluorophenyl)-1,3-benzodioxol-4-yl]piperidin-1-yl}methyl)-7-fluoro-1-[(2*S*)-oxetan-2-ylmethyl]-1*H*-benzimidazole-6-carboxylic acid;
2-({4-[(2*S*)-2-(4-chloro-2-fluorophenyl)-1,3-benzodioxol-4-yl]piperidin-1-yl}methyl)-1-[(2*S*)-oxetan-2-ylmethyl]-1*H*-benzimidazole-6-carboxylic acid;
2-({4-[(2*S*)-2-(4-chloro-2-fluorophenyl)-1,3-benzodioxol-4-yl]piperidin-1-yl}methyl)-7-fluoro-1-[(2*S*)-oxetan-2-ylmethyl]-1*H*-benzimidazole-6-carboxylic acid;
2-({4-[2-(4-chloro-2-fluorophenyl)-2-methyl-1,3-benzodioxol-4-yl]piperidin-1-yl}methyl)-1-[(2*S*)-oxetan-2-ylmethyl]-1*H*-benzimidazole-6-carboxylic acid;
2-({4-[2-(4-cyano-2-fluorophenyl)-2-methyl-1,3-benzodioxol-4-yl]piperidin-1-yl}methyl)-1-[(2*S*)-oxetan-2-ylmethyl]-1*H*-benzimidazole-6-carboxylic acid;
2-({4-[2-(5-chloropyridin-2-yl)-2-methyl-1,3-benzodioxol-4-yl]piperidin-1-yl}methyl)-1-[(2*S*)-oxetan-2-ylmethyl]-1*H*-benzimidazole-6-carboxylic acid;
2-({4-[2-(4-chloro-2-fluorophenyl)-2-methyl-1,3-benzodioxol-4-ylpiperidin-1-yl}methyl)-3-(1,3-oxazol-2-ylmethyl)-3*H*-imidazo[4,5-b]pyridine-5-carboxylic acid;
2-({4-[2-(4-chloro-2-fluorophenyl)-2-methyl-1,3-benzodioxol-4-yl]piperidin-1-yl}methyl)-1-[(1-ethyl-1*H*-imidazol-5-yl)methyl]-1H-benzimidazole-6- carboxylic acid;
2-({4-[2-(4-chloro-2-fluorophenyl)-2-methyl-1,3-benzodioxol-4-ylpiperidin-1-yl}methyl)-1-(1,3-oxazol-4-ylmethyl)-1*H*-benzimidazole-6-carboxylic acid;
2-({4-[2-(4-chloro-2-fluorophenyl)-2-methyl-1,3-benzodioxol-4-ylpiperidin-1-yl}methyl)-1-(pyridin-3-ylmethyl)-1*H*-benzimidazole-6-carboxylic acid;
2-({4-[2-(4-chloro-2-fluorophenyl)-2-methyl-1,3-benzodioxol-4-ylpiperidin-1-yl}methyl)-1-(1,3-oxazol-5-ylmethyl)-1*H*-benzimidazole-6-carboxylic acid;
2-({4-[2-(4-chloro-2-fluorophenyl)-2-methyl-1,3-benzodioxol-4-ylpiperidin-1-yl}methyl)-1-[(1-ethyl-1*H*-1,2,3-triazol-5-yl)methyl]-1*H*-benzimidazole-6-carboxylic acid;
2-({4-[2-(4-chloro-2-fluorophenyl)-2-methyl-1,3-benzodioxol-4-yl]piperidin-1-yl}methyl)-1-(1,3-oxazol-2-ylmethyl)-1*H*-benzimidazole-6-carboxylic acid;
2-({4-[2-(4-chloro-2-fluorophenyl)-7-fluoro-2-methyl-1,3-benzodioxol-4-yl]piperidin-1-yl}methyl)-1-[(2*S*)-oxetan-2-ylmethyl]-1*H*-benzimidazole-6-carboxylic acid;
2-({4-[2-(4-cyano-2-fluorophenyl)-2-methyl-1,3-benzodioxol-4-yl]piperidin-1-yl}methyl)-1-(1,3-oxazol-2-ylmethyl)-1*H*-benzimidazole-6-carboxylic acid;
2-({4-[(2S)-2-(4-chloro-2-fluorophenyl)-2-methyl-1,3-benzodioxol-4-yl]piperidin-1-yl}methyl)-7-fluoro-1-[(2*S*)-oxetan-2-ylmethyl]-1*H*-benzimidazole-6-carboxylic acid;
2-({4-[(2*S*)-2-(4-chloro-2-fluorophenyl)-2-methyl-1,3-benzodioxol-4-yl]piperidin-1-yl}methyl)-1-[(2*S*)-oxetan-2-ylmethyl]-1*H*-benzimidazole-6-carboxylic acid;
2-({4-[(2*S*)-2-(4-chloro-2-fluorophenyl)-2-methyl-1,3-benzodioxol-4-yl]piperidin-1-yl}methyl)-7-fluoro-1-[(2*S*)-oxetan-2-ylmethyl]-1*H*-benzimidazole-6-carboxylic acid;
2-({4-[(2*S*)-2-(4-cyano-2-fluorophenyl)-2-methyl-1,3-benzodioxol-4-yl]piperidin-1-yl}methyl)-1-[(2*S*)-oxetan-2-ylmethyl]-1*H*-benzimidazole-6-carboxylic acid;
2-({4-[(2*S*)-2-(5-chloropyridin-2-yl)-2-methyl-1,3-benzodioxol-4-yl]piperidin-1-yl}methyl)-1-[(2*S*)-oxetan-2-ylmethyl]-1*H*-benzimidazole-6-carboxylic acid;
2-({4-[(2*S*)-2-(4-chloro-2-fluorophenyl)-2-methyl-1,3-benzodioxol-4-yl]piperidin-1-yl}methyl)-1-[(1-ethyl-1*H*-imidazol-5-yl)methyl]-1*H*-benzimidazole-6-carboxylic acid;
2-({4-[(2*R*)-2-(4-chloro-2-fluorophenyl)-2-methyl-1,3-benzodioxol-4-yl]piperidin-1-yl}methyl)-1-[(1-ethyl-1*H*-imidazol-5-yl)methyl]-1*H*-benzimidazole-6-carboxylic acid;
2-({4-[2-(5-chloropyridin-2-yl)-2-methyl-1,3-benzodioxol-4-yl]piperidin-1-yl}methyl)-1-[(2*S*)-oxetan-2-ylmethyl]-1*H*-benzimidazole-6-carboxylic acid;
2-({4-[(2*S*)-2-(5-chloropyridin-2-yl)-2-methyl-1,3-benzodioxol-4-yl]piperidin-1-yl}methyl)-1-[(2*S*)-oxetan-2-ylmethyl]-1*H*-benzimidazole-6-carboxylic acid;
2-({4-[(2*R*)-2-(5-chloropyridin-2-yl)-2-methyl-1,3-benzodioxol-4-yl]piperidin-1-yl}methyl)-1-[(2*S*)-oxetan-2-ylmethyl]-1*H*-benzimidazole-6-carboxylic acid;
2-({4-[2-(5-chloropyridin-2-yl)-2-methyl-1,3-benzodioxol-4-yl]piperidin-1-yl}methyl)-1-[(2*S*)-oxetan-2-ylmethyl]-1*H*-benzimidazole-6-carboxylic acid, DIAST-X2;
2-[(4-{2-[(4-chloro-2-fluorobenzyl)oxy]pyridin-3-yl}piperidin-1-yl)methyl]-1-[(2*S*)-oxetan-2-ylmethyl]-1*H*-benzimidazole-6-carboxylic acid;
2-[(4-{2-[(4-chloro-2-fluorobenzyl)oxy]pyridin-3-yl}piperidin-1-yl)methyl]-1-(1,3-oxazol-2-ylmethyl)-1*H*-benzimidazole-6-carboxylic acid;
2-[(4-{2-[(4-cyano-2-fluorobenzyl)oxy]pyridin-3-yl}piperidin-1-yl)methyl]-1-(1,3-oxazol-2-ylmethyl)-1*H*-benzimidazole-6-carboxylic acid;
2-[(4-{2-[(4-cyano-2-fluorobenzyl)oxy]pyridin-3-yl}piperidin-1-yl)methyl]-1-[(2*S*)-oxetan-2-ylmethyl]-1*H*-benzimidazole-6-carboxylic acid;
2-[(4-{3-[(4-chloro-2-fluorobenzyl)oxy]pyrazin-2-yl}piperidin-1-yl)methyl]-1-[(2*S*)-oxetan-2-ylmethyl]-1*H*-benzimidazole-6-carboxylic acid;
2-(6-{6-[(4-cyano-2-fluorobenzyl)oxy]pyridin-2-yl}-6-azaspiro[2.5]oct-1-yl)-1-[(2*S*)-oxetan-2-ylmethyl]-1*H*-benzimidazole-6-carboxylic acid;
2-(6-{2-[(4-chloro-2-fluorobenzyl)oxy]-5-fluoropyrimidin-4-yl}-6-azaspiro[2.5]oct-1-yl)-1-[(2*S*)-oxetan-2-ylmethyl]-1*H*-benzimidazole-6-carboxylic acid;
2-(6-{2-[(4-chloro-2-fluorobenzyl)oxy]-5-fluoropyrimidin-4-yl}-6-azaspiro[2.5]oct-1-yl)-1-(1,3-oxazol-2-ylmethyl)-1*H*-benzimidazole-6-carboxylic acid;
2-(6-{6-[(4-cyano-2-fluorobenzyl)oxy]-5-fluoropyridin-2-yl}-6-azaspiro[2.5]oct-1-yl)-1-[(2*S*)-oxetan-2-ylmethyl]-1*H*-benzimidazole-6-carboxylic acid;
2-(6-{6-[(4-cyano-2-fluorobenzyl)oxy]-3-fluoropyridin-2-yl}-6-azaspiro[2.5]oct-1-yl)-1-[(2*S*)-oxetan-2-ylmethyl]-1*H*-benzimidazole-6-carboxylic acid;
2-[(4-{2-[(4-chloro-2-fluorobenzyl)oxy]pyrimidin-4-yl}piperidin-1-yl)methyl]-1-[(2*S*)-oxetan-2-ylmethyl]-1*H*-benzimidazole-6-carboxylic acid;
2-{[(2*S*)-4-{2-[(4-chloro-2-fluorobenzyl)oxy]-5-fluoropyrimidin-4-yl}-2-methylpiperazin-1-yl]methyl}-1-[(2*S*)-oxetan-2-ylmethyl]-1*H*-benzimidazole-6-carboxylic acid;
2-{[(2*S*)-4-{2-[(4-chloro-2-fluorobenzyl)oxy]pyrimidin-4-yl}-2-methylpiperazin-1-yl]methyl}-1-[(2*S*)-oxetan-2-ylmethyl]-1*H*-benzimidazole-6-carboxylic acid; and
2-[(4-{6-[(4-cyano-2-fluorobenzyl)oxy]pyridin-2-yl}piperidin-1-yl)methyl]-1-[(2*S*)-oxetan-2-ylmethyl]-1*H*-benzimidazole-6-carboxylic acid, and pharmaceutically acceptable salts thereof.

Exemplary ACC inhibitors include 4-(4-[(1-isopropyl-7-oxo-1,4,6,7-tetrahydro-1'*H-*spiro[indazole-5,4'-piperidin]-1'-yl)carbonyl]-6-methoxypyridin-2-yl)benzoic acid, gemcabene, and firsocostat (GS-0976) and pharmaceutically acceptable salts thereof.

Exemplary FXR agonists include tropifexor (2-[(1*R*,3*R*,5*S*)-3-({5-cyclopropyl-3-[2-(trifluoromethoxy)phenyl]-1,2-oxazol-4-yl}methoxy)-8-azabicyclo[3.2.1]octan-8-yl]-4-fluoro-1,3-benzothiazole-6-carboxylic acid), cilofexor (GS-9674), obeticholic acid, LY2562175, Met409, TERN-101 and EDP-305 and pharmaceutically acceptable salts thereof.

Exemplary KHK inhibitors include [(1*R*,5*S*,6*R*)-3-{2-[(2*S*)-2-methylazetidin-1-yl]-6-(trifluoromethyl)pyrimidin-4-yl}-3-azabicyclo[3.1.0]hex-6-yl]acetic acid and pharmaceutically acceptable salts thereof.

Exemplary DGAT2 inhibitors include (S)-2-(5-((3-ethoxypyridin-2-yl)oxy)pyridin-3-yl)-N-(tetrahydrofuran-3-yl)pyrimidine-5-carboxamide [including its crystalline solid forms (Form 1 and Form 2)]. See U.S. Patent No. 10,071,992.

Exemplary BCKDK inhibitors include those described in US Serial No. 62/868,057 filed June 28, 2019 and US Serial No. 62/868,542 filed June 28, 2019 including the following:
5-(5-chloro-4-fluoro 3-methylthiophen-2-yl)-1*H*-tetrazole;
5-(5-chloro-3-difluoromethylthiophen-2-yl)-1*H*-tetrazole;
5-(5-fluoro-3-methylthiophen-2-yl)-1*H*-tetrazole;
5-(5-chloro-3-methylthiophen-2-yl)-1*H*-tetrazole;
5-(3,5-dichlorothiophen-2-yl)-1*H*-tetrazole;
5-(4-bromo-3-methylthiophen-2-yl)-1*H*-tetrazole;
5-(4-bromo-3-ethylthiophen-2-yl)-1*H*-tetrazole;
5-(4-chloro-3-ethylthiophen-2-yl)-1*H*-tetrazole;
3-chloro-5-fluorothieno[3,2-b]thiophene-2-carboxylic acid;
3-bromo-5-fluorothieno[3,2- b]thiophene-2-carboxylic acid;
3-(difluoromethyl)-5-fluorothieno[3,2-b]thiophene-2-carboxylic acid;
5,6-difluorothieno[3,2-b]thiophene-2-carboxylic acid; and
3,5-difluorothieno[3,2-b]thiophene-2-carboxylic acid;
or a pharmaceutically acceptable salt thereof.

In some embodiments, a compound of this invention may be co-administered with one or more anti-diabetic agents. Suitable anti-diabetic agents include insulin, metformin, GLP-1 receptor agonists (described herein above), an acetyl-CoA carboxylase (ACC) inhibitor (described herein above), SGLT2 inhibitors (described herein above), monoacylglycerol O-acyltransferase inhibitors, phosphodiesterase (PDE)-10 inhibitors, AMPK activators [e.g., ETC-1002 (bempedoic acid)], sulfonylureas (e.g., acetohexamide, chlorpropamide, diabinese, glibenclamide, glipizide, glyburide, glimepiride, gliclazide, glipentide, gliquidone, glisolamide, tolazamide, and tolbutamide), meglitinides, α-amylase inhibitors (e.g., tendamistat, trestatin and AL-3688), an α-glucoside hydrolase inhibitor (e.g., acarbose), α-glucosidase inhibitors (e.g., adiposine, camiglibose, emiglitate, miglitol, voglibose, pradimicin-Q, and salbostatin), PPARγ agonists (e.g., balaglitazone, ciglitazone, darglitazone, englitazone, isaglitazone, pioglitazone and rosiglitazone), PPAR α/γ agonists (e.g., CLX-0940, GW-1536, GW-1929, GW-2433, KRP-297, L-796449, LR-90, MK-0767 and SB-219994), protein tyrosine phosphatase-1B (PTP-1B) inhibitors [e.g., trodusquemine, hyrtiosal extract, and compounds disclosed by Zhang, S. et al., Drug Discovery Today, 12(9/10), 373-381 (2007)], SIRT-1 activators (e.g., resveratrol, GSK2245840 or GSK184072), dipeptidyl peptidase IV (DPP-IV) inhibitors (e.g., those in WO2005116014, sitagliptin, vildagliptin, alogliptin, dutogliptin, linagliptin and saxagliptin), insulin secretagogues, fatty acid oxidation inhibitors, A2 antagonists, c-jun amino-terminal kinase (JNK) inhibitors, glucokinase activators (GKa) such as those described in WO2010103437, WO2010103438, WO2010013161, WO2007122482, TTP-399, TTP-355, TTP-547, AZD1656, ARRY403, MK-0599, TAK-329, AZD5658 or GKM-001, insulin, insulin mimetics, glycogen phosphorylase inhibitors (e.g., GSK1362885), VPAC2 receptor agonists, glucagon receptor modulators such as those described in Demong, D.E. et al., Annual Reports in Medicinal Chemistry 2008, 43, 119-137, GPR119 modulators, particularly agonists, such as those described in WO2010140092, WO2010128425, WO2010128414, WO2010106457, Jones, R.M. et al., Annual Reports in Medicinal Chemistry 2009, 44, 149-170 (e.g., MBX-2982, GSK1292263, APD597 and PSN821), FGF21 derivatives or analogs such as those described in Kharitonenkov, A. et al., Current Opinion in Investigational Drugs 2009, 10(4)359-364, TGR5 (also termed GPBAR1) receptor modulators, particularly agonists, such as those described in Zhong, M., Current Topics in Medicinal Chemistry, 2010, 10(4), 386-396 and INT777, GPR40 agonists, such as those described in Medina, J.C., Annual Reports in Medicinal Chemistry, 2008, 43, 75-85, including but not limited to TAK-875, GPR120 modulators, particularly agonists, high-affinity nicotinic acid receptor (HM74A) activators, and SGLT1 inhibitors, such as GSK1614235. A further representative listing of anti-diabetic agents that can be combined with the compounds of the present invention can be found, for example, at page 28, line 35 through page 30, line 19 of WO2011005611.

Other antidiabetic agents could include inhibitors or modulators of carnitine palmitoyl transferase enzymes, inhibitors of fructose 1,6-diphosphatase, inhibitors of aldose reductase, mineralocorticoid receptor inhibitors, inhibitors of TORC2, inhibitors of CCR2 and/or CCR5, inhibitors of PKC isoforms (e.g., PKCα, PKCβ, PKCγ), inhibitors of fatty acid synthetase, inhibitors of serine palmitoyl transferase, modulators of GPR81, GPR39, GPR43, GPR41, GPR105, Kv1.3, retinol binding protein 4, glucocorticoid receptor, somatostain receptors (e.g., SSTR1, SSTR2, SSTR3 and SSTR5), inhibitors or modulators of PDHK2 or PDHK4, inhibitors of MAP4K4, modulators of IL1 family including IL1beta, modulators of RXRalpha. In addition, suitable anti-diabetic agents include mechanisms listed by Carpino, P.A., Goodwin, B. Expert Opin. Ther. Pat., 2010, 20(12), 1627-51.

The compounds of the present invention may be co-administered with anti-heart failure agents such as ACE inhibitors (e.g., captopril, enalapril, fosinopril, lisinopril, perindopril, quinapril, ramipril, trandolapril), Angiotensin II receptor blockers (e.g., candesartan, losartan, valsartan), Angiotensin-receptor neprilysin inhibitors (sacubitril/valsartan), I_{f} channel blocker Ivabradine, Beta-Adrenergic blocking agents (e.g., bisoprolol, metoprolol succinate, carvedilol), Aldosterone antagonists (e.g., spironolactone, eplerenone), hydralazine and isosorbide dinitrate, diuretics (e.g., furosemide, bumetanide, torsemide, chlorothiazide, amiloride, hydrochlorothiazide, Indapamide, Metolazone, Triamterene), or digoxin.

The compounds of the present invention may also be co-administered with cholesterol or lipid lowering agents including the following exemplary agents: HMG CoA reductase inhibitors (e.g., pravastatin, pitavastatin, lovastatin, atorvastatin, simvastatin, fluvastatin, NK-104 (a.k.a. itavastatin, or nisvastatin or nisbastatin) and ZD-4522 (a.k.a. rosuvastatin, or atavastatin or visastatin); squalene synthetase inhibitors; fibrates (e.g., gemfibrozil, pemafibrate, fenofibrate, clofibrate); bile acid sequestrants (such as questran, colestipol, colesevelam); ACAT inhibitors; MTP inhibitors; lipoxygenase inhibitors; cholesterol absorption inhibitors (e.g., ezetimibe); nicotinic acid agents (e.g., niacin, niacor, slo-niacin); omega-3 fatty acids (e.g., epanova, fish oil, eicosapentaenoic acid); cholesteryl ester transfer protein inhibitors (e.g., obicetrapib) and PCSK9 modulators [e.g., alirocumab, evolocumab, bococizumab, ALN-PCS (inclisiran)].

The compounds of the present invention may also be used in combination with antihypertensive agents and such antihypertensive activity is readily determined by those skilled in the art according to standard assays (e.g., blood pressure measurements). Examples of suitable anti-hypertensive agents include: alpha-adrenergic blockers; beta-adrenergic blockers; calcium channel blockers (e.g., diltiazem, verapamil, nifedipine and amlodipine); vasodilators (e.g., hydralazine), diuretics (e.g., chlorothiazide, hydrochlorothiazide, flumethiazide, hydroflumethiazide, bendroflumethiazide, methylchlorothiazide, trichloromethiazide, polythiazide, benzthiazide, ethacrynic acid tricrynafen, chlorthalidone, torsemide, furosemide, musolimine, bumetanide, triamtrenene, amiloride, spironolactone); renin inhibitors; ACE inhibitors (e.g., captopril, zofenopril, fosinopril, enalapril, ceranopril, cilazopril, delapril, pentopril, quinapril, ramipril, lisinopril); AT-1 receptor antagonists (e.g., losartan, irbesartan, valsartan); ET receptor antagonists (e.g., sitaxsentan, atrsentan and compounds disclosed in U.S. Patent Nos. 5,612,359 and 6,043,265); Dual ET/All antagonist (e.g., compounds disclosed in WO 00/01389); neutral endopeptidase (NEP) inhibitors; vasopepsidase inhibitors (dual NEP-ACE inhibitors) (e.g., gemopatrilat and nitrates). An exemplary antianginal agent is ivabradine.

Examples of suitable calcium channel blockers (L-type or T-type) include diltiazem, verapamil, nifedipine and amlodipine and mybefradil.

Examples of suitable cardiac glycosides include digitalis and ouabain.

In one embodiment, a compound of invention may be co-administered with one or more diuretics. Examples of suitable diuretics include (a) loop diuretics such as furosemide (such as LASIX^{™}), torsemide (such as DEMADEX^{™}), bemetanide (such as BUMEX^{™}), and ethacrynic acid (such as EDECRIN^{™}); (b) thiazide-type diuretics such as chlorothiazide (such as DIURIL^{™}, ESIDRIX^{™} or HYDRODIURIL^{™}), hydrochlorothiazide (such as MICROZIDE^{™} or ORETIC^{™}), benzthiazide, hydroflumethiazide (such as SALURON^{™}), bendroflumethiazide, methychlorthiazide, polythiazide, trichlormethiazide, and indapamide (such as LOZOL^{™}); (c) phthalimidine-type diuretics such as chlorthalidone (such as HYGROTON^{™}), and metolazone (such as ZAROXOLYN^{™}); (d) quinazoline-type diuretics such as quinethazone; and (e) potassium-sparing diuretics such as triamterene (such as DYRENIUM^{™}), and amiloride (such as MIDAMOR^{™} or MODURETIC^{™}).

In another embodiment, a compound of the invention may be co-administered with a loop diuretic. In still another embodiment, the loop diuretic is selected from furosemide and torsemide. In still another embodiment, one or more compounds of Formula I or their pharmaceutically acceptable salts may be co-administered with furosemide. In still another embodiment, one or more compounds of Formula I or their pharmaceutically acceptable salts may be co-administered with torsemide which may optionally be a controlled or modified release form of torsemide.

In another embodiment, a compound of the invention may be co-administered with a thiazide-type diuretic. In still another embodiment, the thiazide-type diuretic is selected from the group consisting of chlorothiazide and hydrochlorothiazide. In still another embodiment, one or more compounds of Formula I or their pharmaceutically acceptable salts may be co-administered with chlorothiazide. In still another embodiment, one or more compounds of Formula I or their pharmaceutically acceptable salts may be co-administered with hydrochlorothiazide.

In another embodiment, one or more compounds of Formula I or their pharmaceutically acceptable salts may be co-administered with a phthalimidine-type diuretic. In still another embodiment, the phthalimidine-type diuretic is chlorthalidone.

Examples of suitable mineralocorticoid receptor antagonists include sprionolactone and eplerenone.

Examples of suitable phosphodiesterase inhibitors include: PDE III inhibitors (such as cilostazol); and PDE V inhibitors (such as sildenafil).

Those skilled in the art will recognize that the compounds of this invention may also be used in conjunction with other cardiovascular or cerebrovascular treatments including PCI, stenting, drug-eluting stents, stem cell therapy and medical devices such as implanted pacemakers, defibrillators, or cardiac resynchronization therapy.

Particularly when provided as a single dosage unit, the potential exists for a chemical interaction between the combined active ingredients. For this reason, when a compound of this invention and a second therapeutic agent are combined in a single dosage unit, they are formulated such that although the active ingredients are combined in a single dosage unit, the physical contact between the active ingredients is minimized (that is, reduced). For example, one active ingredient may be enteric-coated. By enteric-coating one of the active ingredients, it is possible not only to minimize the contact between the combined active ingredients, but also, it is possible to control the release of one of these components in the gastrointestinal tract such that one of these components is not released in the stomach but rather is released in the intestines. One of the active ingredients may also be coated with a material that effects a sustained release throughout the gastrointestinal tract and also serves to minimize physical contact between the combined active ingredients. Furthermore, the sustained-released component can be additionally enteric-coated such that the release of this component occurs only in the intestine. Still another approach would involve the formulation of a combination product in which the one component is coated with a sustained and/or enteric-release polymer, and the other component is also coated with a polymer such as a low viscosity grade of hydroxypropyl methylcellulose (HPMC) or other appropriate materials as known in the art, in order to further separate the active components. The polymer coating serves to form an additional barrier to interaction with the other component.

These as well as other ways of minimizing contact between the components of combination products of the present invention, whether administered in a single dosage form or administered in separate forms but at the same time by the same manner, will be readily apparent to those skilled in the art, once armed with the present disclosure.

In combination therapy treatment, both the compounds of this invention and the other drug therapies are administered to mammals (e.g., humans, male or female) by conventional methods. A compound of Formula I or a salt thereof is adapted to therapeutic use as agents that antagonize (including inhibit) MC4R in mammals, particularly humans, and thus are useful for the treatment of the various conditions (e.g., those described herein) in which such action is implicated.

The disease/disorder/condition that can be treated in accordance with the present invention include, but are not limited to cachexia (e.g., cachexia associated with cancer, AIDS, CHF, and/or CKD); anorexia/anorexia nervosa (e.g., geriatric anorexia, anorexia associated with chemotherapy and/or radiotherapy); nausea; emesis; weight loss (e.g., involuntary weight loss); failure to thrive; sarcopenia; muscle wasting; frailty; osteoporosis; bone disorders (e.g., bone loss); pain; neuropathic pain; anxiety; depression; hypertension; malnutrition; obesity; sexual dysfunction; and inflammatory disease.

Administration of the compounds of this invention can be via any method which delivers a compound of this invention systemically and/or locally. These methods include oral routes, parenteral, intraduodenal routes, buccal, intranasal, etc. Generally, the compounds of this invention are administered orally, but parenteral administration (e.g., intravenous, intramuscular, subcutaneous or intramedullary) may be utilized, for example, where oral administration is inappropriate for the target or where the patient is unable to ingest the drug.

For administration to human patients, an oral daily dose of the compounds herein may be, for example, in the range 0.01 mg to 5000 mg depending, of course, on the mode of and frequency of administration, the disease state, and the age and condition of the patient, etc. An oral daily dose is in the range of 1 mg to 2000 mg (e.g., 3 mg to 2000 mg) may be used. A further oral daily dose is in the range of 5 mg to 1000 mg. For convenience, the compounds of the present invention can be administered in a unit dosage form. If desired, multiple doses per day of the unit dosage form can be used to increase the total daily dose. The unit dosage form, for example, may be a tablet or capsule containing about 0.1, 0.5, 1, 5, 10, 15, 20, 25, 30, 35, 40, 45, 50, 55, 60, 65, 70, 75, 80, 85, 90, 95, 100, 125, 150, 175, 200, 250, 300, 500, or 1000 mg of the compound of the present invention. The total daily dose may be administered in single or divided doses and may, at the physician's discretion, fall outside of the typical ranges given herein.

For administration to human patients, an infusion daily dose of the compounds herein may be in the range 1 mg to 2000 mg depending, of course, on the mode of and frequency of administration, the disease state, and the age and condition of the patient, etc. A further infusion daily dose is in the range of 5 mg to 1000 mg. The total daily dose may be administered in single or divided doses and may, at the physician's discretion, fall outside of the typical ranges given herein.

According to the methods of treatment of the invention, a compound of the present invention or a combination of a compound of the present invention and at least one additional pharmaceutical agent (referred to herein as a "combination") is administered to a subject in need of such treatment, preferably in the form of a pharmaceutical composition. In the combination aspect of the invention, the compound of the present invention and at least one other pharmaceutical agent (e.g., another anti-cachexia or anti-anorexia agent) may be administered either separately or in a pharmaceutical composition comprising both. It is generally preferred that such administration be oral.

When a combination of a compound of the present invention and at least one other pharmaceutical agent are administered together, such administration may be sequential in time or simultaneous. In some embodiments, simultaneous administration of drug combinations is used. For separate or sequential administration, a compound of the present invention and the additional pharmaceutical agent may be administered in any order and each of them can be administered in an independent frequency or dose regimen. In some embodiments, such administration be oral. In some embodiments, such administration can be oral and simultaneous. When a compound of the present invention and the additional pharmaceutical agent are administered sequentially, the administration of each may be by the same or by different methods.

According to the methods of the invention, a compound of the present invention or a combination can be administered in the form of a pharmaceutical composition. Accordingly, a compound of the present invention or a combination can be administered to a patient separately or together in any conventional oral, rectal, transdermal, parenteral (e.g., intravenous, intramuscular or subcutaneous), intracisternal, intravaginal, intraperitoneal, topical (e.g., powder, ointment, cream, spray or lotion), buccal or nasal dosage form (e.g., spray, drops or inhalant).

The compounds of the invention or combinations can be administered alone or be administered in an admixture with one or more suitable pharmaceutical excipients, adjuvants, diluents or carriers known in the art and selected with regard to the intended route of administration and standard pharmaceutical practice. The compound of the invention or combination may be formulated to provide immediate-, delayed-, modified-, sustained-, pulsed- or controlled-release dosage forms depending on the desired route of administration and the specificity of release profile, commensurate with therapeutic needs.

The pharmaceutical composition comprises a compound of the invention or a combination in an amount generally in the range of from about 1% to about 75%, 80%, 85%, 90% or even 95% (by weight) of the composition, usually in the range of about 1%, 2% or 3% to about 50%, 60% or 70%, more frequently in the range of about 1%, 2% or 3% to less than 50% such as about 25%, 30% or 35%.

Methods of preparing various pharmaceutical compositions with a specific amount of active compound are known to those skilled in this art. For examples, see Remington, J.P., The Science and Practice of Pharmacy, Lippincott Williams and Wilkins, Baltimore, Md. 20th ed., 2000.

Compositions suitable for parenteral injection generally include pharmaceutically acceptable, sterile, aqueous or nonaqueous solutions, dispersions, suspensions, or emulsions, and sterile powders for reconstitution into sterile injectable solutions or dispersions. Examples of suitable aqueous and nonaqueous carriers or diluents (including solvents and vehicles) include water, ethanol, polyols (propylene glycol, polyethylene glycol, glycerol, and the like), suitable mixtures thereof, triglycerides including vegetable oils such as olive oil, and injectable organic esters such as ethyl oleate. A preferred carrier is Miglyol.RTM. brand caprylic/capric acid ester with glycerin or propylene glycol (e.g., Miglyol.RTM. 812, Miglyol.RTM. 829, Miglyol.RTM. 840) available from Condea Vista Co., Cranford, N.J. Proper fluidity can be maintained, for example, by the use of a coating such as lecithin, by the maintenance of the required particle size in the case of dispersions, and by the use of surfactants.

These compositions for parenteral injection may also contain excipients such as preserving, wetting, emulsifying, and dispersing agents. Prevention of microorganism contamination of the compositions can be accomplished with various antibacterial and antifungal agents, for example, parabens, chlorobutanol, phenol, sorbic acid, and the like. It may also be desirable to include isotonic agents, for example, sugars, sodium chloride, and the like. Prolonged absorption of injectable pharmaceutical compositions can be brought about by the use of agents capable of delaying absorption, for example, aluminum monostearate and gelatin.

Solid dosage forms for oral administration include capsules, tablets, chews, lozenges, pills, powders, and multi-particulate preparations (granules). In such solid dosage forms, a compound of the present invention or a combination is admixed with at least one inert excipient, diluent or carrier. Suitable excipients, diluents or carriers include materials such as sodium citrate or dicalcium phosphate and/or (a) one or more fillers or extenders (e.g., microcrystalline cellulose (available as Avicel.TM. from FMC Corp.) starches, lactose, sucrose, mannitol, silicic acid, xylitol, sorbitol, dextrose, calcium hydrogen phosphate, dextrin, alpha-cyclodextrin, betacyclodextrin, polyethylene glycol, medium chain fatty acids, titanium oxide, magnesium oxide, aluminum oxide and the like); (b) one or more binders (e.g., carboxymethylcellulose, methylcellulose, hydroxypropylcellulose, hydroxypropylmethylcellulose, gelatin, gum arabic, ethyl cellulose, polyvinyl alcohol, pullulan, pregelatinized starch, agar, tragacanth, alginates, gelatin, polyvinylpyrrolidone, sucrose, acacia and the like); (c) one or more humectants (e.g., glycerol and the like); (d) one or more disintegrating agents (e.g., agar-agar, calcium carbonate, potato or tapioca starch, alginic acid, certain complex silicates, sodium carbonate, sodium lauryl sulphate, sodium starch glycolate (available as Explotab.TM. from Edward Mendell Co.), crosslinked polyvinyl pyrrolidone, croscarmellose sodium A-type (available as Ac-di-sol.TM.), polyacrilin potassium (an ion exchange resin) and the like); (e) one or more solution retarders (e.g., paraffin and the like); (f) one or more absorption accelerators (e.g., quaternary ammonium compounds and the like); (g) one or more wetting agents (e.g., cetyl alcohol, glycerol monostearate and the like); (h) one or more adsorbents (e.g., kaolin, bentonite and the like); and/or one or more lubricants (e.g., talc, calcium stearate, magnesium stearate, stearic acid, polyoxyl stearate, cetanol, hydrogenated castor oil, sucrose esters of fatty acid, dimethylpolysiloxane, microcrystalline wax, yellow beeswax, white beeswax, solid polyethylene glycols, sodium lauryl sulfate and the like). In the case of capsules and tablets, the dosage forms may also comprise buffering agents.

Solid compositions of a similar type may also be used as fillers in soft or hard filled gelatin capsules using such excipients as lactose or milk sugar, as well as high molecular weight polyethylene glycols, and the like.

Solid dosage forms such as tablets, dragees, capsules, and granules may be prepared with coatings and shells, such as enteric coatings and others well known in the art. They may also contain opacifying agents, and can also be of such composition that they release the compound of the present invention and/or the additional pharmaceutical agent in a delayed manner. Examples of embedding compositions that can be used are polymeric substances and waxes. The drug may also be in micro-encapsulated form, if appropriate, with one or more of the above-mentioned excipients.

For tablets, the active agent will typically comprise less than 50% (by weight) of the formulation, for example less than about 10% such as 5% or 2.5% by weight. The predominant portion of the formulation comprises fillers, diluents, disintegrants, lubricants and optionally, flavors. The composition of these excipients is well known in the art. Frequently, the fillers/diluents will comprise mixtures of two or more of the following components: microcrystalline cellulose, mannitol, lactose (all types), starch, and di-calcium phosphate. The filler/diluent mixtures typically comprise less than 98% of the formulation and preferably less than 95%, for example 93.5%. Preferred disintegrants include Ac-di-sol.TM., Explotab.TM., starch and sodium lauryl sulphate. When present a disintegrant will usually comprise less than 10% of the formulation or less than 5%, for example about 3%. A preferred lubricant is magnesium stearate. When present a lubricant will usually comprise less than 5% of the formulation or less than 3%, for example about 1%.

Tablets may be manufactured by standard tabletting processes, for example, direct compression or a wet, dry or melt granulation, melt congealing process and extrusion. The tablet cores may be mono or multi-layer(s) and can be coated with appropriate overcoats known in the art.

Liquid dosage forms for oral administration include pharmaceutically acceptable emulsions, solutions, suspensions, syrups, and elixirs. In addition to the compound of the present invention or the combination, the liquid dosage form may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propylene glycol, 1,3-butylene glycol, dimethylformamide, oils (e.g., cottonseed oil, groundnut oil, corn germ oil, olive oil, castor oil, sesame seed oil and the like), Miglyol.RTM. (available from CONDEA Vista Co., Cranford, N.J.), glycerol, tetrahydrofurfuryl alcohol, polyethylene glycols and fatty acid esters of sorbitan, or mixtures of these substances, and the like.

Besides such inert diluents, the composition may also include excipients, such as wetting agents, emulsifying and suspending agents, sweetening, flavoring, and perfuming agents.

Oral liquid forms of the compounds of the invention or combinations include solutions, wherein the active compound is fully dissolved. Examples of solvents include all pharmaceutically precedented solvents suitable for oral administration, particularly those in which the compounds of the invention show good solubility, e.g., polyethylene glycol, polypropylene glycol, edible oils and glyceryl- and glyceride-based systems. Glyceryl- and glyceride-based systems may include, for example, the following branded products (and corresponding generic products): Captex.TM. 355 EP (glyceryl tricaprylate/caprate, from Abitec, Columbus Ohio), Crodamol.TM. GTC/C (medium chain triglyceride, from Croda, Cowick Hall, UK) or Labrafac.TM. CC (medium chain triglyides, from Gattefosse), Captex.TM. 500P (glyceryl triacetate, i.e., triacetin, from Abitec), Capmul.TM. MCM (medium chain mono- and diglycerides, from Abitec), Migyol.TM. 812 (caprylic/capric triglyceride, from Condea, Cranford N.J.), Migyol.TM. 829 (caprylic/capric/succinic triglyceride, from Condea), Migyol.TM. 840 (propylene glycol dicaprylate/dicaprate, from Condea), Labrafil.TM. M1944CS (oleoyl macrogol-6 glycerides, from Gattefosse), Peceol.TM. (glyceryl monooleate, from Gattefosse) and Maisine.TM. 35-1 (glyceryl monooleate, from Gattefosse). Of particular interest are the medium-chain (about C₈ to C₁₀) triglyceride oils. These solvents frequently make up the predominant portion of the composition, i.e., greater than about 50%, usually greater than about 80%, for example about 95% or 99%. Adjuvants and additives may also be included with the solvents principally as taste-mask agents, palatability and flavoring agents, antioxidants, stabilizers, texture and viscosity modifiers and solubilizers.

Suspensions, in addition to the compound of the present invention or the combination, may further comprise carriers such as suspending agents, e.g., ethoxylated isostearyl alcohols, polyoxyethylene sorbitol and sorbitan esters, microcrystalline cellulose, aluminum metahydroxide, bentonite, agar-agar, and tragacanth, or mixtures of these substances, and the like.

Compositions for rectal or vaginal administration preferably comprise suppositories, which can be prepared by mixing a compound of the present invention or a combination with suitable non-irritating excipients or carriers, such as cocoa butter, polyethylene glycol or a suppository wax which are solid at ordinary room temperature, but liquid at body temperature, and therefore, melt in the rectum or vaginal cavity thereby releasing the active component(s).

Dosage forms for topical administration of the compounds of the present invention or combinations include ointments, creams, lotions, powders and sprays. The drugs are admixed with a pharmaceutically acceptable excipient, diluent or carrier, and any preservatives, buffers, or propellants that may be required.

Some of the present compounds may be poorly soluble in water, e.g., less than about 1 µg/mL. Therefore, liquid compositions in solubilizing, non-aqueous solvents such as the medium-chain triglyceride oils discussed above are a preferred dosage form for these compounds.

Solid amorphous dispersions, including dispersions formed by a spray-drying process, are also a preferred dosage form for the poorly soluble compounds of the invention. By "solid amorphous dispersion" is meant a solid material in which at least a portion of the poorly soluble compound is in the amorphous form and dispersed in a water-soluble polymer. By "amorphous" is meant that the poorly soluble compound is not crystalline. By "crystalline" is meant that the compound exhibits long-range order in three dimensions of at least 100 repeat units in each dimension. Thus, the term amorphous is intended to include not only material which has essentially no order, but also material which may have some small degree of order, but the order is in less than three dimensions and/or is only over short distances. Amorphous material may be characterized by techniques known in the art such as powder X-ray diffraction (PXRD) crystallography, solid-state NMR, or thermal techniques such as differential scanning calorimetry (DSC).

Preferably, at least a major portion (i.e., at least about 60 wt %) of the poorly soluble compound in the solid amorphous dispersion is amorphous. The compound can exist within the solid amorphous dispersion in relatively pure amorphous domains or regions, as a solid solution of the compound homogeneously distributed throughout the polymer or any combination of these states or those states that lie intermediate between them. Preferably, the solid amorphous dispersion is substantially homogeneous so that the amorphous compound is dispersed as homogeneously as possible throughout the polymer. As used herein, "substantially homogeneous" means that the fraction of the compound that is present in relatively pure amorphous domains or regions within the solid amorphous dispersion is relatively small, on the order of less than 20 wt %, and preferably less than 10 wt % of the total amount of drug.

Water-soluble polymers suitable for use in the solid amorphous dispersions should be inert, in the sense that they do not chemically react with the poorly soluble compound in an adverse manner, are pharmaceutically acceptable, and have at least some solubility in aqueous solution at physiologically relevant pHs (e.g., 1-8). The polymer can be neutral or ionizable, and should have an aqueous-solubility of at least 0.1 mg/mL over at least a portion of the pH range of 1-8.

Water-soluble polymers suitable for use with the present invention may be cellulosic or non-cellulosic. The polymers may be neutral or ionizable in aqueous solution. Of these, ionizable and cellulosic polymers are preferred, with ionizable cellulosic polymers being more preferred.

Exemplary water-soluble polymers include hydroxypropyl methyl cellulose acetate succinate (HPMCAS), hydroxypropyl methyl cellulose (HPMC), hydroxypropyl methyl cellulose phthalate (HPMCP), carboxy methyl ethyl cellulose (CMEC), cellulose acetate phthalate (CAP), cellulose acetate trimellitate (CAT), polyvinylpyrrolidone (PVP), hydroxypropyl cellulose (HPC), methyl cellulose (MC), block copolymers of ethylene oxide and propylene oxide (PEO/PPO, also known as poloxamers), and mixtures thereof. Especially preferred polymers include HPMCAS, HPMC, HPMCP, CMEC, CAP, CAT, PVP, poloxamers, and mixtures thereof. Most preferred is HPMCAS. See European Patent Application Publication No. 0 901 786 A2.

The solid amorphous dispersions may be prepared according to any process for forming solid amorphous dispersions that results in at least a major portion (at least 60%) of the poorly soluble compound being in the amorphous state. Such processes include mechanical, thermal and solvent processes. Exemplary mechanical processes include milling and extrusion; melt processes including high-temperature fusion, solvent-modified fusion and melt-congeal processes; and solvent processes including non-solvent precipitation, spray coating and spray drying. See, for example, the following U.S. Patents, Nos. 5,456,923 and 5,939,099, which describe forming dispersions by extrusion processes; Nos. 5,340,591 and 4,673,564, which describe forming dispersions by milling processes; and Nos. 5,707,646 and 4,894,235, which describe forming dispersions by melt congeal processes. In a preferred process, the solid amorphous dispersion is formed by spray drying, as disclosed in European Patent Application Publication No. 0 901 786 A2. In this process, the compound and polymer are dissolved in a solvent, such as acetone or methanol, and the solvent is then rapidly removed from the solution by spray drying to form the solid amorphous dispersion. The solid amorphous dispersions may be prepared to contain up to about 99 wt % of the compound, e.g., 1 wt %, 5 wt %, 10 wt %, 25 wt %, 50 wt %, 75 wt %, 95 wt %, or 98 wt % as desired.

The solid dispersion may be used as the dosage form itself or it may serve as a manufacturing-use-product (MUP) in the preparation of other dosage forms such as capsules, tablets, solutions or suspensions. An example of an aqueous suspension is an aqueous suspension of a 1:1 (w/w) compound/HPMCAS-HF spray-dried dispersion containing 2.5 mg/mL of compound in 2% polysorbate-80. Solid dispersions for use in a tablet or capsule will generally be mixed with other excipients or adjuvants typically found in such dosage forms. For example, an exemplary filler for capsules contains a 2:1 (w/w) compound/HPMCAS-MF spray-dried dispersion (60%), lactose (fast flow) (15%), microcrystalline cellulose (e.g., Avicel.sup.(R0-102) (15.8%), sodium starch (7%), sodium lauryl sulfate (2%) and magnesium stearate (1%).

The HPMCAS polymers are available in low, medium and high grades as Aqoat.sup.(R)-LF, Aqoat.sup.(R)-MF and Aqoat.sup.(R)-HF respectively from Shin-Etsu Chemical Co., LTD, Tokyo, Japan. The higher MF and HF grades are generally preferred.

Conveniently, a compound of the present invention (or combination) can be carried in the drinking water so that a therapeutic dosage of the compound is ingested with the daily water supply. The compound can be directly metered into drinking water, preferably in the form of a liquid, water-soluble concentrate (such as an aqueous solution of a water-soluble salt).

These compounds may also be administered to animals other than humans, for example, for the indications detailed above. The precise dosage administered of each active ingredient will vary depending upon any number of factors, including but not limited to, the type of animal and type of disease state being treated, the age of the animal, and the route(s) of administration.

A dosage of the combination pharmaceutical agents to be used in conjunction with the Formula I compounds or their salts is used that is effective for the indication being treated. Such dosages can be determined by standard assays such as those referenced above and provided herein. The combination agents may be administered simultaneously or sequentially in any order.

These dosages are based on an average human subject having a weight of about 60 kg to 70 kg. The physician will readily be able to determine doses for subjects whose weight falls outside this range, such as infants and the elderly.

Dosage regimens may be adjusted to provide the optimum desired response. For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form, as used herein, refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the chemotherapeutic agent and the particular therapeutic or prophylactic effect to be achieved, and (b) the limitations inherent in the art of compounding such an active compound for the treatment of sensitivity in individuals.

Thus, the skilled artisan would appreciate, based upon the disclosure provided herein, that the dose and dosing regimen is adjusted in accordance with methods well-known in the therapeutic arts. That is, the maximum tolerable dose can be readily established, and the effective amount providing a detectable therapeutic benefit to a patient may also be determined, as can the temporal requirements for administering each agent to provide a detectable therapeutic benefit to the patient. Accordingly, while certain dose and administration regimens are exemplified herein, these examples in no way limit the dose and administration regimen that may be provided to a patient in practicing the present invention.

It is to be noted that dosage values may vary with the type and severity of the condition to be alleviated, and may include single or multiple doses. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition. For example, doses may be adjusted based on pharmacokinetic or pharmacodynamic parameters, which may include clinical effects such as toxic effects and/or laboratory values. Thus, the present invention encompasses intra-patient dose-escalation as determined by the skilled artisan. Determining appropriate dosages and regimens for administration of the chemotherapeutic agent is well-known in the relevant art and would be understood to be encompassed by the skilled artisan once provided the teachings disclosed herein.

The present invention further comprises use of a compound of Formula I or its pharmaceutically acceptable salt for use as a medicament (such as a unit dosage tablet or unit dosage capsule). In another embodiment, the present invention comprises the use of a compound of Formula I or its pharmaceutically acceptable salt for the manufacture of a medicament (such as a unit dosage tablet or unit dosage capsule) to treat one or more of the conditions previously identified in the above sections discussing methods of treatment.

A pharmaceutical composition of the invention may be prepared, packaged, or sold in bulk, as a single unit dose, or as a plurality of single unit doses. As used herein, a "unit dose" is a discrete amount of the pharmaceutical composition comprising a predetermined amount of the active ingredient. The amount of the active ingredient is generally equal to the dosage of the active ingredient which would be administered to a subject or a convenient fraction of such a dosage such as, for example, one-half or one-third of such a dosage.

These agents and compounds of the invention can be combined with pharmaceutically acceptable vehicles such as saline, Ringer's solution, dextrose solution, and the like. The particular dosage regimen, i.e., dose, timing and repetition, will depend on the particular individual and that individual's medical history.

Acceptable carriers, excipients, or stabilizers are nontoxic to recipients at the dosages and concentrations employed, and may comprise buffers such as phosphate, citrate, and other organic acids; salts such as sodium chloride; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens, such as methyl or propyl paraben; catechol; resorcinol; cyclohexanol; 3-pentanol; and m-cresol); low molecular weight (less than about 10 residues) polypeptides; proteins, such as serum albumin, gelatin, or Igs; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrin; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{™}, PLURONICS^{™} or polyethylene glycol (PEG).

Liposomes containing these agents and/or compounds of the invention are prepared by methods known in the art, such as described in U.S. Pat. Nos. 4,485,045 and 4,544,545. Liposomes with enhanced circulation time are disclosed in U.S. Patent No. 5,013,556. Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter.

These agents and/or the compounds of the invention may also be entrapped in microcapsules prepared, for example, by coacervation techniques or by interfacial polymerization, for example, hydroxymethylcellulose or gelatin-microcapsules and poly-(methylmethacrylate) microcapsules, respectively, in colloidal drug delivery systems (for example, liposomes, albumin microspheres, microemulsions, nano-particles and nanocapsules) or in macroemulsions. Such techniques are disclosed in Remington, The Science and Practice of Pharmacy, 20th Ed., Mack Publishing (2000).

Sustained-release preparations may be used. Suitable examples of sustained-release preparations include semi-permeable matrices of solid hydrophobic polymers containing the compound of the invention, which matrices are in the form of shaped articles, e.g., films, or microcapsules. Examples of sustained-release matrices include polyesters, hydrogels [for example, poly(2-hydroxyethyl-methacrylate), or 'poly(vinylalcohol)], polylactides (U.S. Pat. No. 3,773,919), copolymers of L-glutamic acid and ethyl-L-glutamate, non-degradable ethylenevinyl acetate, degradable lactic acid-glycolic acid copolymers such as those used in LUPRON DEPOT^{™} (injectable microspheres composed of lactic acid-glycolic acid copolymer and leuprolide acetate), sucrose acetate isobutyrate, and poly-D-(-)-3-hydroxybutyric acid.

The formulations to be used for intravenous administration must be sterile. This is readily accomplished by, for example, filtration through sterile filtration membranes. Compounds of the invention are generally placed into a container having a sterile access port, for example, an intravenous solution bag or vial having a stopper pierceable by a hypodermic injection needle.

Suitable emulsions may be prepared using commercially available fat emulsions, such as Intralipid^{™}, Liposyn^{™}, Infonutrol^{™}, Lipofundin^{™} and Lipiphysan^{™}. The active ingredient may be either dissolved in a pre-mixed emulsion composition or alternatively it may be dissolved in an oil (e.g., soybean oil, safflower oil, cottonseed oil, sesame oil, corn oil or almond oil) and an emulsion formed upon mixing with a phospholipid (e.g., egg phospholipids, soybean phospholipids or soybean lecithin) and water. It will be appreciated that other ingredients may be added, for example glycerol or glucose, to adjust the tonicity of the emulsion. Suitable emulsions will typically contain up to 20% oil, for example, between 5 and 20%. The fat emulsion can comprise fat droplets between 0.1 and 1.0 µm, particularly 0.1 and 0.5 µm, and have a pH in the range of 5.5 to 8.0.

The emulsion compositions can be those prepared by mixing a compound of the invention with Intralipid^{™} or the components thereof (soybean oil, egg phospholipids, glycerol and water).

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as set out above. In some embodiments, the compositions are administered by the oral or nasal respiratory route for local or systemic effect. Compositions in preferably sterile pharmaceutically acceptable solvents may be nebulized by use of gases. Nebulized solutions may be breathed directly from the nebulizing device or the nebulizing device may be attached to a face mask, tent or intermittent positive pressure breathing machine. Solution, suspension or powder compositions may be administered, preferably orally or nasally, from devices which deliver the formulation in an appropriate manner.

The compounds herein may be formulated for oral, buccal, intranasal, parenteral (e.g., intravenous, intramuscular or subcutaneous) or rectal administration or in a form suitable for administration by inhalation. The compounds of the invention may also be formulated for sustained delivery.

Methods of preparing various pharmaceutical compositions with a certain amount of active ingredient are known, or will be apparent in light of this disclosure, to those skilled in this art. For examples of methods of preparing pharmaceutical compositions see Remington, The Science and Practice of Pharmacy, 20th Edition (Lippincott Williams & Wilkins, 2000).

Pharmaceutical compositions according to the invention may contain 0.1% to 95% of the compound(s) of this invention, preferably 1% to 70%. In any event, the composition to be administered will contain a quantity of a compound(s) according to the invention in an amount effective to treat the disease/condition of the subject being treated.

Since the present invention has an aspect that relates to the treatment of the disease/conditions described herein with a combination of active ingredients which may be administered separately, the invention also relates to combining separate pharmaceutical compositions in kit form. The kit comprises two separate pharmaceutical compositions: a compound of Formula I or its pharmaceutically acceptable salt or a prodrug thereof or a salt of such compound or prodrug and a second compound as described above. The kit comprises a means for containing the separate compositions such as a container, a divided bottle or a divided foil packet. Typically, the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process recesses are formed in the plastic foil. The recesses have the size and shape of the tablets or capsules to be packed. Next, the tablets or capsules are placed in the recesses and the sheet of relatively stiff material is sealed against the plastic foil at the face of the foil which is opposite from the direction in which the recesses were formed. As a result, the tablets or capsules are sealed in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide a memory aid on the kit, e.g., in the form of numbers next to the tablets or capsules whereby the numbers correspond with the days of the regimen which the tablets or capsules so specified should be ingested. Another example of such a memory aid is a calendar printed on the card, e.g., as follows "First Week, Monday, Tuesday, etc.... Second Week, Monday, Tuesday..." etc. Other variations of memory aids will be readily apparent. A "daily dose" can be a single tablet or capsule or several pills or capsules to be taken on a given day. Also, a daily dose of a compound of the present invention can consist of one tablet or capsule while a daily dose of the second compound can consist of several tablets or capsules and vice versa. The memory aid should reflect this.

In another specific embodiment of the invention, a dispenser designed to dispense the daily doses one at a time in the order of their intended use is provided. Preferably, the dispenser is equipped with a memory-aid, so as to further facilitate compliance with the regimen. An example of such a memory-aid is a mechanical counter which indicates the number of daily doses that has been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

Also, as the present invention has an aspect that relates to the treatment of the disease/conditions described herein with a combination of active ingredients which may be administered jointly, the invention also relates to combining separate pharmaceutical compositions in a single dosage form, such as (but not limited to) a single tablet or capsule, a bilayer or multilayer tablet or capsule, or through the use of segregated components or compartments within a tablet or capsule.

The active ingredient may be delivered as a solution in an aqueous or non-aqueous vehicle, with or without additional solvents, co-solvents, excipients, or complexation agents selected from pharmaceutically acceptable diluents, excipients, vehicles, or carriers.

The active ingredient may be formulated as a solid dispersion or as a self-emulsified drug delivery system (SEDDS) with pharmaceutically acceptable excipients.

The active ingredient may be formulated as an immediate release or controlled (e.g., suspended, delayed, or extended) release tablet or capsule. Alternatively, the active ingredient may be delivered as the active ingredient alone within a capsule shell, without additional excipients.

### EXAMPLES

The following illustrate the synthesis of various compounds of the present invention. Additional compounds within the scope of this invention may be prepared using the methods illustrated in these Examples, either alone or in combination with techniques generally known in the art. All starting materials in these Preparations and Examples are either commercially available or can be prepared by methods known in the art or as described herein.

Reactions were performed in air or, when oxygen- or moisture-sensitive reagents or intermediates were employed, under an inert atmosphere (nitrogen or argon). When appropriate, reaction apparatuses were dried under dynamic vacuum using a heat gun, and anhydrous solvents (Sure-Seal^{™} products from Aldrich Chemical Company, Milwaukee, Wisconsin or DriSolv^{™} products from EMD Chemicals, Gibbstown, NJ) were employed. In some cases, commercial solvents were passed through columns packed with 4Å molecular sieves, until the following QC standards for water were attained: a) <100 ppm for dichloromethane, toluene, *N,N*-dimethylformamide, and tetrahydrofuran; b) <180 ppm for methanol, ethanol, 1,4-dioxane, and diisopropylamine. For very sensitive reactions, solvents were further treated with metallic sodium, calcium hydride, or molecular sieves, and distilled just prior to use. Other commercial solvents and reagents were used without further purification. For syntheses referencing procedures in other Examples or Methods, reaction conditions (reaction time and temperature) may vary. Products were generally dried under vacuum before being carried on to further reactions or submitted for biological testing.

When indicated, reactions were heated by microwave irradiation using Biotage Initiator or Personal Chemistry Emrys Optimizer microwave instruments. Reaction progress was monitored using thin-layer chromatography (TLC), liquid chromatography-mass spectrometry (LCMS), high-performance liquid chromatography (HPLC), and/or gas chromatography-mass spectrometry (GCMS) analyses. TLC was performed on pre-coated silica gel plates with a fluorescence indicator (254 nm excitation wavelength) and visualized under UV light and/or with I₂, KMnO₄, CoCl₂, phosphomolybdic acid, and/or ceric ammonium molybdate stains. LCMS data were acquired on an Agilent 1100 Series instrument with a Leap Technologies autosampler, Gemini C18 columns, acetonitrile / water gradients, and either trifluoroacetic acid, formic acid, or ammonium hydroxide modifiers. The column eluent was analyzed using a Waters ZQ mass spectrometer scanning in both positive and negative ion modes from 100 to 1200 Da. Other similar instruments were also used. HPLC data were acquired on an Agilent 1100 Series instrument using Gemini or XBridge C18 columns, acetonitrile / water gradients, and either trifluoroacetic acid or ammonium hydroxide modifiers. GCMS data were acquired using a Hewlett Packard 6890 oven with an HP 6890 injector, HP-1 column (12 m x 0.2 mm x 0.33 µm), and helium carrier gas. The sample was analyzed on an HP 5973 mass selective detector scanning from 50 to 550 Da using electron ionization. Purifications were performed by medium performance liquid chromatography (MPLC) using Isco CombiFlash Companion, AnaLogix IntelliFlash 280, Biotage SP1, or Biotage Isolera One instruments and pre-packed Isco RediSep or Biotage Snap silica cartridges. Chiral purifications were performed by chiral supercritical fluid chromatography (SFC) using Berger or Thar instruments; ChiralPAK-AD, -AS, -IC, Chiralcel-OD, or -OJ columns; and CO₂ mixtures with methanol, ethanol, 2-propanol, or acetonitrile, alone or modified using trifluoroacetic acid or propan-2-amine. UV detection was used to trigger fraction collection. For syntheses referencing procedures in other Examples or Methods, purifications may vary: in general, solvents and the solvent ratios used for eluents / gradients were chosen to provide appropriate R_{f}s or retention times.

Mass spectrometry data are reported from LCMS analyses. Mass spectrometry (MS) was performed via atmospheric pressure chemical ionization (APCI), electrospray Ionization (ESI), electron impact ionization (EI) or electron scatter (ES) ionization sources. Proton nuclear magnetic spectroscopy (¹H NMR) chemical shifts are given in parts per million downfield from tetramethylsilane and were recorded on 300, 400, 500, or 600 MHz Varian, Bruker, or Jeol spectrometers. Chemical shifts are expressed in parts per million (ppm, δ) referenced to the deuterated solvent residual peaks (chloroform, 7.26 ppm; CD₂HOD, 3.31 ppm; acetonitrile-*d*₂, 1.94 ppm; dimethyl sulfoxide-*d*₅, 2.50 ppm; DHO, 4.79 ppm). The peak shapes are described as follows: s, singlet; d, doublet; t, triplet; q, quartet; quin, quintet; m, multiplet; br s, broad singlet; app, apparent. Analytical SFC data were acquired on a Berger analytical instrument as described above. Optical rotation data were acquired on a PerkinElmer model 343 polarimeter using a 1 dm cell. Silica gel chromatography was performed primarily using medium-pressure Biotage or ISCO systems using columns pre-packaged by various commercial vendors including Biotage and ISCO. Microanalyses were performed by Quantitative Technologies Inc. and were within 0.4% of the calculated values.

Unless otherwise noted, chemical reactions were performed at room temperature (about 23 degrees Celsius).

Unless noted otherwise, all reactants were obtained commercially without further purifications or were prepared using methods known in the literature.

The terms "concentrated", "evaporated", and "concentrated *in vacuo"* refer to the removal of solvent at reduced pressure on a rotary evaporator with a bath temperature less than 60 °C. The abbreviation "min" and "h" stand for "minutes" and "hours" respectively. The term "TLC" refers to thin-layer chromatography, "room temperature or ambient temperature" means a temperature between 18 to 25 °C, "GCMS" refers to gas chromatography-mass spectrometry, "LCMS" refers to liquid chromatography-mass spectrometry, "UPLC" refers to ultra-performance liquid chromatography and "HPLC" refers to high-performance liquid chromatography, "SFC" refers to supercritical fluid chromatography.

Hydrogenation may be performed in a Parr Shaker under pressurized hydrogen gas, or in a Thales-nano H-Cube flow hydrogenation apparatus at full hydrogen and a flow rate between 1-2 mL/min at the specified temperature.

HPLC, UPLC, LCMS, GCMS, and SFC retention times were measured using the methods noted in the procedures.

In some examples, chiral separations were carried out to separate enantiomers, diastereomers, or atropisomers (or atropenantiomers) of certain compounds of the invention (in some examples, the separated enantiomers are designated as ENANT-1 and ENANT-2, according to their order of elution; similarly, separated diastereomers are designated as DIAST-1 and DIAST-2, according to their order of elution, and separated atropisomers (or atropenantiomers) are designated as ATROP-1 and ATROP-2, according to their order of elution. In some examples, the optical rotation of an enantiomer or atropisomer (or atropenantiomer) was measured using a polarimeter. According to its observed rotation data (or its specific rotation data), an enantiomer or atropisomer (or atropenantiomer) with a clockwise rotation was designated as the (+)-enantiomer or (+)-atropisomer [or the (+) atropenantiomer] and an enantiomer or atropisomer (or atropenantiomer) with a counter-clockwise rotation was designated as the (-)-enantiomer or (-)-atropisomer [or the (-) atropenantiomer]. Racemic compounds are indicated either by the absence of drawn or described stereochemistry, or by the presence of (+/-) adjacent to the structure; in this latter case, the indicated stereochemistry represents just one of the two enantiomers that make up the racemic mixture.

The compounds and intermediates described below were named using the naming convention provided with ACD/ChemSketch 2019.1.1, File Version C05H41, Build 110712 (Advanced Chemistry Development, Inc., Toronto, Ontario, Canada). The naming convention provided with ACD/ChemSketch 2019.1.1 is well known by those skilled in the art and it is believed that the naming convention provided with ACD/ChemSketch 2019.1.1 generally comports with the IUPAC (International Union for Pure and Applied Chemistry) recommendations on Nomenclature of Organic Chemistry and the CAS Index rules.

### PREPARATIONS

Preparations **P1** to ***P25*** describe preparations of some starting materials or intermediates used for preparation of certain compounds of the invention.

### Preparation P1

### (6-{[(3S)-1-(tert-Butoxycarbonyl)pyrrolidin-3-yl]amino}-2-methylpyridin-3-yl)boronic acid (P1)

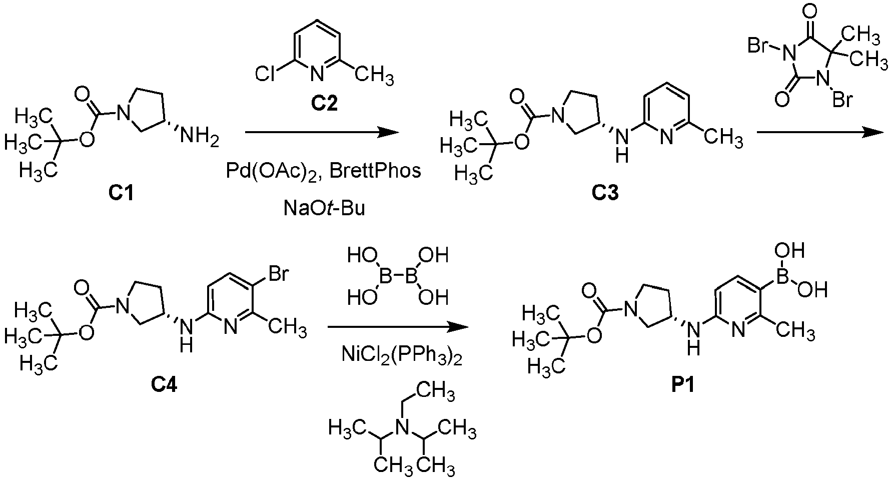

### Step 1. Synthesis of tert-butyl (3S)-3-[(6-methylpyridin-2-yl)amino]pyrrolidine-1-carboxylate (C3).

BrettPhos {dicyclohexyl[3,6-dimethoxy-2',4',6'-tri(propan-2-yl)[1,1'-biphenyl]-2-yl]phosphane; 7.83 g, 14.5 mmol}, palladium(II) acetate (1.57 g, 6.90 mmol), and sodium *tert-*butoxide (216 g, 2.25 mol) were added to tetrahydrofuran (2.0 L) at 20 °C. After the reaction mixture had been stirred for 20 minutes, 2-chloro-6-methylpyridine (**C2**) (191 g, 1.50 mol) and *tert*-butyl (3*S*)-3-aminopyrrolidine-1-carboxylate (**C1**) (293 g, 1.58 mol) were added and the reaction mixture was stirred at 70 °C for 2 hours. The mixture was concentrated under reduced pressure and ice water (2.0 L) was added to the residue. The resulting mixture was extracted with dichloromethane (2 x 3.0 L); the combined organic layers were washed with water (3 x 3.0 L) and with saturated aqueous sodium chloride solution (3.0 L), dried over sodium sulfate, filtered, and concentrated *in vacuo.* Trituration from ethyl acetate / petroleum ether (0.8 L / 1.6 L) provided **C3** as a grey solid. Yield: 396 g, 1.43 mol, 95%. LCMS *m*/*z* 278.3 [M+H]⁺. ¹H NMR (400 MHz, chloroform-*d*) δ 7.34 (dd, *J* = 7.8, 7.8 Hz, 1H), 6.47 (d, *J* = 7.3 Hz, 1H), 6.20 (d, *J =* 8.2 Hz, 1H), 4.77 - 4.55 (m, 1H), 4.33 - 4.12 (m, 1H), 3.78 - 3.62 (m, 1H), 3.57 - 3.35 (m, 2H), 3.33 - 3.11 (m, 1H), 2.36 (s, 3H), 2.26 - 2.12 (m, 1H), 1.96 - 1.79 (m, 1H), 1.45 (s, 9H).

### Step 2. Synthesis of tert-butyl (3S)-3-[(5-bromo-6-methylpyridin-2-yl)amino]pyrrolidine-1-carboxylate (C4).

To a mixture of **C3** (650 g, 2.34 mol) in dichloromethane (19.5 L) was slowly added 1,3-dibromo-5,5-dimethylimidazolidine-2,4-dione (341 g, 1.19 mol) in five portions over 2 hours at 5 °C to 10 °C. The reaction mixture was stirred at 10 °C for 1 hour, whereupon it was diluted with dichloromethane (8.0 L); the resulting mixture was washed with aqueous sodium sulfite solution (5%, 10 L) and with saturated aqueous sodium chloride solution (10 L), dried over sodium sulfate, filtered, and concentrated *in vacuo.* Trituration with tetrahydrofuran / *tert*-butyl methyl ether (150 mL / 1 L) provided **C4** as an off-white solid. Analysis of the ¹H NMR indicated that this material comprised a mixture of rotamers. Yield: 760 g, 2.13 mol, 91%. LCMS *m*/*z* 356.1 (bromine isotope pattern observed) [M+H]⁺. ¹H NMR (400 MHz, DMSO-*d*₆) δ 7.48 (d, *J* = 8.7 Hz, 1H), 6.93 - 6.84 (m, 1H), 6.29 (d, *J* = 8.7 Hz, 1H), 4.36 - 4.17 (m, 1H), 3.60 - 3.47 (m, 1H), 3.44 - 3.22 (m, 2H, assumed; partially obscured by water peak), 3.13 - 3.00 (m, 1H), 2.36 (s, 3H), 2.16 - 2.01 (m, 1H), 1.87 - 1.70 (m, 1H), [1.39 (s) and 1.38 (s), total 9H].

### Step 3. Synthesis of (6-{[(3S)-1-(tert-butoxycarbonyl)pyrrolidin-3-yl]amino}-2-methylpyridin-3-yl)boronic acid (P1).

To a solution of **C4** (930 g, 2.61 mol) in tetrahydrofuran (6.5 L) was added bis(triphenylphosphine)nickel(II) dichloride (51.2 g, 78.3 mmol). A solution of tetrahydroxydiboron (702 g, 7.83 mol) in methanol (6.5 L) was then added dropwise at 25 °C via cannula over a period of 1.5 hours. *N*,*N*-Diisopropylethylamine (1.01 kg, 7.83 mol) was added to the resulting mixture drop-wise over 1 hour at 20 °C to 25 °C, and the reaction mixture was stirred at 20 °C for 2 hours, whereupon it was filtered through a pad of diatomaceous earth, and the filtrate was concentrated *in vacuo.* The residue was dissolved in a mixture of aqueous sodium hydroxide solution (2 M; 12 L) and tert-butyl methyl ether (6 L) at 0 °C and the layers were separated. The organic layer was further extracted with aqueous sodium hydroxide solution (1 M; 4 L), and the combined aqueous layers were stirred while the pH was adjusted to 9 - 10 via addition of 6 M hydrochloric acid. After the mixture had been stirred for an additional 20 minutes, the solid was collected by filtration. The solid was combined with material (75 g) obtained from a similar, smaller-scale experiment and dissolved in ethyl acetate (8 L). The organic layer was washed with water (3 L) and with saturated aqueous sodium chloride solution (3 L), dried over sodium sulfate, filtered, and concentrated *in vacuo.* The crude product was triturated with *tert*-butyl methyl ether / *n*-hexane (1 L / 2 L), then dissolved in tetrahydrofuran (3 L), and concentrated under reduced pressure to afford **P1** as an off-white solid. Combined yield: 635 g, 1.98 mol, 67%. LCMS *m*/*z* 322.1 [M+H]⁺. ¹H NMR (400 MHz, methanol-*d*₄) δ 7.71 (br s, 1H), 6.52 (br s, 1H), 4.43 - 4.29 (m, 1H), 3.73 - 3.62 (m, 1H), 3.57 - 3.37 (m, 2H), 3.28 - 3.22 (m, 1H), 2.47 (s, 3H), 2.31 - 2.16 (m, 1H), 2.00 - 1.86 (m, 1H), 1.46 (br s, 9H).

### Preparation P2

### 6-Methyl-5-(1-methyl-1H-imidazol-4-yl)-N-[(3S)-pyrrolidin-3-yl]pyridin-2-amine, dihydrochloride salt (P2)

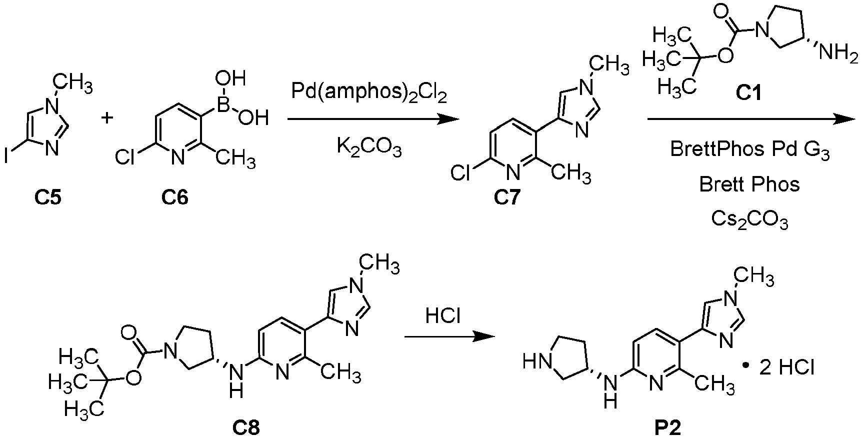

### Step 1. Synthesis of 6-chloro-2-methyl-3-(1-methyl-1H-imidazol-4-yl)pyridine (C7).

To a mixture of 4-iodo-1-methyl-1*H*-imidazole (**C5**; 20.8 g, 100 mmol) in 1,4-dioxane (300 mL) and water (50 mL) were added (6-chloro-2-methylpyridin-3-yl)boronic acid (C6) (17.1 g, 100 mmol), potassium carbonate (27.6 g, 200 mmol), and Pd(amphos)₂Cl₂ {bis[di-tert-butyl(4-dimethylaminophenyl)phosphine]dichloropalladium(II); 3.54 g, 5.00 mmol}. The reaction mixture was stirred at 100 °C for 18 hours, whereupon it was filtered, and the filtrate was concentrated *in vacuo.* Purification via silica gel chromatography (Gradient: 0% to 3% methanol in dichloromethane) provided **C7** as a yellow solid. Yield: 14.7 g, 70.8 mmol, 71%. LCMS *m*/*z* 208.1 (chlorine isotope pattern observed) [M+H]⁺. ¹H NMR (400 MHz, chloroform-d) δ 8.13 (d, *J* = 8.2 Hz, 1H), 7.55 - 7.52 (m, 1H), 7.21 (br d, *J* = 8.2 Hz, 1H), 7.09 (br d, *J* = 1 Hz, 1H), 3.77 (s, 3H), 2.68 (s, 3H).

### Step 2. Synthesis of tert-butyl (3S)-3-{[6-methyl-5-(1-methyl-1H-imidazol-4-yl)pyridin-2-yl]amino}pyrrolidine-1-carboxylate (C8).

A mixture of **C1** (10.1 g, 54.2 mmol), **C7** (13.7 g, 66.0 mmol), BrettPhos Pd G₃ {[(2-dicyclohexylphosphino-3,6-dimethoxy-2',4',6'-triisopropyl-1,1'-biphenyl)-2-(2'-amino-1,1'-biphenyl)]palladium(II) methanesulfonate methanesulfonate; 2.99 g, 3.30 mmol}, BrettPhos (3.54 g, 6.59 mmol), and cesium carbonate (43.0 g, 132 mmol) in 2-methylbutan-2-ol (150 mL) was stirred at 105 °C for 18 hours. The reaction mixture was filtered, and the filtrate was concentrated *in vacuo;* purification via silica gel chromatography (Gradient: 0% to 5% methanol in dichloromethane) provided **C8** as a yellow solid. Yield: 12.7 g, 35.5 mmol, 65%. LCMS *m*/*z* 358.3 [M+H]⁺. ¹H NMR (400 MHz, chloroform-d) δ 7.91 (d, *J* = 8.5 Hz, 1H), 7.48 - 7.45 (m, 1H), 6.92 (d, *J* = 1.1 Hz, 1H), 6.33 (d, *J* = 8.5 Hz, 1H), 4.76 - 4.52 (m, 1H), 4.37 - 4.18 (m, 1H), 3.82 - 3.64 (m, 1H), 3.73 (s, 3H), 3.59 - 3.36 (m, 2H), 3.35 - 3.15 (m, 1H), 2.53 (s, 3H), 2.28 - 2.15 (m, 1H), 1.99 - 1.81 (m, 1H), 1.46 (s, 9H).

### Step 3. Synthesis of 6-methyl-5-(1-methyl-1H-imidazol-4-yl)-N-[(3S)-pyrrolidin-3-yl]pyridin-2-amine, dihydrochloride salt (P2).

To a 25 °C solution of **C8** (12.7 g, 35.5 mmol) in a mixture of dichloromethane (120 mL) and methanol (30 mL) was added a solution of hydrogen chloride in 1,4-dioxane (4 M; 44.3 mL, 177 mmol), and the reaction mixture was stirred at room temperature for 2 hours. The mixture was filtered and the filter cake was washed with ethyl acetate (20 mL) to afford **P2** as a white solid. Yield: 11.5 g, 34.8 mmol, 98%. LCMS *m*/*z* 258.2 [M+H]⁺. ¹H NMR (400 MHz, methanol-*d*₄) δ 9.08 (br s, 1H), 8.01 (d, *J* = 9.2 Hz, 1H), 7.83 (d, *J* = 1.3 Hz, 1H), 7.18 (d, *J* = 9.3 Hz, 1H), 4.86 - 4.77 (m, 1H, assumed; partially obscured by water peak), 4.02 (s, 3H), 3.73 (dd, *J* = 12.5, 6.6 Hz, 1H), 3.65 - 3.56 (m, 1H), 3.54 - 3.42 (m, 2H), 2.67 (s, 3H), 2.61 - 2.49 (m, 1H), 2.29 - 2.17 (m, 1H).

### Preparations P3 and P3-OH

### 2-(4-Fluorophenyl)-1-[(3S)-3-{[6-methyl-5-(4,4,5,5-tetramethyl-1,3,2-dioxaborolan-2-yl)pyridin-2-yl]amino}pyrrolidin-1-yl]ethan-1-one (P3) and [6-({(3S)-1-[(4-Fluorophenyl)acetyl]pyrrolidin-3-yl}amino)-2-methylpyridin-3-yl]boronic acid (P3-OH)

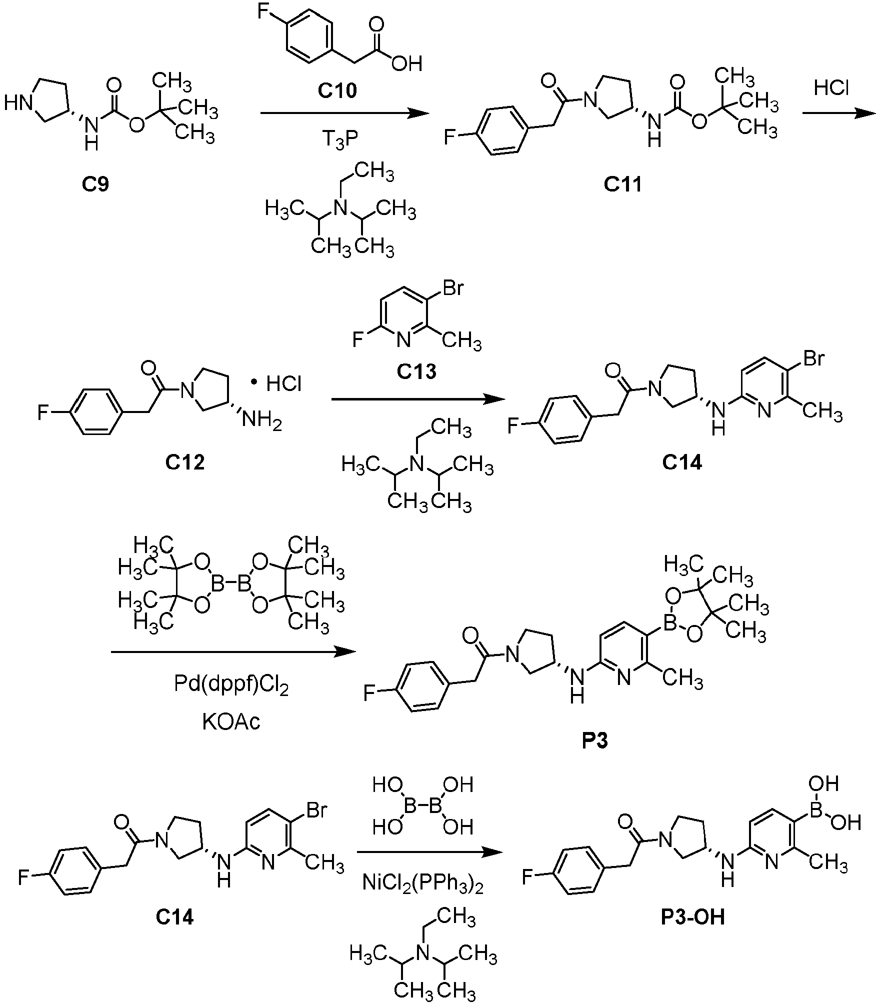

### Step 1. Synthesis of tert-butyl {(3S)-1-[(4-fluorophenyl)acetyl]pyrrolidin-3-yl}carbamate (C11).

To a 25 °C solution of (4-fluorophenyl)acetic acid (**C10**) (20.7 g, 134 mmol) and *tert-*butyl (3*S*)-pyrrolidin-3-ylcarbamate (**C9**) (25 g, 130 mmol) in dichloromethane (300 mL) were added 2,4,6-tripropyl-1,3,5,2,4,6-trioxatriphosphinane 2,4,6-trioxide (T₃P, 50% solution by weight in ethyl acetate; 94.0 g, 148 mmol) and *N*,*N*-diisopropylethylamine (34.7 g, 268 mmol). After the reaction mixture had been stirred for 24 hours, it was concentrated *in vacuo.* The residue was triturated with ethyl acetate to afford **C11** as a white solid. Yield: 32.5 g, 101 mmol, 78%. LCMS *m*/*z* 323.2 [M+H]⁺. ¹H NMR (400 MHz, chloroform-*d*) δ 7.23 (dd, *J* = 8.5, 5.4 Hz, 2H), 7.00 (dd, *J* = 8.6, 8.6 Hz, 2H), 4.57 (br s, 1H), 4.30 - 4.11 (m, 1H), 3.71 (dd, *J* = 11.5, 6.1 Hz, 1H), 3.63 - 3.46 (m, 2H), 3.61 (s, 2H), 3.42 - 3.27 (m, 1H), 2.28 - 2.06 (m, 1H), 2.02 - 1.68 (m, 1H, assumed; partially obscured by water peak), 1.44 (s, 9H).

### Step 2. Synthesis of 1-[(3S)-3-aminopyrrolidin-1-yl]-2-(4-fluorophenyl)ethan-1-one, hydrochloride salt (C12).

To a 0 °C mixture of **C11** (32.5 g, 101 mmol) in dichloromethane (100 mL) was added a solution of hydrogen chloride in 1,4-dioxane (4.0 M; 101 mL, 404 mmol) in a drop-wise manner. After the reaction mixture had been stirred at room temperature for 2 hours, it was concentrated *in vacuo* and triturated with ethyl acetate, providing **C12** as a white solid. Yield: 25.2 g, 97.4 mmol, 96%. LCMS *m*/*z* 223.2 [M+H]⁺. ¹H NMR (400 MHz, DMSO-*d*₆) δ 8.62 - 8.42 (m, 3H), 7.30 - 7.21 (m, 2H), 7.16 - 7.07 (m, 2H), 3.88 - 3.69 (m, 2H), 3.67 - 3.32 (m, 3H), 3.62 (s, 2H), 2.30 - 1.90 (m, 2H).

### Step 3. Synthesis of 1-{(3S)-3-[(5-bromo-6-methylpyridin-2-yl)amino]pyrrolidin-1-yl}-2-(4-fluorophenyl)ethan-1-one (C14).

To a solution of **C12** (12.5 g, 48.3 mmol) and 3-bromo-6-fluoro-2-methylpyridine (**C13**) (9.18 g, 48.3 mmol) in dimethyl sulfoxide (150 mL) was added *N*,*N*-diisopropylethylamine (18.7 g, 145 mmol). After the reaction mixture had been stirred at 140 °C for 12 hours, it was poured into water and extracted with ethyl acetate (2 x 200 mL). The combined organic layers were washed with saturated aqueous sodium chloride solution, dried over sodium sulfate, filtered, and concentrated *in vacuo.* Purification via silica gel chromatography (Gradient: 0% to 100% ethyl acetate in petroleum ether) provided **C14** as a yellow solid. Yield: 7.10 g, 18.1 mmol, 37%. Analysis of the ¹H NMR indicated that this material comprised a mixture of rotamers. LCMS *m*/*z* 394.1 (bromine isotope pattern observed) [M+H]⁺. ¹H NMR (400 MHz, chloroform-*d*) δ [7.50 (d, *J* = 8.6 Hz) and 7.48 (d, *J* = 8.6 Hz), total 1H], 7.26 - 7.17 (m, 2H, assumed; partially obscured by solvent peak), 7.05 - 6.93 (m, 2H), [6.14 (d, *J* = 8.7 Hz) and 6.09 (d, *J* = 8.6 Hz), total 1H], [4.82 - 4.65 (m) and 4.54 - 4.42 (m), total 1H], [4.41 - 4.31 (m) and 4.30 - 4.20 (m), total 1H], 3.87 - 3.79 (m, 1H), 3.74 - 3.52 (m, 4H), [3.45 (dd, *J* = 12.5, 4.3 Hz) and 3.34 (dd, *J* = 10.5, 4.8 Hz), total 1H], 2.47 (br s, 3H), 2.35 - 2.17 (m, 1H), [2.07 - 1.97 (m) and 1.91 - 1.80 (m), total 1H].

### Step 4. Synthesis of 2-(4-fluorophenyl)-1-[(3S)-3-{[6-methyl-5-(4,4,5,5-tetramethyl-1,3,2-dioxaborolan-2-yl)pyridin-2-yl]amino}pyrrolidin-1-yl]ethan-1-one (P3).

A mixture of **C14** (6.7 g, 17.1 mmol), 4,4,4',4',5,5,5',5'-octamethyl-2,2'-bi-1,3,2-dioxaborolane (8.67 g, 34.2 mmol), potassium acetate (4.18 g, 42.7 mmol), and Pd(dppf)Cl₂ {[1,1'-bis(diphenylphosphino)ferrocene]dichloropalladium(II); 1.25 g, 1.71 mmol} in 1,4-dioxane (300 mL) was stirred at 100 °C for 16 hours. After the reaction mixture had been concentrated *in vacuo,* purification via silica gel chromatography (Eluent: 30% ethyl acetate in petroleum ether) provided **P3** as a grey solid. Analysis of the ¹H NMR indicated that this material comprised a mixture of rotamers. Yield: 3.20 g, 7.28 mmol, 43%. LCMS *m*/*z* 440.2 [M+H]⁺. ¹H NMR (400 MHz, chloroform-*d*) δ [7.81 (d, *J* = 8.4 Hz) and 7.79 (d, *J* = 8.4 Hz), total 1H], 7.26 - 7.16 (m, 2H, assumed; partially obscured by solvent peak), 7.05 - 6.92 (m, 2H), [6.19 (d, *J =* 8.3 Hz) and 6.15 (d, *J* = 8.3 Hz), total 1H], [4.92 - 4.75 (m) and 4.63 (br d, *J* = 6.5 Hz), total 1H], [4.45 - 4.35 (m) and 4.33 - 4.23 (m), total 1H], [3.86 (d, *J =* 5.9 Hz) and 3.83 (dd, *J* = 6.0, 3.2 Hz), total 1H], 3.74 - 3.52 (m, 2H), [3.62 (s) and 3.57 (s), total 2H], [3.46 (dd, *J* = 12.5, 4.2 Hz) and 3.33 (dd, *J* = 10.4, 4.9 Hz), total 1H], 2.57 (s, 3H), 2.34 - 2.18 (m, 1H), [2.08 - 1.97 (m) and 1.92 - 1.80 (m), total 1H], [1.32 (s) and 1.31 (s), total 12H].

### Step 5. Synthesis of [6-({(3S)-1-[(4-fluorophenyl)acetyl]pyrrolidin-3-yl}amino)-2-methylpyridin-3-yl]boronic acid (P3-OH).

To a 25 °C mixture of **C14** (5.5 g, 14.0 mmol) in tetrahydrofuran (50 mL) was added bis(triphenylphosphine)nickel(II) dichloride (275 mg, 0.42 mmol) and the mixture was degassed by evacuation followed by back-fill with nitrogen; this degassing procedure was carried out 5 times. A separate reactor was charged with methanol (50 mL) and tetrahydroxydiboron (3.77 g, 42.1 mmol), and the mixture was stirred until fully homogeneous. This solution was degassed in the same manner. The tetrahydroxydiboron solution was then added to the first reactor over 5 minutes via syringe, whereupon *N,N*-diisopropylethylamine (7.0 mL, 42 mmol) was added in a drop-wise manner. After the reaction mixture had been stirred at 25 °C for 2 hours, the reaction was filtered through a pad of diatomaceous earth. The methanol and tetrahydrofuran were removed *in vacuo* and the residue was dissolved in a 0 °C mixture of aqueous sodium hydroxide solution (1 M; 50 mL) and *tert*-butyl methyl ether (50 mL), using ethyl acetate to transfer any residues. The organic layer was extracted with aqueous sodium hydroxide solution (1 M; 2 x 30 mL), and the combined aqueous layers were stirred while 6 M aqueous hydrochloric acid was added drop-wise until solids precipitated (pH approximately 9 to 10). The resulting mixture was stirred at room temperature for 20 minutes, whereupon ethyl acetate (20 mL) was added to provide a solution. This was transferred to a separatory funnel and vigorously shaken; the aqueous layer was extracted with ethyl acetate (2 x 60 mL) and the combined organic layers were washed with saturated aqueous sodium chloride solution, dried over sodium sulfate, filtered through a pad of diatomaceous earth, and concentrated *in vacuo* to provide **P3-OH** as a white powder. Analysis of the ¹H NMR indicated that this material comprised a mixture of rotamers. Yield: 3.46 g, 9.69 mmol, 69%. LCMS *m*/*z* 358.2 [M+H]⁺. ¹H NMR (400 MHz, methanol-*d*₄) δ 7.71 - 7.46 (m, 1H), [7.28 (dd, *J* = 8.3, 5.5 Hz) and 7.23 (dd, *J* = 8.4, 5.5 Hz), total 2H], [7.04 (br dd, *J* = 8.8, 8.7 Hz) and 6.98 (br dd, *J* = 8.8, 8.7 Hz), total 2H], 6.59 - 6.24 (m, 1H), 4.49 - 4.35 (m, 1H), [3.89 (dd, *J* = 10.8, 5.7 Hz) and 3.79 (dd, *J* = 12.4, 6.2 Hz), total 1H], [3.76 - 3.60 (m) and 3.60 - 3.37 (m), total 5H], 2.42 (br s, 3H), 2.39 - 2.18 (m, 1H), 2.09 - 1.88 (m, 1H).

### Preparation P4

### 5-[2-(Difluoromethyl)-1-methyl-1H-imidazol-4-yl]-6-methyl-N-[(3S)-pyrrolidin-3-yl]pyridin-2-amine, dihydrochloride salt (P4)

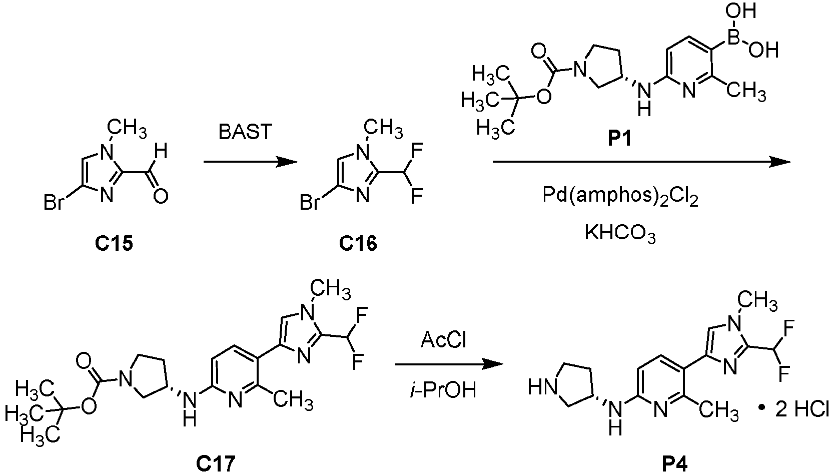

### Step 1: Synthesis of 4-bromo-2-(difluoromethyl)-1-methyl-1H-imidazole (C16)

To a 0 °C solution of 4-bromo-1-methyl-1*H*-imidazole-2-carbaldehyde (**C15**) (1.86 g, 9.84 mmol) in dichloromethane was added [bis(2-methoxyethyl)amino]sulfur trifluoride (BAST; 7.3 mL, 39 mol). After the reaction mixture had been stirred at 25 °C for 7 hours, the mixture was treated with saturated aqueous sodium bicarbonate solution until it became basic (pH of 7 to 8). The aqueous layer was extracted with dichloromethane (3 x 30 mL) and the combined organic layers were washed with saturated aqueous sodium chloride solution, dried over sodium sulfate, filtered, and concentrated *in vacuo.* Purification via silica gel chromatography (Gradient: 0% to 15% ethyl acetate in petroleum ether) provided **C16** as a light-yellow oil that gradually solidified. Yield: 1.79 g, 8.48 mmol, 86%. LCMS *m*/*z* 211.1 (bromine isotope pattern observed) [M+H]⁺. ¹H NMR (400 MHz, methanol-*d*₄) δ 7.29 (s, 1H), 6.81 (t, *J*_{HF} = 52.3 Hz, 1H), 3.82 (s, 3H).

### Step 2: Synthesis of tert-butyl (3S)-3-({5-[2-(difluoromethyl)-1-methyl-1H-imidazol-4-yl]-6-methylpyridin-2-yl}amino)pyrrolidine-1-carboxylate (C17).

A mixture of **C16** (100 mg, 0.47 mmol), **P1** (167 mg, 0.52 mmol), Pd(amphos)₂Cl₂ (17 mg, 24 µmol), and potassium bicarbonate (142 mg, 1.42 mmol) in a mixture of 1,4-dioxane (3.0 mL) and water (0.5 mL) was heated at 80 °C for 17 hours. The reaction mixture was concentrated *in vacuo;* purification of the residue via silica gel chromatography (Gradient: 0% to 5% methanol in dichloromethane) provided **C17** as a white solid. LCMS *m*/*z* 408.2 [M+H]⁺. Analysis of the ¹H NMR indicated that this material comprised a mixture of rotamers. Yield: 133 mg, 0.326 mmol, 69%. ¹H NMR (400 MHz, methanol-*d*₄) δ 7.62 (d, *J* = 8.6 Hz, 1H), 7.28 (s, 1H), 6.87 (t, *J*_{HF} = 52.6 Hz, 1H), 6.44 (d, *J* = 8.6 Hz, 1H), 4.46 - 4.38 (m, 1H), 3.88 (s, 3H), [3.70 (d, *J* = 6.1 Hz), 3.68 (d, *J* = 6.1 Hz), 3.59 - 3.37 (m), 3.24 (br d, *J* = 3.9 Hz), and 3.21 (br d, *J = 3.4* Hz), total 4H], 2.46 (s, 3H), 2.31 - 2.16 (m, 1H), 2.01 - 1.85 (m, 1H), [1.47 (s) and 1.46 (s), total 9H].

### Step 3: Synthesis of 5-[2-(difluoromethyl)-1-methyl-1H-imidazol-4-yl]-6-methyl-N-[(3S)-pyrrolidin-3-yl]pyridin-2-amine, dihydrochloride salt (P4).

Acetyl chloride (3.90 mL, 55.2 mmol) was slowly added to propan-2-ol (15 mL, 200 mmol) to control the anticipated exotherm. The resulting solution was added in one portion to a solution of **C17** (2.25 g, 5.52 mmol) in propan-2-yl acetate (15 mL). After the mixture had been stirred at 25 °C for 4 hours, the solvent was removed *in vacuo* to afford **P4** (2.4 g, assumed quantitative) as a white solid. LCMS *m*/*z* 308.2 [M+H]⁺. ¹H NMR (400 MHz, methanol-*d*₄) δ 8.16 (d, *J* = 9.3 Hz, 1H), 7.85 (s, 1H), 7.27 (t, *J* = 52.1 Hz, 1H), 7.21 (d, *J* = 9.3 Hz, 1H), 4.85 - 4.77 (m, 1H), 4.02 (s, 3H), [3.76 (d, *J* = 6.6 Hz), 3.73 (d, *J* = 6.6 Hz), 3.67 - 3.56 (m), 3.56 - 3.42 (m) total 4H], 2.72 (s, 3H), 2.62 - 2.50 (m, 1H), 2.30 - 2.19 (m, 1H).

### Preparation P5

### 6-Methyl-5-(2-methyl-2H-tetrazol-5-yl)-N-[(3S)-pyrrolidin-3-yl]pyridin-2-amine, dihydrochloride salt (P5)

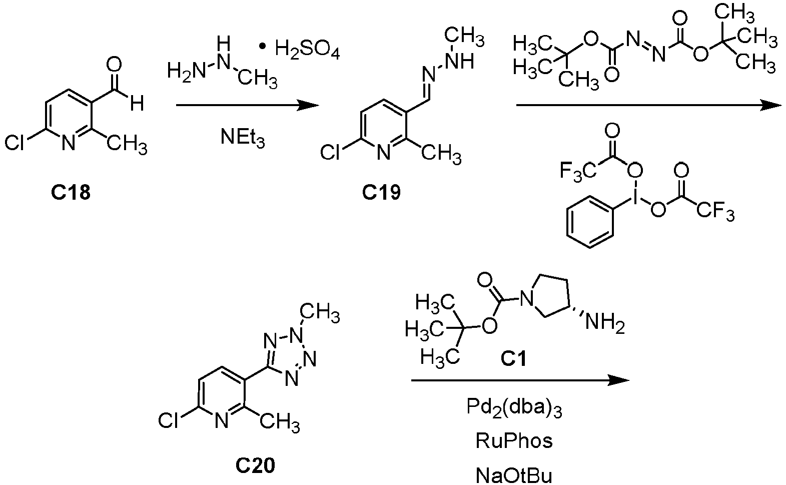

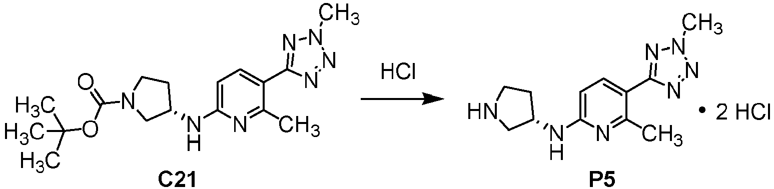

### Step 1: Synthesis of 6-chloro-2-methyl-3-[(E)-(2-methylhydrazinylidene)methyl]pyridine (C19).

A mixture of methylhydrazine sulfate (8.65 g, 60.0 mmol) in methanol (60 mL) and triethylamine (18.2 g, 180 mmol) was added to a solution of 6-chloro-2-methylpyridine-3-carbaldehyde (**C18**) (9.33 g, 60.0 mmol) in methanol (140 mL). The mixture was stirred for 30 minutes at 25 °C; it was then concentrated *in vacuo* and purified via silica gel chromatography (Gradient: 0% to 40% ethyl acetate in petroleum ether), providing **C19** as a yellow solid. Yield: 9.24 g, 50.3 mmol, 84%. LCMS *m*/*z* 184.1 (chlorine isotope pattern observed) [M+H]⁺; ¹H NMR (400 MHz, DMSO-*d*₆) δ 7.97 (d, *J* = 8.3 Hz, 1H), 7.85 - 7.79 (m, 1H), 7.38 (s, 1H), 7.24 (d, *J* = 8.1 Hz, 1H), 2.85 (d, *J* = 4.2 Hz, 3H), 2.50 (s, 3H).

### Step 2: Synthesis of 6-chloro-2-methyl-3-(2-methyl-2H-tetrazol-5-yl)pyridine (C20).

To a solution of **C19** (9.18 g, 50.0 mmol) in a mixture of dichloromethane (50 mL) and 2,2,2-trifluoroethanol (50 mL) were added di-*tert*-butyl azodicarboxylate (13.8 g, 60.0 mmol) and [bis(trifluoroacetoxy)iodo]benzene (43.0 g, 100 mmol). The reaction mixture was stirred at 25 °C for 30 minutes, whereupon it was concentrated *in vacuo.* Purification via silica gel chromatography (Gradient: 0% to 40% ethyl acetate in petroleum ether) provided **C20** as a white solid. Yield: 4.63 g, 22.1 mmol, 44%. LCMS *m*/*z* 210.1 (chlorine isotope pattern observed) [M+H]⁺; ¹H NMR (400 MHz, DMSO-*d*₆) δ 8.32 (d, *J* = 8.3 Hz, 1H), 7.58 - 7.48 (m, 1H), 4.47 (s, 3H), 2.75 (s, 3H).

### Step 3: Synthesis of tert-butyl (3S)-3-{[6-methyl-5-(2-methyl-2H-tetrazol-5-yl)pyridin-2-yl]amino}pyrrolidine-1-carboxylate (C21).

A solution of **C20** (210 mg, 1.00 mmol), **C1** (196 mg, 1.05 mmol), sodium tert-butoxide (193 mg, 2.00 mmol), Pd₂(dba)₃ [tris(dibenzylideneacetone)dipalladium(0); 91.7 mg, 0.100 mmol], and 2-dicyclohexylphosphino-2',6'-diisopropoxybiphenyl (RuPhos; 93.5 mg, 0.200 mmol) in toluene (15 mL) was stirred at 100 °C for 16 hours, whereupon the reaction mixture was concentrated *in vacuo.* Purification via silica gel chromatography (Gradient: 0% to 10% methanol in dichloromethane) provided **C21** as a yellow solid. Analysis of the ¹H NMR indicated that this material comprised a mixture of rotamers. Yield: 270 mg, 0.751 mmol, 75%. LCMS *m*/*z* 360.1 [M+H]⁺. ¹H NMR (400 MHz, methanol-*d*₄) δ 7.95 (d, *J* = 8.8 Hz, 1H), 6.47 (d, *J* = 8.8 Hz, 1H), 4.57 - 4.46 (m, 1H), 4.39 (s, 3H), [3.72 (d, *J* = 6.4 Hz) and 3.69 (d, *J* = 6.1 Hz), total 1H], 3.58 - 3.37 (m, 2H), [3.26 (d, *J* = 4.6 Hz) and 3.23 (d, *J* = 4.6 Hz), total 1H], 2.65 (s, 3H), 2.31 - 2.38 (m, 1H), 2.00 - 1.87 (m, 1H), [1.47 (s) and 1.46 (s), total 9H].

### Step 4: Synthesis of 6-methyl-5-(2-methyl-2H-tetrazol-5-yl)-N-[(3S)-pyrrolidin-3-yl]pyridin-2-amine, dihydrochloride salt (P5).

To a 20 °C solution of **C21** (270 mg, 0.751 mmol) in dichloromethane (5 mL) was added a solution of hydrogen chloride in 1,4-dioxane (4 M; 5 mL, 20 mmol). After the reaction mixture had been stirred for 3 hours, it was concentrated *in vacuo* to afford **P5** as a yellow solid. Yield: 205 mg, 0.617 mmol, 82%. LCMS *m*/*z* 260.1 [M+H]⁺. ¹H NMR (400 MHz, DMSO-*d*₆) δ 9.38 (br s, 2H), 8.27 - 8.06 (m, 1H), 6.99 - 6.73 (m, 1H), 4.98 - 4.46 (m, 3H), 4.42 (s, 3H), 3.57 - 3.46 (m, 1H), 3.45 - 3.32 (m, 1H), 3.32 - 3.21 (m, 1H), 3.21 - 3.11 (m, 1H), 2.75 (br s, 3H), 2.37 - 2.24 (m, 1H), 2.04 - 1.92 (m, 1H).

### Preparation P6

### 5-[5-(1,1-Difluoroethyl)-1-methyl-1H-1,2,4-triazol-3-yl]-6-methyl-N-[(3S)-pyrrolidin-3-yl]pyridin-2-amine, dihydrochloride salt (P6)

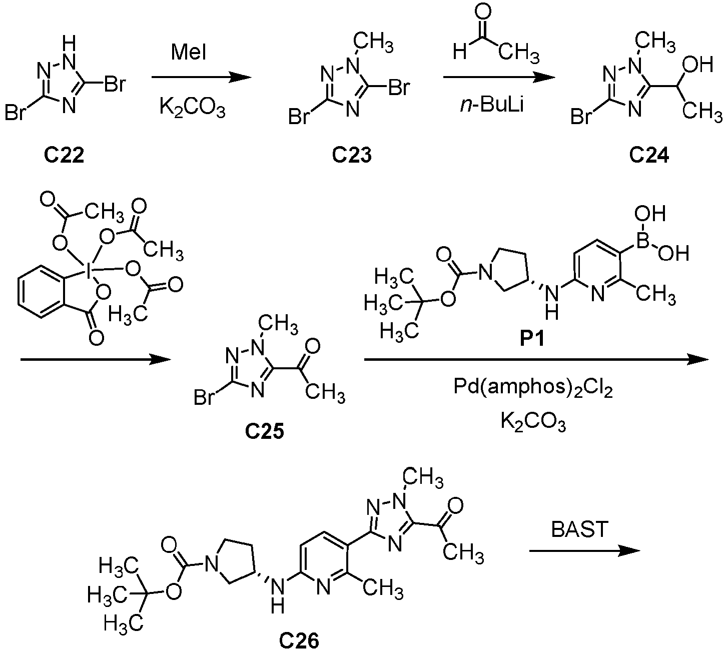

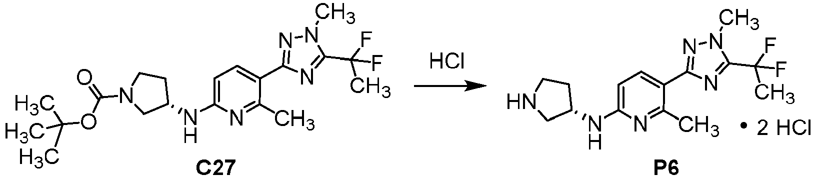

### Step 1: Synthesis of 3,5-dibromo-1-methyl-1H-1,2,4-triazole (C23).

A 25 °C solution of 3,5-dibromo-1*H*-1,2,4-triazole (**C22**) (16.5 g, 72.7 mmol), potassium carbonate (20.1 g, 145 mmol), and iodomethane (15.5 g, 109 mmol) in *N,N*-dimethylformamide (30 mL) was stirred for 2 hours. The reaction mixture was then treated with water (30 mL) and extracted with dichloromethane (2 x 35 mL); the combined organic layers were concentrated *in vacuo* and purified via silica gel chromatography (Eluent: 20% ethyl acetate in petroleum ether) to provide **C23** as a white solid. Yield: 13.6 g, 56.5 mmol, 78%. LCMS *m*/*z* 242.0 (dibromo isotope pattern observed) [M+H]⁺. ¹H NMR (400 MHz, chloroform-d) δ 3.87 (br s, 3H).

### Step 2: Synthesis of 1-(3-bromo-1-methyl-1H-1,2,4-triazol-5-yl)ethan-1-ol (C24).

To a -78 °C solution of **C23** (10.3 g, 42.8 mmol) in tetrahydrofuran (40 mL) was added a solution of n-butyllithium in hexane (2.4 M; 19.6 mL, 47.0 mmol) in a drop-wise manner. The mixture was stirred at -40 °C for 2.5 hours and then a solution of acetaldehyde in tetrahydrofuran (5 M; 17.1 mL, 85.5 mmol) was added at -78 °C. Stirring was continued at -78 °C for 16 hours, whereupon the mixture was diluted with water (20 mL) and concentrated *in vacuo.* Purification via silica gel chromatography (Gradient: 0% to 10% methanol in dichloromethane) afforded **C24** as a yellow oil. Yield: 5.10 g, 24.8 mmol, 58%. LCMS *m*/*z* 206.1 (bromine isotope pattern observed) [M+H]⁺. ¹H NMR (400 MHz, chloroform-d) δ 4.98 (br q, *J* = 6.8 Hz, 1H), 3.91 (s, 3H), 3.56 (br s, 1H), 1.60 (d, *J* = 6.7 Hz, 3H).

### Step 3: Synthesis of 1-(3-bromo-1-methyl-1H-1,2,4-triazol-5-yl)ethan-1-one (C25).

A 25 °C solution of **C24** (5.1 g, 24.7 mmol) and 1,1,1-tris(acetyloxy)-1,1-dihydro-1,2-benziodoxol-3-(1*H*)-one (Dess-Martin periodinane; 11.5 g, 27.2 mmol) in dichloromethane (40 mL) was stirred for 3.5 hours. After the reaction mixture had been concentrated *in vacuo,* silica gel chromatography (Gradient: 0% to 20% ethyl acetate in petroleum ether) provided **C25** as a white solid. Yield: 4.47 g, 21.9 mmol, 89%. LCMS *m*/*z* 204.0 (bromine isotope pattern observed) [M+H]⁺. ¹H NMR (400 MHz, chloroform-d) δ 4.16 (br s, 3H), 2.68 (br s, 3H).

### Step 4: Synthesis of tert-butyl (3S)-3-{[5-(5-acetyl-1-methyl-1H-1,2,4-triazol-3-yl)-6-methylpyridin-2-yl]amino}pyrrolidine-1-carboxylate (C26).

A solution of **C25** (4.57 g, 22.4 mmol), **P1** (7.91 g, 24.6 mmol), potassium carbonate (6.19 g, 44.8 mmol), and Pd(amphos)₂Cl₂ (1.59 g, 2.24 mmol) in a mixture of 1,4-dioxane and water (40:1, 41 mL) was stirred at 90 °C for 16 hours. The reaction mixture was concentrated *in vacuo,* whereupon purification via silica gel chromatography (Gradient: 0% to 40% ethyl acetate in petroleum ether) provided **C26** as a yellow solid. Analysis of the ¹H NMR indicated that this material comprised a mixture of rotamers. Yield: 3.49 g, 8.71 mmol, 39%. LCMS *m*/*z 401.3* [M+H]⁺. ¹H NMR (400 MHz, methanol-*d*₄) δ 7.94 (d, *J* = 8.6 Hz, 1H), 6.45 (d, *J* = 8.6 Hz, 1H), 4.54 - 4.43 (m, 1H), 4.19 (s, 3H), 3.71 (dd, *J* = 11.1, 6.1 Hz, 1H), 3.58 - 3.38 (m, 2H), 3.25 (dd, *J* = 11.1, 4.8 Hz, 1H), 2.69 (s, 3H), 2.66 (s, 3H), 2.31 - 2.17 (m, 1H), 2.01 - 1.87 (m, 1H), [1.47 (s) and 1.46 (s), total 9H].

### Step 5: Synthesis of tert-butyl (3S)-3-({5-[5-(1,1-difluoroethyl)-1-methyl-1H-1,2,4-triazol-3-yl]-6-methylpyridin-2-yl}amino)pyrrolidine-1-carboxylate (C27).

A 25 °C solution of **C26** (3.49 g, 8.71 mmol) in [bis(2-methoxyethyl)amino]sulfur trifluoride (BAST; 20 mL) was stirred for 16 hours. The reaction mixture was diluted with dichloromethane (300 mL), quenched with saturated aqueous sodium bicarbonate solution (300 mL), and extracted with dichloromethane (2 x 30 mL); the combined organic layers were concentrated *in vacuo.* Purification via silica gel chromatography (Gradient: 0% to 40% ethyl acetate in petroleum ether) provided **C27** as a yellow solid. Analysis of the ¹H NMR indicated that this material comprised a mixture of rotamers. Yield: 2.50 g, 5.92 mmol, 68%. LCMS *m*/*z* 423.3 [M+H]⁺. ¹H NMR (400 MHz, methanol-*d*₄) δ 7.89 (d, *J* = 8.7 Hz, 1H), 6.44 (d, *J* = 8.7 Hz, 1H), 4.53 - 4.42 (m, 1H), 4.07 (t, *J* = 1.4 Hz, 3H), 3.70 (dd, *J* = 11.1, 6.1 Hz, 1H), 3.56 - 3.38 (m, 2H), 3.28 - 3.20 (m, 1H), 2.63 (s, 3H), 2.29 - 2.16 (m, 1H), 2.17 (t, *J*_{HF} = 19.2 Hz, 3H), 2.00 - 1.87 (m, 1H), [1.47 (s) and 1.46 (s), total 9H].

### Step 6: Synthesis of 5-[5-(1,1-difluoroethyl)-1-methyl-1H-1,2,4-triazol-3-yl]-6-methyl-N-[(3S)-pyrrolidin-3-yl]pyridin-2-amine, dihydrochloride salt (P6).

To a 25 °C solution of **C27** (2.5 g, 5.92 mmol) in dichloromethane (20 mL) was added a solution of hydrogen chloride in 1,4-dioxane (4.0 M; 5.92 mL, 23.7 mmol). After the reaction had been stirred for 4 hours, it was concentrated *in vacuo.* The residue was washed with ethyl acetate to afford **P6** as a yellow solid. Yield: 1.72 g, 4.35 mmol, 73%. LCMS *m*/*z* 323.2 [M+H]⁺. ¹H NMR (400 MHz, methanol-*d*₄) δ 8.59 (d, *J* = 9.4 Hz, 1H), 7.18 (br d, *J* = 9.4 Hz, 1H), 4.83 - 4.73 (m, 1H), 4.12 (t, *J* = 1.3 Hz, 3H), 3.73 (dd, component of ABX system, *J* = 12.6, 6.7 Hz, 1H), 3.66 - 3.56 (m, 1H), 3.55 - 3.41 (m, 2H), 2.96 (s, 3H), 2.61 - 2.49 (m, 1H), 2.29 - 2.17 (m, 1H), 2.19 (t, *J*_{HF} = 19.2 Hz, 3H).

### Preparation P7

### 6-Methyl-5-(pyrimidin-2-yl)-N-[(3S)-pyrrolid in-3-yl]pyridin-2-amine, dihydrochloride salt (P7)

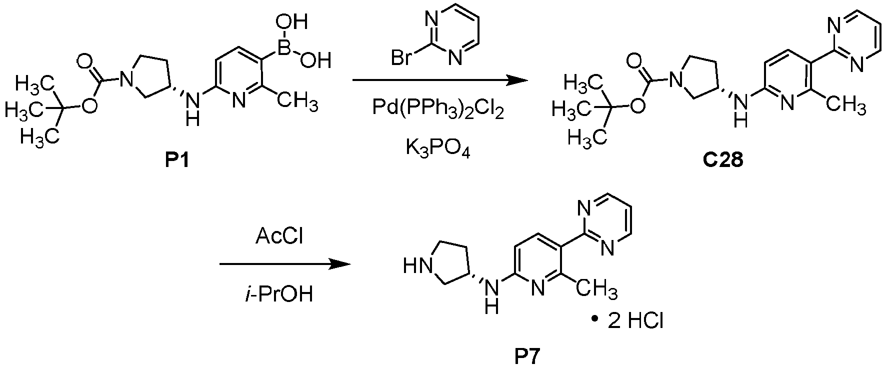

### Step 1. Synthesis of tert-butyl (3S)-3-{[6-methyl-5-(pyrimidin-2-yl)pyridin-2-yl]amino}pyrrolidine-1-carboxylate (C28).

Aqueous tripotassium phosphate solution (2.0 M; 311 mL, 622 mmol) was added to a solution of **P1** (80 g, 250 mmol) and 2-bromopyrimidine (43.6 g, 274 mmol) in tetrahydrofuran (1.0 L), followed by sparging with nitrogen for 10 minutes. Dichlorobis(triphenylphosphine)palladium(II) (3.5 g, 5.0 mmol) was added, and the reaction mixture was again sparged with nitrogen for 10 minutes, whereupon it was heated to 65 °C for 18 hours. The reaction mixture was cooled to room temperature and partitioned between ethyl acetate and water. After the aqueous layer had been extracted three times with ethyl acetate, the combined organic layers were dried over sodium sulfate, filtered through a pad of diatomaceous earth, and concentrated *in vacuo* to afford **C28** as a solid. Yield: 85.0 g, 239 mmol, 96%.LCMS *m*/*z* 356.3 [M+H]⁺. ¹H NMR (400 MHz, methanol-*d*₄), characteristic peaks: δ 8.80 (d, *J* = 4.9 Hz, 2H), 7.93 (d, *J* = 8.7 Hz, 1H), 7.28 (t, *J* = 4.9 Hz, 1H), 6.47 (d, *J* = 8.7 Hz, 1H), 4.56 - 4.45 (m, 1H), 3.25 (dd, *J* = 11.0, 4.5 Hz, 1H), 2.62 (s, 3H), 2.00 - 1.89 (m, 1H), 1.46 (s, 9H).

### Step 2. Synthesis of 6-methyl-5-(pyrimidin-2-yl)-N-[(3S)-pyrrolidin-3-yl]pyridin-2-amine, dihydrochloride salt (P7).

Acetyl chloride (71.6 g, 912 mmol, 64.8 mL) was added to propan-2-ol (174 mL) at 0 °C in a drop-wise manner. In a separate flask, **C28** (81 g, 228 mmol) was dissolved in a 4:1 mixture of propan-2-yl acetate / propan-2-ol (760 mL), which required heating to 50 °C. The hydrogen chloride solution was added in a drop-wise manner to the solution of **C28** at 50 °C and the reaction mixture was stirred at 50 °C for 6 hours. The heat was turned off and the reaction was slowly cooled to room temperature with stirring, whereupon the mixture was filtered to provide **P7** as a solid. Yield: 72 g, 220 mmol, 96%. LCMS *m*/*z* 256.2 [M+H]⁺. ¹H NMR (400 MHz, methanol-*d*₄) δ 8.94 (d, *J* = 5.0 Hz, 2H), 8.73 (d, *J* = 9.3 Hz, 1H), 7.49 - 7.44 (m, 1H), 7.23 (d, *J =* 9.4 Hz, 1H), 4.86 - 4.77 (m, 1H), 3.76 (dd, *J* = 12.5, 6.6 Hz, 1H), 3.67 - 3.57 (m, 1H), 3.57 - 3.42 (m, 2H), 2.97 (s, 3H), 2.63 - 2.51 (m, 1H), 2.32 - 2.19 (m, 1H).

### Preparation P8

### 5-[5-(Difluoromethyl)-1-methyl-1H-1,2,4-triazol-3-yl]-6-methyl-N-[(3S)-pyrrolidin-3-yl]pyridin-2-amine, dihydrochloride salt (P8)

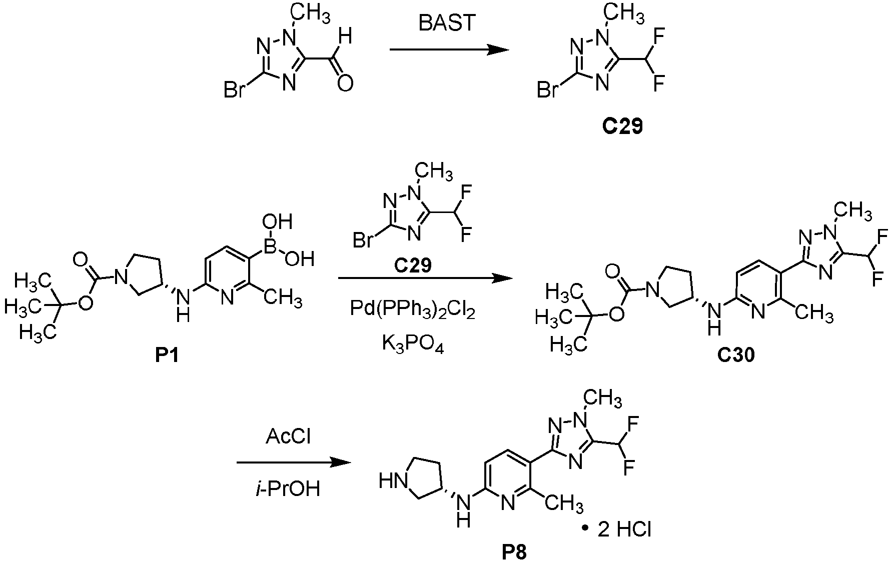

### Step 1. Synthesis of 3-bromo-5-(difluoromethyl)-1-methyl-1H-1,2,4-triazole (C29).

[Bis(2-methoxyethyl)amino]sulfur trifluoride (BAST; 47.0 mL, 255 mmol) was added drop-wise to a 0 °C mixture of 3-bromo-1-methyl-1*H*-1,2,4-triazole-5-carbaldehyde (24.2 g, 127 mmol) in dichloromethane (400 mL). The reaction mixture was allowed to warm to 20 °C and stirred at 20 °C for 16 hours, whereupon it was treated drop-wise with aqueous sodium bicarbonate solution and extracted with dichloromethane (3 x 300 mL). The combined organic layers were washed with saturated aqueous sodium chloride solution, dried over sodium sulfate, and filtered. After the filtrate had been concentrated to dryness *in vacuo* below 38 °C, the residue was purified via chromatography on silica gel (Gradient: 50% to 70% dichloromethane in petroleum ether) to provide **C29** as a light-yellow oil. Yield: 17.7 g, 83.5 mmol, 66%. LCMS *m*/*z* 212.0 (bromine isotope pattern observed) [M+H]⁺. ¹H NMR (400 MHz, methanol-*d*₄) δ 7.06 (t, *J*_{HF} = 52.2 Hz, 1H), 4.02 (s, 3H).

### Step 2. Synthesis of tert-butyl (3S)-3-({5-[5-(difluoromethyl)-1-methyl-1H-1,2,4-triazol-3-yl]-6-methylpyridin-2-yl}amino)pyrrolidine-1-carboxylate (C30).

To a nitrogen-sparged, 25 °C mixture of **P1** (110 g, 342 mmol) and **C29** (68.0 g, 321 mmol) in tetrahydrofuran (800 mL) was added aqueous tripotassium phosphate solution (2.5 M; 311 mL, 778 mmol), followed by dichlorobis(triphenylphosphine)palladium(II) (1.09 g, 1.56 mmol). The reaction mixture was sparged with nitrogen for 10 minutes, heated to 70 °C for 18 hours, and cooled to room temperature. After the reaction mixture had been partitioned between ethyl acetate (250 mL) and water (250 mL), the aqueous layer was extracted with ethyl acetate. The combined organic layers were washed sequentially with saturated aqueous sodium bicarbonate and saturated aqueous sodium chloride solution, dried over magnesium sulfate, filtered, and concentrated *in vacuo;* the resulting material was dissolved in hot ethyl acetate, treated with heptane, and allowed to cool. Collection of the precipitate via filtration provided **C30** as a white solid. Yield: 105 g, 257 mmol, 80%. LCMS *m*/*z* 409.3 [M+H]⁺. ¹H NMR (400 MHz, chloroform-*d*) δ 8.00 (d, *J* = 8.6 Hz, 1H), 6.86 (t, *J*_{HF} = 52.6 Hz, 1H), 6.31 (d, *J* = 8.6 Hz, 1H), 4.80 - 4.67 (m, 1H), 4.41 - 4.23 (m, 1H), 4.08 (s, 3H), 3.80 - 3.65 (m, 1H), 3.57 - 3.38 (m, 2H), 3.36 - 3.15 (m, 1H), 2.70 (s, 3H), 2.29 - 2.16 (m, 1H), 1.99 - 1.81 (m, 1H), 1.46 (s, 9H).

### Step 3. Synthesis of 5-[5-(difluoromethyl)-1-methyl-1H-1,2,4-triazol-3-yl]-6-methyl-N-[(3S)-pyrrolidin-3-yl]pyridin-2-amine, dihydrochloride salt (P8).

Acetyl chloride (2.99 mL, 42.1 mmol) was added to 0 °C propan-2-ol (8.0 mL) in a drop-wise manner. In a separate flask, **C30** (4.30 g, 10.5 mmol) was dissolved in propan-2-ol (30 mL) by heating to 60 °C. Once the material had dissolved, the temperature was lowered to 50 °C and the hydrogen chloride solution was added at 50 °C in a drop-wise manner. After the reaction mixture had been stirred at 50 °C for 3 hours, it was slowly cooled to room temperature with stirring; collection of the precipitate via filtration afforded **P8** as a white solid. Yield: 2.85 g, 7.48 mmol, 71%. LCMS *m*/*z* 309.3 [M+H]⁺. ¹H NMR (400 MHz, methanol-*d*₄) δ 8.58 (d, *J* = 9.4 Hz, 1H), 7.19 (d, *J* = 9.4 Hz, 1H), 7.17 (t, *J*_{HF} = 52.2 Hz, 1H), 4.83 - 4.74 (m, 1H), 4.11 (br s, 3H), 3.74 (dd, *J* = 12.6, 6.7 Hz, 1H), 3.66 - 3.56 (m, 1H), 3.55 - 3.42 (m, 2H), 2.96 (s, 3H), 2.62 - 2.49 (m, 1H), 2.29 - 2.18 (m, 1H).

### Preparation P9

### 6-Methyl-N-[(3S)-pyrrolidin-3-yl]-5-[5-(trifluoromethyl)-1,2,4-oxadiazol-3-yl]pyridin-2-amine, dihydrochloride salt (P9)

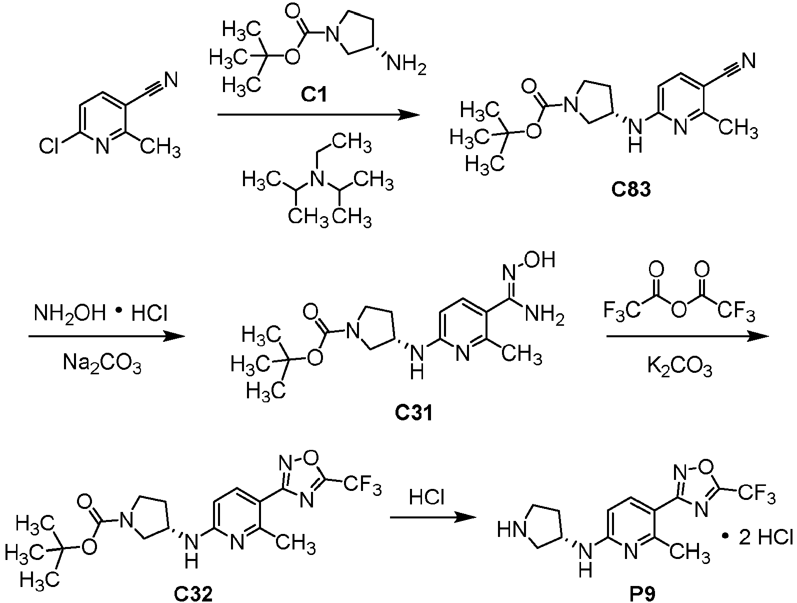

### Step 1. Synthesis of tert-butyl (3S)-3-[(5-cyano-6-methylpyridin-2-yl)amino]pyrrolidine-1-carboxylate (C83).

A solution of 6-chloro-2-methylpyridine-3-carbonitrile (5.00 g, 32.8 mmol), **C1** (6.10 g, 32.8 mmol), and *N,N*-diisopropylethylamine (8.47 g, 65.5 mmol) in dimethyl sulfoxide (25 mL) was stirred at 120 °C for 16 hours. The reaction mixture was then diluted with water (30 mL) and extracted with ethyl acetate (2 x 35 mL); the combined organic layers were concentrated *in vacuo* to afford **C83** as a yellow solid. Yield: 8.50 g, 28.1 mmol, 86%. LCMS *m*/*z* 303.0 [M+H]⁺. ¹H NMR (400 MHz, chloroform-d) δ 7.53 (d, *J* = 8.7 Hz, 1H), 6.24 (d, *J* = 8.6 Hz, 1H), 5.04 (br s, 1H), 4.50 - 4.31 (m, 1H), 3.78 - 3.64 (m, 1H), 3.57 - 3.38 (m, 2H), 3.36 - 3.14 (m, 1H), 2.56 (s, 3H), 2.31 - 2.15 (m, 1H), 1.98 - 1.82 (m, 1H), 1.46 (s, 9H).

### Step 2. Synthesis of tert-butyl (3S)-3-{[5-(N'-hydroxycarbamimidoyl)-6-methylpyridin-2-yl]amino}pyrrolidine-1-carboxylate (C31).

A mixture of **C83** (8.50 g, 28.1 mmol), hydroxylamine hydrochloride (7.81 g, 112 mmol), and sodium carbonate (11.9 g, 112 mmol) in ethanol (150 mL) was stirred at 90 °C for 16 hours. After the reaction mixture had been concentrated *in vacuo,* it was diluted with saturated aqueous sodium bicarbonate solution (40 mL) and extracted with ethyl acetate (2 x 50 mL). The combined organic layers were concentrated under reduced pressure to afford **C31** as a yellow solid. Yield: 8.68 g, 25.9 mmol, 92%. LCMS *m*/*z* 336.2 [M+H]⁺. ¹H NMR (400 MHz, chloroform-*d*) δ 7.46 (br d, *J* = 8.4 Hz, 1H), 6.22 (d, *J* = 8.5 Hz, 1H), 5.52 (br s, 1H), 4.78 (br s, 2H), 4.26 - 4.09 (m, 1H), 3.74 - 3.61 (m, 1H), 3.49 - 3.29 (m, 2H), 3.28 - 3.09 (m, 1H), 2.48 (s, 3H), 2.22 - 2.08 (m, 1H), 1.87 - 1.69 (m, 1H), 1.45 (s, 9H).

### Step 3. Synthesis of tert-butyl (3S)-3-({6-methyl-5-[5-(trifluoromethyl)-1,2,4-oxadiazol-3-yl]pyridin-2-yl}amino)pyrrolidine-1-carboxylate (C32).

To a 25 °C solution of **C31** (100 mg, 0.298 mmol) in 1,4-dioxane (2.0 mL) was added potassium carbonate (124 mg, 0.894 mmol), followed by trifluoroacetic anhydride (150 mg, 0.1 mL, 0.714 mmol). After the reaction mixture had been stirred for 20 hours, it was filtered and concentrated *in vacuo;* silica gel chromatography (Gradient: 0% to 5% methanol in dichloromethane) provided **C32** as a yellow solid. Yield: 64.0 mg, 0.155 mmol, 52%. LCMS *m*/*z* 414.3 [M+H]⁺. ¹H NMR (400 MHz, methanol-*d*₄) δ 8.47 (d, *J* = 9.5 Hz, 1H), 7.16 (d, *J* = 9.5 Hz, 1H), 4.58 - 4.44 (m, 1H), 3.82 - 3.67 (m, 1H), 3.63 - 3.47 (m, 2H), 3.43 (dd, *J* = 11.6, 3.8 Hz, 1H), 2.91 (s, 3H), 2.42 - 2.28 (m, 1H), 2.19 - 2.04 (m, 1H), 1.47 (s, 9H).

### Step 4. Synthesis of 6-methyl-N-[(3S)-pyrrolidin-3-yl]-5-[5-(trifluoromethyl)-1,2,4-oxadiazol-3-yl]pyridin-2-amine, dihydrochloride salt (P9).

To a 25 °C solution of **C32** (64.0 mg, 0.155 mmol) in methanol (5 mL) was added a solution of hydrogen chloride in 1,4-dioxane (4.0 M; 2 mL, 8 mmol). The reaction mixture was stirred for 3 hours, whereupon it was concentrated *in vacuo* to afford **P9** as a yellow solid. Yield: 41.0 mg, 0.106 mmol, 68%. LCMS *m*/*z* 314.1 [M+H]⁺. ¹H NMR (400 MHz, methanol-*d*₄), characteristic peaks: δ 8.54 (d, *J =* 9.1 Hz, 1H), 7.32 (d, *J* = 9.2 Hz, 1H), 3.79 (dd, *J* = 12.5, 6.2 Hz, 1H), 3.69 - 3.59 (m, 1H), 3.59 - 3.47 (m, 2H), 2.98 (s, 3H), 2.66 - 2.53 (m, 1H), 2.35 - 2.22 (m, 1H).

### Preparation P10

### 5-[2-(1,1-Difluoroethyl)-1-methyl-1H-imidazol-4-yl]-6-methyl-N-[(3S)-pyrrolidin-3-yl]pyridin-2-amine, dihydrochloride salt (P10)

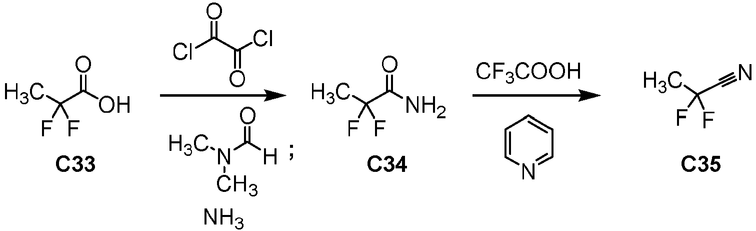

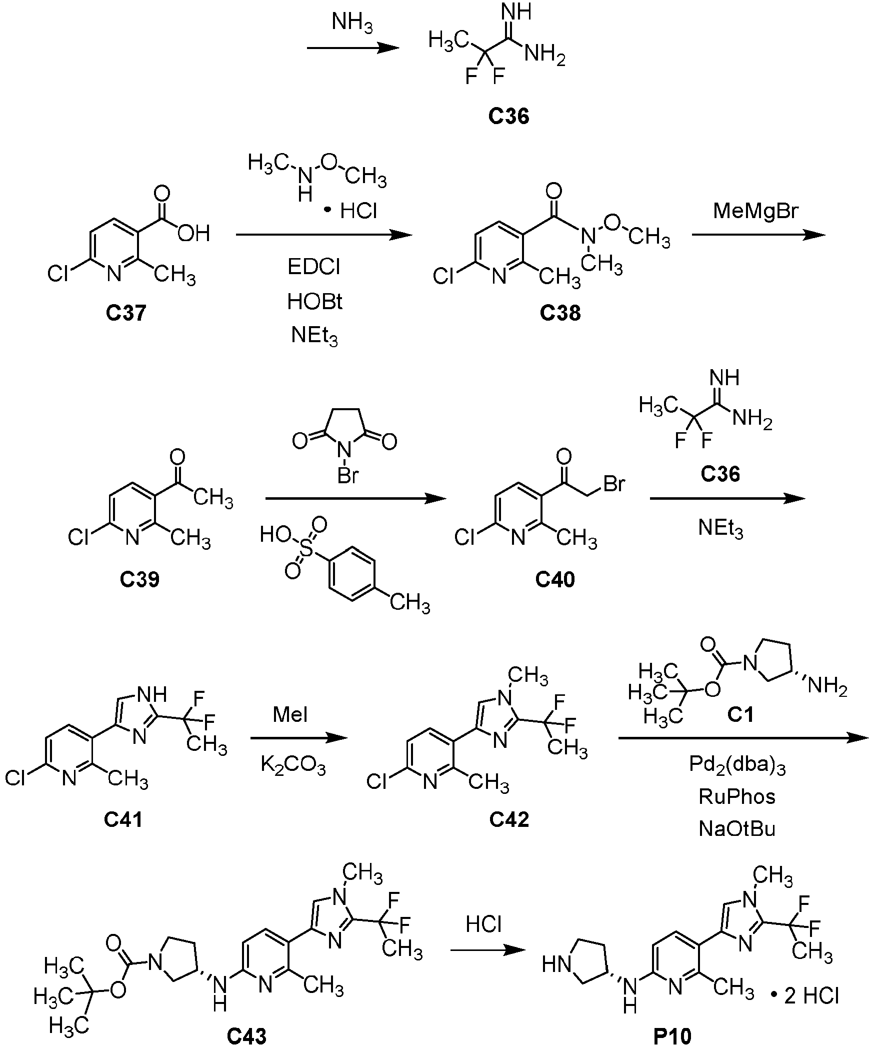

### Step 1. Synthesis of 2,2-difluoropropanamide (C34).

To a 0 °C solution of 2,2-difluoropropanoic acid (**C33**) (10.0 g, 90.9 mmol) in tetrahydrofuran (150 mL) was added oxalyl chloride (8.46 mL, 99.9 mmol) in a drop-wise manner, followed by three drops of *N,N*-dimethylformamide. The reaction mixture was stirred at 25 °C for 2 hours and then added drop-wise over 20 minutes to aqueous ammonia (25%, 100 mL) at 0 °C. After this reaction mixture had been stirred for 16 hours at 25 °C, it was extracted with ethyl acetate (2 x 100 mL). The combined organic layers were washed with saturated aqueous sodium chloride solution, dried over sodium sulfate, filtered, and concentrated *in vacuo* to afford **C34** as a white solid. Yield: 5.88 g, 53.9 mmol, 59%. LCMS *m*/*z* 110.2 [M+H]⁺. ¹H NMR (400 MHz, methanol-*d*₄) δ 1.75 (t, *J*_{HF} = 19.1 Hz, 3H).

### Step 2. Synthesis of 2,2-difluoropropanenitrile (C35).

To a 25 °C solution of **C34** (10.2 g, 93.5 mmol) in pyridine (60 mL) was added a solution of trifluoroacetic acid (14.3 mL, 103 mmol) in pyridine (30 mL) in a drop-wise manner. The reaction mixture was heated at 110 °C, and the product was directly distilled at this temperature into a receiving flask cooled in an ice bath, affording **C35** as a colorless oil. Yield: 5.90 g, 64.8 mmol, 69%. ¹H NMR (400 MHz, methanol-*d*₄) δ 1.83 (t, *J*_{HF} = 19.0 Hz, 3H).

### Step 3. Synthesis of 2,2-difluoropropanimidamide (C36).

Starting material **C35** (5.90 g, 64.8 mmol) was condensed into a flask immersed in a dry ice-ethanol bath and equipped with a dry ice-ethanol condenser. Liquid ammonia (approximately 30 mL) was added to the flask, the cooling bath was removed, and the reaction mixture was allowed to reflux for 1 hour. The ammonia was then allowed to vent off, providing **C36** as a colorless oil. Yield: 5.4 g, 50 mmol, 77%. LCMS *m*/*z* 109.2 [M+H]⁺. ¹H NMR (400 MHz, methanol-*d*₄) δ 1.83 (t, *J*_{HF} = 19.0 Hz, 3H).

### Step 4. Synthesis of 6-chloro-N-methoxy-N,2-dimethylpyridine-3-carboxamide (C38).

To a 25 °C solution of 6-chloro-2-methylpyridine-3-carboxylic acid (**C37**) (5.0 g, 29 mmol), 1*H*-benzotriazol-1-ol (HOBt; 5.91 g, 43.7 mmol), 1-[3-(dimethylamino)propyl]-3-ethylcarbodiimide hydrochloride (EDCl; 8.38 g, 43.7 mmol), and *N,O*-dimethylhydroxylamine hydrochloride (3.41 g, 35.0 mmol) in dichloromethane (100 mL) was added triethylamine (12.6 mL, 87.4 mmol). After the reaction mixture had been stirred for 2 hours, it was washed sequentially with saturated aqueous ammonium chloride solution (50 mL), water (100 mL), and saturated aqueous sodium chloride solution (50 mL), then concentrated *in vacuo* to provide **C38** as a colorless oil (6.45 g). Most of this material was progressed to the following step. LCMS *m*/*z* 215.1 (chlorine isotope pattern observed) [M+H]⁺. ¹H NMR (400 MHz, methanol-*d*₄) δ 7.76 (d, *J* = 8.1 Hz, 1H), 7.36 (d, *J* = 8.1 Hz, 1H), 3.50 (br s, 3H), 3.37 (br s, 3H), 2.47 (s, 3H).

### Step 5. Synthesis of 1-(6-chloro-2-methylpyridin-3-yl)ethan-1-one (C39).

To a 10 °C solution of **C38** (from the previous step; 6.10 g, ≤27.4 mmol) in tetrahydrofuran (60 mL) was added a solution of methylmagnesium bromide in tetrahydrofuran (3.0 M; 23.7 mL, 71.1 mmol) in a drop-wise manner, whereupon the reaction mixture was stirred at 25 °C for 1 hour. It was then cooled below 10 °C, quenched by addition of saturated aqueous ammonium chloride solution, and poured into water (200 mL). The resulting mixture was extracted with ethyl acetate (2 x 100 mL); the combined organic layers were washed with saturated aqueous sodium chloride solution, dried over sodium sulfate, filtered, and concentrated *in vacuo.* Purification via silica gel chromatography (Gradient: 0% to 30% ethyl acetate in petroleum ether) provided **C39** as a colorless oil. Yield: 3.49 g, 20.6 mmol, 75% over 2 steps. LCMS *m*/*z* 170.1 (chlorine isotope pattern observed) [M+H]⁺. ¹H NMR (400 MHz, methanol-*d*₄) δ 8.20 (d, *J* = 8.3 Hz, 1H), 7.40 (d, *J* = 8.2 Hz, 1H), 2.66 (s, 3H), 2.59 (s, 3H).

### Step 6. Synthesis of 2-bromo-1-(6-chloro-2-methylpyridin-3-yl)ethan-1-one (C40).

To a 25 °C solution of **C39** (3.50 g, 20.6 mmol) in acetonitrile (60 mL) was added *N-*bromosuccinimide (4.41 g, 24.8 mmol) and *p*-toluenesulfonic acid (355 mg, 2.06 mmol), whereupon the reaction mixture was stirred at 80 °C for 5 hours and then concentrated *in vacuo.* Purification via silica gel chromatography (Gradient: 0% to 10% ethyl acetate in petroleum ether) provided **C40** as a brown oil. Yield: 3.23 g, 13.0 mmol, 63%. LCMS *m*/*z* 247.9 (bromo chloro isotope pattern observed) [M+H]⁺. ¹H NMR (400 MHz, methanol-*d*₄) δ 8.21 (d, *J* = 8.3 Hz, 1H), 7.42 (d, *J* = 8.3 Hz, 1H), 4.61 (s, 2H), 2.65 (s, 3H).

### Step 7. Synthesis of 6-chloro-3-[2-(1,1-difluoroethyl)-1H-imidazol-4-yl]-2-methylpyridine (C41)

A mixture of **C40** (3.23 g, 13.0 mmol), triethylamine (1.82 mL, 13.1 mmol), and **C36** (2.11 g, 19.5 mmol) in tetrahydrofuran (40 mL) was stirred at reflux for 4 hours. The reaction mixture was poured into water (60 mL) and extracted with ethyl acetate (80 mL); the organic layer was concentrated *in vacuo* and subjected to silica gel chromatography (Gradient: 0% to 30% ethyl acetate in petroleum ether), providing **C41** as a colorless gum. Yield: 1.18 g, 4.58 mmol, 35%. LCMS *m*/*z* 258.1 (chlorine isotope pattern observed) [M+H]⁺. ¹H NMR (400 MHz, methanol-*d*₄) δ 8.05 (d, *J* = 8.2 Hz, 1H), 7.46 (s, 1H), 7.33 (d, *J* = 8.2 Hz, 1H), 2.64 (s, 3H), 2.09 (t, *J*_{HF} = 18.6 Hz, 3H).

### Step 8. Synthesis of 6-chloro-3-[2-(1,1-difluoroethyl)-1-methyl-1H-imidazol-4-yl]-2-methylpyridine (C42).

A mixture of **C41** (1.47 g, 5.70 mmol), iodomethane (0.426 mL, 6.84 mmol), and potassium carbonate (1.57 g, 11.4 mmol) in *N,N*-dimethylformamide (20 mL) was stirred at 50 °C for 5 hours. After the reaction mixture had cooled, it was poured into water and extracted with ethyl acetate (2 x 100 mL). The combined organic layers were washed with saturated aqueous sodium chloride solution, dried over sodium sulfate, filtered, concentrated *in vacuo,* and purified via silica gel chromatography (Gradient: 0% to 10% ethyl acetate in petroleum ether) to afford **C42** as a white solid. Yield: 900 mg, 3.31 mmol, 58%. LCMS *m*/*z* 272.1 (chlorine isotope pattern observed) [M+H]⁺. ¹H NMR (400 MHz, methanol-*d*₄) δ 8.08 (d, *J* = 8.2 Hz, 1H), 7.50 (s, 1H), 7.32 (d, *J* = 8.2 Hz, 1H), 3.91 (s, 3H), 2.64 (s, 3H), 2.15 (t, *J*_{HF} = 19.0 Hz, 3H). Step 9. Synthesis of *tert*-butyl (3*S*)-3-({5-[2-(1,1-difluoroethyl)-1-methyl-1*H*-imidazol-4-yl]-6-methylpyridin-2-yl}amino)pyrrolidine-1-carboxylate (**C43**).

A mixture of **C1** (617 mg, 3.31 mmol), **C42** (900 mg, 3.31 mmol), Pd₂(dba)₃ (303 mg, 0.331 mmol), 2-dicyclohexylphosphino-2',6'-diisopropoxybiphenyl (RuPhos; 309 mg, 0.663 mmol), and sodium *tert*-butoxide (637 mg, 6.63 mmol) in toluene (20 mL) was stirred at 100 °C for 10 hours. The reaction mixture was concentrated *in vacuo* and subjected to silica gel chromatography (Gradient: 0% to 60% ethyl acetate in petroleum ether), providing **C43** as a white solid. Analysis of the ¹H NMR indicated that this material comprised a mixture of rotamers. Yield: 850 mg, 2.02 mmol, 61%. LCMS *m*/*z* 422.2 [M+H]⁺. ¹H NMR (400 MHz, methanol-*d*₄) δ 7.62 (d, *J* = 8.5 Hz, 1H), 7.22 (s, 1H), 6.43 (d, *J* = 8.5 Hz, 1H), 4.48 - 4.38 (m, 1H), 3.88 (br t, *J* = 1 Hz, 3H), 3.69 (dd, *J* = 11.1, 6.1 Hz, 1H), 3.57 - 3.37 (m, 2H), 3.23 (br dd, *J* = 11.1, 4.5 Hz, 1H), 2.45 (s, 3H), 2.29 - 2.17 (m, 1H), 2.12 (t, *J*_{HF} = 18.9 Hz, 3H), 1.99 - 1.85 (m, 1H), [1.47 (s) and 1.46 (s), total 9H].

### Step 10. Synthesis of 5-[2-(1,1-difluoroethyl)-1-methyl-1H-imidazol-4-yl]-6-methyl-N-[(3S)-pyrrolidin-3-yl]pyridin-2-amine, dihydrochloride salt (P10).

To a 25 °C solution of **C43** (850 mg, 2.02 mmol) in methanol (5 mL) was added a solution of hydrogen chloride in 1,4-dioxane (4 M; 5 mL, 20 mmol). The reaction mixture was stirred for 2 hours, whereupon it was concentrated *in vacuo* to afford **P10** as a grey solid. Yield: 746 mg, 1.89 mmol, 94%. LCMS *m*/*z* 322.2 [M+H]⁺. ¹H NMR (400 MHz, methanol-*d*₄) δ 8.30 (d, *J* = 9.3 Hz, 1H), 7.61 (s, 1H), 7.13 (d, *J* = 9.3 Hz, 1H), 4.78 - 4.70 (m, 1H), 3.94 (br t, *J* = 1 Hz, 3H), 3.72 (dd, *J* = 12.6, 6.6 Hz, 1H), 3.64 - 3.55 (m, 1H), 3.53 - 3.46 (m, 1H), 3.43 (dd, *J* = 12.6, 4.2 Hz, 1H), 2.75 (s, 3H), 2.60 - 2.48 (m, 1H), 2.27 - 2.16 (m, 1H), 2.17 (t, *J*_{HF} = 19.0 Hz, 3H).

### Preparation P12

### 6-Methyl-5-[1-methyl-5-(trifluoromethyl)-1H-1,2,4-triazol-3-yl]-N-[(3S)-pyrrolidin-3-yl]pyridin-2-amine, dihydrochloride salt (P12)

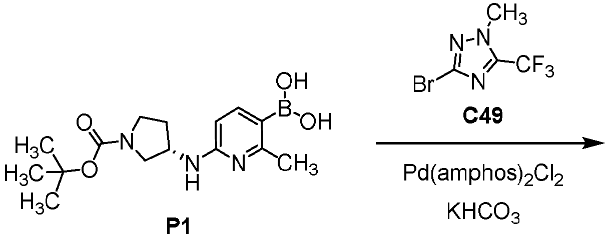

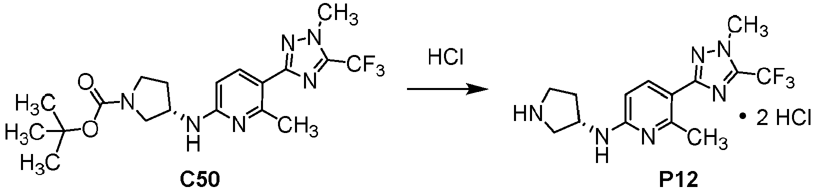

### Step 1. Synthesis of tert-butyl (3S)-3-({6-methyl-5-[1-methyl-5-(trifluoromethyl)-1H-1,2,4-triazol-3-yl]pyridin-2-yl}amino)pyrrolidine-1-carboxylate (C50).

A mixture of 3-bromo-1-methyl-5-(trifluoromethyl)-1*H*-1,2,4-triazole (**C49**) (8.95 g, 37.4 mmol), **P1** (12.0 g, 37.4 mmol), Pd(amphos)₂Cl₂ (2.65 g, 3.74 mmol), and potassium bicarbonate (11.2 g, 112 mmol) in 1,4-dioxane (150 mL) and water (35 mL) was heated at 100 °C for 4 hours. The reaction mixture was concentrated *in vacuo* and purified using silica gel chromatography (Gradient: 0% to 50% ethyl acetate in petroleum ether), affording **C50** as a white solid. Analysis of the ¹H NMR indicated that this material comprised a mixture of rotamers. Yield: 9.82 g, 23.0 mmol, 61%. LCMS *m*/*z* 427.2 [M+H]⁺. ¹H NMR (400 MHz, methanol-*d*₄) δ 7.92 (d, *J* = 8.7 Hz, 1H), 6.45 (d, *J* = 8.6 Hz, 1H), 4.55 - 4.43 (m, 1H), 4.10 (q, *J* = 1.0 Hz, 3H), 3.70 (dd, *J* = 11.1, 6.1 Hz, 1H), 3.57 - 3.38 (m, 2H), 3.24 (dd, *J* = 11.1, 4.7 Hz, 1H), 2.65 (s, 3H), 2.30 - 2.17 (m, 1H), 2.00 - 1.86 (m, 1H), [1.47 (s) and 1.46 (s), total 9H].

### Step 2. Synthesis of 6-methyl-5-[1-methyl-5-(trifluoromethyl)-1H-1,2,4-triazol-3-yl]-N-[(3S)-pyrrolidin-3-yl]pyridin-2-amine, dihydrochloride salt (P12).

To a 25 °C solution of **C50** (9.8 g, 23 mmol) in methanol (40 mL) was added a solution of hydrogen chloride in 1,4-dioxane (4.0 M; 57.5 mL, 230 mmol) and the reaction mixture was stirred for 2 hours. It was then concentrated *in vacuo* to give **P12** as a white solid. Yield: 9.05 g, 22.7 mmol, 99%. LCMS *m*/*z* 327.1 [M+H]⁺. ¹H NMR (400 MHz, DMSO-*d*₆), characteristic peaks: δ 4.78 - 4.57 (m, 1H), 4.13 - 4.07 (m, 3H), 3.58 - 3.46 (m, 1H), 3.44 - 3.32 (m, 1H), 3.32 - 3.21 (m, 1H), 3.21 - 3.11 (m, 1H), 2.77 (br s, 3H), 2.37 - 2.24 (m, 1H), 2.03 - 1.92 (m, 1H).

### Preparation P13

### 2-(5-Chloro-2-methoxypyridin-4-yl)propanoic acid (P13)

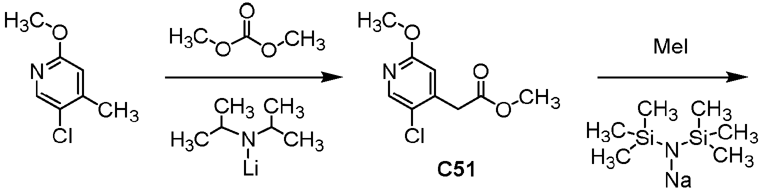

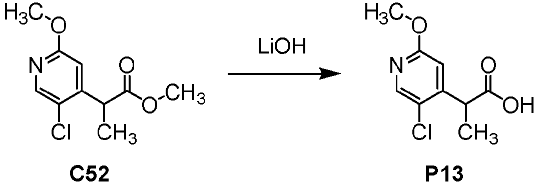

### Step 1. Synthesis of methyl (5-chloro-2-methoxypyridin-4-yl)acetate (C51).

A solution of lithium diisopropylamide in tetrahydrofuran (2 M; 1.9 L, 3.8 mol) was added in a drop-wise manner to a -30 °C solution of 5-chloro-2-methoxy-4-methylpyridine (197 g, 1.25 mol) in tetrahydrofuran (1.4 L). After the reaction mixture had been stirred at -30 °C for 1 hour, dimethyl carbonate (338 g, 3.75 mol) was added drop-wise; at the end of the addition, the reaction mixture was warmed to 25 °C and stirred for 1 hour. It was then poured into hydrochloric acid (0.5 M; 7 L, 3.5 mol) and extracted with ethyl acetate (2 x 1.5 L); the combined organic layers were washed with saturated aqueous sodium chloride solution, dried over sodium sulfate, filtered, and concentrated *in vacuo.* Purification via silica gel chromatography (Gradient: 0% to 20% ethyl acetate in petroleum ether) provided **C51** as a yellow oil. Yield: 203 g, 0.941 mol, 75%. LCMS *m*/*z* 216.1 (chlorine isotope pattern observed) [M+H]⁺. ¹H NMR (400 MHz, methanol-*d*₄) δ 8.10 (s, 1H), 6.82 (s, 1H), 3.90 (s, 3H), 3.79 (s, 2H), 3.71 (s, 3H).

### Step 2. Synthesis of methyl 2-(5-chloro-2-methoxypyridin-4-yl)propanoate (C52).

To a -78 °C solution of **C51** (175 g, 0.812 mol) in tetrahydrofuran (1.2 L) was added a solution of sodium bis(trimethylsilyl)amide in tetrahydrofuran (2 M; 455 mL, 0.910 mol) in a drop-wise manner. The reaction mixture was stirred at -78 °C for 1 hour, whereupon a solution of iodomethane (172.6 g, 1.216 mol) in tetrahydrofuran (100 mL) was added drop-wise at -78 °C, and stirring was continued at this temperature for 2 hours. The reaction mixture was then poured into saturated aqueous ammonium chloride solution (500 mL) and extracted with ethyl acetate (2 x 100 mL); the combined organic layers were washed with saturated aqueous sodium chloride solution, dried over sodium sulfate, filtered, and concentrated *in vacuo* to provide **C52** as a brown oil. By ¹H NMR and LCMS analysis, this material was contaminated with some of the dimethylated side product methyl 2-(5-chloro-2-methoxypyridin-4-yl)-2-methylpropanoate. Yield: 136 g, ≤0.592 mol, 73%. LCMS *m*/*z* 230.1 (chlorine isotope pattern observed) [M+H]⁺. ¹H NMR (400 MHz, methanol-*d*₄), product peaks only: δ 8.10 (s, 1H), 6.76 (s, 1H), 4.10 (q, *J* = 7.2 Hz, 1H), 3.89 (s, 3H), 3.69 (s, 3H), 1.48 (d, *J* = 7.2 Hz, 3H).

### Step 3. Synthesis of 2-(5-chloro-2-methoxypyridin-4-yl)propanoic acid (P13).

To a 25 °C solution of **C52** (168 g, 0.732 mol) in tetrahydrofuran (1 L) was added, in a drop-wise manner, a solution of lithium hydroxide monohydrate (61.4 g, 0.146 mol) in water (300 mL). The reaction mixture was stirred for 2 hours, whereupon it was concentrated *in vacuo.* The aqueous residue was poured into water (500 mL) and washed with *tert*-butyl methyl ether (2 x 500 mL). The aqueous layer was then adjusted to pH 4 by addition of 3 M hydrochloric acid and extracted with ethyl acetate (2 x 500 mL); the combined ethyl acetate layers were washed with saturated aqueous sodium chloride solution, dried over sodium sulfate, filtered, and concentrated under reduced pressure to provide **P13** as a white solid. Yield: 122 g, 0.566 mol, 77%. LCMS *m*/*z* 216.1 (chlorine isotope pattern observed) [M+H]⁺. ¹H NMR (400 MHz, methanol-*d*₄) δ 8.10 (s, 1H), 6.79 (s, 1H), 4.06 (q, *J* = 7.1 Hz, 1H), 3.89 (s, 3H), 1.48 (d, *J* = 7.2 Hz, 3H).

### Preparations P14 and P15

### (2R)-2-(5-Chloro-2-methoxypyridin-4-yl)propanoic acid (P14) and (2S)-2-(5-Chloro-2-methoxypyridin-4-yl)propanoic acid (P15)

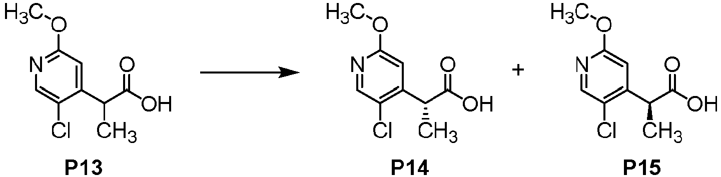

Separation of **P13** (5.00 g, 23.2 mmol) into its component enantiomers was carried out via supercritical fluid chromatography (Column: Chiral Technologies Chiralpak IG, 30 x 250 mm, 5 µm; Mobile phase: 95:5 carbon dioxide / methanol; Flow rate: 80 mL/minute; Back pressure: 120 bar). The first-eluting enantiomer, an oil which solidified on standing, was designated as **P14,** and the second-eluting enantiomer as **P15.** The indicated absolute stereochemistry was assigned via X-ray crystal structure determination of Example 6 (see below), which was synthesized using **P14.**

**P14** - Yield: 2.4 g, 11.1 mmol, 48%. ¹H NMR (400 MHz, chloroform-*d*) δ 8.13 (s, 1H), 6.75 (s, 1H), 4.12 (q, *J* = 7.2 Hz, 1H), 3.91 (s, 3H), 1.53 (d, *J* = 7.2 Hz, 3H). Retention time: 3.98 minutes (Analytical conditions. Column: Chiral Technologies Chiralpak IG, 4.6 x 250 mm, 5 µm; Mobile phase A: carbon dioxide; Mobile phase B: methanol; Gradient: 5% B for 1.00 minute, then 5% to 60% B over 8 minutes; Flow rate: 3.0 mL/minute; Back pressure: 120 bar).

**P15** - Yield: 2.4 g, 11.1 mmol, 48%. Retention time: 4.22 minutes (Analytical conditions identical to those used for **P14**).

### Preparation P16

### 2-(5-Fluoro-2-methoxypyridin-4-yl)propanoic acid (P16)

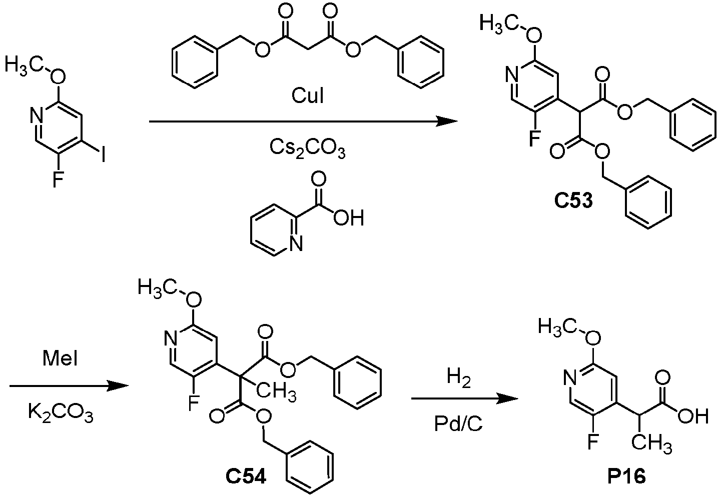

### Step 1. Synthesis of dibenzyl (5-fluoro-2-methoxypyridin-4-yl)propanedioate (C53).

This reaction was carried out in three parallel batches. To a 25 °C solution of dibenzyl propanedioate (607 g, 2.13 mol) in tetrahydrofuran (1.5 L) was added pyridine-2-carboxylic acid (35.0 g, 284 mmol), followed by copper(I) iodide (27.1 g, 142 mmol), and then freshly ground cesium carbonate (1.39 kg, 4.27 mol). After the reaction mixture had been heated to 70 °C, it was treated in a drop-wise manner with a solution of 5-fluoro-4-iodo-2-methoxypyridine (360 g, 1.42 mol) in tetrahydrofuran (800 mL), whereupon stirring was continued for 16 hours at 70 °C. The three reaction mixtures were combined at this point and filtered through diatomaceous earth. The filter pad was rinsed with ethyl acetate (3 x 500 mL), and the combined filtrates were concentrated *in vacuo,* while keeping the internal temperature below 40 °C. The residue was dissolved in ethyl acetate (2 L), washed sequentially with saturated aqueous ammonium chloride solution (500 mL) and saturated aqueous sodium chloride solution (500 mL), dried over sodium sulfate, filtered, and concentrated under reduced pressure at 40 °C. Chromatography on silica gel (Gradient: 1% to 8% ethyl acetate in petroleum ether) afforded **C53** (1.87 kg) as a yellow oil. By ¹H NMR analysis, this material was contaminated with dibenzyl propanedioate; a portion of it was used in the following step. LCMS *m*/*z* 410.1 [M+H]⁺. ¹H NMR (400 MHz, chloroform-d), product peaks only: δ 8.01 (d, *J* = 1.3 Hz, 1H), 7.40 - 7.25 (m, 10H, assumed; partially obscured by residual dibenzyl propanedioate), 6.83 (d, *J* = 4.8 Hz, 1H), 5.20 (AB quartet, *J*_{AB}= 12.2 Hz, Δv_{AB}= 11.9 Hz, 4H), 5.00 (s, 1H), 3.89 (s, 3H).

### Step 2. Synthesis of dibenzyl (5-fluoro-2-methoxypyridin-4-yl)(methyl)propanedioate (C54).

This reaction was carried out in two parallel batches. A solution of **C53** (from the previous step; 575 g, ≤1.31 mol) in acetonitrile (1.5 L) was stirred in an ice-water bath for 20 minutes, whereupon potassium carbonate (582 g, 4.21 mol) was added. Stirring was continued for an additional 10 minutes. lodomethane (302 g, 2.13 mol) was then added to the reaction mixture at 0 °C, and the reaction was allowed to proceed until LCMS analysis indicated conversion to **C54**. After the two reaction mixtures had been combined, they were filtered through diatomaceous earth, and the filter cake was washed with acetonitrile (2 x 1 L). The combined filtrates were concentrated at 40 °C and the residue was partitioned between ethyl acetate (2 L) and water (500 mL). The aqueous layer was extracted with ethyl acetate (2 x 1 L), and the combined organic layers were washed with saturated aqueous sodium chloride solution (1 L), dried over sodium sulfate, filtered, and concentrated under reduced pressure at 40 °C. The resulting crude product was dissolved in petroleum ether (1.5 L) and stirred at 0 °C for 2 hours; a solid was collected via filtration. The filtrate was concentrated *in vacuo,* and the residue was taken up in petroleum ether (500 mL), then cooled to 0 °C to provide additional solid, which was isolated via filtration. The two solids were combined, suspended in petroleum ether (800 mL), and stirred at 20 °C for 16 hours. Subsequent collection via filtration afforded **C54** as a yellow solid. Yield: 670 g, 1.58 mol, 60% over 2 steps. LCMS *m*/*z* 423.8 [M+H]⁺. ¹H NMR (400 MHz, chloroform-*d*) δ 7.94 (d, *J* = 2.6 Hz, 1H), 7.36 - 7.20 (m, 10H), 6.54 (d, *J* = 5.1 Hz, 1H), 5.18 (s, 4H), 3.87 (s, 3H), 1.85 (s, 3H).

### Step 3. Synthesis of 2-(5-fluoro-2-methoxypyridin-4-yl)propanoic acid (P16).

This reaction was carried out in four parallel batches. To a 25 °C solution of **C54** (200 g, 472 mmol) in ethyl acetate (1 L) was added 10% palladium on carbon (wet; 40 g). The mixture was degassed under vacuum and then purged with nitrogen; this evacuation-purge cycle was carried out a total of three times. The mixture was again degassed under vacuum and then purged with hydrogen; this evacuation-purge cycle was also carried out a total of three times. The mixture was hydrogenated (30 psi) at 50 °C for 16 hours, whereupon the four reaction mixtures were combined and filtered through a pad of diatomaceous earth, and the filtrate was concentrated *in vacuo* at 45 °C. Chromatography on silica gel (Gradient: 10% to 20% ethyl acetate in petroleum ether) provided **P16** as a white solid. Combined yield: 270 g, 1.36 mmol, 72%. LCMS *m*/*z* 199.7 [M+H]⁺. ¹H NMR (400 MHz, chloroform-d) δ 7.98 (d, *J* = 1.7 Hz, 1H), 6.70 (d, *J* = 4.9 Hz, 1H), 3.97 (q, *J* = 7.3 Hz, 1H), 3.89 (s, 3H), 1.53 (d, *J* = 7.3 Hz, 3H).

### Preparations P17 and P18

### (2R)-2-(5-Fluoro-2-methoxypyridin-4-yl)propanoic acid (P17) and (2S)-2-(5-Fluoro-2-methoxypyridin-4-yl)propanoic acid (P18)

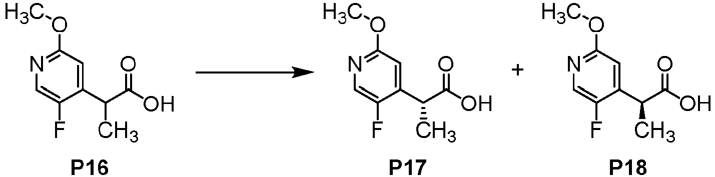

Separation of **P16** (700 g, 3.51 mol) into its component enantiomers was carried out by supercritical fluid chromatography (Column: Chiral Technologies Chiralpak AD-H, 50 x 250 mm, 5 µm; Mobile phase: 9:1 carbon dioxide / propan-2-ol; Flow rate: 250 mL/minute; Back pressure: 120 bar). The first-eluting enantiomer was designated as **P17,** and the second-eluting enantiomer as **P18**; both were isolated as solids. The indicated absolute stereochemistries for **P17** and **P18** were assigned on the basis of an X-ray crystal structure determination carried out on Example 7 (see below); **7** was synthesized using a sample of **P17** that was separated from **P18** using the same chromatographic conditions described above.

**P17** - Yield: 260 g, 1.30 mol, 37%. Retention time: 3.17 minutes (Analytical conditions. Column: Chiral Technologies Chiralpak AD-H, 4.6 x 250 mm, 5 µm; Mobile phase A: carbon dioxide; Mobile phase B: propan-2-ol; Gradient: 5% B for 1.00 minute, then 5% to 60% B over 8 minutes; Flow rate: 3.0 mL/minute; Back pressure: 120 bar).

**P18** - Yield: 400 g, 2.01 mol, 57%. Retention time: 3.36 minutes (Analytical conditions identical to those used for **P17**).

### First Alternate Preparation of P17

### (2R)-2-(5-Fluoro-2-methoxypyridin-4-yl)propanoic acid (P17)

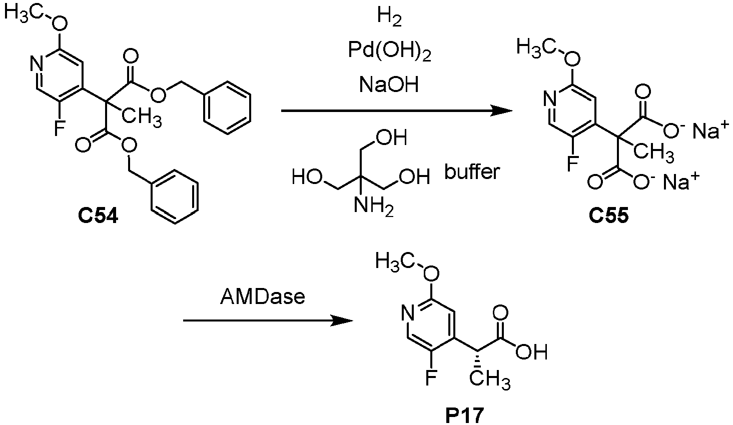

### Step 1. Synthesis of disodium (5-fluoro-2-methoxypyridin-4-yl)(methyl)propanedioate (C55).

A 1.0 M, pH 8.0 buffer solution was prepared in the following manner: a solution of 2-amino-2-(hydroxymethyl)propane-1,3-diol (Tris; 121 g, 1.00 mol) in water (900 mL) was adjusted to pH 8.0 by addition of hydrochloric acid (37.5 weight%, approximately 40 mL), and then brought to a volume of 1 L by addition of water.

A hydrogenation reactor was charged with palladium hydroxide on carbon (10%; 5.00 g). To this was added a solution of **C54** (50.0 g, 118 mmol) in toluene (50 mL, 1 volume); additional toluene (50 mL) was used to rinse the flask, and this was also added to the reaction mixture. A mixture of aqueous sodium hydroxide solution (2.0 M; 118 mL, 236 mmol), the pH 8.0 buffer solution described above (1.0 M; 250 mL, 250 mmol), and water (132 mL) was added, and the resulting mixture was purged with nitrogen (3.5 bar) followed by hydrogen (3.5 bar); this purging process was carried out a total of three times. After the mixture had been brought to 20 °C, stirring at 100 rpm, it was pressurized with hydrogen to 3.45 bar, whereupon the rate of stirring was increased to 750 rpm. After the hydrogenation had proceeded for 4 hours at 20 °C, the stirring rate was decreased to 250 rpm and the reaction was purged three times with nitrogen (3.5 bar). The catalyst was removed via filtration, and the reactor was rinsed with water (100 mL), which was then used to wash the filter cake. The aqueous phase of the combined filtrates (590 mL, pH 8.2), containing **C55**, was progressed directly to the following step. LCMS *m*/*z* 244.2 [M+H]⁺.

### Step 2. Synthesis of (2R)-2-(5-fluoro-2-methoxypyridin-4-yl)propanoic acid (P17).

A 2 L jacketed vessel (set to a 20 °C jacket temperature) with overhead stirrer was charged with **C55** (aqueous solution from the previous step; ≤118 mmol), and the stirring rate was set at 200 rpm. A solution of *Bordetella bronchiseptica* AMDase lyophilized cell-free extract powder (1.75 gm) [This aryl malonate decarboxylase (AMDase) from *Bordetella bronchiseptica* is a wild-type enzyme described in the literature with accession number Q05115, which was recombinantly expressed in *E*. *coli* and charged as a lyophilized cell-free extract powder. Literature references: S. K. Gaßmeyer et al., ChemCatChem, 2016, 8, 916 - 921; K. Okrasa et al., Angew. Chem. Int. Ed. 2009, 48, 7691 -7694] in water (17.5 mL) was then charged to the reactor, along with a water rinse of the enzyme vessel (5 mL). After 15 hours, the stirring speed was lowered to 100 rpm, and the pH of the reaction mixture was adjusted to pH 6.0 by sequential additions of hydrochloric acid (4.0 M; 5 mL portions, 38 mL). At this point, the mixture was stirred for 1.5 hours to allow off-gassing to subside, whereupon it was acidified to a pH of ≤2.0 via further addition of hydrochloric acid (4.0 M; total of 85 mL). *tert*-Butyl methyl ether (300 mL) was added and stirring was continued at 200 rpm for 15 minutes. The mixture was then filtered through diatomaceous earth (25 g), using a Büchner funnel and filter paper; the reactor was rinsed with *tert*-butyl methyl ether (100 mL), which was then used to wash the filter cake. The aqueous layer of the combined filtrates was extracted in the same manner with *tert*-butyl methyl ether (300 mL). The combined organic layers were dried over sodium sulfate (50 g) and filtered; the filter cake was washed with *tert*-butyl methyl ether (25 mL). The combined filtrates were concentrated *in vacuo* at 30 °C to provide an oil, which solidified under vacuum drying overnight to afford **P17** as an off-white solid. Yield: 18.88 g, 94.8 mmol, 80% over 2 steps. ¹H NMR (400 MHz, chloroform-d) δ 11.4 - 10.3 (br s, 1H), 7.98 (d, *J* = 1.6 Hz, 1H), 6.70 (d, *J* = 4.9 Hz, 1H), 3.97 (q, *J* = 7.2 Hz, 1H), 3.89 (s, 3H), 1.53 (d, *J* = 7.2 Hz, 3H).

Combination of **P17** from this step (18.6 g, 93.4 mmol) and **P17** (24.9 g, 125 mmol) from a similar reaction of **C55** with AMDase afforded a slightly pink solid, with an enantiomeric excess of 98.5%. ¹H NMR (400 MHz, methanol-*d*₄) δ 7.94 (d, *J* = 1.9 Hz, 1H), 6.74 (d, *J* = 5.0 Hz, 1H), 3.93 (q, *J* = 7.3 Hz, 1H), 3.87 (s, 3H), 1.48 (d, *J* = 7.3 Hz, 3H). Retention time: 2.86 minutes [Column: Chiral Technologies Chiralpak IG, 4.6 x 250 mm, 5 µm; Mobile phase A: carbon dioxide; Mobile phase B: methanol; Gradient: 5% B for 1 minute, then 5% to 60% B over 7 minutes; Flow rate: 3 mL/minute; Back pressure: 120 bar].

The absolute configuration of this sample of **P17** was confirmed via comparison to the sample of **P17** isolated from Preparations P17 and P18:
Retention time for **P17** from Preparations P17 and P18: 2.86 minutes.
Retention time for **P17** from First Alternate Preparation of **P17**: 2.86 minutes.
Retention times for a racemic mixture of **P17** and **P18**: 2.87 and 3.16 minutes.
These three analyses were run using the same analytical method: [Column: Chiral Technologies Chiralpak IG, 4.6 x 250 mm, 5 µm; Mobile phase A: carbon dioxide; Mobile phase B: methanol; Gradient: 5% B for 1 minute, then 5% to 60% B over 7 minutes; Flow rate: 3 mL/minute; Back pressure: 120 bar].

### Second Alternate Preparation of P17

### (2R)-2-(5-Fluoro-2-methoxypyridin-4-yl)propanoic acid (P17)

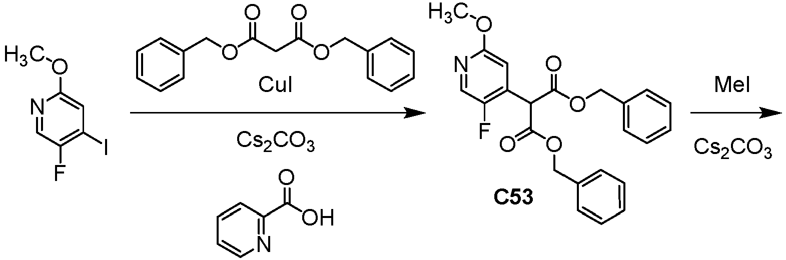

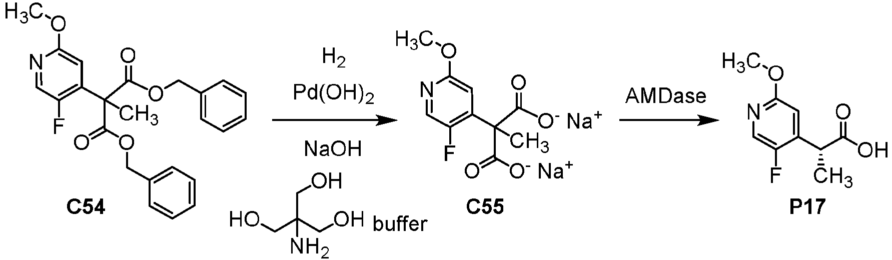

### Step 1. Synthesis of dibenzyl (5-fluoro-2-methoxypyridin-4-yl)propanedioate (C53).

A mixture of pyridine-2-carboxylic acid (24.6 g, 0.200 mol), copper(I) iodide (19.1 g, 0.100 mol), and cesium carbonate (977 g, 3.00 mol) in tetrahydrofuran (1.26 L; 5 volumes) was heated to an internal temperature of 60 °C to 70 °C, whereupon a solution of 5-fluoro-4-iodo-2-methoxypyridine (253 g, 1.00 mol) and dibenzyl propanedioate (426 g, 1.50 mol) in tetrahydrofuran (250 mL, 1 volume) was added. After the reaction mixture had been heated at 60 °C to 70 °C for approximately 3 to 6 hours, it was allowed to cool to 15 °C to 30 °C and filtered through diatomaceous earth (250 g). The filter cake was washed with tetrahydrofuran (500 mL, 2 volumes) and the combined filtrates, containing **C53**, were used directly in the following step. Representative ¹H NMR (500 MHz, chloroform-*d*) δ 8.00 (d, *J* = 1.3 Hz, 1H), 7.40 - 7.24 (m, 10H, assumed; partially obscured by residual dibenzyl propanedioate), 6.82 (d, *J* = 4.8 Hz, 1H), 5.20 (AB quartet, *J*_{AB}= 12.3 Hz, Δ*ν*_{AB}= 14.9 Hz, 4H), 4.99 (s, 1H), 3.88 (s, 3H).

### Step 2. Synthesis of dibenzyl (5-fluoro-2-methoxypyridin-4-yl)(methyl)propanedioate (C54).

Iodomethane (284 g, 2.00 mol) was slowly added to a 10 °C to 20 °C mixture of cesium carbonate (977 g, 3.00 mol) and a solution of **C53** (from the previous step, solution in tetrahydrofuran; ≤1.00 mol). After the reaction mixture had been stirred at 10 °C to 20 °C for approximately 10 to 12 hours, it was filtered through diatomaceous earth (250 g). The filter cake was washed with tetrahydrofuran (500 mL, 1.2 volumes), and the combined filtrates were concentrated to 1 to 2 volumes. The resulting mixture was diluted with propan-2-yl acetate (1.25 L, 3.1 volumes), washed sequentially with water (750 mL, 1.8 volumes), aqueous ammonium chloride solution (20%; 750 mL), and aqueous sodium chloride solution (20%; 750 mL), and concentrated *in vacuo.* The remaining solvent was exchanged with heptane, and precipitation was allowed to proceed from heptane (2 to 3 volumes) at 15 °C to 25 °C. The resulting solid was collected via filtration and triturated with a mixture of heptane (450 mL) and propan-2-yl acetate (50 mL) to afford **C54** as a solid. Three batches of the chemistry in steps 1 and 2 were carried out, and the final lots of **C54** were combined. Combined yield: 675 g, 1.59 mol, approximately 53% over 2 steps. Representative ¹H NMR (500 MHz, DMSO-*d*₆) δ 8.15 (d, *J* = 2.0 Hz, 1H), 7.39 - 7.21 (m, 10H), 6.75 (d, *J* = 5.0 Hz, 1H), 5.21 (s, 4H), 3.81 (s, 3H), 1.81 (s, 3H).

### Step 3. Synthesis of disodium (5-fluoro-2-methoxypyridin-4-yl)(methyl)propanedioate (C55).

A buffer solution [pH 8.0; 2-amino-2-(hydroxymethyl)propane-1,3-diol (Tris; 121 g, 1.00 mol) and concentrated hydrochloric acid (46 mL, 0.23 volumes) in water (1 L, 5 volumes)] and palladium hydroxide on carbon (10%, 20 g) were added to a 15 °C to 25 °C mixture of **C54** (200 g, 0.472 mol) in toluene (400 mL, 2 volumes). A solution of sodium hydroxide (38.8 g, 0.970 mol) in water (1 L, 5 volumes) was added, whereupon the mixture was stirred for approximately 10 to 20 minutes. After the reactor had been purged with nitrogen, then purged with hydrogen, the reaction mixture was stirred at 15 °C to 30 °C under a bag of hydrogen (approximately 10 L), until HPLC analysis indicated ≤0.5% of **C54** was present (approximately 22 hours) (Retention time: 11.44 minutes. HPLC conditions. Column: Agilent Technologies ZORBAX Eclipse Plus C18, 4.6 x 100 mm, 3.5 µm; Mobile phase A: 0.1% phosphoric acid in water; Mobile phase B: acetonitrile; Gradient: 5% B for 3 minutes, then 5% to 100% B over 9 minutes, then 100% B for 3 minutes; Flow rate: 1.5 mL/minute). The reaction mixture was filtered, and the filter cake was washed with water (2.6 volumes); the aqueous layer of the filtrate, containing **C55**, was taken directly to the following step.

### Step 4. Synthesis of (2R)-2-(5-fluoro-2-methoxypyridin-4-yl)propanoic acid (P17).

A mixture of AMDase (7 g) in water (70 mL, 0.35 volumes) and **C55** (from the previous step, as a solution in water, ≤0.472 mol) was stirred at 15 °C to 30 °C until HPLC analysis indicated that ≤0.5% of **C55** was present (approximately 16 hours) [Retention time: 5.80 minutes. HPLC conditions identical to those described in Step 3, Synthesis of disodium (5-fluoro-2-methoxypyridin-4-yl)(methyl)propanedioate (**C55**)]. Hydrochloric acid (4.0 M) was then slowly added until the pH of the reaction mixture reached 6.0, whereupon stirring was continued for 1.5 hours. The pH was then adjusted to ≤2.0 (range, 1.5 to 2.0) by further addition of hydrochloric acid (4.0 M). After addition of *tert*-butyl methyl ether (1.2 L, 6 volumes), the mixture was filtered through diatomaceous earth (100 g), and the aqueous phase of the filtrate was extracted with *tert*-butyl methyl ether (800 mL, 4 volumes). The combined organic layers were washed with aqueous sodium chloride solution (15%; 600 mL, 3 volumes), and concentrated to 2 to 2.5 volumes at a temperature of ≤45 °C and a pressure of ≤ -0.08 MPa. n-Heptane (600 mL, 3 volumes) was added, and the mixture was concentrated to 3 to 5 volumes at a temperature of ≤45 °C and a pressure of ≤ -0.08 MPa; this heptane dilution / concentration was carried out a total of 3 times. After the resulting mixture had been stirred at 0 °C to 10 °C for approximately 1 to 2 hours, the precipitate was collected via filtration, providing **P17** as an off-white solid with an enantiomeric excess of 99.8%. Yield: 80.0 g, 0.402 mol, 85% over 2 steps. Representative ¹H NMR (500 MHz, chloroform-d) δ 11.68 (v br s, 1H), 7.99 (br s, 1H), 6.70 (d, *J* = 4.9 Hz, 1H), 3.97 (q, *J =* 7.2 Hz, 1H), 3.88 (s, 3H), 1.52 (d, *J* = 7.3 Hz, 3H).

### Preparation P19

### 2-[3-(Difluoromethoxy)-5-methoxyphenyl]propanoic acid (P19)

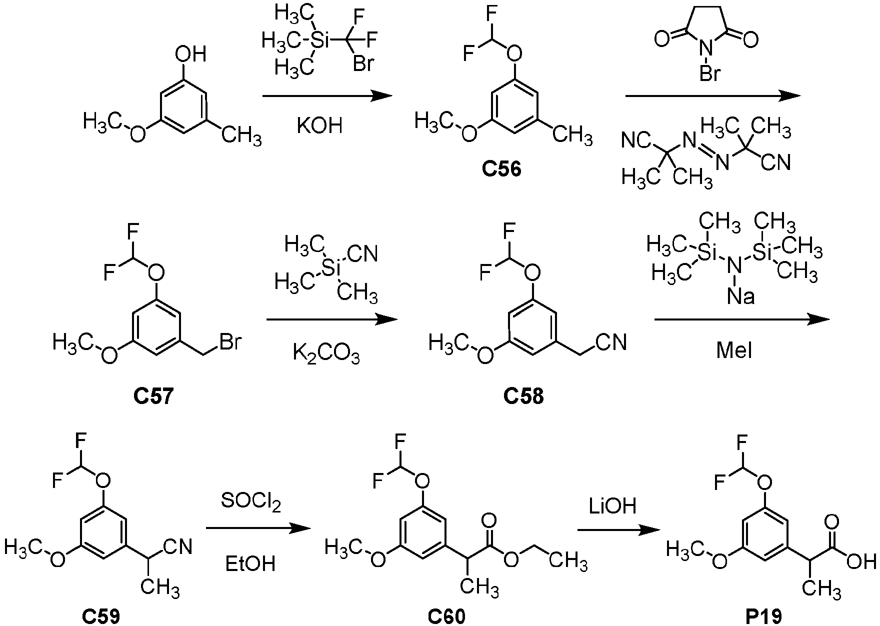

### Step 1. Synthesis of 1-(difluoromethoxy)-3-methoxy-5-methylbenzene (C56).

Aqueous potassium hydroxide solution (20%; 60.9 g, 217 mmol) and [bromo(difluoro)methyl](trimethyl)silane (11.3 mL, 72.7 mmol) were sequentially added to a 0 °C solution of 3-methoxy-5-methylphenol (5.00 g, 36.2 mmol) in dichloromethane (50 mL). After the reaction mixture had been stirred at 0 °C for 4.5 hours, it was diluted with water (50 mL) and extracted with dichloromethane (3 x 100 mL). The combined organic layers were washed with saturated aqueous sodium chloride solution, dried over sodium sulfate, filtered, concentrated *in vacuo,* and purified via chromatography on silica gel (Gradient: 0% to 5% ethyl acetate in petroleum ether), affording **C56** as a colorless oil. Yield: 6.27 g, 33.3 mmol, 92%. ¹H NMR (400 MHz, methanol-*d*₄) δ 6.76 (t, *J*_{HF} = 74.4 Hz, 1H), 6.60 (br s, 1H), 6.53 (br s, 1H), 6.49 - 6.46 (m, 1H), 3.76 (s, 3H), 2.30 (s, 3H).

### Step 2. Synthesis of 1-(bromomethyl)-3-(difluoromethoxy)-5-methoxybenzene (C57).

A mixture of **C56** (3.00 g, 15.9 mmol), 2,2'-azobisisobutyronitrile (262 mg, 1.60 mmol), and N-bromosuccinimide (2.84 g, 15.9 mmol) in tetrachloromethane (90 mL) was stirred at 80 °C for 8 hours. Concentration *in vacuo* provided **C57** as a yellow oil (4.0 g), which was used in the following step. ¹H NMR (400 MHz, methanol-*d*₄), characteristic product peaks: δ 6.84 (s, 1H), 6.77 (s, 1H), 6.63 - 6.60 (m, 1H), 4.50 (s, 2H), 3.81 (s, 3H).

### Step 3. Synthesis of [3-(difluoromethoxy)-5-methoxyphenyl]acetonitrile (C58).

To a solution of **C57** (from the previous step; 4.0 g, ≤15 mmol) in acetonitrile (150 mL) were sequentially added potassium carbonate (3.11 g, 22.5 mmol) and trimethylsilyl cyanide (2.2 g, 22 mmol). The resulting mixture was stirred at 80 °C for 16 hours, at which time LCMS analysis indicated the presence of **C58**: LCMS *m*/*z* 214.1 [M+H]⁺. The reaction mixture was concentrated under reduced pressure, diluted with water (50 mL), and extracted with ethyl acetate (3 x 50 mL). The combined organic layers were washed with saturated aqueous sodium chloride solution, dried over sodium sulfate, filtered, concentrated *in vacuo,* and purified using silica gel chromatography (Gradient: 0% to 30% ethyl acetate in petroleum ether) to afford **C58** as a yellow oil. Yield: 1.20 g, 5.63 mmol, 35% over 2 steps. ¹H NMR (400 MHz, methanol-*d*₄) δ 6.84 (t, *J*_{HF} = 73.9 Hz, 1H), 6.81 (br s, 1H), 6.73 (br s, 1H), 6.68 - 6.66 (m, 1H), 3.89 (s, 2H), 3.82 (s, 3H).

### Step 4. Synthesis of 2-[3-(difluoromethoxy)-5-methoxyphenyl]propanenitrile (C59).

To a -78 °C solution of **C58** (3.00 g, 14.1 mmol) in tetrahydrofuran (150 mL) was added a solution of sodium bis(trimethylsilyl)amide (2 M; 8.4 mL, 17 mmol). After the reaction mixture had been stirred at -78 °C for 1 hour, iodomethane (0.876 mL, 14.1 mmol) was added and stirring was continued at -78 °C for 2 hours, whereupon LCMS analysis indicated conversion to **C59**: LCMS *m*/*z* 228.1 [M+H]⁺. The reaction was quenched by addition of saturated aqueous ammonium chloride solution (10 mL), and the resulting mixture was extracted with ethyl acetate (3 x 50 mL). The combined organic layers were washed with saturated aqueous sodium chloride solution, dried over sodium sulfate, filtered, concentrated in vacuo, and purified via silica gel chromatography (Gradient: 0% to 5% ethyl acetate in petroleum ether), providing **C59** as a yellow oil. Yield: 1.00 g, 4.40 mmol, 31%. LCMS *m*/*z* 228.1 [M+H]⁺. ¹H NMR (400 MHz, methanol-*d*₄) δ 6.85 (t, *J*_{HF} = 73.9 Hz, 1H), 6.84 - 6.82 (m, 1H), 6.77 - 6.74 (m, 1H), 6.69 - 6.66 (m, 1H), 4.11 (q, *J* = 7.2 Hz, 1H), 3.82 (s, 3H), 1.60 (d, *J* = 7.3 Hz, 3H).

### Step 5. Synthesis of ethyl 2-[3-(difluoromethoxy)-5-methoxyphenyl]propanoate (C60).

Thionyl chloride (5.3 mL, 73 mmol) was added in a drop-wise manner to a 0 °C solution of **C59** (900 mg, 3.96 mmol) in ethanol (40 mL). The reaction mixture was stirred at 85 °C for 16 hours, whereupon it was diluted with water (50 mL) and extracted with ethyl acetate (3 x 30 mL). The combined organic layers were washed with saturated aqueous sodium chloride solution, dried over sodium sulfate, filtered, and concentrated *in vacuo.* Silica gel chromatography (Gradient: 0% to 6% ethyl acetate in petroleum ether) provided **C60** as a yellow oil. Yield: 700 mg, 2.55 mmol, 64%. LCMS *m*/*z* 275.1 [M+H]⁺. ¹H NMR (400 MHz, methanol-*d*₄) δ 6.80 (t, *J*_{HF} = 74.2 Hz, 1H), 6.74 - 6.71 (m, 1H), 6.65 (br s, 1H), 6.59 (dd, *J* = 2.2, 2.2 Hz, 1H), 4.19 - 4.05 (m, 2H), 3.79 (s, 3H), 3.72 (q, *J* = 7.2 Hz, 1H), 1.44 (d, *J* = 7.2 Hz, 3H), 1.20 (t, *J* = 7.1 Hz, 3H).

### Step 6. Synthesis of 2-[3-(difluoromethoxy)-5-methoxyphenyl]propanoic acid (P19).

To a solution of **C60** (700 mg, 2.55 mmol) in tetrahydrofuran (30 mL) was added a solution of lithium hydroxide monohydrate (535 mg, 12.8 mmol) in water (10 mL). After the reaction mixture had been stirred at 25 °C for 16 hours, it was concentrated *in vacuo,* diluted with water (20 mL), and washed with dichloromethane (3 x 25 mL). These organic layers were discarded. The aqueous layer was adjusted to a pH of approximately 2 using 2 M hydrochloric acid; it was then extracted with dichloromethane (3 x 25 mL). The combined organic layers were washed with saturated aqueous sodium chloride solution (10 mL), dried over sodium sulfate, filtered, and concentrated under reduced pressure, affording **P19** as a yellow oil. Yield: 628 mg, 2.55 mmol, quantitative. LCMS *m*/*z* 247.1 [M+H]⁺. ¹H NMR (400 MHz, methanol-*d*₄) δ 6.79 (t, *J*_{HF} = 74.2 Hz, 1H), 6.77 - 6.73 (m, 1H), 6.69 - 6.66 (m, 1H), 6.59 (dd, *J* = 2.2*,* 2.2 Hz, 1H), 3.79 (s, 3H), 3.69 (q, *J* = 7.2 Hz, 1H), 1.44 (d, *J* = 7.2 Hz, 3H).

### Preparation P20

### Lithium 2-(6-methoxy-2-methylpyrimidin-4-yl)propanoate (P20)

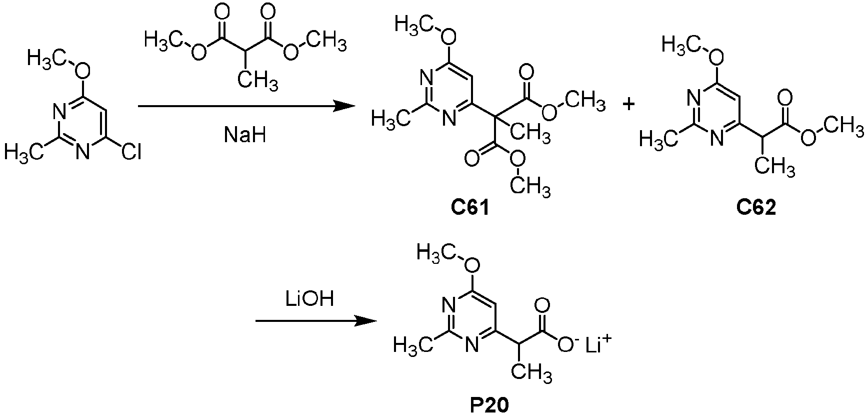

### Step 1. Synthesis of dimethyl (6-methoxy-2-methylpyrimidin-4-yl)(methyl)propanedioate (C61) and methyl 2-(6-methoxy-2-methylpyrimidin-4-yl)propanoate (C62).

Sodium hydride (60% in mineral oil; 1.14 g, 28.5 mmol) was added to a solution of dimethyl methylpropanedioate (5.53 g, 37.8 mmol) in *N,N*-dimethylformamide (25 mL). After 30 minutes, 4-chloro-6-methoxy-2-methylpyrimidine (3.00 g, 18.9 mmol) was added, whereupon the reaction mixture was heated at 100 °C for 16 hours. It was then diluted with water (150 mL) and extracted with ethyl acetate (3 x 50 mL); the combined organic layers were washed with saturated aqueous sodium chloride solution, dried over sodium sulfate, filtered, concentrated *in vacuo,* and purified via silica gel chromatography (Gradient: 0% to 10% ethyl acetate in petroleum ether), affording the product (2.60 g) as a yellow oil. On the basis of NMR and LCMS analysis, this was judged to be an impure mixture of **C61** and **C62**, which was taken directly into the following step. LCMS *m*/*z* 211.1 [M+H]⁺ and 269.2 [M+H]⁺. ¹H NMR (400 MHz, methanol-*d*₄), characteristic peaks: δ 6.68 (s), 6.60 (s), 3.96 (s), 3.94 (s), 3.81 (q, *J* = 7.2 Hz), 3.75 (s), 3.68 (s), 2.54 (s), 2.52 (s), 1.79 (s), 1.47 (d, *J* = 7.3 Hz).

### Step 2. Synthesis of lithium 2-(6-methoxy-2-methylpyrimidin-4-yl)propanoate (P20).

A solution of **C61** and **C62** (from the previous step; 2.60 g, ≤18.9 mmol) and lithium hydroxide monohydrate (1.22 g, 29.1 mmol) in a mixture of tetrahydrofuran (45 mL) and water (15 mL) was stirred at 45 °C for 3 hours. After the reaction mixture had been concentrated *in vacuo,* the residue was subjected to lyophilization, providing **P20** as a white solid. Yield: 2.3 g, 11 mmol, 58% over 2 steps. LCMS *m*/*z* 197.1 [M+H]⁺. ¹H NMR (400 MHz, methanol-*d*₄) δ 6.66 (s, 1H), 3.94 (s, 3H), 3.61 (q, *J* = 7.3 Hz, 1H), 2.53 (s, 3H), 1.44 (d, *J* = 7.2 Hz, 3H).

### Preparation P21

### 2-[2-(Difluoromethoxy)-6-methoxypyridin-4-yl]propanoic acid (P21)

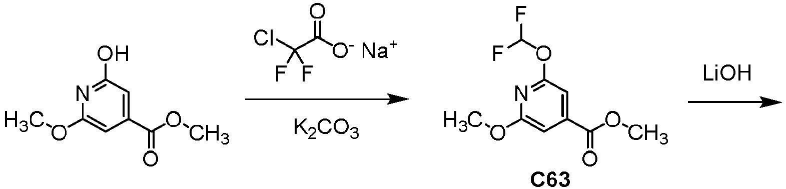

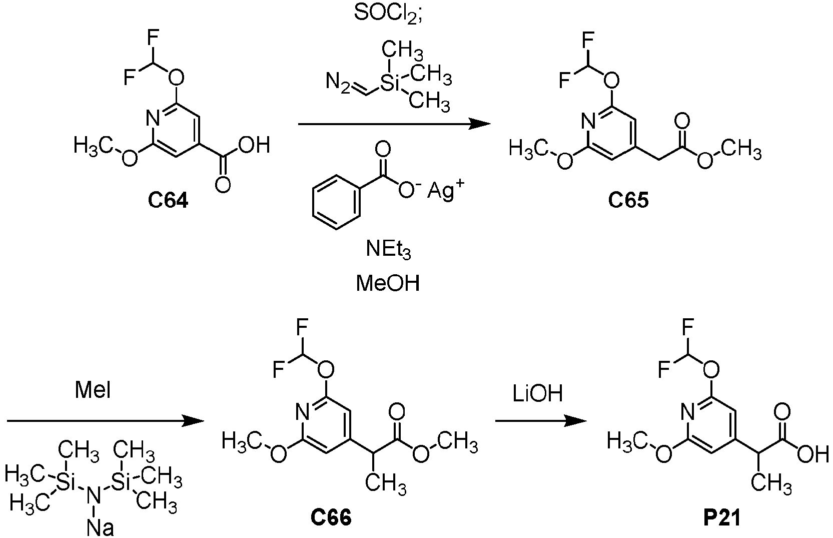

### Step 1. Synthesis of methyl 2-(difluoromethoxy)-6-methoxypyridine-4-carboxylate (C63).

To a 25 °C solution of methyl 2-hydroxy-6-methoxypyridine-4-carboxylate (900 mg, 4.91 mmol) in *N,N*-dimethylformamide (15 mL) were added sodium chloro(difluoro)acetate (1.12 g, 7.35 mmol) and potassium carbonate (1.36 g, 9.84 mmol), whereupon the reaction mixture was heated at 50 °C for 16 hours. After addition of water (100 mL), the mixture was extracted with ethyl acetate (3 x 50 mL); the combined organic layers were washed with saturated aqueous sodium chloride solution, dried over sodium sulfate, filtered, and concentrated *in vacuo.* Chromatography on silica gel (Gradient: 0% to 8% ethyl acetate in petroleum ether) provided **C63** as a colorless oil. Yield: 720 mg, 3.09 mmol, 63%. LCMS *m*/*z* 234.1 [M+H]⁺. ¹H NMR (400 MHz, chloroform-d) δ 7.39 (t, *J*_{HF} = 73.0 Hz, 1H), 7.10 (br s, 1H), 7.00 (br s, 1H), 3.94 (s, 3H), 3.93 (s, 3H).

### Step 2. Synthesis of 2-(difluoromethoxy)-6-methoxypyridine-4-carboxylic acid (C64).

To a solution of **C63** (1.10 g, 4.72 mmol) in tetrahydrofuran (30 mL) was added a solution of lithium hydroxide monohydrate (990 mg, 23.6 mmol) in water (10 mL). After the reaction mixture had been stirred at 25 °C for 5 hours, it was concentrated *in vacuo.* The residue was diluted with water (20 mL) and extracted with dichloromethane (3 x 20 mL). The organic extracts were discarded, and the aqueous layer was adjusted to a pH of approximately 2 by addition of 2 M hydrochloric acid. The resulting mixture was extracted with dichloromethane (3 x 20 mL); the combined organic layers were washed with saturated aqueous sodium chloride solution (20 mL), dried over sodium sulfate, filtered, and concentrated *in vacuo* to afford **C64** as a white solid. Yield: 980 mg, 4.47 mmol, 95%. LCMS *m*/*z* 220.1 [M+H]⁺. ¹H NMR (400 MHz, chloroform-d) δ 7.41 (t, *J*_{HF} = 72.8 Hz, 1H), 7.15 (d, *J* = 1.1 Hz, 1H), 7.05 (d, *J* = 1.0 Hz, 1H), 3.95 (s, 3H).

### Step 3. Synthesis of methyl [2-(difluoromethoxy)-6-methoxypyridin-4-yl]acetate (C65).

A solution of **C64** (980 mg, 4.47 mmol) in thionyl chloride (6.49 mL, 89.0 mmol) was stirred at 70 °C for 2.5 hours, whereupon it was concentrated under reduced pressure. After the resulting acyl chloride had been dissolved in a mixture of tetrahydrofuran (8 mL) and acetonitrile (8 mL), it was cooled to 0 °C and treated with freshly distilled triethylamine (0.87 mL, 6.2 mmol), followed by (diazomethyl)(trimethyl)silane (2 M solution in diethyl ether; 3.35 mL, 6.70 mmol). The reaction mixture was stirred at 0 °C for 8 hours, whereupon it was diluted with diethyl ether (25 mL) and washed sequentially with 10% aqueous citric acid solution (5 mL), saturated aqueous sodium bicarbonate solution (15 mL), and saturated aqueous sodium chloride solution (25 mL). The organic layer was dried over sodium sulfate, filtered, and concentrated under reduced pressure to provide the crude diazoketone. This material was suspended in methanol (10 mL) in an ultrasonic bath; a solution of silver benzoate (512 mg, 2.24 mmol) in triethylamine (1.86 mL, 13.3 mmol) was gradually added at room temperature while the reaction mixture was sonicated. After 30 minutes, volatiles were removed *in vacuo,* and the residue was purified using chromatography on silica gel (Gradient: 0% to 10% ethyl acetate in petroleum ether) to provide **C65** as a colorless oil. Yield: 340 mg, 1.38 mmol, 31%. LCMS *m*/*z* 248.0 [M+H]⁺. ¹H NMR (400 MHz, chloroform-d) δ 7.40 (t, *J*_{HF} = 73.4 Hz, 1H), 6.45 (br s, 1H), 6.40 (br s, 1H), 3.88 (s, 3H), 3.71 (s, 3H), 3.57 (s, 2H).

### Step 4. Synthesis of methyl 2-[2-(difluoromethoxy)-6-methoxypyridin-4-yl]propanoate (C66).

To a -78 °C solution of **C65** (230 mg, 0.930 mmol) in tetrahydrofuran (20 mL) was added sodium bis(trimethylsilyl)amide (2 M solution in tetrahydrofuran; 0.56 mL, 1.1 mmol), and the reaction mixture was stirred at -78 °C for 1 hour. Iodomethane (57.9 µL, 0.93 mmol) was then added and stirring was continued for 2 hours at -78 °C. After addition of saturated aqueous ammonium chloride solution (10 mL), the mixture was combined with a similar reaction carried out using **C65** (100 mg, 0.405 mmol) and extracted with ethyl acetate (3 x 20 mL). The combined organic layers were washed with saturated aqueous sodium chloride solution, dried over sodium sulfate, filtered, and concentrated *in vacuo;* silica gel chromatography (Gradient: 0% to 4% ethyl acetate in petroleum ether) afforded **C66** as a colorless oil. Combined yield: 150 mg, 0.574 mmol, 43%. LCMS *m*/*z* 262.1 [M+H]⁺. ¹H NMR (400 MHz, methanol-*d*₄) δ 7.51 (t, *J*_{HF} = 73.3 Hz, 1H), 6.50 (br d, *J* = 1 Hz, 1H), 6.43 (br d, *J* = 1 Hz, 1H), 3.88 (s, 3H), 3.78 (q, *J* = 7.2 Hz, 1H), 3.68 (s, 3H), 1.44 (d, *J* = 7.2 Hz, 3H).

### Step 5. Synthesis of 2-[2-(difluoromethoxy)-6-methoxypyridin-4-yl]propanoic acid (P21).

A solution of lithium hydroxide monohydrate (125 mg, 2.98 mmol) in water (3.0 mL) was added to a solution of **C66** (130 mg, 0.498 mmol) in tetrahydrofuran (9.0 mL), and the reaction mixture was stirred at 25 °C for 2 hours. After concentration of the reaction mixture *in vacuo,* the residue was diluted with water (10 mL) and washed with dichloromethane (3 x 15 mL). The aqueous layer was then adjusted to a pH of approximately 5 by addition of 2 M hydrochloric acid, and extracted with dichloromethane (3 x 15 mL); these 3 organic layers were combined and concentrated *in vacuo* to provide **P21** as a colorless oil. Yield: 101 mg, 0.409 mmol, 82%. LCMS *m*/*z* 248.0 [M+H]⁺. ¹H NMR (400 MHz, methanol-*d*₄) δ 7.51 (t, *J*_{HF} = 73.3 Hz, 1H), 6.53 (br d, *J* = 1.1 Hz, 1H), 6.46 (br d, *J* = 1.1 Hz, 1H), 3.88 (s, 3H), 3.72 (q, *J* = 7.1 Hz, 1H), 1.44 (d, *J* = 7.2 Hz, 3H).

### Preparation P22

### 2-Fluoro-2-(2-methoxypyridin-4-yl)propanoic acid (P22)

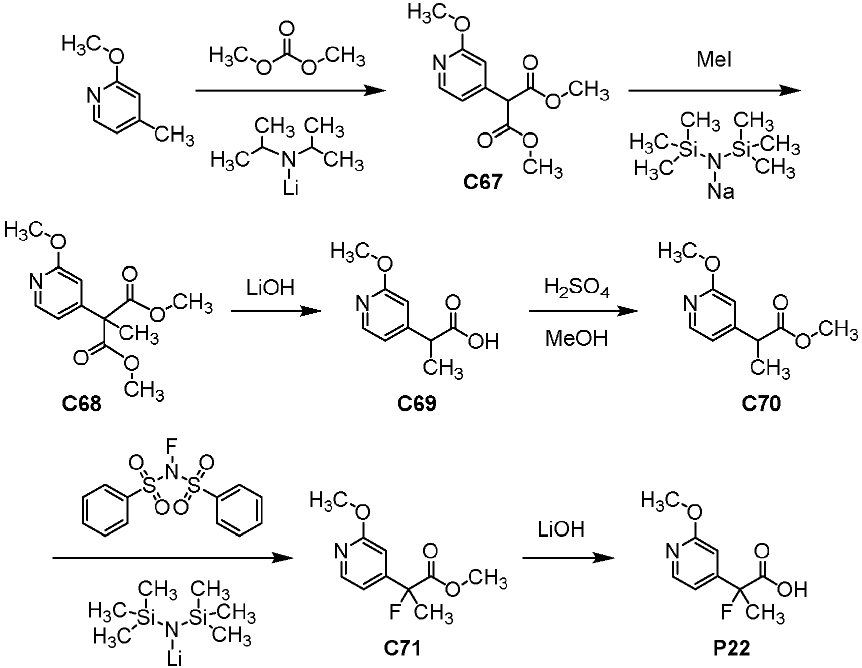

### Step 1. Synthesis of dimethyl (2-methoxypyridin-4-yl)propanedioate (C67).

To a -10 °C solution of 2-methoxy-4-methylpyridine (5.00 g, 40.6 mmol) in tetrahydrofuran (30 mL) was added lithium diisopropylamide (2 M solution in tetrahydrofuran; 81.2 mL, 162 mmol). After the reaction mixture had been stirred at -10 °C for 1.5 hours, dimethyl carbonate (14.6 g, 162 mmol) was added and stirring was continued at -10 °C for 1.5 hours. The reaction mixture was then warmed to 25 °C and allowed to stir for 16 hours, whereupon it was quenched by addition of aqueous ammonium chloride solution. The resulting mixture was extracted with ethyl acetate (3 x 30 mL), and the combined organic layers were dried over sodium sulfate, filtered, and concentrated *in vacuo.* Purification via chromatography on silica gel (Gradient: 0% to 20% ethyl acetate in petroleum ether) provided **C67** as a yellow oil. Yield: 4.92 g, 20.6 mmol, 51%. LCMS *m*/*z* 240.1 [M+H]⁺. ¹H NMR (400 MHz, chloroform-d) δ 8.17 (d, *J* = 5.0 Hz, 1H), 6.95 (d, *J* = 4.8 Hz, 1H), 6.80 (s, 1H), 4.59 (s, 1H), 3.96 (s, 3H), 3.77 (s, 6H).

Also obtained from the chromatographic purification was the product of mono-acylation, methyl (2-methoxypyridin-4-yl)acetate. Yield: 1.29 g, 7.12 mmol, 18%. LCMS *m*/*z* 182.1 [M+H]⁺. ¹H NMR (400 MHz, chloroform-d) δ 8.11 (br d, *J* = 5.3 Hz, 1H), 6.81 (dd, *J* = 5.4, 1.5 Hz, 1H), 6.68 - 6.66 (m, 1H), 3.93 (s, 3H), 3.71 (s, 3H), 3.57 (s, 2H).

### Step 2. Synthesis of dimethyl (2-methoxypyridin-4-yl)(methyl)propanedioate (C68).

Sodium bis(trimethylsilyl)amide (2 M solution in tetrahydrofuran; 14.0 mL, 28.0 mmol) was added to a -78 °C solution of **C67** (4.47 g, 18.7 mmol) in tetrahydrofuran (30 mL). After the reaction mixture had been stirred at -78 °C for 1 hour, iodomethane (1.40 mL, 22.5 mmol) was added. The reaction mixture was then warmed to -40 °C, stirred for 2 hours, warmed to 25 °C, and stirred for a further 16 hours, whereupon it was quenched with aqueous ammonium chloride solution. The resulting mixture was extracted with ethyl acetate (2 x 30 mL), and the combined organic layers were dried over sodium sulfate, filtered, and concentrated *in vacuo.* Silica gel chromatography (Gradient: 0% to 10% ethyl acetate in petroleum ether) afforded **C68** as a yellow oil. Yield: 3.29 g, 13.0 mmol, 70%. LCMS *m*/*z* 254.1 [M+H]⁺. ¹H NMR (400 MHz, chloroform-d) δ 8.15 (d, *J* = 5.5 Hz, 1H), 6.88 (br d, *J* = 5.5 Hz, 1H), 6.74 (br s, 1H), 3.95 (s, 3H), 3.78 (s, 6H), 1.83 (s, 3H).

### Step 3. Synthesis of 2-(2-methoxypyridin-4-yl)propanoic acid (C69).

A solution of **C68** (3.28 g, 13.0 mmol) and lithium hydroxide (1.24 g, 51.8 mmol) in a mixture of tetrahydrofuran (20 mL) and water (10 mL) was stirred at 45 °C for 5 hours. LCMS analysis indicated conversion to **C69**: LCMS *m*/*z* 182.1 [M+H]⁺, and the reaction mixture was concentrated *in vacuo,* providing **C69** as a white solid (2.40 g). This material was used directly in the following step.

### Step 4. Synthesis of methyl 2-(2-methoxypyridin-4-yl)propanoate (C70).

A mixture of **C69** (from the previous step; 2.40 g, ≤13.0 mmol) and sulfuric acid (2.5 mL) in methanol (25 mL) was stirred at 60 °C for 16 hours. The reaction mixture was then concentrated *in vacuo,* washed with aqueous sodium bicarbonate solution, and extracted with ethyl acetate (2 x 20 mL). The combined organic layers were dried over sodium sulfate, filtered, and concentrated under reduced pressure to afford **C70** as a colorless oil. Yield: 1.56 g, 7.99 mmol, 61% over 2 steps. LCMS *m*/*z* 196.2 [M+H]⁺. ¹H NMR (400 MHz, chloroform-d) δ 8.10 (d, *J* = 5.4 Hz, 1H), 6.81 (dd, *J* = 5.4, 1.5 Hz, 1H), 6.67 (br s, 1H), 3.93 (s, 3H), 3.67 (s, 3H), 3.66 (q, *J* = 7.1 Hz, 1H), 1.47 (d, *J* = 7.2 Hz, 3H).

### Step 5. Synthesis of methyl 2-fluoro-2-(2-methoxypyridin-4-yl)propanoate (C71).

To a -78 °C solution of **C70** (500 mg, 2.56 mmol) in tetrahydrofuran (13 mL) was added a solution of lithium bis(trimethylsilyl)amide in tetrahydrofuran (1 M; 3.33 mL, 3.33 mmol). After the reaction mixture had been stirred at -78 °C for 30 minutes, a solution of N-(benzenesulfonyl)-*N*-fluorobenzenesulfonamide (969 mg, 3.07 mmol) in tetrahydrofuran (2 mL) was added. The reaction mixture was stirred at -10 °C for 3 hours, whereupon it was quenched with aqueous ammonium chloride solution and extracted with ethyl acetate (3 x 20 mL). The combined organic layers were dried over sodium sulfate, filtered, and concentrated *in vacuo;* silica gel chromatography (Gradient: 0% to 10% ethyl acetate in petroleum ether) afforded **71** as a yellow oil. Yield: 400 mg, 1.88 mmol, 73%. LCMS *m*/*z* 214.1 [M+H]⁺. ¹H NMR (400 MHz, chloroform-d) δ 8.18 (d, *J* = 5.4 Hz, 1H), 7.00 (dd, *J* = 5.5, 1.6 Hz, 1H), 6.88 (br d, *J* = 1.5 Hz, 1H), 3.96 (s, 3H), 3.78 (s, 3H), 1.89 (d, *J*_{HF} = 22.3 Hz, 3H).

### Step 6. Synthesis of 2-fluoro-2-(2-methoxypyridin-4-yl)propanoic acid (P22).

A solution of **C71** (400 mg, 1.88 mmol) and lithium hydroxide (89.9 mg, 3.75 mmol) in a mixture of tetrahydrofuran (10 mL) and water (2 mL) was stirred at 45 °C for 4 hours. The reaction mixture was then concentrated *in vacuo,* diluted with water (12 mL), and adjusted to pH 6 by addition of 3 M hydrochloric acid. The resulting mixture was extracted with ethyl acetate (2 x 20 mL), and the combined organic layers were dried over sodium sulfate, filtered, and concentrated under reduced pressure to provide **P22** as a yellow oil. Yield: 300 mg, 1.51 mmol, 80%. LCMS *m*/*z* 200.1 [M+H]⁺. ¹H NMR (400 MHz, chloroform-d) δ 9.9 - 9.4 (br s, 1H), 8.21 (d, *J* = 5.6 Hz, 1H), 7.08 (dd, *J* = 5.6, 1.6 Hz, 1H), 6.95 (br s, 1H), 3.95 (s, 3H), 1.92 (d, *J*_{HF} = 22.2 Hz, 3H).

### Preparation P23

### 2-Bromo-6,6-difluoro-6,7-dihydro-5H-[1,2,4]triazolo[5,1-b][1,3]oxazine (P23)

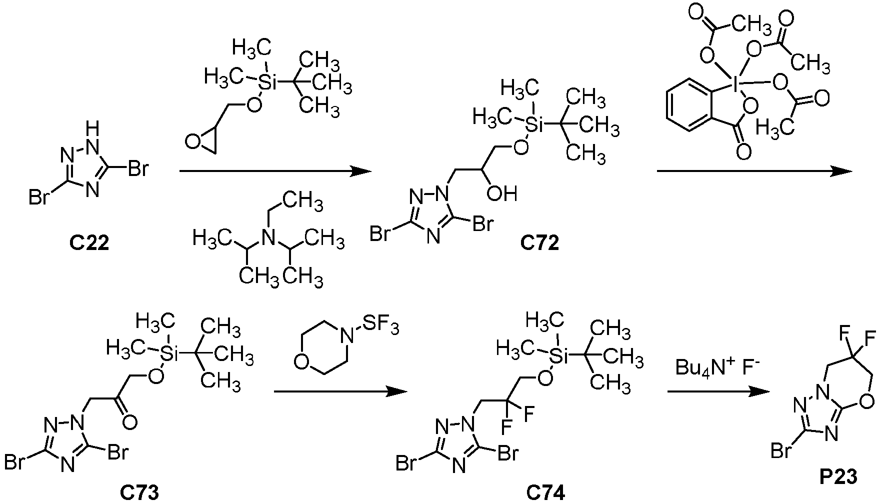

### Step 1. Synthesis of 1-{[tert-butyl(dimethyl)silyl]oxy}-3-(3,5-dibromo-1H-1,2,4-triazol-1-yl)propan-2-ol (C72).

To a solution of **C22** (4.00 g, 17.6 mmol) in toluene (100 mL) were added *N,N-*diisopropylethylamine (3.5 mL, 21.2 mmol) and *tert*-butyl(dimethyl)[(oxiran-2-yl)methoxy]silane (3.65 g, 19.4 mmol). The reaction mixture was stirred at 80 °C for 16 hours, whereupon it was concentrated *in vacuo* and purified via silica gel chromatography (Gradient: 0% to 20% ethyl acetate in petroleum ether) to afford **C72** as a colorless oil. Yield: 4.06 g, 9.78 mmol, 56%. LCMS *m*/*z* 413.9 (dibromo isotope pattern observed) [M+H]⁺. ¹H NMR (400 MHz, methanol-*d*₄) δ 4.32 (dd, component of ABX system, *J* = 14.0, 4.0 Hz, 1H), 4.22 (dd, component of ABX system, *J* = 14.1, 8.1 Hz, 1H), 4.05 (dddd, *J* = 8.3, 6.4, 4.2, 4.2 Hz, 1H), 3.72 (dd, component of ABX system, *J* = 10.5, 4.5 Hz, 1H), 3.65 (dd, component of ABX system, *J* = 10.5, 6.4 Hz, 1H), 0.93 (s, 9H), 0.11 (s, 3H), 0.11 (s, 3H).

### Step 2. Synthesis of 1-{[tert-butyl(dimethyl)silyl]oxy}-3-(3,5-dibromo-1H-1,2,4-triazol-1-yl)propan-2-one (C73).

To a 25 °C solution of **C72** (4.06 g, 9.78 mmol) in dichloromethane (120 mL) was added 1,1,1-tris(acetyloxy)-1,1-dihydro-1,2-benziodoxol-3-(1*H*)-one (8.30 g, 19.6 mmol) portion-wise. After the reaction mixture had been stirred for 16 hours, it was diluted with saturated aqueous sodium sulfate solution (100 mL) and extracted with dichloromethane (100 mL). The organic layer was concentrated *in vacuo* and purified via silica gel chromatography (Gradient: 0% to 10% ethyl acetate in petroleum ether) to provide **C73** as a colorless oil. By ¹H NMR analysis, this was presumed to exist as a mixture of keto and enol forms. Yield: 3.82 g, 9.24 mmol, 94%. LCMS *m*/*z* 412.0 (dibromo isotope pattern observed) [M+H]⁺. ¹H NMR (400 MHz, methanol-*d*₄) δ 5.36 - 5.30 (m, enol, 1H), 4.46 (s, keto, approximately 2H), 4.34 - 4.29 (m, enol, 2H), 3.63 (AB quartet, keto, *J*_{AB} = 11.0 Hz, Δ*ν*_{AB}= 50.6 Hz, 2H), [0.97 (s) and 0.94 (s), total 9H for keto and enol], [0.16 (s), 0.13 (s), and 0.12 (s), total 6H for keto and enol].

### Step 3. Synthesis of 3,5-dibromo-1-(3-{[tert-butyl(dimethyl)silyl]oxy}-2,2-difluoropropyl)-1H-1,2,4-triazole (C74).

To a solution of **C73** (2.00 g, 4.84 mmol) in dichloromethane (100 mL) was added 4-(trifluoro-λ⁴-sulfanyl)morpholine (Morpho-DAST; 0.592 mg, 4.84 mmol), whereupon the reaction mixture was stirred at 30 °C for 16 hours. It was then poured into saturated aqueous sodium bicarbonate solution (100 mL) and extracted with dichloromethane (2 x 100 mL). The combined organic layers were washed with saturated aqueous sodium chloride solution, dried over sodium sulfate, filtered, and concentrated *in vacuo*; purification via silica gel chromatography (Gradient: 0% to 5% ethyl acetate in petroleum ether) provided **C74** as a colorless oil. Yield: 910 mg, 2.09 mmol, 43%. LCMS *m*/*z* 436.0 (dibromo isotope pattern observed) [M+H]⁺. ¹H NMR (400 MHz, methanol-*d*₄) δ 4.75 (t, *J*_{HF} = 13.3 Hz, 2H), 3.92 (t, *J*_{HF} = 12.2 Hz, 2H), 0.94 (s, 9H), 0.14 (s, 6H).

### Step 4. Synthesis of 2-bromo-6,6-difluoro-6,7-dihydro-5H-[1,2,4]triazolo[5,1-b][1,3]oxazine (P23).

To a 25 °C solution of **C74** (910 mg, 2.09 mmol) in tetrahydrofuran (15 mL) was added tetrabutylammonium fluoride (1.64 g, 6.27 mmol), whereupon the reaction mixture was stirred for 2 hours. The solvent was then evaporated *in vacuo,* and the residue was diluted with ethyl acetate (100 mL) before being washed sequentially with saturated aqueous ammonium chloride and saturated aqueous sodium chloride solution, dried over sodium sulfate, filtered, and concentrated *in vacuo.* Silica gel chromatography (Gradient: 0% to 10% methanol in dichloromethane) provided **P23** as a white solid. Yield: 310 mg, 1.29 mmol, 62%. LCMS *m*/*z* 239.9 (bromine isotope pattern observed) [M+H]⁺. ¹H NMR (400 MHz, methanol-*d*₄) δ 4.73 (tt, *J* = 10.8, 1 Hz, 2H), 4.64 (tt, *J* =12.0, 1 Hz, 2H).

### Preparation P24

### 3-Bromo-1-(²H₃) m ethyl-5-(trifluoromethyl)-1H-1,2,4-triazole (P24)

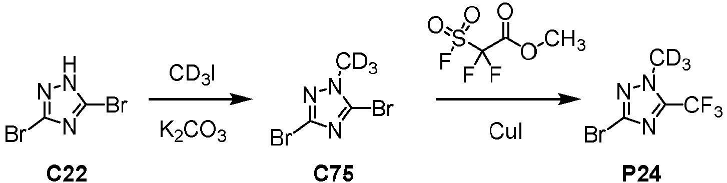

### Step 1. Synthesis of 3,5-dibromo-1-(²H₃)methyl-1H-1,2,4-triazole (C75).

A 25 °C mixture of **C22** (25 g, 110 mmol), potassium carbonate (30.5 g, 220 mmol), and iodo(²H₃)methane (25.6 g, 176 mmol) in acetonitrile (200 mL) was stirred for 20 hours. The reaction mixture was diluted with ethyl acetate, filtered through a pad of diatomaceous earth, and concentrated *in vacuo.* Purification via silica gel chromatography (Gradient: 0% to 30% ethyl acetate in heptane) provided **C75** as a white solid. Yield: 24.3 g, 99.6 mmol, 90%. GCMS *m*/*z* 244.0 (dibromo isotope pattern observed) [M]⁺.

### Step 2. Synthesis of 3-bromo-1-(²H₃)methyl-5-(trifluoromethyl)-1H-1,2,4-triazole (P24)

To a mixture of **C75** (2.0 g, 8.20 mmol) and copper(I) iodide (2.34 g, 12.3 mmol) in *N,N-*dimethylformamide (20 mL) was added methyl difluoro(fluorosulfonyl)acetate (4.73 g, 3.1 mL, 24.6 mmol) in one portion and the reaction mixture was heated at 110 °C for 22 hours. The cooled reaction mixture was partitioned between diethyl ether (200 mL) and a mixture of water and concentrated ammonium hydroxide solution (1:1, approximately 100 mL), whereupon the aqueous layer was extracted with *tert*-butyl methyl ether. The combined organic layers were washed with water, dried over magnesium sulfate, filtered, and concentrated *in vacuo* to afford **P24** as a light-yellow syrup; this material was used directly in further chemistry. Yield: 1.67 g, 7.17 mmol, 87%. GCMS *m*/*z* 232.1 (bromine isotope pattern observed) [M]⁺.

### Preparation P25

### 3-Bromo-5-(difluoromethyl)-1-(²H₃)methyl-1H-1,2,4-triazole (P25)

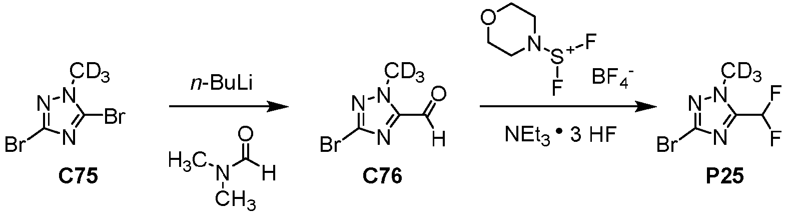

### Step 1. Synthesis of 3-bromo-1-(²H₃)methyl-1H-1,2,4-triazole-5-carbaldehyde (C76).

To a -45 °C solution of **C75** (7.60 g, 31.2 mmol) in tetrahydrofuran (60 mL) was added a solution of n-butyllithium in hexane (2.5 M; 15.0 mL, 37.5 mmol) in a drop-wise manner. After the reaction mixture had been stirred for 30 minutes at -45 °C, additional *n*-butyllithium solution (2.5 M; 1.0 mL, 2.5 mmol) was added and stirring was continued at -40 °C for 30 minutes. The reaction mixture was then cooled to -78 °C, and treated with *N,N*-dimethylformamide (3.12 mL, 40.3 mmol), whereupon stirring was continued at -78 °C for an additional 30 minutes. The reaction mixture was then slowly warmed to room temperature; hydrochloric acid (1 M; 60 mL, 60 mmol) was added and stirring was continued for 10 minutes. The resulting precipitate slowly dissolved, and sodium carbonate was added until gas evolution ceased. The mixture was then separated; the aqueous layer was extracted twice with ethyl acetate, and the combined organic layers were dried over magnesium sulfate, filtered, and concentrated *in vacuo.* Purification via silica gel chromatography (Gradient: 20% to 70% ethyl acetate in heptane) afforded **C76** as a white solid. Yield: 3.06 g, 15.8 mmol, 51%. ¹H NMR (400 MHz, chloroform-d) δ 9.91 (s, 1H).

### Step 2. Synthesis of 3-bromo-5-(difluoromethyl)-1-(²H₃)methyl-1H-1,2,4-triazole (P25).

To a 0 °C solution of **C76** (3.06 g, 15.8 mmol) in dichloromethane (60 mL) were added difluoro(morpholin-4-yl)sulfanium tetrafluoroborate (XtalFluor-M^{®}; 7.7 g, 31.7 mmol) and triethylamine trihydrofluoride (TREAT-HF; 5.11 g, 31.7 mmol). After the reaction mixture had been stirred at room temperature for 18 hours, it was treated with saturated aqueous sodium bicarbonate solution and extracted with dichloromethane. The organic layer was dried over magnesium sulfate, filtered, concentrated *in vacuo,* and purified using silica gel chromatography (Gradient: 0% to 20% ethyl acetate in heptane) to afford **P25** as a yellow oil that solidified at low temperature under vacuum. Yield: 1.88 g, 8.74 mmol, 55%. GCMS *m*/*z* 214.0 (bromine isotope pattern observed) [M+H]⁺. ¹H NMR (400 MHz, chloroform-d) δ 6.77 (t, *J*_{HF} = 52.3 Hz, 1H).

### General Procedure 1

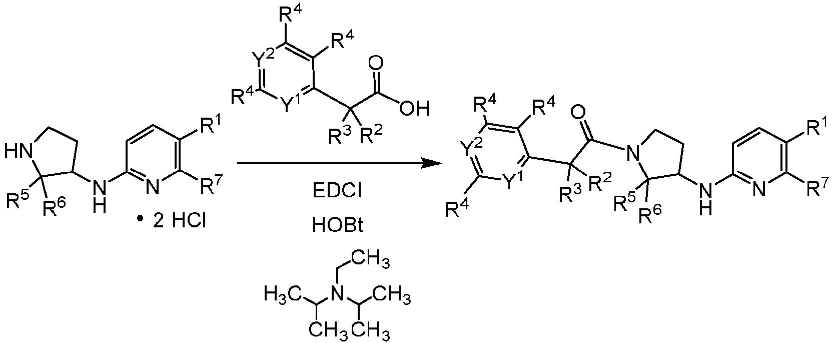

To a solution of carboxylic acid (1 equivalent), *N,N*-diisopropylethylamine (2 equivalents) and amine dihydrochloride salt (1 equivalent) in *N,N*-dimethylformamide (0.1 M) were added 1-[3-(dimethylamino)propyl]-3-ethylcarbodiimide hydrochloride (1.5 equivalents) and 1*H-*benzotriazol-1-ol (1.1 equivalents). The reaction mixture was stirred at 25 °C for 16 hours, whereupon it was diluted with water and extracted with ethyl acetate. The combined organic layers were washed with saturated aqueous sodium chloride solution, dried over sodium sulfate, filtered, and concentrated *in vacuo.* The residue was purified by either silica gel chromatography or reversed-phase HPLC to afford the product.

### Example 1

### (2R)-2-(5-Chloro-2-methoxypyridin-4-yl)-1-[(3S)-3-{[6-methyl-5-(2-methyl-2H-tetrazol-5-yl)pyridin-2-yl]amino}pyrrolidin-1-yl]propan-1-one (1)

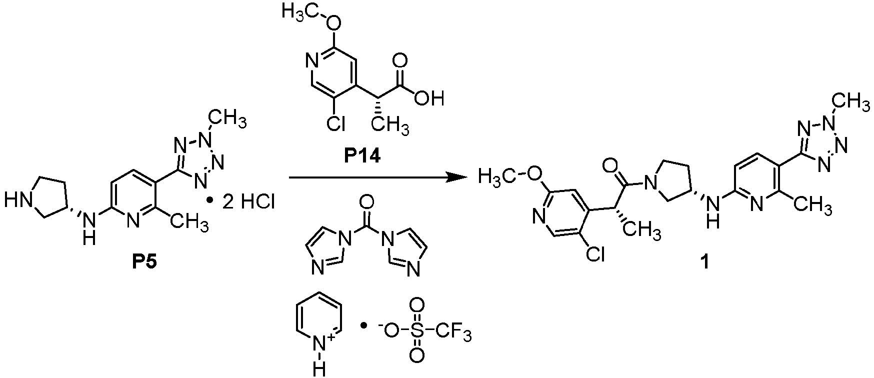

To a 25 °C solution of **P14** (50 mg, 0.23 mmol) in acetonitrile (10.0 mL) was added pyridinium trifluoromethanesulfonate (133 mg, 0.58 mmol), followed by 1,1'-carbonyldiimidazole (40 mg, 0.24 mmol). After the resulting mixture had been stirred for 1 hour, **P5** (69 mg, 0.21 mmol) was added and the reaction mixture was stirred for 4 hours, whereupon it was added to water (20 mL) and extracted with ethyl acetate (2 x 20 mL). The combined organic layers were washed sequentially with saturated aqueous ammonium chloride (30 mL), saturated aqueous sodium bicarbonate (30 mL), and saturated aqueous sodium chloride solution (30 mL), dried over sodium sulfate, filtered, and concentrated *in vacuo.* The residue was purified via preparative thin-layer chromatography on silica gel (Eluent: 5% dichloromethane in methanol) to provide (2R)-2-(5-chloro-2-methoxypyridin-4-yl)-1-[(3S)-3-{[6-methyl-5-(2-methyl-2H-tetrazol-5-yl)pyridin-2-yl]amino}pyrrolidin-1-yl]propan-1-one (**1**) as a white solid. Analysis of the ¹H NMR indicated that this material comprised a mixture of rotamers. Yield: 25 mg, 55 µmol, 26%. LCMS *m*/*z* 457.0 [M+H]⁺. ¹H NMR (400 MHz, methanol-*d*₄) δ [8.15 (s) and 8.13 (s), total 1H], 7.94 (d, *J* = 8.6 Hz, 1H), [6.77 (s) and 6.75 (s), total 1H], [6.46 (d, *J* = 8.8 Hz) and 6.43 (d, *J* = 8.6 Hz), total 1H], [4.56 - 4.42 (m), 4.40 (s), and 4.39 (s), total 4H], [4.29 (q, *J* = 6.8 Hz) and 4.21 (q, *J* = 6.8 Hz), total 1H], [3.90 (s) and 3.88 (s), total 3H], [3.88 - 3.82 (m), 3.80 - 3.64 (m), and 3.58 - 3.39 (m), total 4H], [2.64 (s) and 2.61 (s), total 3H], [2.40 - 2.29 (m), 2.28 - 2.15 (m), and 2.04 - 1.91 (m), total 2H], [1.42 (d, *J* = 7.1 Hz) and 1.38 (d, *J* = 7.1 Hz), total 3H].

### Example 2

### (2R)-2-(5-Fluoro-2-methoxypyridin-4-yl)-1-[(3S)-3-({6-methyl-5-[1-methyl-5-(trifluoromethyl)-1H-1,2,4-triazol-3-yl]pyridin-2-yl}amino)pyrrolidin-1-yl]propan-1-one (2)

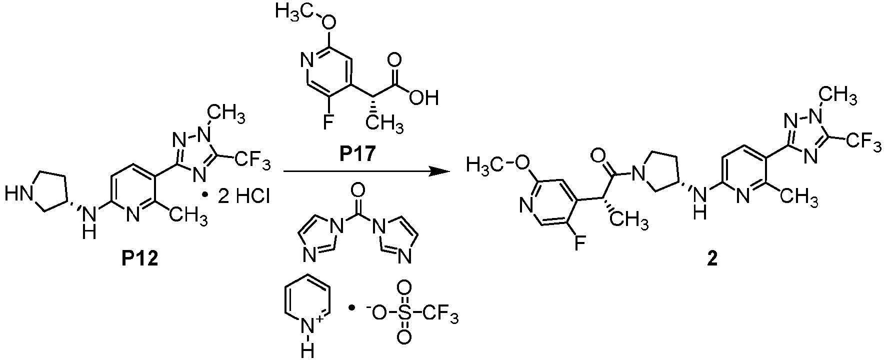

Pyridinium trifluoromethanesulfonate (39.1 g, 171 mmol) was added to a mixture of **P17** (90%, 18.0 g, 81.3 mmol) in acetonitrile (300 mL) and the mixture was stirred until a solution was obtained. 1,1'-Carbonyldiimidazole (13.8 g, 85.4 mmol) was then added; after the reaction mixture had been stirred for 45 minutes, **P12** (32.5 g, 81.4 mmol) was added and stirring was continued for 3 hours. The reaction mixture was then partitioned between ethyl acetate and water. The organic layer was washed sequentially with water (twice), saturated aqueous ammonium chloride solution, saturated aqueous sodium bicarbonate solution, and saturated aqueous sodium chloride solution, dried over sodium sulfate, filtered, and concentrated *in vacuo.* The resulting foam was dissolved in ethyl acetate (50 mL) with heating (80 °C), and heptane (180 mL) was added until the mixture became cloudy; heating was discontinued and the mixture was allowed to cool to room temperature. An oil appeared, so the mixture was reheated to 80 °C, whereupon additional ethyl acetate (10 mL) was added and the mixture was again allowed to cool to room temperature. The resulting precipitate was collected via filtration to provide (2*R*)-2-(5-fluoro-2-methoxypyridin-4-yl)-1-[(3S)-3-({6-methyl-5-[1-methyl-5-(trifluoromethyl)-1*H*-1,2,4-triazol-3-yl]pyridin-2-yl}amino)pyrrolidin-1-yl]propan-1-one (**2**) as an off-white solid. Yield: 31.8 g, 62.7 mmol, 77%. LCMS *m*/*z* 508.3 [M+H]⁺. ¹H NMR (400 MHz, methanol-*d*₄) δ [7.98 (d, *J* = 1.0 Hz) and 7.97 (d, *J* = 1.0 Hz), total 1H], 7.91 (d, *J* = 8.6 Hz, 1H), [6.74 (d, *J* = 4.4 Hz) and 6.73 (d, *J* = 4.6 Hz), total 1H], [6.43 (d, *J* = 9.0 Hz) and 6.41 (d, *J* = 9.0 Hz), total 1H], 4.53 - 4.42 (m, 1H), [4.25 (q, *J* = 7.1 Hz) and 4.16 (q, *J* = 6.8 Hz), total 1H], 4.10 (br s, 3H), [3.90 - 3.84 (m), 3.88 (s), 3.86 (s), 3.82 - 3.63 (m), 3.58 - 3.46 (m), 3.41 (d, *J* = 4.6 Hz), and 3.38 (d, *J* = 4.9 Hz), total 7H], [2.64 (s) and 2.61 (s), total 3H], [2.39 - 2.28 (m) and 2.25 - 2.15 (m), total 1H], 2.05 - 1.90 (m, 1H), [1.44 (d, *J* = 6.8 Hz) and 1.40 (d, *J* = 6.8 Hz), total 3H].

### Examples 3A and 3B

### 1-[(3R)-2,2-Dimethyl-3-{[6-methyl-5-(1-methyl-1H-imidazol-4-yl)pyridin-2-yl]amino}pyrrolidin-1-yl]-2-(4-fluorophenyl)ethan-1-one (3A) and 1-[(3S)-2,2-Dimethyl-3-{[6-methyl-5-(1-methyl-1H-imidazol-4-yl)pyridin-2-yl]amino}pyrrolidin-1-yl]-2-(4-fluorophenyl)ethan-1-one (3B)

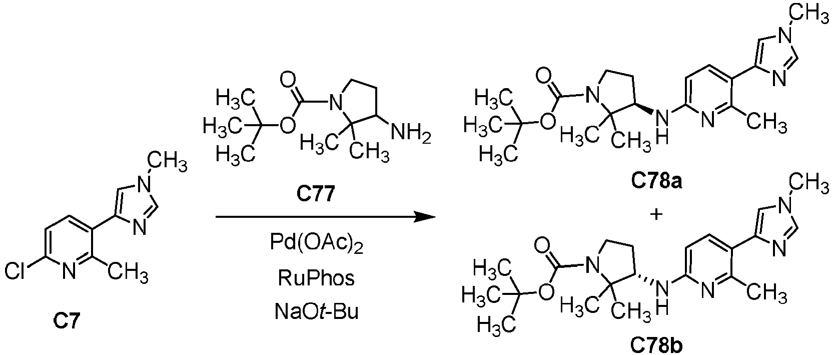

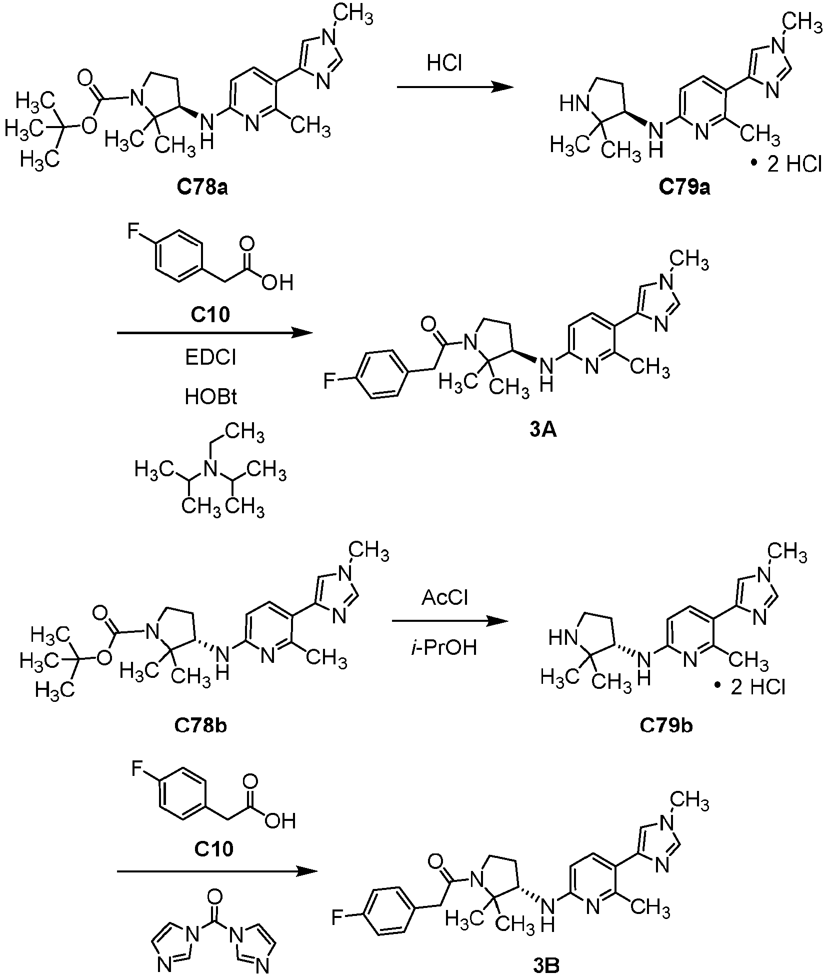

### Step 1: Synthesis of tert-butyl (3R)-2,2-dimethyl-3-{[6-methyl-5-(1-methyl-1H-imidazol-4-yl)pyridin-2-yl]amino}pyrrolidine-1-carboxylate (C78a) and tert-butyl (3S)-2,2-dimethyl-3-{[6-methyl-5-(1-methyl-1H-imidazol-4-yl)pyridin-2-yl]amino}pyrrolidine-1-carboxylate (C78b).

To a solution of 2-dicyclohexylphosphino-2',6'-diisopropoxybiphenyl (RuPhos; 2.52 g, 5.39 mmol) and palladium(II) acetate (605 mg, 2.70 mmol) in 2-methylbutan-2-ol (450 mL) was added sodium *tert*-butoxide (25.9 g, 270 mmol) and the reaction mixture was heated to 80 °C. *tert*-Butyl 3-amino-2,2-dimethylpyrrolidine-1-carboxylate (**C77**) (28.9 g, 135 mmol) and **C7** (28 g, 130 mmol) were then added and the reaction was heated at 80 °C for 3 hours. It was then filtered through a pad of diatomaceous earth, which was rinsed twice with ethyl acetate. The filtrate was washed with water, and the aqueous layer was extracted three times with ethyl acetate. The combined organic layers were washed with saturated aqueous sodium chloride solution, dried over sodium sulfate, filtered, and concentrated *in vacuo* to provide crude material (40 g). This was separated into its component enantiomers via supercritical fluid chromatography {Column: Chiral Technologies ChiralCel OD-H, 30 x 250 mm, 5 µm; Mobile phase: 77.5 / 22.5 carbon dioxide / [methanol containing 0.2% (7 M ammonia in methanol)]; Flow rate: 80 mL/minute; Back pressure: 150 bar}. The first-eluting enantiomer was designated as **C78a,** and the second-eluting enantiomer as **C78b**; both were obtained as oils. The indicated absolute stereochemistry was assigned via X-ray crystal structure determination of **3A,** which was synthesized using **C78a** (see below).

**C78a** -Yield: 17.7 g, 45.9 mmol, 35%. Retention time: 5.18 minutes (Analytical conditions identical to those used for **C78b**).

**C78b** -Yield: 18.1 g, 47.0 mmol, 36%. LCMS *m*/*z* 386.4 [M+H]⁺. ¹H NMR (400 MHz, methanol-*d*₄) δ 7.63 (s, 1H), 7.61 (d, *J = 8.3* Hz, 1H), 7.11 (d, *J = 1.2* Hz, 1H), 6.50 (d, *J* = 8.6 Hz, 1H), 4.50 - 4.34 (m, 1H), 3.77 (s, 3H), 3.51 (ddd, *J* = 2.9, 8.5, 11.2 Hz, 1H), 3.39 - 3.31 (m, 1H), 2.44 (s, 3H), 2.18 - 2.08 (m, 1H), 1.81 - 1.68 (m 1H), 1.49 (br s, 3H), 1.46 (br s, 9H), 1.26 (br s, 3H). Retention time: 6.03 minutes (Analytical conditions. Column: Chiral Technologies ChiralCel OD-H, 4.6 x 250 mm, 5 µm; Mobile phase A: carbon dioxide; Mobile phase B: methanol containing 0.2% (7 M ammonia in methanol); Gradient: 5% B for 1.00 minute, then 5% to 60% B over 8.0 minutes; Flow rate: 3.0 mL/minute; Back pressure: 120 bar).

### Step 2. Synthesis of N-[(3R)-2,2-dimethylpyrrolidin-3-yl]-6-methyl-5-(1-methyl-1H-imidazol-4-yl)pyridin-2-amine, dihydrochloride salt (C79a).

A mixture of **C78a** (40 mg, 0.10 mmol) and a solution of hydrogen chloride in 1,4-dioxane (4 M; 0.26 mL, 1.0 mmol) was heated to 50 °C for 1 hour, whereupon LCMS analysis indicated the presence of **C79a**: LCMS *m*/*z* 286.3 [M+H]⁺. The reaction mixture was combined with a similar reaction carried out using **C78a** (46 mg, 0.12 mmol) and concentrated *in vacuo,* providing **C79a** as a solid; this material was used directly in the following step.

### Step 3. Synthesis of 1-[(3R)-2,2-dimethyl-3-{[6-methyl-5-(1-methyl-1H-imidazol-4-yl)pyridin-2-yl]amino}pyrrolidin-1-yl]-2-(4-fluorophenyl)ethan-1-one (3A).

To a solution of **C79a** (from the previous step; ≤0.22 mmol) in *N,N-*dimethylformamide (2.0 mL) were added **C10** (48.4 mg, 0.314 mmol), 1H-benzotriazol-1-ol (50.9 mg, 0.377 mmol), 1-[3-(dimethylamino)propyl]-3-ethylcarbodiimide hydrochloride (92.3 mg, 0.481 mmol), and *N,N-*diisopropylethylamine (0.365 mL, 2.09 mmol). After the reaction mixture had been stirred for 3 hours at ambient temperature, it was partitioned between ethyl acetate and saturated aqueous sodium bicarbonate solution. The organic layer was washed with saturated aqueous sodium chloride solution, dried over magnesium sulfate, filtered, and concentrated *in vacuo.* The residue was subjected to chromatography on silica gel (Gradient: ethyl acetate in hexane), and the resulting material was partitioned between 1 M hydrochloric acid and ethyl acetate. The aqueous layer was adjusted to pH 10 by addition of 1 M aqueous sodium hydroxide solution, whereupon it was extracted three times with dichloromethane. The combined dichloromethane layers were washed with saturated aqueous sodium chloride solution, dried over sodium sulfate, filtered, and concentrated under reduced pressure to afford 1-[(3*R*)-2,2-dimethyl-3-{[6-methyl-5-(1-methyl-1*H*-imidazol-4-yl)pyridin-2-yl]amino}pyrrolidin-1-yl]-2-(4-fluorophenyl)ethan-1-one (**3A**) as a solid. Yield: 63 mg, 0.15 mmol, 68% over 2 steps. LCMS *m*/*z* 422.4 [M+H]⁺. ¹H NMR (400 MHz, methanol-*d*₄) δ 7.65 - 7.58 (m, 2H), 7.27 (dd, *J* = 8.6, 5.4 Hz, 2H), 7.11 (d, *J =* 1.3 Hz, 1H), 7.05 (dd, *J* = 8.8, 8.8 Hz, 2H), 6.49 (d, *J* = 8.5 Hz, 1H), 4.44 (dd, *J* = 10.1, 6.1 Hz, 1H), 3.76 (s, 3H), 3.72 - 3.64 (m, 1H), 3.65 (s, 2H), 3.53 (ddd, *J* = 10, 10, 7.1 Hz, 1H), 2.43 (s, 3H), 2.23 - 2.14 (m, 1H), 1.88 - 1.74 (m, 1H), 1.55 (s, 3H), 1.35 (s, 3H).

This material was dissolved in a minimal amount of propan-2-ol and slurried for 12 hours; the solid was then collected via filtration to afford 1-[(3*R*)-2,2-dimethyl-3-{[6-methyl-5-(1-methyl-1*H*-imidazol-4-yl)pyridin-2-yl]amino}pyrrolidin-1-yl]-2-(4-fluorophenyl)ethan-1-one (**3A**) as a crystalline solid. Melting point: 196-198 °C. A crystal from this lot was used in the single-crystal X-ray structure determination below.

### Single-crystal X-ray structural determination of 3A

### Single Crystal X-Ray Analysis

Data collection was performed on a Bruker D8 Quest diffractometer at room temperature. Data collection consisted of omega and phi scans.

The structure was solved by intrinsic phasing using SHELX software suite in the orthorhombic class space group *P*2₁2₁2₁. The structure was subsequently refined by the full-matrix least squares method. All non-hydrogen atoms were found and refined using anisotropic displacement parameters.

The hydrogen atoms located on nitrogen were found from the Fourier difference map and refined with distances restrained. The remaining hydrogen atoms were placed in calculated positions and were allowed to ride on their carrier atoms. The final refinement included isotropic displacement parameters for all hydrogen atoms.

Analysis of the absolute structure using likelihood methods (Hooft, 2008) was performed using PLATON (Spek). The results indicate that the absolute structure has been correctly assigned. The method calculates that the probability that the structure is correctly assigned is 100%. The Hooft parameter is reported as 0.05 with an esd (estimated standard deviation) of 0.006 and the Parson's parameter is reported as 0.02 with an esd of 0.005.

The final R-index was 4.2%. A final difference Fourier revealed no missing or misplaced electron density.

### Software and References

**SHELXTL**, Version 5.1, Bruker AXS, 1997.
**PLATON**, A. L. Spek, J. Appl. Cryst. 2003, 36, 7-13.
**MERCURY**, C. F. Macrae, P. R. Edington, P. McCabe, E. Pidcock, G. P. Shields, R. Taylor, M. Towler, and J. van de Streek, J. Appl. Cryst. 2006, 39, 453-457.
**OLEX2**, O. V. Dolomanov, L. J. Bourhis, R. J. Gildea, J. A. K. Howard, and H. Puschmann, J. Appl. Cryst. 2009, 42, 339-341.
R. W. W. Hooft, L. H. Straver, and A. L. Spek, J. Appl. Cryst. 2008, 41, 96-103.
H. D. Flack, Acta Cryst. 1983, A39, 867-881.

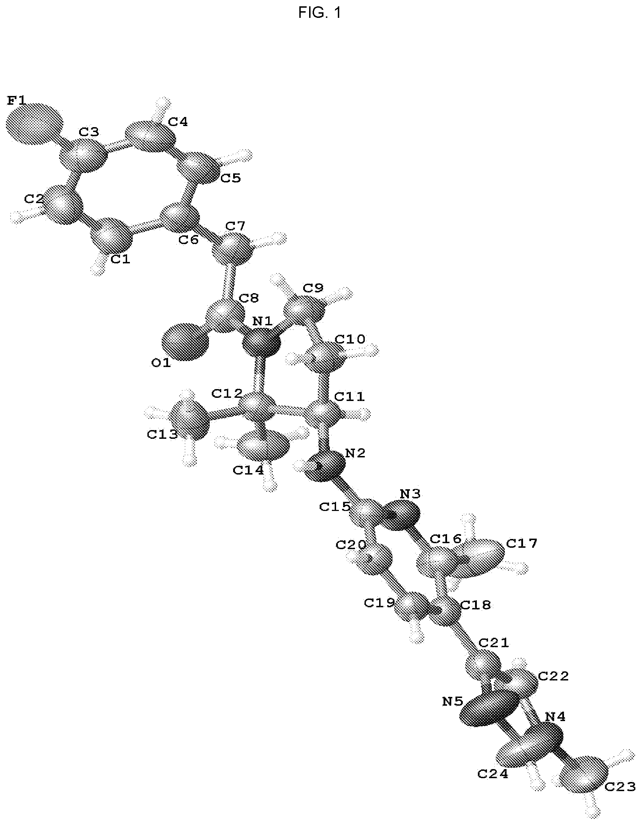

The absolute stereochemistry of compound Example 3A was determined by single crystal X-ray crystallography (using pertinent data/parameters/information used/collected, including, e.g., pertinent crystal data and refinement information; atomic coordinates; bond lengths; bond angles; and displacement parameters). Figure 1 (FIG. 1) is an obtained single crystal X-ray structure (ORTEP drawing) of the crystalline compound Example 3A: 1-[(3*R*)-2,2-Dimethyl-3-{[6-methyl-5-(1-methyl-1*H*-imidazol-4-yl)pyridin-2-yl]amino}pyrrolidin-1-yl]-2-(4-fluorophenyl)ethan-1-one.

In some embodiments, the present invention provides a crystalline form of 1-[(3*R*)-2,2-Dimethyl-3-{[6-methyl-5-(1-methyl-1*H*-imidazol-4-yl)pyridin-2-yl]amino}pyrrolidin-1-yl]-2-(4-fluorophenyl)ethan-1-one. In some further embodiments, the crystalline form of 1-[(3*R*)-2,2-Dimethyl-3-{[6-methyl-5-(1-methyl-1*H*-imidazol-4-yl)pyridin-2-yl]amino}pyrrolidin-1-yl]-2-(4-fluorophenyl)ethan-1-one is the one described (and/or as prepared) in Example 3A.

### Step 4. Synthesis of N-[(3S)-2,2-dimethylpyrrolidin-3-yl]-6-methyl-5-(1-methyl-1H-imidazol-4-yl)pyridin-2-amine, dihydrochloride salt (C79b).

Acetyl chloride (14.7 g, 188 mmol) was slowly added to propan-2-ol (36 mL) with stirring at 0 °C. In a separate flask **C78b** (18.1 g, 47.0 mmol) was dissolved in propan-2-ol (36 mL), which required heating up to 50 °C. Once the material had dissolved, the hydrogen chloride solution was added in a drop-wise manner, whereupon the reaction mixture was heated at 50 °C for 3 hours. The heat was turned off and the reaction was allowed to slowly cool to room temperature with stirring, then to stir at room temperature for 2 hours. The solids were collected by vacuum filtration to provide **C79b**, **dihydrochloride salt** (15.8 g) as a white solid. This material was dissolved in dichloromethane and transferred to a separatory funnel. Saturated aqueous sodium bicarbonate solution was added, and the mixture was shaken for 3 minutes. The layers were separated, and the organic layer was dried over sodium sulfate, filtered, and concentrated *in vacuo* to provide C79b as a solid. Yield: 10.2 g, 35.7 mmol, 76%. ¹H NMR (400 MHz, methanol-*d*₄) δ 7.62 (s, 1H), 7.61 (d, *J* = 8.6 Hz, 1H), 7.10 (d, *J =* 1.2 Hz, 1H), 6.44 (d, *J =* 8.6 Hz, 1H), 4.12 (t, *J* = 7.7 Hz, 1H), 3.76 (s, 3H), 3.08 - 2.91 (m, 2H), 2.43 (s, 3H), 2.37 (dtd, *J* = 4.9, 8.5, 13.1 Hz, 1H), 1.72 (tdd, *J* = 7.0, 9.4, 13.5 Hz, 1H), 1.24 (s, 3H), 1.10 (s, 3H).

### Step 5: Synthesis of 1-[(3S)-2,2-dimethyl-3-{[6-methyl-5-(1-methyl-1H-imidazol-4-yl)pyridin-2-yl]amino}pyrrolidin-1-yl]-2-(4-fluorophenyl)ethan-1-one (3B).

To a 25 °C solution of **C10** (5.35 g, 34.7 mmol) in tetrahydrofuran (158 mL) was added 1,1-carbonyldiimidazole (5.62 g, 34.7 mmol). After the reaction mixture had been stirred for 1 hour, a solution of **C79b** (9.00 g, 31.5 mmol) in a minimal volume of tetrahydrofuran was added, and stirring was continued at room temperature for 4 hours. The reaction mixture was then diluted with 1 M hydrochloric acid and ethyl acetate, and the aqueous layer was adjusted to pH 10 by addition of 1 M aqueous sodium hydroxide solution. The aqueous layer was extracted three times with dichloromethane and the combined organic layers were washed with saturated aqueous sodium chloride solution, dried over sodium sulfate, filtered, and concentrated *in vacuo.* The resulting material was dissolved in a minimal amount of hot propan-2-ol, which was then allowed to cool to room temperature with stirring. Solids were collected by vacuum filtration to provide 1-[(3S)-2,2-dimethyl-3-{[6-methyl-5-(1-methyl-1*H*-imidazol-4-yl)pyridin-2-yl]amino}pyrrolidin-1-yl]-2-(4-fluorophenyl)ethan-1-one (**3B**) as a solid. Yield: 8.85 g, 21.0 mmol, 67%. LCMS *m*/*z* 422.3 [M+H]^{+ 1}H NMR (400 MHz, methanol-*d*₄) δ 7.62 (s, 1H), 7.61 (d, *J* = 8.3 Hz, 1H), 7.23 - 7.20 (m, 2H), 7.11 (d, *J* = 1.2 Hz, 1H), 7.09 - 7.01 (m, 2H), 6.49 (d, *J* = 8.6 Hz, 1H), 4.45 (dd, *J* = 6.2, 10.1 Hz, 1H), 3.76 (s, 3H), 3.68 (ddd, *J* = 2.9, 8.3, 11.0 Hz, 1H), 3.65 (s, 2H), 3.54 (dt, *J* = 7.2, 10.0 Hz, 1H), 2.43 (s, 3H), 2.19 (dtd, *J* = 3.1, 6.4, 12.5 Hz, 1H), 1.90 - 1.74 (m, 1H), 1.55 (s, 3H), 1.35 (s, 3H).

### Example 4

### (2R)-1-[(3S)-3-({5-[5-(1,1-Difluoroethyl)-1-methyl-1H-1,2,4-triazol-3-yl]-6-methylpyridin-2-yl}amino)pyrrolidin-1-yl]-2-(5-fluoro-2-methoxypyridin-4-yl)propan-1-one (4)

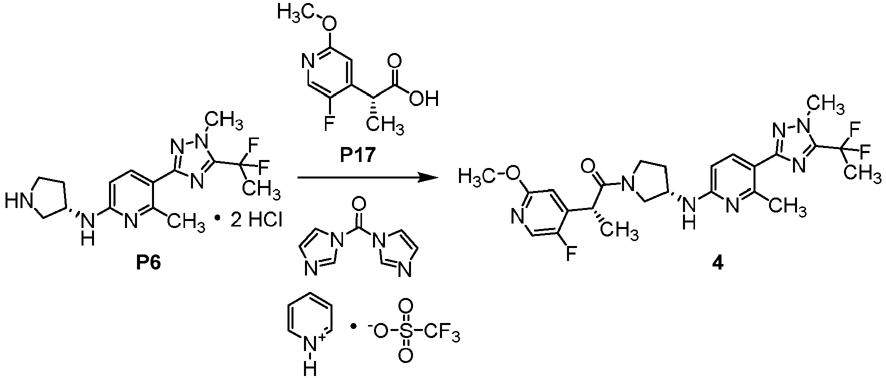

To a solution of **P17** (40 mg, 0.18 mmol) in acetonitrile (10.0 mL) were added pyridinium trifluoromethanesulfonate (115 mg, 0.502 mmol) and 1,1'-carbonyldiimidazole (34 mg, 0.21 mmol). After the reaction mixture had been stirred at 25 °C for 1 hour, **P6** (72 mg, 0.20 mmol) was added and stirring was continued at 20 °C for 24 hours. Water (20 mL) was added and the resulting mixture was extracted with ethyl acetate (2 x 20 mL); the combined organic layers were washed sequentially with saturated aqueous ammonium chloride solution (30 mL), saturated aqueous sodium bicarbonate solution (30 mL), and saturated aqueous sodium chloride solution (30 mL), dried over sodium sulfate, filtered, and concentrated *in vacuo.* Purification using reversed-phase HPLC (Column: Phenomenex Gemini C18, 21.2 x 100 mm, 5 µm; Mobile phase A: water containing 0.1% formic acid; Mobile phase B: acetonitrile; Gradient: 20% to 30% B; Flow rate: 25 mL/minute) provided (2R)-1-[(3S)-3-({5-[5-(1,1-difluoroethyl)-1-methyl-1H-1,2,4-triazol-3-yl]-6-methylpyridin-2-yl}amino) pyrrolidin-1-yl]-2-(5-fluoro-2-methoxypyridin-4-yl)propan-1-one (**4**) as a white solid. Analysis of the ¹H NMR indicated that this material comprised a mixture of rotamers. Yield: 29 mg, 58 µmol, 32%. LCMS *m*/*z* 504.1 [M+H]⁺. ¹H NMR (400 MHz, methanol-*d*₄) δ [7.98 (d, *J* = 1.5 Hz) and 7.97 (d, *J* = 1.7 Hz), total 1H], 7.88 (d, *J* = 8.6 Hz, 1H), [6.74 (d, *J* = 4.9 Hz), and 6.73 (d, *J* = 4.4 Hz), total 1H], [6.43 (d, *J* = 8.8 Hz) and 6.41 (d, *J* = 9.1 Hz), total 1H], 4.53 - 4.41 (m, 1H), [4.25 (q, *J* = 6.8 Hz) and 4.16 (q, *J* = 7.1 Hz), total 1H], 4.08 - 4.06 (m, 3H), [3.90 - 3.84 (m), 3.88 (s), 3.86 (s), 3.81 - 3.65 (m), 3.57 - 3.47 (m), and 3.42 - 3.36 (m), total 7H], [2.63 (s) and 2.60 (s), total 3H], [2.38 - 2.28 (m), 2.26 - 2.09 (m), 2.17 (t, *J* = 19.1 Hz), and 2.17 (t, *J* = 19.1 Hz), total 4H], 2.04 - 1.91 (m, 1H), [1.44 (d, *J* = 6.8 Hz) and 1.41 (d, *J* = 6.8 Hz), total 3H].

### Example 5

### (2R)-2-(5-Fluoro-2-methoxypyridin-4-yl)-1-[(3S)-3-{[6-methyl-5-(2-methyl-2H-tetrazol-5-yl)pyridin-2-yl]amino}pyrrolidin-1-yl]propan-1-one (5)

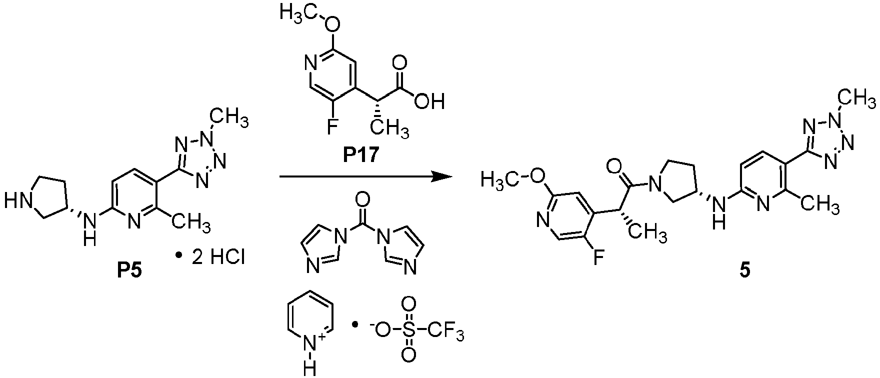

To a 25 °C solution of **P17** (40 mg, 0.20 mmol) in acetonitrile (10.0 mL) were added pyridine trifluoromethanesulfonate (115 mg, 0.502 mmol) and 1,1'-carbonyldiimidazole (34 mg, 0.21 mmol). The reaction mixture was stirred for 1 hour, whereupon **P5** (59 mg, 0.18 mmol) was added and stirring was continued for 24 hours. Water (20 mL) was added to the reaction mixture and the resulting mixture was extracted with ethyl acetate (2 x 20 mL). The combined organic layers were washed sequentially with saturated aqueous ammonium chloride (30 mL), saturated aqueous sodium bicarbonate solution (30 mL), and saturated aqueous sodium chloride solution (30 mL), dried over sodium sulfate, filtered, and concentrated *in vacuo.* Reversed-phase HPLC (Column: Phenomenex Gemini C18, 21.2 x 100 mm, 5 µm; Mobile phase A: water containing 0.1% formic acid; Mobile phase B: acetonitrile; Gradient: 20% to 30% B; Flow rate: 25 mL/minute) afforded (2*R*)-2-(5-fluoro-2-methoxypyridin-4-yl)-1-[(3S)-3-{[6-methyl-5-(2-methyl-2*H*-tetrazol-5-yl)pyridin-2-yl]amino}pyrrolidin-1-yl]propan-1-one (**5**) as a white solid. Analysis of the ¹H NMR indicated that this material comprised a mixture of rotamers. Yield: 10 mg, 23 µmol, 13%. LCMS *m*/*z* 441.1 [M+H]⁺. ¹H NMR (400 MHz, methanol-*d*₄) δ [7.98 (d, *J* = 1.7 Hz) and 7.97 (d, *J* = 1.5 Hz), total 1H], [7.943 (d, *J* = 8.6 Hz) and 7.937 (d, *J* = 8.8 Hz), total 1H], 6.76 - 6.72 (m, 1H), [6.47 (d, *J* = 8.8 Hz) and 6.43 (d, *J* = 8.8 Hz), total 1H], 4.56 - 4.43 (m, 1H), [4.40 (s) and 4.39 (s), total 3H], [4.25 (q, *J* = 6.9 Hz) and 4.17 (q, *J* = 6.8 Hz), total 1H], [3.90 - 3.83 (m), 3.82 - 3.64 (m), 3.61 - 3.47 (m), and 3.40 (dd, *J* = 4.9, 12.5 Hz), total 7H], [2.64 (s) and 2.61 (s), total 3H], [2.39 - 2.29 (m) and 2.27 - 2.15 (m), total 1H], 2.05 - 1.93 (m, 1H), [1.44 (d, *J* = 6.8 Hz) and 1.41 (d, *J* = 6.8 Hz), total 3H].

### Example 6

### (2R)-2-(5-Chloro-2-methoxypyridin-4-yl)-1-[(3S)-3-{[6-methyl-5-(pyrimidin-2-yl)pyridin-2-yl]amino}pyrrolidin-1-yl]propan-1-one (6)

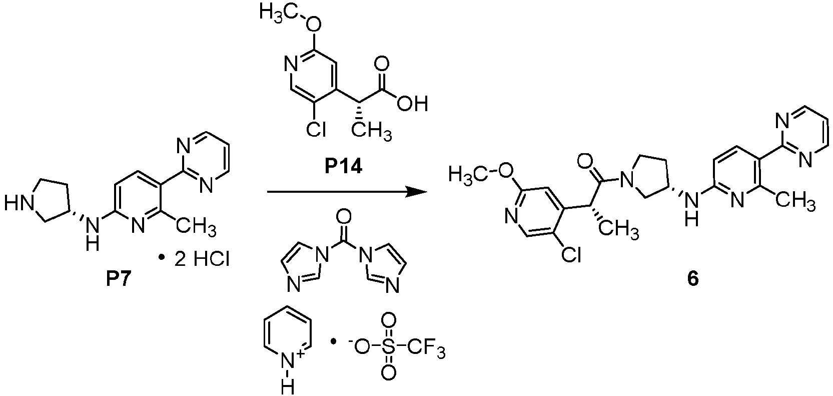

To a 25 °C solution of **P14** (33.2 g, **154** mmol) and pyridinium trifluoromethanesulfonate (74.1 g, 323 mmol) in acetonitrile (620 mL) was added 1,1-carbonyldiimidazole (25.0 g, 154 mmol). After the reaction mixture had been stirred for 30 minutes, **P7** (70% purity; 72.2 g, 154 mmol) was added and stirring was continued for 2 hours. The reaction mixture was poured into water, and the organic layer was washed twice with water; the combined aqueous layers were then extracted three times with ethyl acetate. The combined organic layers were washed sequentially with saturated aqueous ammonium chloride solution, saturated aqueous sodium bicarbonate solution, and saturated aqueous sodium chloride solution, dried over sodium sulfate, filtered, and concentrated *in vacuo.* The residue was dissolved in a minimal amount of propan-2-ol (400 mL) at 60 °C and the solution was cooled to room temperature and slurried over 2 days. Collection of the precipitate via vacuum filtration afforded (2R)-2-(5-chloro-2-methoxypyridin-4-yl)-1-[(3S)-3-{[6-methyl-5-(pyrimidin-2-yl)pyridin-2-yl]amino}pyrrolidin-1-yl]propan-1-one (**6**) as a white solid. Analysis of the ¹H NMR indicated that this material comprised a mixture of rotamers. Yield: 38.5 g, 85.0 mmol, 55%. Diastereomeric excess: 100%. LCMS *m*/*z* 453.2 [M+H]⁺. ¹H NMR (400 MHz, methanol-*d*₄) δ 8.81 (d, *J* = 4.9 Hz, 1H), 8.80 (d, *J* = 4.9 Hz, 1H), 8.13 (d, *J* = 5.9 Hz, 1H), 7.91 (d, *J* = 8.6 Hz, 1H), 7.30 (dt, *J* = 2.7, 4.9 Hz, 1H), 6.76 (d, *J* = 6.6 Hz, 1H), 6.45 (dd, *J* = 8.8, 10.5 Hz, 1H), [4.51 (quin, *J* = 5.6 Hz) and 4.45 (quin, *J* = 5.6 Hz), total 1H], [4.29 (q, *J* = 6.8 Hz) and 4.21 (q, *J* = 6.9 Hz), total 1H], [3.89 (d, *J* = 5.9 Hz) and 3.82 - 3.64 (m), total 5H], 3.59 - 3.38 (m, 2H), [2.61 (s) and 2.57 (s), total 3H], [2.40 - 2.28 (m) and 2.27 - 2.15 (m), total 1H], 1.99 (qd, *J* = 6.5, 13.1 Hz, 1H), [1.42 (d, *J* = 6.8 Hz) and 1.38 (d, *J* = 6.8 Hz), total 3H].

A smaller-scale reaction using this procedure was carried out using the same lot of **P14** (4.40 g, 20.4 mmol); the crude product was dissolved in a minimal amount of propan-2-ol (approximately 40 mL) at 50 °C. The solution was allowed to cool to room temperature and slurried overnight, whereupon the solid was isolated via filtration. This yielded a crystal for X-ray structural determination of**6**.

### Single-crystal X-ray structural determination of 6

### Single Crystal X-Ray Analysis

Data collection was performed on a Bruker D8 Venture diffractometer at -100 °C. Data collection consisted of omega and phi scans.

The structure was solved by intrinsic phasing using SHELX software suite in the monoclinic class space group *P*2₁. The structure was subsequently refined by the full-matrix least squares method. All non-hydrogen atoms were found and refined using anisotropic displacement parameters.

The hydrogen atoms located on nitrogen were found from the Fourier difference map and refined with distances restrained. The remaining hydrogen atoms were placed in calculated positions and were allowed to ride on their carrier atoms. The final refinement included isotropic displacement parameters for all hydrogen atoms.

TWIN Law as matrix: -1 0 0 0 -1 0 0.997 0 1 with BASF of 0.18 was applied to the refinement L.S. cycle to improve fitting the molecular module into the experimental data.

Analysis of the absolute structure using likelihood methods (Hooft, 2008) was performed using PLATON (Spek). The results indicate that the absolute structure has been correctly assigned. The method calculates that the probability that the structure is correctly assigned is 100%. The Hooft parameter is reported as 0.054 with an esd (estimated standard deviation) of (8) and the Parson's parameter is reported as 0.150 with an esd of (8).

The asymmetric unit is comprised of four molecules of **6**. The absolute configurations at C7_C12 / C30_C35 / C53_C58 / C76_C81 were confirmed as (-R)_(-S) for all four molecules in the asymmetric unit.

The final R-index was 4.6%. A final difference Fourier revealed no missing or misplaced electron density.

### Software and References

See list provided above for Single-crystal X-ray structural determination of **3A**.

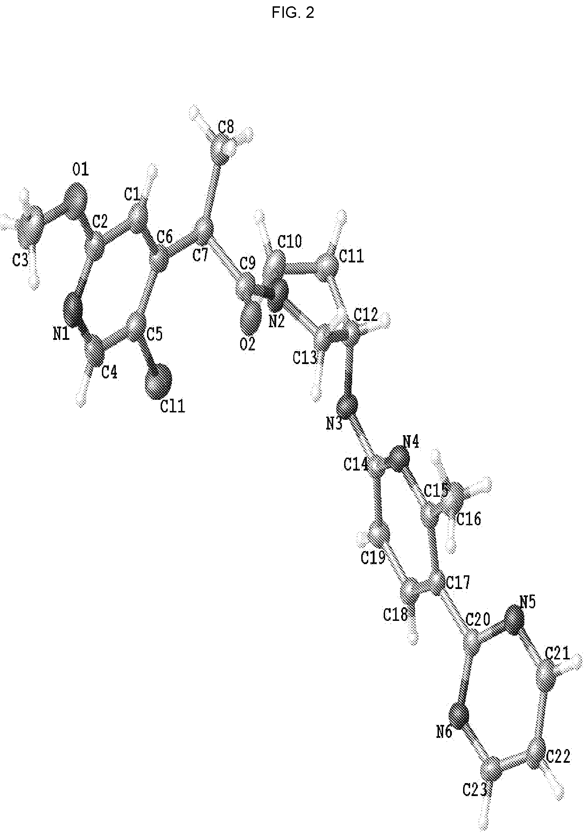

The absolute stereochemistry of compound Example 6 was determined by single crystal X-ray crystallography (using pertinent data/parameters/information used/collected, including, e.g., pertinent crystal data and refinement information; atomic coordinates; bond lengths; bond angles; and displacement parameters). Figure 2 (FIG. 2) is an obtained single crystal X-ray structure (ORTEP drawing) of the crystalline compound Example 6: (2*R*)-2-(5-Chloro-2-methoxypyridin-4-yl)-1-[(3*S*)-3-{[6-methyl-5-(pyrimidin-2-yl)pyridin-2-yl]amino}pyrrolidin-1-yl]propan-1-one.

In some embodiments, the present invention provides a crystalline form of (2*R*)-2-(5-Chloro-2-methoxypyridin-4-yl)-1-[(3*S*)-3-{[6-methyl-5-(pyrimidin-2-yl)pyridin-2-yl]amino}pyrrolidin-1-yl]propan-1-one. In some further embodiments, the crystalline form of (2R)-2-(5-Chloro-2-methoxypyridin-4-yl)-1-[(3*S*)-3-{[6-methyl-5-(pyrimidin-2-yl)pyridin-2-yl]amino}pyrrolidin-1-yl]propan-1-one is the one described (and/or as prepared) in Example 6.

### Example 7

### (2R)-1-[(3S)-3-({5-[5-(Difluoromethyl)-1-methyl-1H-1,2,4-triazol-3-yl]-6-methylpyridin-2-yl}amino)pyrrolidin-1-yl]-2-(5-fluoro-2-methoxypyridin-4-yl)propan-1-one (7)

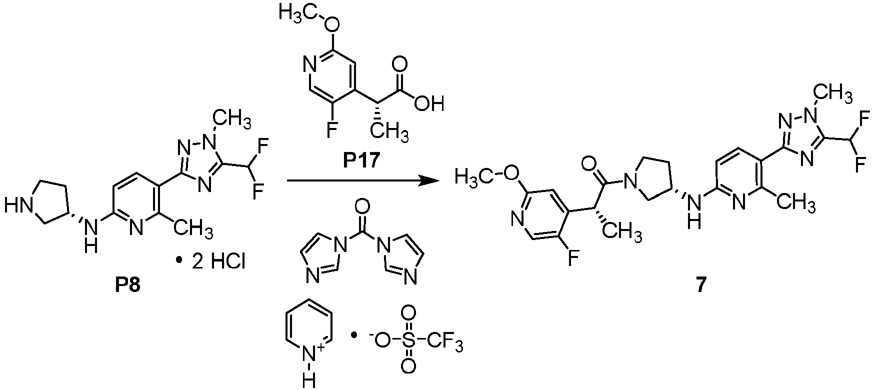

To a room temperature solution of **P17** (31 g, 160 mmol) and pyridinium trifluoromethanesulfonate (74.9 g, 327 mmol) in acetonitrile (620 mL) was added 1,1-carbonyldiimidazole (25.2 g, 156 mmol). After the reaction mixture had been stirred for 25 minutes at room temperature, **P8** (48.0 g, 126 mmol) was added and stirring was continued for 3 hours. The reaction mixture was poured into water and the organic layer was washed twice with water; the combined aqueous layers were extracted three times with ethyl acetate, and the combined organic layers were washed with saturated aqueous sodium bicarbonate solution and with saturated aqueous sodium chloride solution, then dried over sodium sulfate, filtered, and concentrated *in vacuo.* Dichloromethane was then added to the residue, followed by silica gel (150 g) and the resulting slurry was concentrated *in vacuo.* This mixture was loaded onto a filter pad and eluted with 70% ethyl acetate in heptane. Concentration of the eluent *in vacuo* provided a residue (45 g), which was treated with ethyl acetate (100 mL) and heated to 50 °C. Heptane (100 mL) was added slowly with stirring until the mixture remained cloudy, whereupon a small amount of ethyl acetate was added until the mixture just clarified. Heating was ceased at this point, and the mixture was allowed to cool slowly to room temperature. It was then heated to 40 °C, cooled, and held at 30 °C until solids appeared, whereupon the mixture was allowed to cool to room temperature and stir overnight. The solids were collected via filtration to provide (2*R*)-1-[(3*S*)-3-({5-[5-(difluoromethyl)-1-methyl-1H-1,2,4-triazol-3-yl]-6-methylpyridin-2-yl}amino)pyrrolidin-1-yl]-2-(5-fluoro-2-methoxypyridin-4-yl)propan-1-one (**7**) as a solid. Analysis of the ¹H NMR indicated that this material comprised a mixture of rotamers. Yield: 26.0 g, 53.1 mmol, 42%. LCMS *m*/*z* 490.5 [M+H]⁺. ¹H NMR (400 MHz, methanol-*d*₄) δ [7.98 (d, *J* = 1.0 Hz) and 7.97 (d, *J* = 1.0 Hz), total 1H], 7.87 (d, *J* = 8.6 Hz, 1H), [7.11 (t, *J =* 52.3 Hz) and 7.10 (t, *J* = 52.3 Hz), total 1H], [6.74 (d, *J* = 4.7 Hz) and 6.73 (d, *J* = 4.7 Hz), total 1H], [6.43 (d, *J* = 9.0 Hz) and 6.40 (d, *J* = 9.0 Hz), total 1H], 4.53 - 4.41 (m, 1H), [4.25 (q, *J* = 6.9 Hz) and 4.16 (q, *J* = 6.9 Hz), total 1H], 4.07 (br s, 3H), [3.90 - 3.84 (m), 3.88 (s), 3.86 (s), 3.82 - 3.63 (m), 3.58 - 3.46 (m), and 3.42 - 3.46 (m), total 7H], [2.63 (s) and 2.60 (s), total 3H], [2.39 - 2.28 (m) and 2.25 - 2.15 (m), total 1H], 2.05 - 1.90 (m, 1H), [1.44 (d, *J* = 6.8 Hz) and 1.41 (d, *J* = 7.1 Hz), total 3H].

A sample of Example 7 was dissolved in a minimal amount of ethyl acetate with stirring, whereupon heptane was slowly added until the mixture became cloudy. A solution of 20% ethyl acetate in heptane was slowly added until there was sufficient solvent to allow the mixture to slurry; stirring was then carried out for 2 hours. Collection of the solid via vacuum filtration, followed by drying at 50 °C on a rotary evaporator for 1 hour, followed by evacuation using high vacuum, afforded crystals of Example 7 appropriate for single-crystal X-ray structure determination.

### Single-crystal X-ray structural determination of 7

### Single Crystal X-Ray Analysis

Data collection was performed on a Bruker D8 Venture diffractometer at -150 °C. Data collection consisted of omega and phi scans.

The structure was solved by intrinsic phasing using SHELX software suite in the monoclinic class space group *P*2₁. The structure was subsequently refined by the full-matrix least squares method. All non-hydrogen atoms were found and refined using anisotropic displacement parameters.

The hydrogen atoms located on nitrogen were found from the Fourier difference map and refined with distances restrained. The remaining hydrogen atoms were placed in calculated positions and were allowed to ride on their carrier atoms. The final refinement included isotropic displacement parameters for all hydrogen atoms.

Analysis of the absolute structure using likelihood methods (Hooft, 2008) was performed using PLATON (Spek). The results indicate that the absolute structure has been correctly assigned. The method calculates that the probability that the structure is correctly assigned is 100%. The Hooft parameter is reported as 0.04 with an esd (estimated standard deviation) of (5) and the Parson's parameter is reported as 0.08 with an esd of (4).

The asymmetric unit is comprised of two molecules of **7**. The absolute configurations at C7_C12 / C30_C35 were confirmed as (-R)_(-S) / (-R)_(-S) for both molecules in the asymmetric unit.

The final R-index was 4.0%. A final difference Fourier revealed no missing or misplaced electron density.

### Software and References

### See list provided above for Single-crystal X-ray structural determination of 3A.

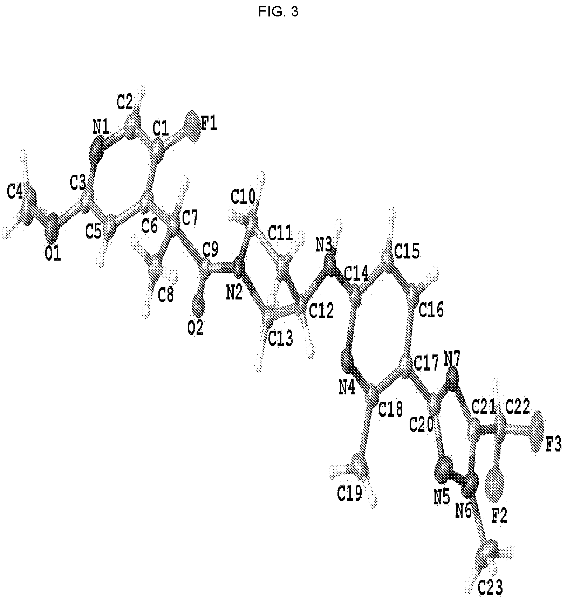

The absolute stereochemistry of compound Example 7 was determined by single crystal X-ray crystallography (using pertinent data/parameters/information used/collected, including, e.g., pertinent crystal data and refinement information; atomic coordinates; bond lengths; bond angles; and displacement parameters). Figure 3 (FIG. 3) is an obtained single crystal X-ray structure (ORTEP drawing) of the crystalline compound Example 7: (2*R*)-1-[(3*S*)-3-({5-[5-(Difluoromethyl)-1-methyl-1*H*-1,2,4-triazol-3-yl]-6-methylpyridin-2-yl}amino)pyrrolidin-1-yl]-2-(5-fluoro-2-methoxypyridin-4-yl)propan-1-one.

In some embodiments, the present invention provides a crystalline form of (2*R*)-1-[(3*S*)-3-({5-[5-(Difluoromethyl)-1-methyl-1*H*-1,2,4-triazol-3-yl]-6-methylpyridin-2-yl}amino)pyrrolidin-1-yl]-2-(5-fluoro-2-methoxypyridin-4-yl)propan-1-one. In some further embodiments, the crystalline form of (2*R*)-1-[(3*S*)-3-({5-[5-(Difluoromethyl)-1-methyl-1*H*-1,2,4-triazol-3-yl]-6-methylpyridin-2-yl}amino)pyrrolidin-1-yl]-2-(5-fluoro-2-methoxypyridin-4-yl)propan-1-one is the one described (and/or as prepared) in Example 7.

### Example 8

### (2R)-1-[(3S)-3-({5-[1-Cyclopropyl-5-(difluoromethyl)-1H-1,2,4-triazol-3-yl]-6-methylpyridin-2-yl}amino)pyrrolidin-1-yl]-2-(5-fluoro-2-methoxypyridin-4-yl)propan-1-one (8)

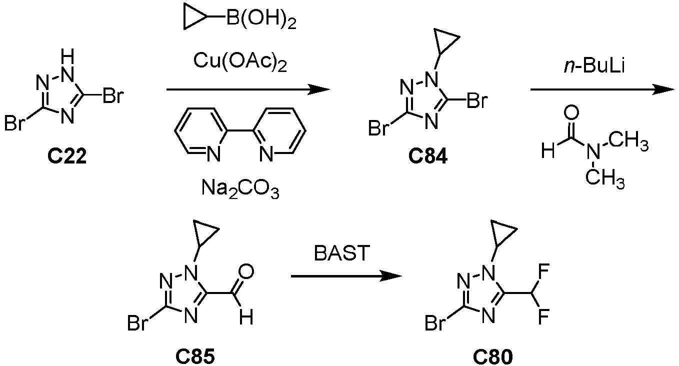

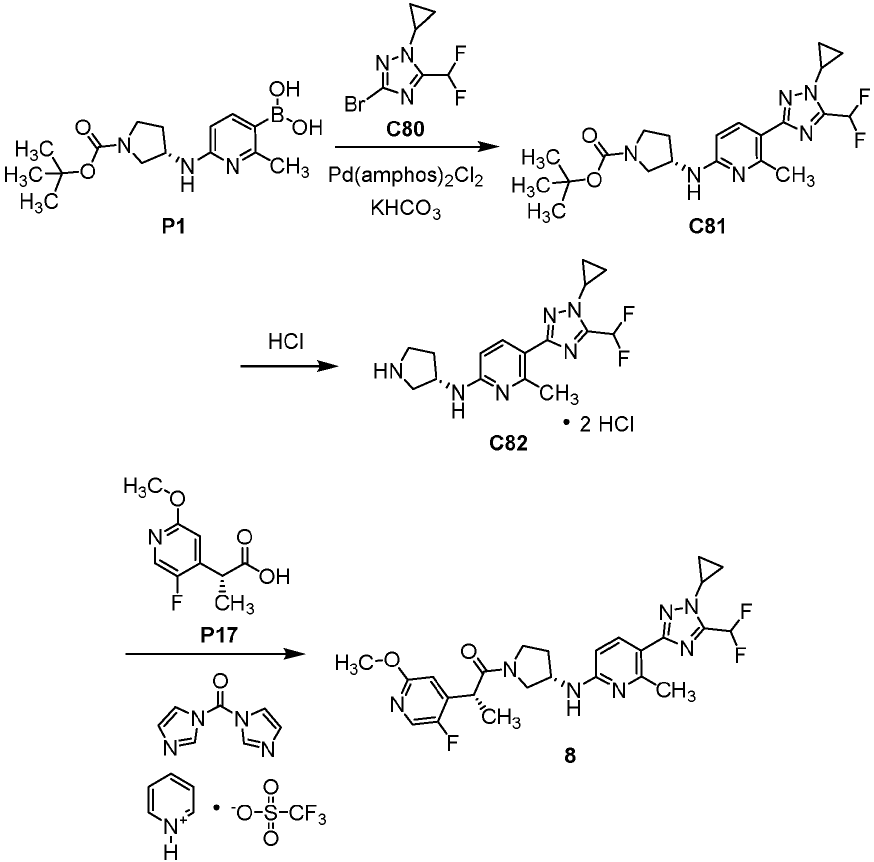

### Step 1. Synthesis of 3,5-dibromo-1-cyclopropyl-1H-1,2,4-triazole (C84).

A mixture of **C22** (6.00 g, 26.4 mmol), cyclopropylboronic acid (4.54 g, 52.8 mmol), 2,2'-bipyridine (4.13 g, 26.4 mmol), copper(II) acetate (4.79 g, 26.4 mmol), and sodium carbonate (5.60 g, 52.8 mmol) in 1,2-dichloroethane (100 mL) was stirred at 70 °C for 3 hours. After the reaction mixture had been concentrated *in vacuo,* the residue was purified using silica gel chromatography (Gradient: 0% to 10% ethyl acetate in petroleum ether) to afford **C84** as a colorless oil. Yield: 1.55 g, 5.81 mmol, 22%. LCMS *m*/*z* 265.9 (dibromo isotope pattern observed) [M+H]⁺. ¹H NMR (400 MHz, methanol-*d*₄) δ 3.63 (tt, *J* = 6.5, 4.2 Hz, 1H), 1.23 - 1.14 (m, 4H).

### Step 2. Synthesis of 3-bromo-1-cyclopropyl-1H-1,2,4-triazole-5-carbaldehyde (C85).

A solution of *n*-butyllithium (2.5 M; 2.47 mL, 6.18 mmol) was added drop-wise to a -78 °C solution of **C84** (1.50 g, 5.62 mmol) in diethyl ether (10 mL). After the reaction mixture had been stirred at -78 °C for 50 minutes, N,N-dimethylformamide (820 mg, 11.2 mmol) was added, and stirring was continued at -40 °C for 30 minutes. The reaction mixture was then poured into aqueous ammonium chloride solution and extracted with ethyl acetate (20 mL), whereupon the organic layer was washed with saturated aqueous sodium chloride solution, dried over sodium sulfate, filtered, and concentrated *in vacuo.* Chromatography on silica gel (Gradient: 0% to 10% ethyl acetate in petroleum ether) provided **C85** as a colorless oil. Yield: 620 mg, 2.87 mmol, 51%. ¹H NMR (400 MHz, chloroform-d) δ 9.94 (s, 1H), 4.42 (tt, *J* = 7.6, 3.9 Hz, 1H), 1.39 - 1.32 (m, 2H), 1.21 - 1.14 (m, 2H).

### Step 3. Synthesis of 3-bromo-1-cyclopropyl-5-(difluoromethyl)-1H-1,2,4-triazole (C80).

[Bis(2-methoxyethyl)amino]sulfur trifluoride (BAST; 1.06 mL, 5.75 mmol) was added drop-wise to a solution of **C85** (620 mg, 2.87 mmol) in dichloromethane (10 mL), while the reaction temperature was maintained below 10 °C. After the reaction mixture had been stirred at 25 °C for 16 hours, it was poured into aqueous sodium bicarbonate solution (50 mL) and extracted with dichloromethane (30 mL). The organic layer was washed with saturated aqueous sodium chloride solution, dried over sodium sulfate, filtered, and concentrated *in vacuo* to provide **C80** as a colorless oil. Yield: 390 mg, 1.64 mmol, 57%. LCMS *m*/*z* 238.0 (bromine isotope pattern observed) [M+H]⁺. ¹H NMR (400 MHz, methanol-*d*₄) δ 7.14 (t, *J*_{HF} = 52.1 Hz, 1H), 3.87 - 3.78 (m, 1H), 1.31 - 1.23 (m, 2H), 1.21 - 1.14 (m, 2H).

### Step 4. Synthesis of tert-butyl (3S)-3-({5-[1-cyclopropyl-5-(difluoromethyl)-1H-1,2,4-triazol-3-yl]-6-methylpyridin-2-yl}amino)pyrrolidine-1-carboxylate (C81).

A mixture of **C80** (130 mg, 0.546 mmol), **P1** (175 mg, 0.546 mmol), Pd(amphos)₂Cl₂ (38.7 mg, 54.6 µmol), and potassium bicarbonate (164 mg, 1.64 mmol) in 1,4-dioxane (3 mL) and water (0.7 mL) was heated at 95 °C for 8 hours, whereupon the reaction mixture was concentrated *in vacuo,* and the residue was poured into water and extracted with ethyl acetate (2 x 50 mL). The combined organic layers were washed with saturated aqueous sodium chloride solution, dried over sodium sulfate, filtered, and concentrated *in vacuo.* Purification via silica gel chromatography (Gradient: 0% to 50% ethyl acetate in petroleum ether) provided **C81** as a colorless gum (76 mg) that contained an unknown impurity. Most of this material was taken directly to the following step, without further purification. LCMS *m*/*z* 435.3 [M+H]⁺. ¹H NMR (400 MHz, methanol-*d*₄), product peaks only: δ 7.87 (d, *J* = 8.6 Hz, 1H), 7.18 (t, *J*_{HF} = 52.6 Hz, 1H), 6.44 (d, *J* = 8.6 Hz, 1H), 4.53 - 4.43 (m, 1H), 3.86 (tt, *J* = 3.6, 7.2 Hz, 1H), [3.74 - 3.63 (m), 3.59 - 3.37 (m), and 3.24 (dd, *J* = 4.6, 11.0 Hz), total 4H], 2.61 (s, 3H), 2.30 - 2.16 (m, 1H), 2.00 - 1.85 (m, 1H), [1.47 (s) and 1.46 (s), total 9H], 1.36 - 1.28 (m, 2H), 1.22 - 1.15 (m, 2H).

### Step 5. Synthesis of 5-[1-cyclopropyl-5-(difluoromethyl)-1H-1,2,4-triazol-3-yl]-6-methyl-N-[(3S)-pyrrolidin-3-yl]pyridin-2-amine, dihydrochloride salt (C82).

To a 25 °C solution of **C81** (from the previous step; 70 mg, <0.16 mmol) in methanol (2 mL) was added a solution of hydrogen chloride in 1,4-dioxane (4 M; 0.40 mL, 1.6 mmol) and the reaction mixture was stirred for 3 hours. Concentration *in vacuo* afforded **C82** as a colorless gum (70 mg), which was progressed to the next step without purification. LCMS *m*/*z* 335.2 [M+H]⁺.

### Step 6. Synthesis of (2R)-1-[(3S)-3-({5-[1-cyclopropyl-5-(difluoromethyl)-1H-1,2,4-triazol-3-yl]-6-methylpyridin-2-yl}amino)pyrrolidin-1-yl]-2-(5-fluoro-2-methoxypyridin-4-yl)propan-1-one (8).

A 25 °C solution of **P17** (32.8 mg, 0.165 mmol), 1,1'-carbonyldiimidazole (29.3 mg, 0.181 mmol) and pyridinium trifluoromethanesulfonate (75.3 mg, 0.329 mmol) in acetonitrile (2 mL) was stirred for 10 minutes, whereupon **C82** (from the previous step; 67 mg, ≤0.15 mmol) was added and the reaction mixture was stirred for 16 hours. It was then poured into water and extracted with ethyl acetate (2 x 30 mL); the combined organic layers were washed with saturated aqueous sodium chloride solution, dried over sodium sulfate, filtered, and concentrated *in vacuo.* Purification via reversed-phase C18 chromatography (Gradient: 0% to 50% acetonitrile in water) provided (2*R*)-1-[(3*S*)-3-({5-[1-cyclopropyl-5-(difluoromethyl)-1H-1,2,4-triazol-3-yl]-6-methylpyridin-2-yl}amino)pyrrolidin-1-yl]-2-(5-fluoro-2-methoxypyridin-4-yl)propan-1-one (**8**) as a white solid. Analysis of the ¹H NMR indicated that this material comprised a mixture of rotamers. Yield: 39 mg, 76 µmol, 16% over 3 steps. LCMS *m*/*z* 516.3 [M+H]⁺. ¹H NMR (400 MHz, methanol-*d*₄) δ [7.98 (d, *J* = 1.5 Hz) and 7.97 (d, *J* = 1.5 Hz), total 1H], 7.86 (d, *J* = 8.6 Hz, 1H), [7.19 (t, *J*_{HF} = 52.3 Hz) and 7.18 (t, *J*_{HF} = 52.3 Hz), total 1H], [6.74 (d, *J* = 4.4 Hz) and 6.73 (t, *J* = 4.7 Hz), total 1H], [6.42 (d, *J* = 8.8 Hz) and 6.40 (d, *J* = 8.8 Hz), total 1H], 4.52 - 4.41 (m, 1H), [4.25 (q, *J* = 7.0 Hz) and 4.16 (q, *J* = 7.1 Hz), total 1H], [3.90 - 3.83 (m), 3.88 (s), 3.86 (s), 3.82 - 3.64 (m), 3.57 - 3.46 (m), and 3.42 - 3.36 (m), total 8H], [2.61 (s) and 2.58 (s), total 3H], [2.38 - 2.28 (m) and 2.26 - 2.14 (m), total 1H], 2.03 - 1.92 (m, 1H), [1.44 (d, *J* = 6.8 Hz) and 1.40 (d, *J* = 6.8 Hz), total 3H], 1.37 - 1.29 (m, 2H), 1.23 - 1.14 (m, 2H).

**Table 1. Method of synthesis, structure, and physicochemical data for Examples 9 - 44B.**

| Exam ple Numb er | Method of synthesis; Non-commercial starting materials | Structure | ¹H NMR (400 MHz, methanol-*d*₄) δ; Mass spectrum, observed ion *m*/*z* [M+H]⁺ or HPLC retention time; Mass spectrum *m*/*z* [M+H]⁺ (unless otherwise indicated) |
|---|---|---|---|
| **9** | Example 3B; **P4** | 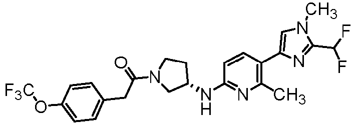 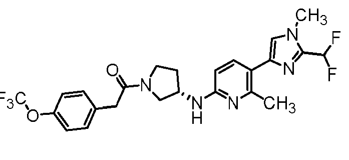 | 7.62 (dd, *J* = 2.2, 8.6 Hz, 1H), 7.38 (d, *J* = 8.6 Hz, 1H), 7.33 (d, *J* = 8.8 Hz, 1H), 7.28 (s, 1H), 7.23 (d, *J = 8.1* Hz, 1H), 7.17 (d, *J* = 8.1 Hz, 1H), [6.88 (t, *J* = 52.6 Hz), 6.87 (t, *J* = 52.6 Hz) total 1H], 6.42 (t, *J* = 8.7 Hz, 1H), 4.54 - 4.40 (m, 1H), [3.93 (br dd, *J* = 5.6, 10.8 Hz), 3.90-3.85 (m), 3.85-3.65 (m), 3.59-3.47 (m), 3.42 (dd, *J =* 4.5, 12.3 Hz), total 9H], [2.46 (s), 2.45 (s), total 3H], 2.39 - 2.19 (m, 1H), 2.11 - 1.89 (m, 1H); 510.2 |
| **10** | Example 3B; **P4** | 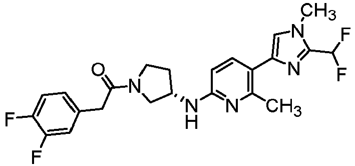 | [7.62 (d, *J* = 8.5 Hz), 7.61 (d, *J* = 8.5 Hz) total 1H], 7.28 (s, 1H), 7.25 - 6.97 (m, 3H), [6.88 (t, *J* = 52.6 Hz), 6.87 (t, *J* = 52.6 Hz) total 1H], [6.43 (d, *J* = 8.6 Hz), 6.40 (d, *J* = 8.5 Hz), total 1H], 4.53 - 4.41 (m, 1H), [3.93 - 3.85 (m), 3.85 - 3.62 (m), 3.60 - 3.47 (m), 3.42 (dd, *J* = 4.4, 12.2 Hz), total 9H], [2.46 (s), 2.45 (s), total 3H], 2.39 - 2.18 (m, 1H), 2.11 - 1.90 (m, 1H); 462.2 |
| **11** | Example 3B; **P4** | 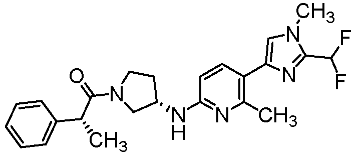 | 7.60 (d, *J* = 8.6 Hz, 1H), 7.37 - 7.20 (m, 6H), [6.87 (t, *J* = 52.6 Hz), 6.87 (t, *J* = 52.6 Hz) total 1H], [6.37 (d, *J* = 8.5 Hz), 6.37 (d, *J* = 8.5 Hz) total 1H], [4.41 (quin, *J* = 5.9 Hz), 4.31 (quin, *J* = 5.8 Hz), total 1H], [3.94 (q, *J* = 6.9 Hz), 3.92 - 3.82 (m), 3.77 - 3.59 (m), 3.54 - 3.33 (m), total 8H], [2.44 (s), 2.41 (s) total 3H], [2.28 (dt, *J* = 6.2, 13.1 Hz), 2.20 - 2.08 (m), total 1H], 1.97 - 1.81 (m, 1H), [1.40 (d, *J* = 7.0 Hz), 1.38 (d, *J =* 7.0 Hz) total 3H]; 440.2 |
| **12A** | Example 3B^{1,2,3}; **P4** | 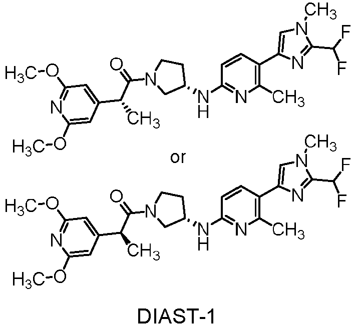 | 2.23 minutes⁴; 501.5 |
| **12B** | Example 3B^{1,2,3}; **P4** | 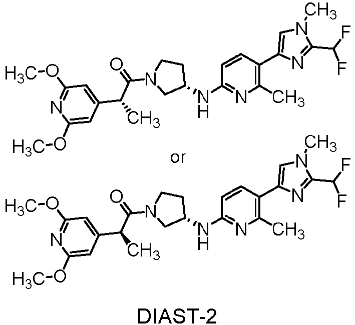 | 2.41 minutes⁴; 501.5 |
| **13A** | Example 3B^{1,5}; **P2** | 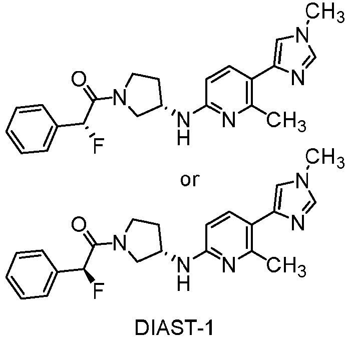 | 2.99 minutes⁶; 394.4 |
| **13B** | Example 3B^{1,5}; **P2** | 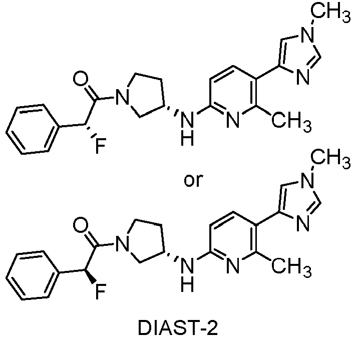 | 3.69 minutes⁶; 394.4 |
| **14A** | Synthesis of **C11**⁷; **P10** | 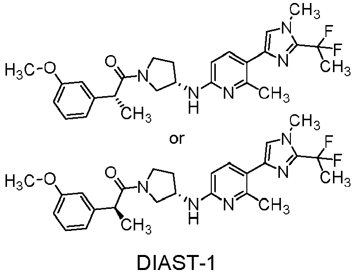 | 3.17 minutes⁸; 484.3 |
| **14B** | Synthesis of **C11⁷**; **P10** | 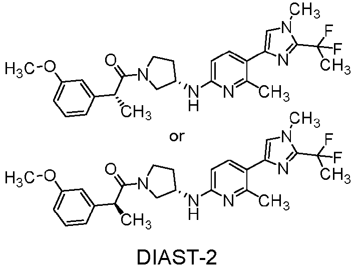 | 7.60 (d, *J = 8.6* Hz, 1H), 7.27 - 7.19 (m, 2H), 6.89 - 6.84 (m, 2H), 6.84 - 6.78 (m, 1H), [6.37 (d, *J =* 8.3 Hz), 6.37 (d, *J* = 8.6 Hz) total 1H], [4.40 (quin, *J* = 5.7 Hz), 4.31 (quin, *J* = 5.8 Hz) total 1H], [3.98 - 3.75 (m), 3.75 - 3.61 (m), 3.54 - 3.38 (m) total 11H], [2.44 (s), 2.40 (s) total 3H], [2.27 (quin, *J* = 6.4 Hz), 2.21 - 2.13 (m), 2.123 (t, *J =* 19.1 Hz), 2.116 (t, *J* = 18.8 Hz) total 4H], 1.98 - 1.81 (m, 1H), [1.39 (d, *J* = 7.3 Hz), 1.36 (d, *J* = 7.1 Hz) total 3H]; 3.18 minutes⁸; 484.2 |
| **15A** | General Procedure 1⁹; **P10** | 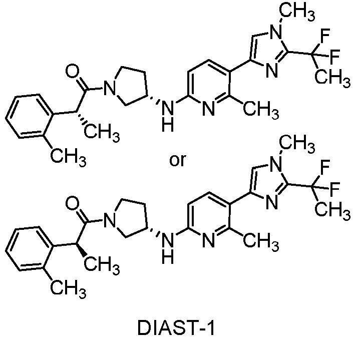 | 3.19 minutes⁸; 468.3 |
| **15B** | General Procedure 1⁹; **P10** | 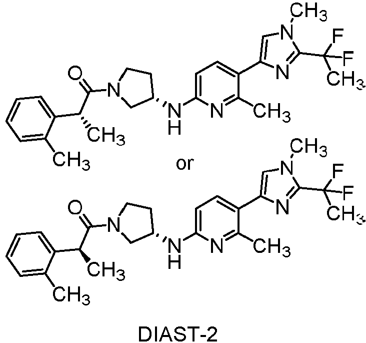 | [7.60 (d, *J* = 8.3 Hz), 7.58 (d, *J =* 8.6 Hz) total 1H], 7.24 - 7.09 (m, 5H), [6.36 (d, *J* = 8.1 Hz), 6.34 (d, *J* = 8.3 Hz) total 1H], [4.39 (quin, *J* = 5.7 Hz), 4.20 (quin, *J* = 5.7 Hz), total 1H], [4.06 (q, *J* = 6.8 Hz), 3.96 (q, *J* = 6.9 Hz) total 1H], [3.92 - 3.85 (m), 3.74 - 3.60 (m), 3.55 - 3.45 (m), 3.45 - 3.33 (m), 3.18 - 3.07 (m), total 7H], [2.44 (s), 2.40 (s), total 3H], [2.38 (s), 2.36 (s) total 3H], [2.31 - 2.19 (m), 2.17 - 2.09 (m), 2.122 (t, *J* = 18.8 Hz), 2.117 (t, *J* = 19.1 Hz) total 4H], [1.97 - 1.86 (m), 1.85 - 1.75 (m) total 1H], [1.34 (d, *J* = 6.8 Hz), 1.31 (d, *J* = 6.8 Hz) total 3H]; 3.31 minutes⁸; 468.2 |
| **16A** | General Procedure 1^{10,11}; **P10** | 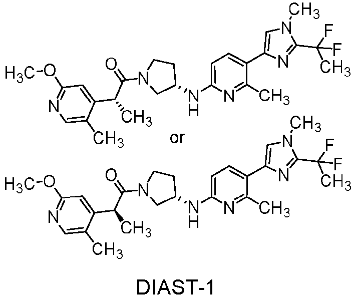 | 3.02 minutes⁸; 499.2 |
| **16B** | General Procedure 1^{10,11}; **P10** | 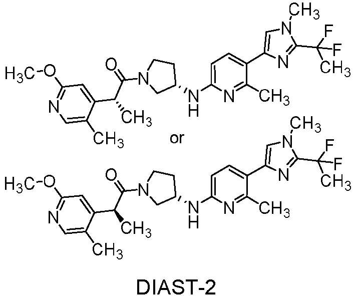 | [7.93 (s), 7.92 (s) total 1H], [7.60 (d, *J =* 8.3 Hz), 7.59 (d, *J* = 8.6 Hz) total 1H], 7.21 (s, 1H), [6.62 (s), 6.61 (s) total 1H], [6.380 (d, *J* = 8.3 Hz), 6.376 (d, *J* = 8.3 Hz) total 1H], [4.41 (quin, J = 5.6 Hz), 4.29 (quin, J = 5.8 Hz) total 1H], [4.05 (q, *J* = 6.8 Hz), 3.96 (q, *J =* 6.8 Hz) total 1H], [3.90 - 3.84 (m), 3.86 (s), 3.84 (s), 3.77 - 3.65 (m), 3.57 - 3.47 (m), 3.47 - 3.38 (m), 3.29 - 3.21 (m) total 10H], [2.44 (s), 2.39 (s) total 3H], [2.34 - 2.25 (m), 2.28 (s), 2.26 (s), 2.24 - 2.13 (m), 2.122 (t, *J* = 19.1 Hz), 2.118 (t, *J* = 19.1 Hz) total 7H], 1.99 - 1.85 (m, 1H), [1.37 (d, *J* = 6.8 Hz), 1.33 (d, *J* = 6.8 Hz) total 3H]; 3.07 minutes⁸; 499.3 |
| **17** | Example 1¹²; **P17, P25, P1** | 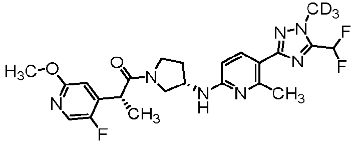 | ¹H NMR (400 MHz, chloroform-d) δ 8.03 (br d, *J* = 8.6 Hz, 1H), 7.97 (d, *J* = 1.2 Hz, 1H), [6.88 (t, *J =* 52.7 Hz), 6.87 (t, *J* = 52.7 Hz) total 1H], [6.79 (d, *J* = 4.7 Hz), 6.78 (d, *J* = 5.1 Hz) total 1H], [6.33 (br d, *J* = 8.6 Hz), 6.31 (br d, *J* = 8.6 Hz) total 1H], 4.91 (br s, 1H), 4.41 - 4.32 (m, 1H), [4.13 - 4.06 (m), 4.06 - 3.93 (m), 3.79 - 3.54 (m), 3.50 - 3.35 (m) total 5H], 3.90 (s, 3H), [2.714 (s), 2.706 (s), total 3H], [2.40 - 2.30 (m), 2.29 - 2.19 (m) total 1H], [2.05 - 1.96 (m), 1.96 - 1.86 (m), total 1H], [1.47 (d, *J* = 7.0 Hz), 1.44 (d, *J* = 6.6 Hz) total 3H]; 493.2 |
| **18** | Synthesis of **C79b**, Example 1¹³; **P14, P1** | 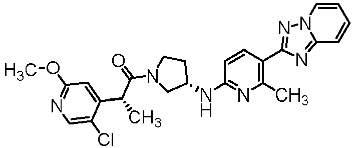 | ¹H NMR (400 MHz, DMSO-*d*₆) δ 8.95 - 8.91 (m, 1H), [8.25 (s), 8.22 (s) total 1H], [8.096 (d, *J* = 8.3 Hz), 8.092 (d, *J* = 8.8 Hz) total 1H], 7.82 - 7.77 (m, 1H), 7.67 - 7.60 (m, 1H), [7.18 - 7.10 (m), 7.01 (br d, *J* = 5.6 Hz) total 2H], [6.74 (s), 6.71 (s) total 1H], [6.46 (d, *J* = 8.6 Hz)*,* 6.43 (d, *J* = 8.3 Hz) total 1H], 4.41 - 4.27 (m, 1H), [4.16 (q, *J* = 6.6 Hz), 4.10 (q, *J =* 6.8 Hz) total 1H], [3.85 (s), 3.84 (s) total 3H], [3.75 - 3.64 (m), 3.64 - 3.48 (m), 3.48 - 3.35 (m), 3.30 - 3.24 (m) total 4H], [2.75 (s), 2.70 (s) total 3H], [2.31 - 2.17 (m), 2.17 - 2.03 (m) total 1H], 1.98 - 1.80 (m, 1H), [1.33 (d, *J* = 6.8 Hz), 1.29 (d, *J* = 6.8 Hz) total 3H]. 492.3 |
| **20A** | **P2**^{14,15,16} | 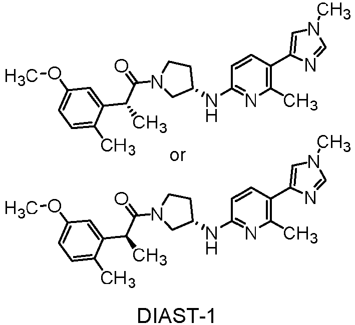 | 1.35 minutes¹⁷; 434.3 |
| **20B** | **P2**^{14,15,16} | 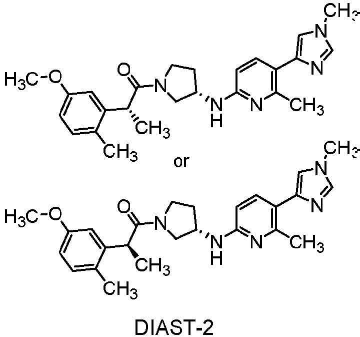 | [7.704 (br s), 7.695 (br s) total 1H], [7.60 (d, *J* = 8.6 Hz), 7.58 (d, *J* = 8.6 Hz) total 1H], [7.144 (d, *J* = 1.2 Hz), 7.136 (d, *J* = 1.2 Hz) total 1H], [7.11 (d, *J* = 8.3 Hz), 7.10 (d, *J* = 7.8 Hz) total 1H], 6.76 - 6.67 (m, 2H), [6.37 (d, *J* = 8.8 Hz), 6.35 (d, *J* = 8.8 Hz) total 1H], [4.39 (quin, *J* = 5.7 Hz), 4.21 (quin, *J* = 5.7 Hz) total 1H], [4.02 (q, *J* = 6.8 Hz), 3.92 (q, *J =* 5.9 Hz) total 1H], [3.90 - 3.83 (m), 3.78 (s), 3.74 (s), 3.73 - 3.67 (m), 3.69 (s), 3.55 - 3.45 (m), 3.42 - 3.35 (m), 3.20 - 3.10 (m) total 10H], [2.44 (s), 2.39 (s) total 3H], [2.32 (s), 2.28 (s) total 3H], [2.28 - 2.23 (m), 2.19 - 2.08 (m), 1.97 - 1.87 (m), 1.86 - 1.77 (m) total 2H], [1.33 (d, *J* = 6.8 Hz), 1.30 (d, *J =* 6.8 Hz) total 3H]; 2.51 minutes⁸; 434.3 |
| **21A** | General Procedure 1¹⁸; **P16**, **P10** | 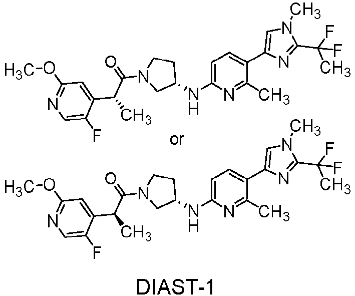 | 3.12 minutes⁸; 503.2 |
| **21B** | General Procedure 1¹⁸; **P16**, **P10** | 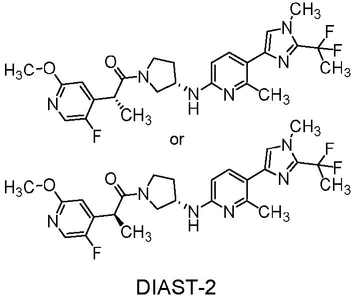 | [7.98 (d, *J* = 1.5 Hz), 7.97 (d, *J* = 1.5 Hz) total 1H], [7.621 (d, *J =* 8.3 Hz), 7.618 (d, *J* = 8.6 Hz) total 1H], [7.218 (s), 7.216 (s), total 1H], [6.74 (d, *J* = 4.9 Hz), 6.73 (d, *J* = 4.9 Hz) total 1H], [6.42 (d, *J =* 8.6 Hz), 6.40 (d, *J* = 8.3 Hz) total 1H], 4.49 - 4.36 (m, 1H), [4.25 (q, *J* = 6.7 Hz), 4.16 (q, *J* = 6.9 Hz) total 1H], [3.93 - 3.81 (m), 3.81 - 3.63 (m), 3.57 - 3.46 (m), 3.42 - 3.36 (m), total 10H], [2.45 (s), 2.43 (s), total 3H], [2.39 - 2.26 (m), 2.26 - 2.18 (m), 2.123 (t, *J* = 18.8 Hz), 2.118 (t, *J* = 18.8 Hz), 2.05 - 1.89 (m) total 5H], [1.44 (d, *J* = 6.8 Hz), 1.40 (d, *J* = 6.8 Hz) total 3H]; 3.14 minutes⁸; 503.2 |
| **22A** | General Procedure 1^{10,19}; **P7** | 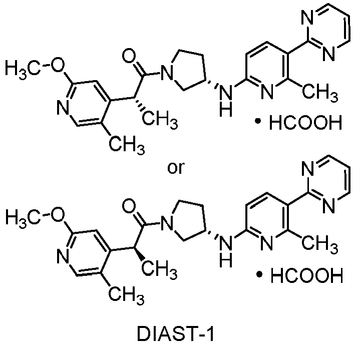 | 2.69 minutes⁸; 433.3 |
| **22B** | General Procedure 1^{10,19}; **P7** | 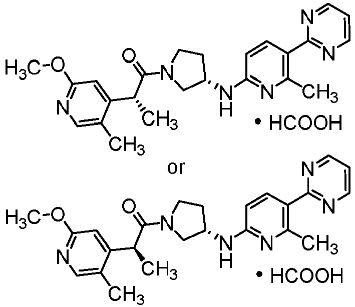 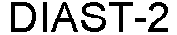 | 8.83 - 8.79 (m, 2H), 7.99 - 7.91 (m, 2H), 7.30 (t, *J* = 4.9 Hz, 1H), [6.63 (s), 6.62 (s) total 1H], [6.47 (d, *J* = 8.6 Hz), 6.46 (d, *J = 8.6* Hz) total 1H], [4.49 (quin, *J* = 5.6 Hz), 4.36 (quin, *J* = 5.7 Hz) total 1H], [4.06 (q, *J* = 6.8 Hz), 3.97 (q, *J* = 6.8 Hz) total 1H], 3.92 - 3.80 (m), 3.76 - 3.66 (m), 3.58 - 3.40 (m), 3.31 - 3.23 (m) total 7H], [2.63 (s), 2.56 (s) total 3H], [2.37 - 2.15 (m), 2.29 (s), 2.27 (s), total 4H], 2.03 - 1.87 (m, 1H), [1.37 (d, *J* = 6.8 Hz), 1.34 (d, *J* = 6.8 Hz) total 3H]; 2.71 minutes⁸; 433.3 |
| **23** | **P4**²⁰ | 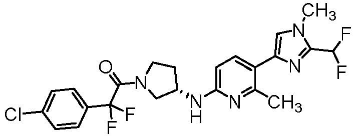 | 2.50 minutes²¹; 496 |
| **24A** | Example 3B^{1,22}; **P13, P8** | 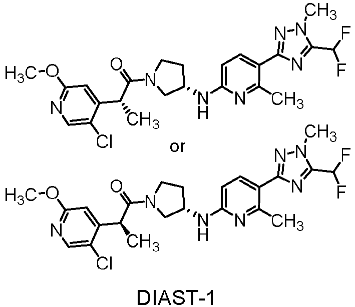 | 3.07 minutes⁸; 506.2 (chlorine isotope pattern observed) |
| **24B** | Example 3B^{1,22}; **P13, P8** | 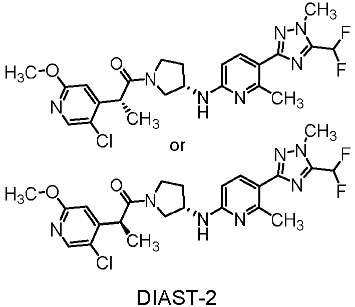 | [8.14 (s), 8.12 (s) total 1H], 7.87 (d, *J =* 8.6 Hz, 1H), [7.11 (t, *J* = 52.3 Hz), 7.10 (t, *J* = 52.3 Hz) total 1H], [6.76 (s), 6.76 (s), total 1H], [6.43 (d, *J* = 9.0 Hz), 6.41 (d, *J* = 9.0 Hz) total 1H], [4.48 (quin, *J* = 5.6 Hz), 4.41 (quin, *J* = 5.7 Hz) total 1H], [4.28 (q, *J* = 7.0 Hz), 4.20 (q, *J* = 7.0 Hz), total 1H], [4.07 (s), 4.06 (s), total 3H], [3.90 (s), 3.88 (s), total 3H], [3.87 - 3.82 (m), 3.81 - 3.63 (m), 3.57-3.39 (m), total 4H], [2.62 (s), 2.60 (s), total 3H], [2.39-2.29 (m), 2.25-2.15 (m), total 1H], 2.03 - 1.92 (m, 1H), [1.42 (d, *J* = 6.6 Hz), 1.38 (d, *J* = 7.0 Hz), total 3H]; 3.11 minutes⁸; 506.2 (chlorine isotope pattern observed) |
| **25A** | General Procedure 1²³, **P13, P12** | 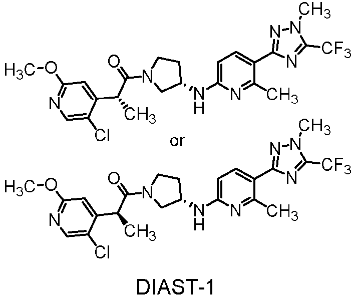 | 3.30 minutes⁸; 524.2 (chlorine isotope pattern observed) |
| **25B** | General Procedure 1²³, **P13, P12** | 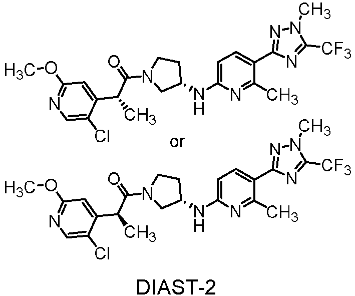 | 8.13 (d, *J* = 7.6 Hz, 1H), 7.91 (d, *J* = 8.8 Hz, 1H), 6.76 (d, *J* = 5.6 Hz, 1H), [6.43 (d, *J* = 8.8 Hz), 6.40 (d, *J* = 8.8 Hz) total 1H], [4.49 (quin, *J* = 5.6 Hz), 4.42 (quin, *J* = 5.7 Hz) total 1H], [4.28 (q, *J* = 6.8 Hz), 4.20 (q, *J* = 6.9 Hz) total 1H], 4.10 (br s, 3H), [3.90 (s), 3.88 (s), total 3H], [3.88 - 3.82 (m), 3.80 - 3.64 (m), 3.58 - 3.38 (m) total 4H], [2.64 (s), 2.61 (s) total 3H], [2.34 (dt, *J* = 7.1, 13.2 Hz), 2.26 - 2.15 (m) total 1H], 2.03 - 1.91 (m, 1H), [1.42 (d, *J* = 6.8 Hz), 1.38 (d, *J =* 6.8 Hz) total 3H]; 3.29 minutes⁸; 524.2 |
| **26A** | General Procedure 1^{10,24} ; **P5** | 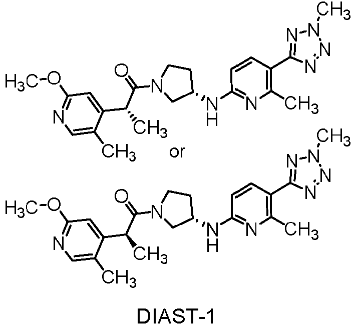 | 2.77 minutes⁸; 437.3 |
| **26B** | General Procedure 1^{10,24} ; **P5** | 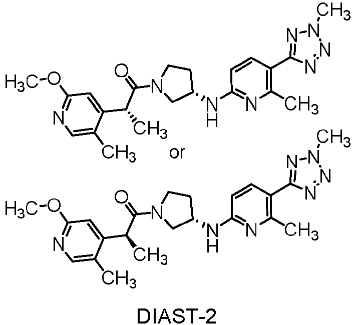 | 7.96 - 7.90 (m, 2H), [6.62 (s), 6.61 (s), total 1H], [6.44 (d, *J* = 8.6 Hz), 6.42 (d, *J* = 8.8 Hz) total 1H], [4.53 - 4.46 (m), 4.40 (s), 4.39 (s), 4.38 - 4.32 (m), total 4H], [4.06 (q, *J* = 6.9 Hz), 3.97 (q, *J* = 6.8 Hz) total 1H], 3.91-3.85 (m), 3.86 (s), 3.84 (s), 3.77 - 3.65 (m), 3.58 - 3.48 (m), 3.48 - 3.39 (m), 3.29 - 3.22 (m) total 7H], [2.64 (s), 2.58 (s) total 3H], [2.35 - 2.28 (m), 2.29 (s), 2.26 (s), 2.24 - 2.13 (m), total 4H], 2.02 - 1.85 (m, 1H), [1.37 (d, *J* = 6.8 Hz), 1.34 (d, *J* = 6.8 Hz) total 3H]; 2.81 minutes⁸; 437.3 |
| **27** | General Procedure 1; **P9** | 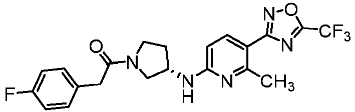 | [8.01 (d, *J* = 8.3 Hz), 7.99 (d, *J* = 8.6 Hz) total 1H], [7.33 - 7.26 (m), 7.26 - 7.19 (m) total 2H], [7.10 - 7.01 (m), 6.99 - 6.91 (m) total 2H], [6.48 (d, *J* = 8.8 Hz), 6.44 (d, *J =* 8.6 Hz) total 1H], 4.62 - 4.52 (m, 1H), [3.91 - 3.78 (m), 3.78 - 3.61 (m), 3.61 - 3.51 (m), 3.45 (d, *J =* 4.4 Hz), 3.42 (d, *J* = 4.4 Hz) total 6H], [2.69 (s), 2.67 (s) total 3H], [2.39 - 2.29 (m), 2.29 - 2.20 (m) total 1H], [2.12 - 2.02 (m), 2.02 - 1.92 (m) total 1H]; 450.1 |
| **28** | Example 3B; **P4** | 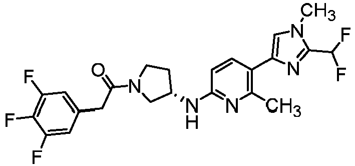 | [7.62 (d, *J* = 8.6 Hz), 7.61 (d, *J* = 8.6 Hz) total 1H], 7.28 (s, 1H), 7.09 - 7.03 (m, 1H), 7.03 - 6.96 (m, 1H), [6.88 (t, *J* = 52.6 Hz), 6.87 (t, *J* = 52.6 Hz), total 1H], [6.43 (d, *J* = 8.6 Hz), 6.40 (d, *J* = 8.6 Hz) total 1H], 4.48 (qd, J *=* 5.5, 10.5 Hz, 1H), [3.89 (m), 3.84 - 3.74 (m), 3.74 - 3.62 (m), 3.59 - 3.50 (m), 3.42 (dd, *J* = 4.8, 12.3 Hz) total 9H], [2.46 (s), 2.45 (s) total 3H], [2.39 - 2.29 (m), 2.29 - 2.19 (m) total 1H], [2.12 - 2.02 (m), 2.02 - 1.91 (m) total 1H]; 480.1 |
| **29** | Synthesis of **C17;P3-OH, P23** | 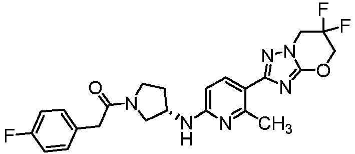 | [7.79 (d, *J =* 8.6 Hz), 7.77 (d, *J =* 8.6 Hz) total 1H], [7.32 - 7.26 (m), 7.26 - 7.19 (m) total 2H], [7.08 - 7.01 (m), 7.00 - 6.93 (m) total 2H], [6.41 (d, *J = 8.8* Hz), 6.38 (d, *J =* 8.6 Hz) total 1H], 4.80 - 4.64 (m, 4H), 4.54 - 4.46 (m, 1H), [3.90 (d, *J* = 5.9 Hz), 3.88 (d, *J* = 5.6 Hz), 3.82 (d, *J* = 6.1 Hz), 3.81 - 3.77 (m), 3.77 - 3.60 (m), 3.59 - 3.47 (m), 3.43 (d, *J* = 4.6 Hz), 3.40 (d, *J* = 4.4 Hz) total 6H], [2.61 (s), 2.60 (s), total 3H], [2.37 - 2.28 (m), 2.28 - 2.18 (m) total 1H], 2.10 - 2.00 (m), 2.00 - 1.89 (m) total 1H]; 473.2 |
| **30A** | General Procedure 1²⁵; **P21, P7** | 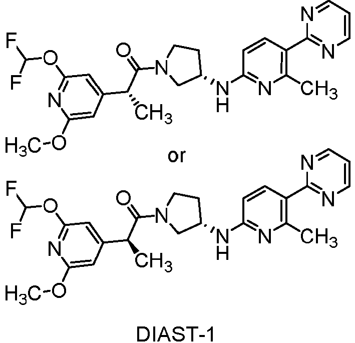 | 1.63 minutes¹⁷; 485.3 |
| **30B** | General Procedure 1²⁵; **P21, P7** | 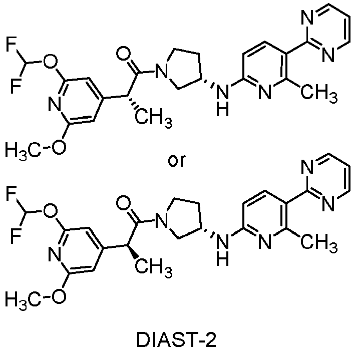 | 8.81 (d, *J* = 4.9 Hz, 1H), 8.80 (d, *J* = 4.9 Hz, 1H), 7.91 (d, *J* = 8.6 Hz, 1H), [7.52 (t, *J* = 73.4 Hz), 7.52 (t, *J* = 73.4 Hz) total 1H], [7.30 (t, *J* = 4.9 Hz), 7.30 (t, *J* = 4.9 Hz) total 1H], 6.56 - 6.52 (m, 1H), 6.51 - 6.39 (m, 2H), [4.52 (quin, *J* = 5.6 Hz), 4.44 (quin, *J* = 5.8 Hz) total 1H], [3.99 (q, *J* = 6.8 Hz), 3.95 - 3.84 (m), 3.82 - 3.63 (m) total 6H], [3.61 - 3.44 (m), 3.39 (dd, *J* = 4.8, 12.6 Hz) total 2H], [2.60 (s), 2.57 (s), total 3H], [2.38 - 2.27 (m), 2.26 - 2.15 (m) total 1H], 2.07 - 1.89 (m, 1H), [1.41 (d, *J* = 7.0 Hz), 1.38 (d, *J* = 7.0 Hz) total 3H]; 1.65 minutes¹⁷; 485.3 |
| **31** | **P2**¹⁵ | 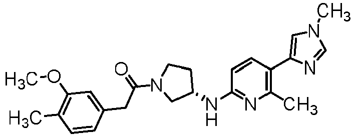 | 1.72 minutes²⁶; 420.3 |
| **32** | Synthesis of C17; **P3-OH** | 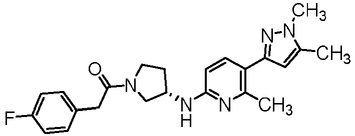 | ¹H NMR (400 MHz, DMSO-*d*₆) δ 7.52 (d, *J* = 8.6 Hz, 1H), 7.30 - 7.20 (m, 2H), 7.16 - 7.05 (m, 2H), [6.72 (d, *J* = 6.5 Hz), 6.68 (d, *J =* 6.2 Hz) total 1H], 6.34 (d, *J* = 8.2 Hz, 1H), 6.16 (d, *J* = 1.6 Hz, 1H), [4.46 - 4.37 (m), 4.36 - 4.27 (m) total 1H], [3.86 (br dd, *J* = 6.0, 10.3 Hz), 3.72 (d, *J* = 1.2 Hz), 3.70 - 3.56 (m), 3.56 - 3.46 (m) total 8H], [3.42 - 3.35 (m), 3.25 - 3.18 (m) total 1H], 2.44 (s, 3H), 2.26 (br s, 3H), [2.25 - 2.15 (m), 2.15 - 2.03 (m) total 1H], [1.99 - 1.88 (m), 1.87-1.76 (m) total 1H]; 408.1 |
| **33A** | General Procedure 1^{27,28}; **P8** | 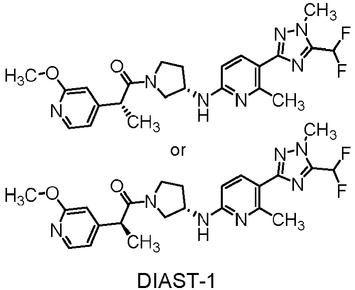 | [8.07 (d, *J* = 5.4 Hz) and 7.84 (d, *J* = 5.3 Hz), total 1H], [7.88 (d, *J* = 8.6 Hz) and 7.83 (d, *J* = 8.7 Hz), total 1H], [7.12 (t, *J* = 52.4 Hz) and 7.11 (t, *J* = 52.4 Hz), total 1H], [6.93 (dd, *J* = 5.4, 1.5 Hz), 6.78 - 6.74 (m), and 6.60 - 6.58 (m), total 2H], [6.44 (d, *J* = 8.6 Hz) and 6.27 (d, *J* = 8.6 Hz), total 1H], 4.47 - 4.38 (m, 1H), [4.07 (s) and 4.06 (s), total 3H], [3.97 (q, *J* = 6.9 Hz), 3.89 - 3.73 (m), and 3.71 - 3.64 (m), total 3H], [3.90 (s) and 3.72 (s), total 3H], [3.57 (ddd, *J* = 12.7, 8.7, 4.9 Hz) and 3.41 - 3.33 (m), total 1H], 3.51 - 3.43 (m, 1H), [2.62 (s) and 2.61 (s), total 3H], 2.30 - 2.15 (m, 1H), [2.08 - 1.98 (m) and 1.95 - 1.84 (m), total 1H], [1.41 (d, *J* = 6.9 Hz) and 1.36 (d, *J* = 6.9 Hz), total 3H]; 472.2 |
| **33B** | General Procedure 1^{27,28}; **P8** | 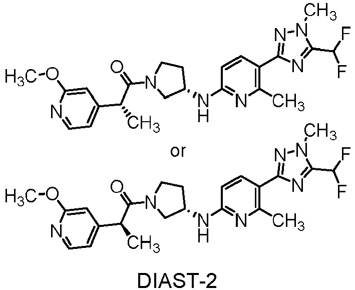 | [8.07 (d, *J* = 5.6 Hz), 8.05 (d, *J* = 5.9 Hz) total 1H], 7.86 (d, *J* = 8.6 Hz, 1H), [7.11 (t, *J* = 52.3 Hz), 7.10 (t, *J* = 52.3 Hz) total 1H], 6.93 - 6.89 (m, 1H), 6.75 - 6.73 (m, 1H), 6.42 - 6.36 (m, 1H), [4.46 (quin, *J* = 5.7 Hz), 4.39 (quin, *J* = 5.8 Hz) total 1H], 4.11 - 4.02 (m, 3H), [3.97 (q, *J* = 7.0 Hz), 3.92 - 3.83 (m), 3.89 (s), 3.88 (s), 3.80 - 3.63 (m), 3.55 - 3.40 (m), 3.39 (d, *J* = 4.6 Hz), 3.35 (d, *J* = 4.9 Hz) total 8H], [2.62 (s), 2.58 (s) total 3H], [2.35 - 2.24 (m), 2.23 - 2.11 (m) total 1H], 2.00 - 1.87 (m, 1H), [1.40 (d, *J* = 6.8 Hz), 1.38 (d, *J* = 6.8 Hz) total 3H]; 472.2 |
| **34** | **P4**²⁰ | 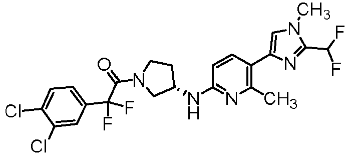 | 2.65 minutes²¹; 530 |
| **35** | Example 3B¹; **P2** | 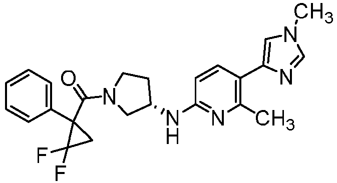 | 2.18 minutes²⁹; LCMS *m*/*z* 460.4 [M+Na⁺] |
| **36A** | General Procedure 1³⁰; **P22, P4** | 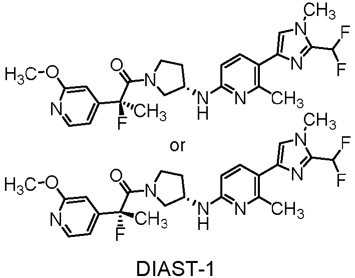 | 2.98 minutes⁸; 489.2 |
| **36B** | General Procedure 1³⁰; **P22, P4** | 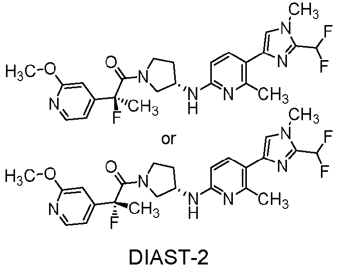 | 8.20 - 8.15 (m, 1H), [7.60 (d, *J* = 8.6 Hz), 7.58 (d, *J* = 8.6 Hz) total 1H], 7.27 (br s, 1H), [7.01 - 6.96 (m), 6.88 - 6.86 (m), 6.84 - 6.82 (m), 6.82 - 6.81 (m), 6.74 - 6.73 (m) total 3H], [6.38 (d, *J* = 8.6 Hz), 6.35 (d, *J* = 8.6 Hz) total 1H], [4.40 (quin, *J* = 6.3 Hz), 4.26 - 4.17 (m) total 1H], [3.97 - 3.84 (m), 3.84 - 3.64 (m), 3.61 - 3.51 (m), 3.46 - 3.36 (m) total 10H], [2.45 (s), 2.36 (s) total 3H], 2.30 - 2.11 (m, 1H), 1.89 - 1.69 (m, 4H); 3.03 minutes⁸; 489.2 |
| **37A** | General Procedure 1³¹; **P13, P6** | 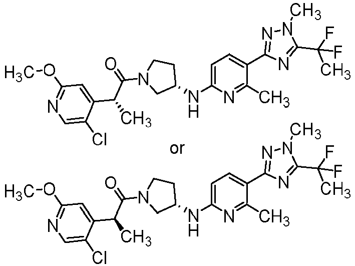 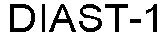 | 3.27 minutes⁸; 520.2 (chlorine isotope pattern observed) |
| **37B** | General Procedure 1³¹; **P13, P6** | 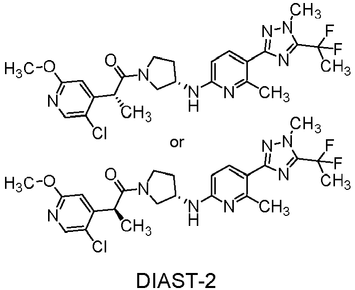 | [8.14 (s), 8.12 (s), total 1H], 7.87 (d, *J* = 8.8 Hz, 1H), [6.76 (s), 6.75 (s) total 1H], [6.42 (d, *J* = 8.6 Hz), 6.39 (d, *J* = 8.6 Hz) total 1H], [4.48 (quin, *J* = 5.7 Hz), 4.41 (quin, *J* = 5.7 Hz) total 1H], [4.28 (q, *J* = 6.8 Hz), 4.20 (q, *J* = 6.8 Hz) total 1H], 4.08 - 4.06 (m, 3H), [3.90 (s), 3.88 (s) total 3H], [3.88 - 3.82 (m), 3.80 - 3.64 (m), 3.57 - 3.46 (m), 3.46 - 3.39 (m) total 4H], [2.63 (s), 2.59 (s) total 3H], [2.38 - 2.28 (m), 2.25 - 2.16 (m), total 1H], [2.172 (t, *J* = 19.3 Hz), 2.167 (t, *J* = 19.1 Hz) total 3H], 2.03 - 1.91 (m, 1H), [1.42 (d, *J* = 6.8 Hz), 1.38 (d, *J* = 6.8 Hz) total 3H]; 3.28 minutes⁸; 520.2 |
| **38A** | General Procedure 1³²; **P16, P4** | 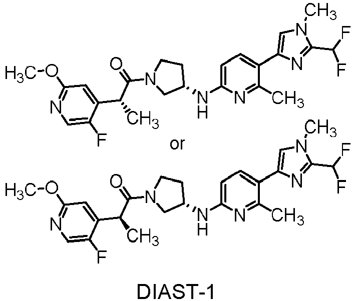 | 2.94 minutes⁸; 489.2 |
| **38B** | General Procedure 1³², **P16, P4** | 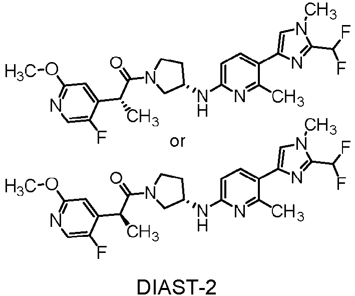 | 8.00 - 7.95 (m, 1H), 7.61 (d, *J* = 8.6 Hz, 1H), 7.28 (br s, 1H), [6.87 (t, *J* = 52.6 Hz), 6.87 (t, *J* = 52.6 Hz), total 1H], 6.76 - 6.70 (m, 1H), [6.42 (d, *J* = 8.6 Hz), 6.40 (d, *J* = 8.6 Hz) total 1H], 4.43 (tt, *J* = 5.7, 12.0 Hz, 1H), [4.25 (q, *J* = 6.8 Hz), 4.16 (q, *J* = 6.8 Hz) total 1H], [3.92 - 3.81 (m), 3.81 - 3.64 (m), 3.56 - 3.46 (m), 3.38 (dd, *J* = 4.9, 12.5 Hz) total 10H], [2.45 (s), 2.43 (s) total 3H], [2.38 - 2.26 (m), 2.25 - 2.14 (m) total 1H], 1.97 (tt, *J* = 6.8, 12.9 Hz, 1H), [1.44 (d, *J* = 6.8 Hz), 1.40 (d, *J* = 7.1 Hz) total 3H]; 2.97 minutes⁸; 489.2 |
| **39** | Example 1^{33,34}; **P12** | 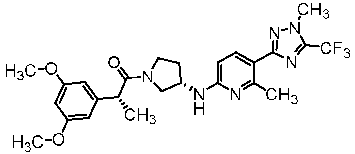 | 2.37 minutes²⁶; 519.5 |
| **40A** | General Procedure 1³⁵; **P19, P6** | 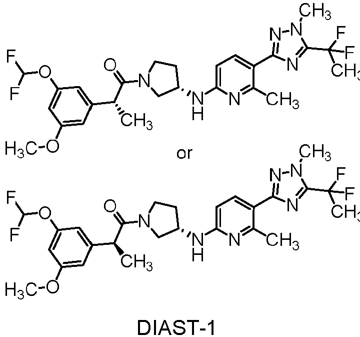 | 3.34 minutes⁸; 551.2 |
| **40B** | General Procedure 1³⁵; **P19, P6** | 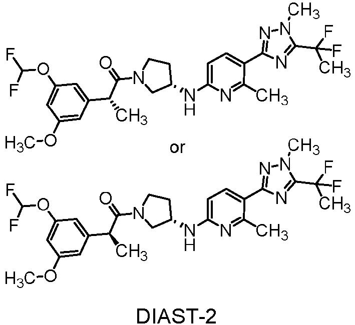 | 7.87 (d, *J* = 8.6 Hz, 1H), [6.82 (t, *J* = 74.1 Hz), 6.81 (t, *J* = 74.1 Hz) total 1H], 6.77 - 6.73 (m, 1H), 6.70 - 6.65 (m, 1H), 6.61 - 6.57 (m, 1H), [6.40 (d, *J* = 8.3 Hz), 6.38 (d, *J* = 8.1 Hz) total 1H], [4.51 - 4.43 (m), 4.43 - 4.34 (m) total 1H], 4.10 - 4.03 (m, 3H), [3.95 (q, *J* = 6.9 Hz), 3.91 - 3.82 (m), 3.80 (s), 3.78 (s), 3.75 - 3.62 (m), 3.54 - 3.41 (m), 3.37 (br d, *J* = 4.9 Hz), 3.34 (br d, *J* = 5.4 Hz) total 8H], [2.62 (s), 2.59 (s) total 3H], [2.37 - 2.25 (m), 2.17 (t, *J* = 19.3 Hz), 2.17 (t, *J* = 19.3 Hz), total 4H], 2.00 - 1.84 (m, 1H), [1.40 (d, *J* = 7.1 Hz), 1.38 (d, *J* = 6.8 Hz) total 3H]; 3.44 minutes⁸; 551.2 |
| **41A** | General Procedure 1³⁶; **P20, P8** | 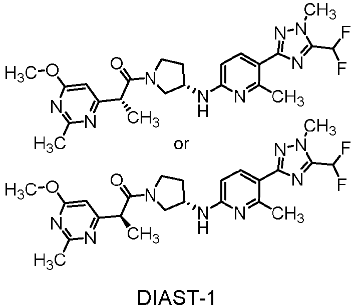 | 2.77 minutes⁸; 487.3 |
| **41B** | General Procedure 1³⁶; **P20, P8** | 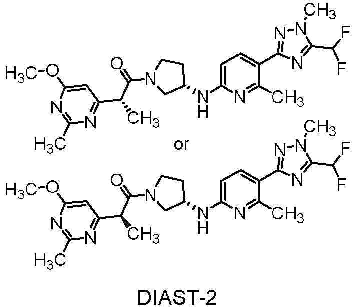 | 8.15 - 7.93 (m, 1H), 7.12 (t, *J* = 52.3 Hz, 1H), [6.63 (s), 6.61 (s), total 1H], 6.63 - 6.54 (m, 1H) 4.55 - 4.45 (m, 1H), 4.08 (s, 3H), [4.03 (q, *J* = 7.0 Hz), 3.97 (s), 3.96 (s), 3.95 (q, *J* = 6.9 Hz) total 4H], [3.90 - 3.81 (m), 3.81 - 3.73 (m), 3.73 - 3.62 (m), 3.61 - 3.50 (m), 3.50 - 3.42 (m) total 4H], [2.72 (s), 2.67 (s) total 3H], 2.56 (s, 3H), [2.42 - 2.32 (m), 2.30 - 2.19 (m) total 1H], 2.09 - 1.93 (m, 1H), [1.46 (d, *J* = 7.1 Hz), 1.42 (d, *J* = 7.1 Hz) total 3H]; 2.80 minutes⁸; 487.3 |
| **42** | Example 1³⁷; **P17, P1, P24** | 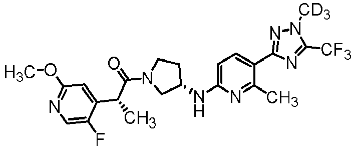 | [7.98 (d, *J* = 1.2 Hz), 7.97 (d, *J* = 1.2 Hz) total 1H], [7.91 (d, *J* = 8.6 Hz, 1H), [6.74 (d, *J* = 4.7 Hz), 6.73 (d, *J* = 4.6 Hz) total 1H], [6.43 (d, *J* = 8.9 Hz), 6.41 (d, *J* = 8.9 Hz) total 1H], 4.54 - 4.41 (m, 1H), [4.25 (q, *J* = 7.1 Hz), 4.16 (q, *J* = 6.9 Hz) total 1H], [3.91 - 3.83 (m), 3.88 (s), 3.86 (s), 3.82 - 3.64 (m), 3.57 - 3.46 (m), 3.41 (d, *J* = 4.6 Hz), 3.38 (d, *J* = 4.6 Hz) total 7H], [2.64 (s), 2.61 (s) total 3H], [2.38 - 2.28 (m), 2.26 - 2.15 (m) total 1H], 2.04 - 1.92 (m, 1H), [1.44 (d, *J* = 6.8 Hz), 1.41 (d, *J* = 6.8 Hz) total 3H]; 511.1 |
| **43** | Example 2; **P17, P1** | 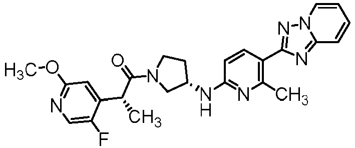 | ¹H NMR (400 MHz, chloroform-d) δ 8.63 - 8.57 (m, 1H), 8.23 (d, *J* = 8.6 Hz, 1H), 7.98 (br s, 1H), [7.76 - 7.74 (m), 7.74 - 7.72 (m) total 1H], 7.55 - 7.48 (m, 1H), 7.04 - 6.96 (m, 1H), [6.80 (d, *J* = 5.1 Hz), 6.79 (d, *J* = 5.1 Hz) total 1H], [6.36 (br d, *J* = 8.6 Hz), 6.35 (br d, *J* = 8.6 Hz) total 1H], 4.76 (br s, 1H), 4.46 - 4.36 (m, 1H), [4.11 (q, *J* = *6.9 Hz),* 4.04 (q, *J* = 7.7 Hz) total 1H], [4.00 (d, *J* = 6.6 Hz), 3.97 (d, *J* = 6.2 Hz), 3.90 (s), 3.80 - 3.55 (m), 3.51 - 3.38 (m) total 7H], [2.84 (s), 2.83 (s) total 3H], [2.41 - 2.31 (m), 2.31 - 2.20 (m) total 1H], 2.08 - 1.86 (m, 1H), [1.47 (d, *J* = 6.8 Hz), 1.44 (d, *J* = 7.0 Hz) total 3H]; 476.1 |
| **44A** | General Procedure 1³⁸; **P19, P7** | 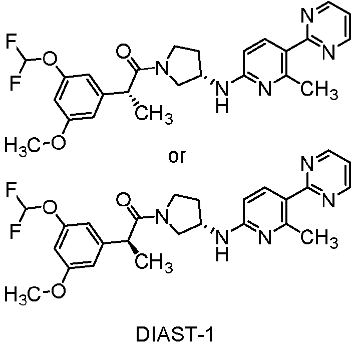 | 3.06 minutes⁸; 484.2 |
| **44B** | General Procedure 1³⁸; **P19, P7** | 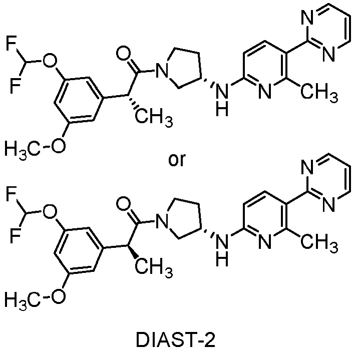 | [8.81 (d, *J* = 4.9 Hz), 8.80 (d, *J* = 4.9 Hz) total 2H], 7.91 (d, *J* = 8.8 Hz, 1H), [7.30 (t, *J* = 4.9 Hz), 7.30 (t, *J* = 4.9 Hz) total 1H], [6.82 (t, *J* = 74.1 Hz), 6.81 (t, *J* = 74.1 Hz), total 1H], 6.76 - 6.74 (m, 1H), 6.69 - 6.66 (m, 1H), 6.59 (q, *J* = 2.4 Hz, 1H), 6.43 (t, *J* = 8.6 Hz, 1H), [4.50 (quin, *J* = 5.8 Hz), 4.42 (quin, *J* = 5.7 Hz) total 1H], [3.99 - 3.83 (m), [3.81 - 3.66 (m), 3.78 (d, *J* = 5.9 Hz) total 6H], [3.55 - 3.42 (m), 3.37 (dd, *J* = 5.0, 12.3 Hz) total 2H], [2.60 (s), 2.56 (s) total 3H], [2.32 (dt, *J* = 6.5, 13.3 Hz), 2.23 - 2.12 (m), total 1H], 2.00 - 1.88 (m, 1H), [1.39 (d, *J* = 7.1 Hz), 1.39 (d, *J* = 7.1 Hz) total 3H]; 3.12 minutes⁸; 484.2 |

1. Acetonitrile was used as solvent, rather than tetrahydrofuran.

2. 2,6-Dimethoxy-4-methylpyridine was converted to the requisite 2-(2,6-dimethoxypyridin-4-yl)propanoic acid using the method described in Preparation P13. LCMS m/z 212.1 [M+H]⁺. ¹H NMR (400 MHz, chloroform-d) δ 6.26 (s, 2H), 3.90 (s, 6H), 3.63 (q, *J* = 7.2 Hz, 1H), 1.47 (d, *J* = 7.1 Hz, 3H).

3. The product was separated into its component diastereomers using supercritical fluid chromatography {Column: Chiral Technologies Chiralpak IA, 21 x 250 mm, 5 µm; Mobile phase: 85:15 carbon dioxide / [methanol containing 0.2% ammonium hydroxide (v/v)]; Flow rate: 75 mL/minute; Back pressure: 200 bar}. The first-eluting diastereomer was designated as Example 12A, and the second-eluting diastereomer as Example 12B.

4. Analytical conditions. Column: Chiral Technologies Chiralpak IA, 4.6 x 100 mm, 5 µm; Mobile phase 7:3 carbon dioxide / (methanol containing 0.2% ammonium hydroxide); Flow rate: 1.5 mL/minute; Back pressure: 200 bar.

5. The product was separated into its component diastereomers using supercritical fluid chromatography [Column: Chiral Technologies Chiralcel OJ-H, 21 x 250 mm, 5 µm; Mobile phase: 4:1 carbon dioxide / (methanol containing 0.2% ammonium hydroxide); Flow rate 75 mL/minute; Back pressure: 200 bar]. The first-eluting diastereomer was designated as Example 13A, and the second-eluting diastereomer as Example 13B.

6. Analytical conditions. Column: Chiral Technologies Chiralcel OJ-H, 4.6 x 100 mm, 5 µm; Mobile phase: 7:3 carbon dioxide / (methanol containing 0.2% ammonium hydroxide); Flow rate 1.5 mL/minute; Back pressure: 200 bar.

7. The product was separated into its component diastereomers using reversed-phase HPLC (Column: Waters XBridge C18, 19 x 150 mm; 5 µm; Mobile phase A: water containing 0.05% ammonium hydroxide; Mobile phase B: acetonitrile; Gradient: 43% to 51% B; Flow rate: 20 mL/minute). The first-eluting diastereomer was designated as Example 14A, and the second-eluting diastereomer as Example 14B.

8. Analytical conditions. Column: Waters XBridge C18, 2.1 x 50 mm, 5 µm; Mobile phase A: water containing 0.1% formic acid; Mobile phase B: acetonitrile containing 1% formic acid; Gradient: 1% B for 0.6 minutes, then 1% to 100% B over 3.4 minutes; Flow rate: 0.8 mL/minute.

9. The product was separated into its component diastereomers using reversed-phase HPLC (Column: Waters XBridge C18, 19 x 150 mm; 5 µm; Mobile phase A: water containing 0.05% ammonium hydroxide; Mobile phase B: acetonitrile; Gradient: 45% to 57% B; Flow rate: 20 mL/minute). The first-eluting diastereomer was designated as Example 15A, and the second-eluting diastereomer as Example 15B.

10. Conversion of 2-methoxy-4,5-dimethylpyridine to the requisite 2-(2-methoxy-5-methylpyridin-4-yl)propanoic acid was carried out using the method described in Preparation P13. LCMS *m*/*z* 196.1 [M+H]⁺. ¹H NMR (400 MHz, methanol-*d*₄) δ 7.89 (br s, 1H), 6.69 (s, 1H), 3.90 (q, *J* = 7.1 Hz, 1H), 3.86 (s, 3H), 2.26 (br s, 3H), 1.44 (d, *J* = 7.2 Hz, 3H).

11. The product was separated into its component diastereomers using reversed-phase HPLC (Column: Waters XBridge C18, 19 x 150 mm; 5 µm; Mobile phase A: water containing 0.05% ammonium hydroxide; Mobile phase B: acetonitrile; Gradient: 40% to 48% B; Flow rate: 20 mL/minute). The first-eluting diastereomer was designated as Example 16A, and the second-eluting diastereomer as Example 16B.

12. Coupling of **P1** and **P25** was effected using [1,1'-bis(diphenylphosphino)ferrocene]dichloropalladium(II) and tripotassium phosphate in tetrahydrofuran at 70 °C. The resulting *tert*-butyl (3*S*)-3-({5-[5-(difluoromethyl)-1-(²H₃)methyl-1*H-*1,2,4-triazol-3-yl]-6-methylpyridin-2-yl}amino)pyrrolidine-1-carboxylate was deprotected at 50 °C using hydrogen chloride generated from acetyl chloride in propan-2-ol.

13. Suzuki conditions: A mixture of **P1** (2.23 g, 6.94 mmol), 2-bromo-[1,2,4]triazolo[1,5-a]pyridine (1.25 g, 6.31 mmol), an aqueous solution of tripotassium phosphate (2.5 M; 6.31 mL, 15.8 mmol), and tetrahydrofuran (16 mL) was degassed with nitrogen for 10 minutes. Pd(dppf)Cl₂, dichloromethane complex (77.3 mg, 94.7 µmol) was then added, and degassing was continued for 3 minutes. The reaction mixture was heated to 70 °C and stirred for 18 hours before being partitioned between water and ethyl acetate. The aqueous layer was extracted three times with ethyl acetate, and the combined organic layers were dried over magnesium sulfate, filtered, and concentrated in vacuo; silica gel chromatography (Gradient: 0% to 100% ethyl acetate in heptane) afforded the requisite tert-butyl (3S)-3-{[6-methyl-5-([1,2,4]triazolo[1,5-a]pyridin-2-yl)pyridin-2-yl]amino}pyrrolidine-1-carboxylate as a white solid. Yield: 1.20 g, 3.04 mmol, 48%. LCMS *m*/*z* 395.3 [M+H]⁺. ¹H NMR (400 MHz, chloroform-d) δ 8.59 (d, *J* = 6.8 Hz, 1H), 8.24 (d, *J* = 8.6 Hz, 1H), 7.73 (d, *J* = 9.0 Hz, 1H), 7.50 (dd, *J* = 8.0, 7.8 Hz, 1H), 6.99 (dd, *J* = 6.9, 6.8 Hz, 1H), 6.38 (d, *J* = 8.6 Hz, 1H), 5.00 - 4.74 (m, 1H), 4.45 - 4.25 (m, 1H), 3.84 - 3.67 (m, 1H), 3.62 - 3.39 (m, 2H), 3.39 - 3.17 (m, 1H), 2.84 (s, 3H), 2.32 - 2.19 (m, 1H), 2.02 - 1.84 (m, 1H), 1.47 (s, 9H).

14. Reaction of (5-methoxy-2-methylphenyl)methanol with thionyl chloride was followed by displacement of the resulting primary chloride using sodium cyanide in N,N-dimethylformamide at room temperature. The derived (5-methoxy-2-methylphenyl)acetonitrile was subjected to sodium hydride and iodomethane in tetrahydrofuran at room temperature, followed by ester hydrolysis with sodium hydroxide in aqueous ethanol at room temperature, to afford the requisite 2-(5-methoxy-2-methylphenyl)propanoic acid: LCMS *m*/*z* 195.1 [M+H]⁺. ¹H NMR (400 MHz, methanol-*d*₄) δ 7.06 (d, *J* = 8.4 Hz, 1H), 6.80 (d, *J* = 2.7 Hz, 1H), 6.70 (dd, *J* = 8.4, 2.7 Hz, 1H), 3.91 (q, *J* = 7.1 Hz, 1H), 3.74 (s, 3H), 2.28 (s, 3H), 1.40 (d, *J* = 7.1 Hz, 3H).

15. The final coupling was effected using *O*-(7-azabenzotriazol-1-yl)-*N,N,N',N'-*tetramethyluronium hexafluorophosphate (HATU) and *N,N*-diisopropylethylamine in *N,N-*dimethylformamide at 25 °C.

16. The product was separated into its component diastereomers using reversed-phase HPLC (Column: Phenomenex Gemini C18, 21.2 x 100 mm, 5 µm; Mobile phase A: water containing 0.1% formic acid; Mobile phase B: acetonitrile; Gradient: 10% to 20% B; Flow rate: 25 mL/minute). The first-eluting diastereomer was designated as Example 20A, and the second-eluting diastereomer as Example 20B.

17. Analytical conditions. Column: Waters Acquity BEH C18, 2.1 x 50 mm, 1.7 µm; Mobile phase A: water containing 0.05% trifluoroacetic acid; Mobile phase B: acetonitrile containing 0.05% trifluoroacetic acid; Gradient: 5% to 95% B over 2 minutes, then 95% B for 0.5 minutes; Flow rate: 0.5 mL/minute.

18. The product was separated into its component diastereomers using reversed-phase HPLC [Column: Waters XBridge C18, 19 x 150 mm; 5 µm; Mobile phase A: water containing 0.05% ammonium hydroxide; Mobile phase B: acetonitrile; Gradient: 42% to 95% B; Flow rate: 20 mL/minute]. The first-eluting diastereomer was designated as Example 21A, and the second-eluting diastereomer as Example 21B.

19. The product was separated into its component diastereomers using reversed-phase HPLC [Column: Nouryon Kromasil^{®} C18, 21.5 x 100 mm, 5 µm; Mobile phase A: water containing 0.1% formic acid; Mobile phase B: acetonitrile; Gradient: 13% to 23% B; Flow rate: 25 mL/minute]. The first-eluting diastereomer was designated as Example 22A, and the second-eluting diastereomer as Example 22B.

20. The final coupling was carried out using *O*-(7-azabenzotriazol-1-yl)-*N,N,N',N'-*tetramethyluronium hexafluorophosphate (HATU) and *N,N*-diisopropylethylamine in *N,N-*dimethylacetamide at 80 °C.

21. Analytical conditions. Column: Waters XBridge C18, 2.1 x 50 mm, 5 µm; Mobile phase A: water containing 0.0375% trifluoroacetic acid; Mobile phase B: acetonitrile containing 0.01875% trifluoroacetic acid; Gradient: 10% B for 0.50 minutes, then 10% to 100% B over 3.50 minutes; Flow rate: 0.8 mL/minute

22. The product was separated into its component diastereomers using reversed-phase HPLC (Column: Nouryon Kromasil^{®} C18, 21.2 x 100 mm, 5 µm; Mobile phase A: water containing 0.1% formic acid; Mobile phase B: acetonitrile; Gradient: 45% to 55% B). The first-eluting diastereomer was designated as Example 24A, and the second-eluting diastereomer as Example 24B.

23. The product was separated into its component diastereomers using reversed-phase HPLC (Column: Nouryon Kromasil^{®} C18, 21.2 x 100 mm, 5 µm; Mobile phase A: water containing 0.05% ammonium hydroxide; Mobile phase B: acetonitrile; Gradient: 55% to 65% B). The first-eluting diastereomer was designated as Example 25A, and the second-eluting diastereomer as Example 25B.

24. The product was separated into its component diastereomers using reversed-phase HPLC (Column: Nouryon Kromasil^{®} C18, 21.5 x 100 mm, 5 µm; Mobile phase A: water containing 0.1% formic acid; Mobile phase B: acetonitrile; Gradient: 18% to 28% B; Flow rate: 25 mL/minute). The first-eluting diastereomer was designated as Example 26A, and the second-eluting diastereomer as Example 26B.

25. The product was separated into its component diastereomers using supercritical fluid chromatography [Column: Chiral Technologies ChiralCel OD-H, 20 x 250 mm, 5 µm; Mobile phase: 78:22 carbon dioxide / (methanol containing 0.2% ammonium hydroxide); Flow rate: 50 mL/minute]. The first-eluting diastereomer was designated as Example 30A, and the second-eluting diastereomer as Example 30B.

26. Analytical conditions. Column: Waters Atlantis dC18, 4.6 x 50 mm, 5 µm; Mobile phase A: water containing 0.05% trifluoroacetic acid (v/v); Mobile phase B: acetonitrile containing 0.05% trifluoroacetic acid (v/v); Gradient: 5.0% to 95% B, linear over 4.0 minutes, then 95% B for 1.0 minute; Flow rate: 2 mL/minute.

27. Methyl (2-methoxypyridin-4-yl)acetate was converted to the requisite 2-(2-methoxypyridin-4-yl)propanoic acid using the method described in Preparation P13. LCMS m/z 182.1 [M+H]⁺. ¹H NMR (400 MHz, DMSO-*d*₆) δ 12.53 (br s, 1H), 8.09 (d, *J* = 5.3 Hz, 1H), 6.90 (d, *J* = 5.3 Hz, 1H), 6.71 (s, 1H), 3.83 (s, 3H), 3.69 (q, *J* = 7.1 Hz, 1H), 1.34 (d, *J* = 7.1 Hz, 3H).

28. The product was separated into its component diastereomers using reversed-phase HPLC (Column: Waters XBridge C18, 19 x 150 mm, 5 µm; Mobile phase A: water containing 0.05% ammonium hydroxide; Mobile phase B: acetonitrile; Gradient: 33% to 38% B; Flow rate: 20 mL/minute). The first-eluting diastereomer was designated as Example 33A, and the second-eluting diastereomer as Example 33B.

29. Analytical conditions. Column: Chiral Technologies Chiralcel OJ-H, 4.6 x 100 mm, 5 µm; Mobile phase: 7:3 carbon dioxide / (methanol containing 0.2% ammonium hydroxide); Flow rate 1.5 mL/minute; Back pressure: 120 bar.

30. The product was separated into its component diastereomers using reversed-phase HPLC (Column: Nouryon Kromasil^{®} C18, 21.2 x 100 mm, 5 µm; Mobile phase A: water containing 0.05% ammonium hydroxide; Mobile phase B: acetonitrile; Gradient: 40% to 50% B). The first-eluting diastereomer was designated as Example 36A, and the second-eluting diastereomer as Example 36B.

31. The product was separated into its component diastereomers using reversed-phase HPLC (Column: Nouryon Kromasil^{®} C18, 21.2 x 100 mm, 5 µm; Mobile phase A: water containing 0.05% ammonium hydroxide; Mobile phase B: acetonitrile; Gradient: 50% to 60% B). The first-eluting diastereomer was designated as Example 37A, and the second-eluting diastereomer as Example 37B.

32. The product was separated into its component diastereomers using reversed-phase HPLC (Column: Waters XBridge C18, 19 x 150 mm, 5 µm; Mobile phase A: water containing 0.05% ammonium hydroxide; Mobile phase B: acetonitrile; Gradient: 35% to 95% B; Flow rate: 20 mL/minute). The first-eluting diastereomer was designated as Example 38A, and the second-eluting diastereomer as Example 38B.

33. No pyridine trifluoromethanesulfonate was added.

34. Reaction of 1-bromo-3,5-dimethoxybenzene and dibenzyl propanedioate was mediated by Pd₂(dba)₃, tri-*tert*-butylphosphine, and tripotassium phosphate in toluene at 70 °C. The resulting dibenzyl (3,5-dimethoxyphenyl)propanedioate was methylated using iodomethane and potassium carbonate in *N,N*-dimethylformamide at room temperature; subsequent hydrogenolysis of the esters was carried out over palladium on carbon in ethyl acetate to provide (3,5-dimethoxyphenyl)(methyl)propanedioic acid: LCMS *m*/*z* 254.9 [M+H]⁺. ¹H NMR (400 MHz, DMSO-*d*₆) δ 6.49 (d, *J* = 2.2 Hz, 2H), 6.45 (t, *J* = 2.2 Hz, 1H), 3.72 (s, 6H), 1.67 (s, 3H). This material was subjected to AMDase in 2-amino-2-(hydroxymethyl)propane-1,3-diol (Tris) buffer, pH 8.5, at room temperature (see Production of (*R*)-2-(3,5-dimethoxyphenyl)propanoic acid using an aryl malonate decarboxylase from *Bordetella bronchiseptica*; S.P. France et al., *Applied Biocatalysis* **2021,** 259-261) to afford the requisite (2R)-2-(3,5-dimethoxyphenyl)propanoic acid: ¹H NMR (400 MHz, DMSO-*d*₆) δ 12.26 (br s, 1H), 6.42 (d, *J* = 2.3 Hz, 2H), 6.38 (t, *J* = 2.3 Hz, 1H), 3.72 (s, 6H), 3.59 (q, *J* = 7.1 Hz, 1H), 1.32 (d, *J* = 7.1 Hz, 3H).

35. The product was separated into its component diastereomers using reversed-phase HPLC (Column: Waters XBridge C18, 19 x 150 mm; 5 µm; Mobile phase A: water containing 0.05% ammonium hydroxide; Mobile phase B: acetonitrile; Gradient: 50% to 55% B; Flow rate: 20 mL/minute). The first-eluting diastereomer was designated as Example 40A, and the second-eluting diastereomer as Example 40B.

36. The product was separated into its component diastereomers using reversed-phase HPLC (Column: Waters XBridge C18, 19 x 150 mm; 5 µm; Mobile phase A: water containing 0.05% ammonium hydroxide; Mobile phase B: acetonitrile; Gradient: 30% to 40% B; Flow rate: 20 mL/minute). The first-eluting diastereomer was designated as Example 41A, and the second-eluting diastereomer as Example 41B.

37. Coupling of **P1** and **P24** was effected using [1,1'-bis(diphenylphosphino)ferrocene]dichloropalladium(II) and tripotassium phosphate in tetrahydrofuran at 70 °C. The resulting *tert*-butyl (3*S*)-3-({6-methyl-5-[1-(²H₃)methyl-5-(trifluoromethyl)-1*H*-1,2,4-triazol-3-yl]pyridin-2-yl}amino)pyrrolidine-1-carboxylate was deprotected at 50 °C using hydrogen chloride generated from acetyl chloride in propan-2-ol.

38. The product was separated into its component diastereomers using reversed-phase HPLC (Column: Waters XBridge C18, 19 x 150 mm; 5 µm; Mobile phase A: water containing 0.1% formic acid; Mobile phase B: acetonitrile; Gradient: 23% to 33% B; Flow rate: 20 mL/minute). The first-eluting diastereomer was designated as Example 44A, and the second-eluting diastereomer as Example 44B.

### Example AA. In Vitro Binding Affinity Assay Using hMC4R

The binding affinity of test compounds at the α-melanocyte-stimulating hormone receptor (hMC4R) was assessed using a radioligand competition binding assay. Recombinant Chinese hamster ovaries (CHO) cells stably expressing hMC4R (PerkinElmer # ES-191-C) were used for competitive binding. hMC4R membranes were grown in Dulbecco's Modified Essential Medium and Ham's F-12 Medium (DMEM/F12), 10% heat inactivated fetal bovine serum (FBS), 0.4 mg/mL Geneticin and 2 mM L-glutamine. Cell membranes were bulked and frozen until the assay was performed.

Compounds were solubilized in 100% dimethyl sulfoxide (DMSO) to a concentration of 30 mM. A 10-point intermediate dilution series using half log dilutions was created in 100% DMSO with a top concentration of 0.03 mM. The serially diluted compounds were spotted as 1 µL/well, in 96-well Costar 3363 plates. The final compound range in the assay was 300 nM to 0.01 nM with a final DMSO concentration of 1%. Control wells, containing 1 µL of 2 mM (2 µM final) alpha-melanocyte stimulating hormone (α-MSH-Tocris # 2584) was added to the non-specific binding wells and 1 µL 100% DMSO for the total binding control wells. This was followed by the addition of 80 µL of assay buffer [25 mM HEPES, 5 mM MgCl₂, 2.5 mM CaCl₂, 150 mM NaCl, Complete EDTA-free Protease Inhibitor Tablet (Thermo Scientific #11873580001) and 0.25% BSA]. 10 µL of [¹²⁵I]-(Nle4, D-Phe7)-α-MSH (PerkinElmer #NEX3520) was added to all wells at 10-fold the final concentration of 0.5 nM. The radioligand concentration used was below the equilibrium dissociation constant (K_{d}) of 2.59 nM. The exact concentration of radioligand used for each experiment was determined by liquid scintillation counting and adjusted if necessary.

Frozen hMC4R cell membranes were thawed and Dounce homogenized. Homogenates were resuspended in assay buffer to a concentration of 2 µg per well. The competition binding reaction was initiated by the addition of 10 µL MC4R membrane solution to the assay-ready plates containing test compound and [¹²⁵I]-(Nle4, D-Phe7)-α-MSH. The plates were incubated for 2 hours at room temperature. Assay samples were then rapidly filtered through Unifilter-96 GF/B PEI coated filter plates using a filter plate harvester (PerkinElmer) and rinsed with ice-cold wash buffer [25 mM (4-(2-hydroxyethyl)-1-piperazineethanesulfonic acid (HEPES), 1 mM MgCl₂, 2.5 mM CaCl₂, and 500 mM NaCl]. Filter plates were dried overnight at room temperature. Plates were then bottom-sealed prior to the addition of 50 µL/well Ultima Gold XR scintillation fluid (PerkinElmer 6013111). Plates were then top-sealed, incubated for 60 minutes at room temperature and then the amount of radioactivity present was determined by liquid scintillation counting on a Microbeta Trilux (PerkinElmer # 2450-0060).

The raw data (expressed as counts per minute) were analyzed using ActivityBase (IDBS Data Management Software). The percent effect at each concentration of compound was calculated by ActivityBase based on the values for the uninhibited wells (total binding controls) and fully inhibited wells (non-specific binding controls) on each assay plate. A concentration required for 50% inhibition (IC₅₀) value was determined from these data using a 4-parameter logistic model. Equilibrium dissociation constant for inhibitor of ligand and receptor interaction (Kᵢ) values were then calculated from the IC₅₀ values using the Cheng-Prusoff equation: Kᵢ = IC₅₀ / (1 + ([L]/ K_{d})), where [L] is the concentration of the radioligand used in the experiment and K_{d} is the affinity of the radioligand (determined in separate saturation experiments).

### Example BB. Functional In Vitro MC4R Antagonist Potency Assay

The functional *in vitro* antagonist potency for test compounds was determined by monitoring intracellular cyclic adenosine monophosphate (cAMP) levels in Chinese hamster ovary (CHO-) cells stably expressing the human Melanocortin-4 receptor (MC4R). Following agonist activation, human MC4R associates with the G-protein complex causing the Gα subunit to exchange bound GDP for GTP, followed by dissociation of the Gα-GTP complex. The activated Gα subunit can couple to downstream effectors to regulate the levels of second messengers or cAMP within the cell. Thereby, determination of intracellular cAMP levels allows for pharmacological characterization. Intracellular cAMP levels are quantitated using a homogenous assay utilizing the Homogeneous Time-Resolved Fluorescence (HTRF) technology from CisBio. The method is a competitive immunoassay between native cAMP produced by cells and the cAMP labelled with the acceptor dye, d2. The two entities then compete for binding to a monoclonal anti-cAMP antibody labeled with cryptate. The specific signal is inversely proportional to the concentration of cAMP in the cells.

Test compounds were solubilized to 30 mM in 100% dimethyl sulfoxide (DMSO) and stored. An 11-point dilution series using 1 in 3 162-fold serial dilutions was created in 100% DMSO with a top concentration of 800 µM. The serially diluted compound was spotted into a 384-well plate (Greiner, Cat No. 781280) at 40 nL/well with duplicate points at each concentration, and then diluted 1 in 1000 with 40 µL assay buffer containing HBSS, 20 mM HEPES (Invitrogen), 0.1% BSA, and 250 µM IBMX (Sigma Aldrich) to create an intermediate plate at 2x final assay concentration (FAC). The final compound concentration range in the assay was 400 nM to 4 pM, with a final DMSO concentration of 0.1%.

In-house generated CHO cells stably expressing the Gs-coupled human MC4R receptor were plated in 384-well assay plates (Corning, Cat No. 3570) in 50 µL/well of Ham's F-12 containing 10% heat inactivated FBS, 1x penicillin/streptomycin, 1 mM Glutamax (Invitrogen) at a density of 2,500 cells per well and incubated at 37 °C (95% O₂: 5% CO₂) overnight, with micro-clime lids (Labcyte, Cat No. LLS-0310). On day of assay, media was removed from the assay plate through gentle flicking and blotting plate on a paper towel and replaced with 5 µL of 2x antagonist compound in assay buffer (HBSS, 20 mM HEPES, 0.1% BSA, 250 µM IBMX) and 0.1% DMSO. Cells were incubated with compound for 30 minutes at 37 °C (95% O₂: 5% CO₂) before addition of 5 µL EC₈₀ agonist stimulation (200 nM α-melanocyte stimulating hormone, αMSH, Bachem) and another 30-minute incubation at 37 °C (95% O₂: 5% CO₂). Intracellular cAMP levels were quantified as per Cisbio's protocol (5 µL of D2 and then 5 µL Cryptate, incubated for 1-2 hours at room temperature). Samples were measured on an Envision plate reader (PerkinElmer Life and Analytical Sciences; excitation, 320 nm; emission, 665 nm/620 nm).

Data were analyzed using the ratio of fluorescence intensity at 620 and 665 nm for each well, extrapolated from the cAMP standard curve to express data as nM cAMP for each well. Data expressed as nM cAMP were then normalized to control wells using Activity Base (IDBS). Zero percent effect (ZPE) was defined as nM of cAMP generated from EC₈₀ agonist stimulation (200 nM αMSH). In the absence of an antagonist control compound, one hundred percent effect (HPE) was defined as nM of cAMP generated from assay buffer/vehicle only. The concentration and % effect values for each compound were plotted by Activity Base using a four-parameter logistic dose response equation, and the concentration required for 50% inhibition (IC₅₀) was determined. Equilibrium dissociation constant (K_{b}) values were then calculated according to the Leff-Dougall equation: K_{b} = [IC₅₀] / ((2+ ([A]/[EC₅₀])ⁿ)^{1/n} -1 ), wherein A is the concentration of the agonist challenge used in the experiment (200 nM) and n= the slope.

Table 2 lists biological activities (Kᵢ values, see Example AA; and K_{b} values, see Example BB) and compound names for Examples 1 to 44B.

**Table 2. Biological activity and Compound name for Examples 1 to 44B.**

| Example Number | Kᵢ (nM) geometric mean | Count used (Kᵢ) | K_{b} (nM) Antagoni st geometri c mean | Count used (K_{b}) | Compound Name |
|---|---|---|---|---|---|
| 1 | 0.88 | 4 | 5.1 | 4 | (2*R*)-2-(5-chloro-2-methoxypyridin-4-yl)-1-[(3*S*)-3-{[6-methyl-5-(2-methyl-2*H-*tetrazol-5-yl)pyridin-2-yl]amino}pyrrolidin-1-yl]propan-1-one |
| 2 | 1.2 | 13 | 7.2 | 3 | (2*R*)-2-(5-fluoro-2-methoxypyridin-4-yl)-1-[(3*S*)-3-({6-methyl-5-[1-methyl-5-(trifluoromethyl)-1*H*-1,2,4-triazol-3-yl]pyridin-2-yl}amino)pyrrolidin-1-yl]propan-1-one |
| 3A | 520 | 2 | N.D.¹ | | 1-[(3*R*)-2,2-dimethyl-3-{[6-methyl-5-(1-methyl-1*H*-imidazol-4-yl)pyridin-2-yl]amino}pyrrolidin-1-yl]-2-(4-fluorophenyl)ethan-1-one |
| 3B | 4.2 | 7 | 33 | 2 | 1-[(3*S*)-2,2-d imethyl-3-{[6-methyl-5-(1-methyl-1*H*-imidazol-4-yl)pyridin-2-yl]amino}pyrrolidin-1-yl]-2-(4-fluorophenyl)ethan-1-one |
| 4 | 1.0 | 4 | 9.6 | 3 | (2*R*)-1-[(3*S*)-3-({5-[5-(1,1-difluoroethyl)-1-methyl-1*H*-1,2,4-triazol-3-yl]-6-methylpyridin-2-yl}amino)pyrrolidin-1-yl]-2-(5-fluoro-2-methoxypyridin-4-yl)propan-1-one |
| 5 | 2.6 | 3 | 17 | 3 | (2*R*)-2-(5-fluoro-2-methoxypyridin-4-yl)-1-[(3*S*)-3-{[6-methyl-5-(2-methyl-2*H-*tetrazol-5-yl)pyridin-2-yl]amino}pyrrolidin-1-yl]propan-1-one |
| 6 | 1.1 | 7 | 2.2 | 3 | (2*R*)-2-(5-chloro-2-methoxypyridin-4-yl)-1-[(3*S*)-3-{[6-methyl-5-(pyrimidin-2-yl)pyridin-2-yl]amino}pyrrolidin-1-yl]propan-1-one |
| 7 | 0.77 | 26 | 3.3 | 5 | (2*R*)-1-[(3*S*)-3-({5-[5-(difluoromethyl)-1-methyl-1*H*-1,2,4-triazol-3-yl]-6-methylpyridin-2-yl}amino)pyrrolidin-1-yl]-2-(5-fluoro-2-methoxypyridin-4-yl)propan-1-one |
| 8 | 1.2 | 4 | 19 | 3 | (2*R*)-1-[(3*S*)-3-({5-[1-cyclopropyl-5-(difluoromethyl)-1*H*-1,2,4-triazol-3-yl]-6-methylpyridin-2-yl}amino)pyrrolidin-1-yl]-2-(5-fluoro-2-methoxypyridin-4-yl)propan-1-one |
| **9** | 0.46 | 2 | N.D. | | 1-[(3*S*)-3-({5-[2-(difluoromethyl)-1-methyl-1*H*-imidazol-4-yl]-6-methylpyridin-2-yl}amino)pyrrolidin-1-yl]-2-[4-(trifluoromethoxy)phenyl]ethan-1-one |
| **10** | 0.31 | 3 | N.D. | | 1-[(3*S*)-3-({5-[2-(difluoromethyl)-1-methyl-1*H*-imidazol-4-yl]-6-methylpyridin-2-yl}amino)pyrrolidin-1-yl]-2-(3,4-difluorophenyl)ethan-1-one |
| **11** | 1.1 | 3 | N.D. | | (2*R*)-1-[(3*S*)-3-({5-[2-(difluoromethyl)-1-methyl-1*H*-imidazol-4-yl]-6-methylpyridin-2-yl}amino)pyrrolidin-1-yl]-2-phenylpropan-1-one |
| **12A** | 0.31 | 3 | 1.9 | 2 | 1-[(3uS)-3-({5-[2-(difluoromethyl)-1-methyl-1*H*-imidazol-4-yl]-6-methylpyridin-2-yl}amino)pyrrolidin-1-yl]-2-(2,6-dimethoxypyridin-4-yl)propan-1-one, DIAST-1 |
| **12B** | 7.7 | 1 | N.D. | | 1-[(3*S*)-3-({5-[2-(difluoromethyl)-1-methyl-1*H*-imidazol-4-yl]-6-methylpyridin-2-yl}amino)pyrrolidin-1-yl]-2-(2,6-dimethoxypyridin-4-yl)propan-1-one, DIAST-2 |
| **13A** | N.D. | | N.D. | | 2-fluoro-1-[(3*S*)-3-{[6-methyl-5-(1-methyl-1*H*-imidazol-4-yl)pyridin-2-yl]amino}pyrrolidin-1-yl]-2-phenylethan-1-one, DIAST-1 |
| **13B** | 0.34 | 4 | 2.9 | 1 | 2-fluoro-1-[(3*S*)-3-{[6-methyl-5-(1-methyl-1*H*-imidazol-4-yl)pyridin-2-yl]amino}pyrrolidin-1-yl]-2-phenylethan-1-one, DIAST-2 |
| **14A** | 530 | 1 | N.D. | | 1-[(3*S*)-3-({5-[2-(1,1-difluoroethyl)-1-methyl-1*H*-imidazol-4-yl]-6-methylpyridin-2-yl}amino)pyrrolidin-1-yl]-2-(3-methoxyphenyl)propan-1-one, DIAST-1 |
| **14B** | 0.45 | 2 | 14 | 1 | 1-[(3*S*)-3-({5-[2-(1,1-difluoroethyl)-1-methyl-1*H*-imidazol-4-yl]-6-methylpyridin-2-yl}amino)pyrrolidin-1-yl]-2-(3-methoxyphenyl)propan-1-one, DIAST-2 |
| **15A** | 150 | 1 | N.D. | | 1-[(3*S*)-3-({5-[2-(1,1-difluoroethyl)-1-methyl-1*H*-imidazol-4-yl]-6-methylpyridin-2-yl}amino)pyrrolidin-1-yl]-2-(2-methylphenyl)propan-1-one, DIAST-1 |
| **15B** | 0.83 | 1 | N.D. | | 1-[(3*S*)-3-({5-[2-(1,1-difluoroethyl)-1-methyl-1*H*-imidazol-4-yl]-6-methylpyridin-2-yl}amino)pyrrolidin-1-yl]-2-(2-methylphenyl)propan-1-one, DIAST 2 |
| **16A** | >220 | 1 | N.D. | | 1-[(3*S*)-3-({5-[2-(1,1-difluoroethyl)-1-methyl-1*H*-imidazol-4-yl]-6-methylpyridin-2-yl}amino)pyrrolidin-1-yl]-2-(2-methoxy-5-methylpyridin-4-yl)propan-1-one, DIAST-1 |
| **16B** | 0.23 | 4 | 2.1 | 1 | 1-[(3*S*)-3-({5-[2-(1,1-difluoroethyl)-1-methyl-1*H*-imidazol-4-yl]-6-methylpyridin-2-yl}amino)pyrrolidin-1-yl]-2-(2-methoxy-5-methylpyridin-4-yl)propan-1-one, DIAST-2 |
| **17** | 0.46 | 3 | 2.0 | 3 | (2*R*)-1-[(3*S*)-3-({5-[5-(difluoromethyl)-1-(²H₃)methyl-1*H*-1,2,4-triazol-3-yl]-6-methylpyridin-2-yl}amino)pyrrolidin-1-yl]-2-(5-fluoro-2-methoxypyridin-4-yl)propan-1-one |
| **18** | 0.51 | 5 | 2.1 | 4 | (2*R*)-2-(5-chloro-2-methoxypyridin-4-yl)-1-[(3*S*)-3-{[6-methyl-5-([1,2,4]triazolo[1,5-*a*]pyridin-2-yl)pyridin-2-yl]amino}pyrrolidin-1-yl]propan-1-one |
| **20A** | 1100 | 1 | N.D. | | 2-(5-methoxy-2-methylphenyl)-1-[(3*S*)-3-{[6-methyl-5-(1-methyl-1*H*-imidazol-4-yl)pyridin-2-yl]amino}pyrrolidin-1-yl]propan-1-one, DIAST-1 |
| **20B** | 0.23 | 3 | 2.9 | 27 | 2-(5-methoxy-2-methylphenyl)-1-[(3*S*)-3-{[6-methyl-5-(1-methyl-1*H*-imidazol-4-yl)pyridin-2-yl]amino}pyrrolidin-1-yl]propan-1-one, DIAST-2 |
| **21A** | 190 | 1 | N.D. | | 1-[(3*S*)-3-({5-[2-(1,1-difluoroethyl)-1-methyl-1*H*-imidazol-4-yl]-6-methylpyridin-2-yl}amino)pyrrolidin-1-yl]-2-(5-fluoro-2-methoxypyridin-4-yl)propan-1-one, DIAST-1 |
| **21B** | 0.48 | 4 | 2.6 | 2 | 1-[(3*S*)-3-({5-[2-(1,1-difluoroethyl)-1-methyl-1*H*-imidazol-4-yl]-6-methylpyridin-2-yl}amino)pyrrolidin-1-yl]-2-(5-fluoro-2-methoxypyridin-4-yl)propan-1-one, DIAST-2 |
| **22A** | 120 | 3 | >110 | 1 | 2-(2-methoxy-5-methylpyridin-4-yl)-1-[(3*S*)-3-{[6-methyl-5-(pyrimidin-2-yl)pyridin-2-yl]amino}pyrrolidin-1-yl]propan-1-one, formate salt, DIAST-1 |
| **22B** | 2.1 | 3 | 8.1 | 3 | 2-(2-methoxy-5-methylpyridin-4-yl)-1-[(3*S*)-3-{[6-methyl-5-(pyrimidin-2-yl)pyridin-2-yl]amino}pyrrolidin-1-yllpropan-1-one, formate salt, DIAST-2 |
| **23** | 1.1 | 2 | N.D. | | 2-(4-chlorophenyl)-1-[(3*S*)-3-({5-[2-(difluoromethyl)-1-methyl-1*H*-imidazol-4-yl]-6-methylpyridin-2-yl}amino)pyrrolidin-1-yl]-2,2-difluoroethan-1-one |
| **24A** | 20 | 1 | N.D. | | 2-(5-chloro-2-methoxypyridin-4-yl)-1-[(3*S*)-3-({5-[5-(difluoromethyl)-1-methyl-1*H*-1,2,4-triazol-3-yl]-6-methylpyridin-2-yl}amino)pyrrolidin-1-yl]propan-1-one, DIAST-1 |
| **24B** | 0.49 | 8 | 0.96 | 3 | 2-(5-chloro-2-methoxypyridin-4-yl)-1-[(3*S*)-3-({5-[5-(difluoromethyl)-1-methyl-1*H*-1,2,4-triazol-3-yl]-6-methylpyridin-2-yl}amino)pyrrolidin-1-yl]propan-1-one, DIAST-2 |
| **25A** | 88 | 1 | N.D. | | 2-(5-chloro-2-methoxypyridin-4-yl)-1-[(3*S*)-3-({6-methyl-5-[1-methyl-5-(trifluoromethyl)-1*H*-1,2,4-triazol-3-yl]pyridin-2-yl}amino)pyrrolidin-1-yl]propan-1-one, DIAST-1 |
| **25B** | 0.49 | 3 | 1.8 | 3 | 2-(5-chloro-2-methoxypyridin-4-yl)-1-[(3*S*)-3-({6-methyl-5-[1-methyl-5-(trifluoromethyl)-1*H*-1,2,4-triazol-3-yl]pyridin-2-yl}amino)pyrrolidin-1-yl]propan-1-one, DIAST-2 |
| **26A** | >170 | 3 | >150 | 1 | 2-(2-methoxy-5-methylpyridin-4-yl)-1-[(3*S*)-3-{[6-methyl-5-(2-methyl-2*H-*tetrazol-5-yl)pyridin-2-yl]amino}pyrrolidin-1-yl]propan-1-one, DIAST-1 |
| **26B** | 2.1 | 3 | 5.6 | 3 | 2-(2-methoxy-5-methylpyridin-4-yl)-1-[(3*S*)-3-{[6-methyl-5-(2-methyl-2*H-*tetrazol-5-yl)pyridin-2-yl]amino}pyrrolidin-1-yl]propan-1-one, DIAST-2 |
| **27** | 950 | 1 | N.D. | | 2-(4-fluorophenyl)-1-[(3*S*)-3-({6-methyl-5-[5-(trifluoromethyl)-1,2,4-oxadiazol-3-yl]pyridin-2-yl}amino)pyrrolidin-1-yl]ethan-1-one |
| **28** | 0.25 | 2 | 1.3 | 63 | 1-[(3*S*)-3-({5-[2-(difluoromethyl)-1-methyl-1*H*-imidazol-4-yl]-6-methylpyridin-2-yl}amino)pyrrolidin-1-yl]-2-(3,4,5-trifluorophenyl)ethan-1-one |
| **29** | 6.9 | 1 | N.D. | | 1-[(3*S*)-3-{[5-(6,6-difluoro-6,7-dihydro-5*H*-[1,2,4]triazolo[5,1-*b*][1,3]oxazin-2-yl)-6-methylpyridin-2-yl]amino}pyrrolidin-1-yl]-2-(4-fluorophenyl)ethan-1-one |
| **30A** | >200 | 2 | >62 | 1 | 2-[2-(difluoromethoxy)-6-methoxypyridin-4-yl]-1-[(3*S*)-3-{[6-methyl-5-(pyrimidin-2-yl)pyridin-2-yl]amino}pyrrolidin-1-yl]propan-1-one, DIAST-1 |
| **30B** | 0.93 | 2 | 6.8 | 3 | 2-[2-(difluoromethoxy)-6-methoxypyridin-4-yl]-1-[(3*S*)-3-{[6-methyl-5-(pyrimidin-2-yl)pyridin-2-yl]amino}pyrrolidin-1-yl]propan-1-one, DIAST-2 |
| **31** | 1.0 | 2 | N.D. | | 2-(3-methoxy-4-methylphenyl)-1-[(3*S*)-3-{[6-methyl-5-(1-methyl-1*H*-imidazol-4-yl)pyridin-2-yl]amino}pyrrolidin-1-yl]ethan-1-one |
| **32** | 5.5 | 1 | N.D. | | 1-[(3*S*)-3-{[5-(1,5-dimethyl-1*H*-pyrazol-3-yl)-6-methylpyridin-2-yl]amino}pyrrolidin-1-yl]-2-(4-fluorophenyl)ethan-1-one |
| **33A** | 1100 | 1 | N.D. | | 1-[(3*S*)-3-({5-[5-(difluoromethyl)-1-methyl-1*H*-1,2,4-triazol-3-yl]-6-methylpyridin-2-yl}amino)pyrrolidin-1-yl]-2-(2-methoxypyridin-4-yl)propan-1-one, DIAST-1 |
| **33B** | 1.1 | 3 | 3.3 | 3 | 1-[(3*S*)-3-({5-[5-(difluoromethyl)-1-methyl-1*H*-1,2,4-triazol-3-yl]-6-methylpyridin-2-yl}amino)pyrrolidin-1-yl]-2-(2-methoxypyridin-4-yl)propan-1-one, DIAST-2 |
| **34** | 0.88 | 3 | N.D. | | 2-(3,4-dichlorophenyl)-1-[(3*S*)-3-({5-[2-(difluoromethyl)-1-methyl-1*H*-imidazol-4-yl]-6-methylpyridin-2-yl}amino)pyrrolidin-1-yl]-2,2-difluoroethan-1-one |
| **35** | 0.95 | 2 | N.D. | | (2,2-difluoro-1-phenylcyclopropyl)[(3*S*)-3-{[6-methyl-5-(1-methyl-1*H*-imidazol-4-yl)pyridin-2-yl]amino}pyrrolidin-1-yl]methanone |
| **36A** | N.D. | | N.D. | | 1-[(3*S*)-3-({5-[2-(difluoromethyl)-1-methyl-1*H*-imidazol-4-yl]-6-methylpyridin-2-yl}amino)pyrrolidin-1-yl]-2-fluoro-2-(2-methoxypyridin-4-yl)propan-1-one, DIAST-1 |
| **36B** | 4.3 | 1 | N.D. | | 1-[(3*S*)-3-({5-[2-(difluoromethyl)-1-methyl-1*H*-imidazol-4-yl]-6-methylpyridin-2-yl}amino)pyrrolidin-1-yl]-2-fluoro-2-(2-methoxypyridin-4-yl)propan-1-one, DIAST-2 |
| **37A** | 150 | 1 | N.D. | | 2-(5-chloro-2-methoxypyridin-4-yl)-1-[(3*S*)-3-({5-[5-(1,1-difluoroethyl)-1-methyl-1*H*1 ,2,4-triazol-3-yl]-6-methylpyridin-2-yl}amino)pyrrolidin-1-yl]propan-1-one, DIAST-1 |
| **37B** | 0.53 | 4 | 4.1 | 3 | 2-(5-chloro-2-methoxypyridin-4-yl)-1-[(3*S*)-3-({5-[5-(1,1-difluoroethyl)-1-methyl-1*H*-1 ,2,4-triazol-3-yl]-6-methylpyridin-2-yl}amino)pyrrolidin-1-yl]propan-1-one, DIAST-2 |
| **38A** | 62 | 1 | N.D. | | 1-[(3*S*)-3-({5-[2-(difluoromethyl)-1-methyl-1*H*-imidazol-4-yl]-6-methylpyridin-2-yl}amino)pyrrolidin-1-yl]-2-(5-fluoro-2-methoxypyridin-4-yl)propan-1-one, DIAST-1 |
| **38B** | 0.31 | 3 | 3.2 | 1 | 1-[(3*S*)-3-({5-[2-(difluoromethyl)-1-methyl-1*H*-imidazol-4-yl]-6-methylpyridin-2-yl}amino)pyrrolidin-1-yl]-2-(5-fluoro-2-methoxypyridin-4-yl)propan-1-one, DIAST-2 |
| **39** | 0.28 | 2 | 3.3 | 1 | (2*R*)-2-(3,5-dimethoxyphenyl)-1-[(3*S*)-3-({6-methyl-5-[1-methyl-5-(trifluoromethyl)-1*H*-1,2,4-triazol-3-yl]pyridin-2-yl}amino)pyrrolidin-1-yl]propan-1-one |
| **40A** | 54 | 1 | N.D. | | 1-[(3*S*)-3-({5-[5-(1,1-difluoroethyl)-1-methyl-1*H*-1,2,4-triazol-3-yl]-6-methylpyridin-2-yl}amino)pyrrolidin-1-yl]-2-[3-(difluoromethoxy)-5-methoxyphenyl]propan-1-one, DIAST-1 |
| **40B** | 0.29 | 3 | 7.2 | 1 | 1-[(3*S*)-3-({5-[5-(1,1-difluoroethyl)-1-methyl-1*H*-1,2,4-triazol-3-yl]-6-methylpyridin-2-yl}amino)pyrrolidin-1-yl]-2-[3-(difluoromethoxy)-5-methoxyphenyl]propan-1-one, DIAST-2 |
| **41A** | 240 | 1 | N.D. | | 1-[(3*S*)-3-({5-[5-(difluoromethyl)-1-methyl-1*H*-1,2,4-triazol-3-yl]-6-methylpyridin-2-yl}amino)pyrrolidin-1-yl]-2-(6-methoxy-2-methylpyrimidin-4-yl)propan-1-one, DIAST-1 |
| **41B** | 2.0 | 4 | 3.6 | 3 | 1-[(3*S*)-3-({5-[5-(difluoromethyl)-1-methyl-1*H*-1,2,4-triazol-3-yl]-6-methylpyridin-2-yl}amino)pyrrolidin-1-yl]-2-(6-methoxy-2-methylpyrimidin-4-yl)propan-1-one, DIAST-2 |
| **42** | 1.3 | 5 | 5.4 | 4 | (2*R*)-2-(5-fluoro-2-methoxypyridin-4-yl)-1-[(3*S*)-3-({6-methyl-5-[1-(²H₃)methyl-5-(trifluoromethyl)-1*H*-1,2,4-triazol-3-yl]pyridin-2-yl}amino)pyrrolidin-1-yl]propan-1-one |
| **43** | 0.91 | 4 | 9.5 | 5 | (2*R*)-2-(5-fluoro-2-methoxypyridin-4-yl)-1-[(3*S*)-3-{[6-methyl-5-([1,2,4]triazolo[1,5-*a*]pyridin-2-yl)pyridin-2-yl]amino}pyrrolidin-1-yl]propan-1-one |
| **44A** | >220 | 2 | >97 | 1 | 2-[3-(difluoromethoxy)-5-methoxyphenyl]-1-[(3*S*)-3-{[6-methyl-5-(pyrimidin-2-yl)pyridin-2-yl]amino}pyrrolidin-1-yl]propan-1-one, DIAST-1 |
| **44B** | 0.77 | 2 | 4.1 | 2 | 2-[3-(difluoromethoxy)-5-methoxyphenyl]-1-[(3*S*)-3-{[6-methyl-5-(pyrimidin-2-yl)pyridin-2-yl]amino}pyrrolidin-1-yl]propan-1-one, DIAST-2 |

| | | | | | |
|---|---|---|---|---|---|
| a. ND: Not determined | | | | | |

Throughout this application, various publications are referenced.

It will be apparent to those skilled in the art that various modifications and variations can be made in the present invention without departing from the scope of the invention. Other embodiments of the invention will be apparent to those skilled in the art from consideration of the specification and practice of the invention disclosed herein. It is intended that the specification and examples be considered as exemplary only, with a true scope of the invention being indicated by the following claims.

## Claims

1. A compound of Formula I: 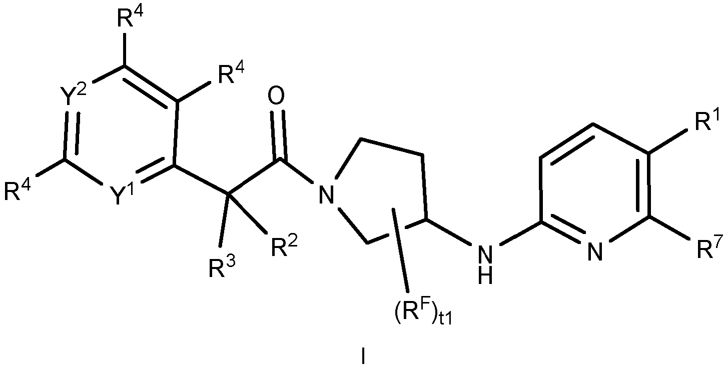 or a pharmaceutically acceptable salt thereof, wherein:
R¹ is 1,2,4-triazolyl, tetrazolyl, 1,2,4-oxadiazolyl, imidazolyl, 6,7-dihydro-[1,2,4]triazolo[5,1-b][1,3]oxazinyl, [1,2,4]triazolo[1,5-a]pyridinyl-, pyrazolyl, or pyrimidinyl, each of which is optionally substituted with 1, 2, or 3 substituents each independently selected from halogen, C₁₋₄ alkyl, C₁₋₄ haloalkyl, and C₃₋₄ cycloalkyl;
each of R² and R³ is independently H, halogen, C₁₋₄ alkyl, or C₁₋₄ haloalkyl;
or R² and R³ together with the carbon atom to which they are attached form a C₃₋₄ cycloalkyl optionally substituted with 1, 2, 3, 4, or 5 substituents each independently selected from halogen;
each of Y¹ and Y² is independently CR⁴ or N;
each R⁴ is independently H, halogen, C₁₋₄ alkyl, C₁₋₄ haloalkyl, C₁₋₄ alkoxy, or C₁₋₄ haloalkoxy;
each R^{F} is independently halogen, -OH, C₁₋₄ alkyl, C₁₋₄ haloalkyl, C₁₋₄ alkoxy, C₁₋₄ haloalkoxy, C₃₋₄ cycloalkyl, or (C₃₋₄ cycloalkyl)-C₁₋₄ alkyl-;
or when two R^{F} groups are substituted on the same carbon atom of the pyrrolidine ring of Formula I, they together with the carbon atom to which they are attached optionally form a C₃₋₆ cycloalkyl optionally substituted with 1, 2, 3, 4, or 5 substituents each independently selected from halogen, -OH, C₁₋₄ alkyl, C₁₋₄ haloalkyl, C₁₋₄ alkoxy, and C₁₋₄ haloalkoxy;
R⁷ is methyl or ethyl; and
t1 is 0, 1, 2, 3, or 4.

2. The compound of claim 1 wherein the compound is a compound of Formula I-a: 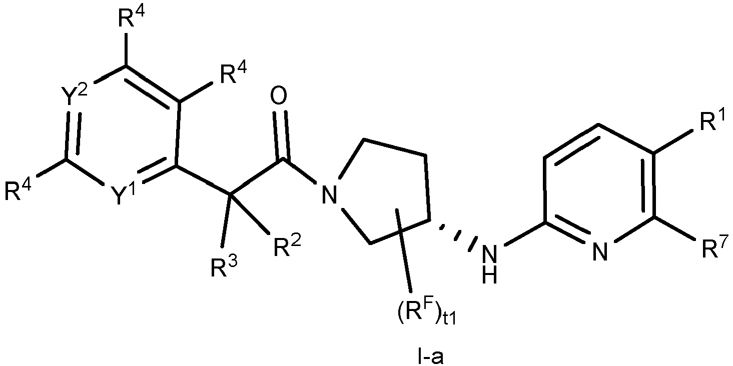 or a pharmaceutically acceptable salt thereof.

3. The compound of claim 1 or 2 wherein the compound is a compound of Formula I-b: 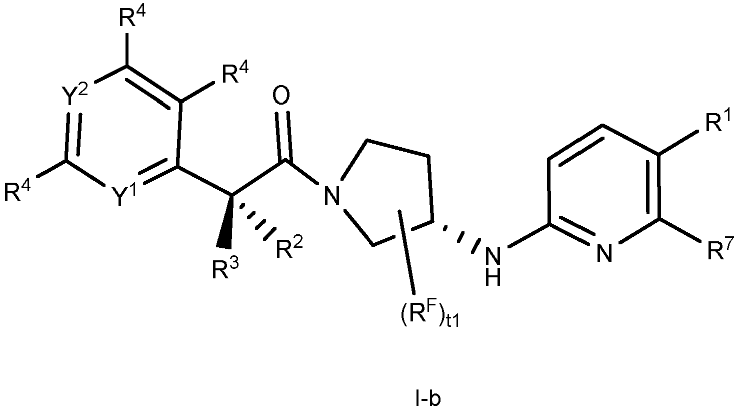 or a pharmaceutically acceptable salt thereof.

4. The compound of claim 1 wherein the compound is a compound of Formula II: 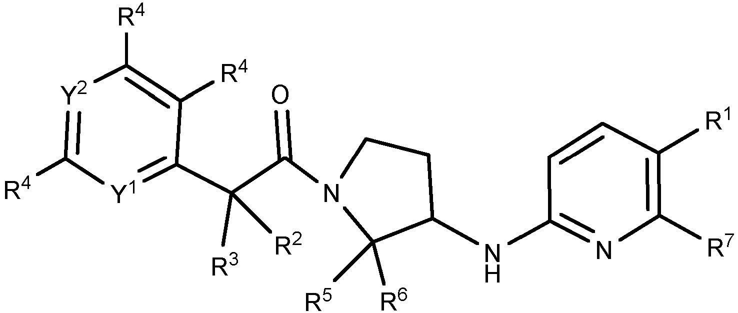
or pharmaceutically acceptable salt thereof, wherein:
each of R⁵ and R⁶ is independently H, methyl, or ethyl;
or R⁵ and R⁶ together with the carbon atom to which they are attached form a C₃₋₆ cycloalkyl optionally substituted with 1, 2, 3, 4, or 5 substituents each independently selected from halogen, - OH, C₁₋₄ alkyl, C₁₋₄ haloalkyl, C₁₋₄ alkoxy, and C₁₋₄ haloalkoxy.

5. The compound or pharmaceutically acceptable salt of claim 1 or 4 wherein the compound is a compound of Formula III: 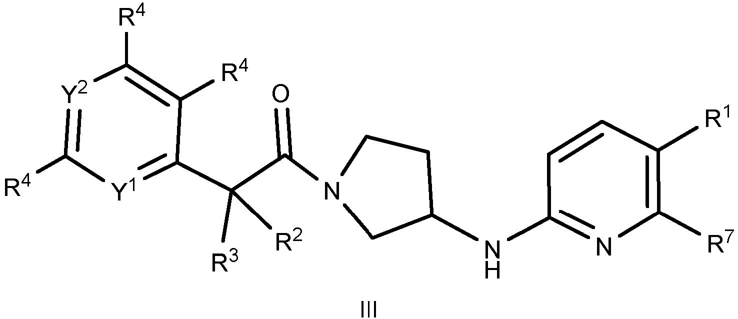 or a pharmaceutically acceptable salt thereof.

6. The compound or pharmaceutically acceptable salt of any one of claims 1 to 5 wherein R¹ is 1,2,4-triazolyl, tetrazolyl, 1,2,4-oxadiazolyl,or imidazolyl, each of which is optionally substituted with 1, 2, or 3 substituents each independently selected from halogen, C₁₋₄ alkyl, C₁₋₄ haloalkyl, and C₃₋₄ cycloalkyl.

7. The compound or pharmaceutically acceptable salt of any one of claims 1 to 5 wherein R¹ is 6,7-dihydro-[1,2,4]triazolo[5,1-b][1,3]oxazinyl or [1,2,4]triazolo[1,5-a]pyridinyl-, each of which is optionally substituted with 1, 2, or 3 substituents each independently selected from halogen, C₁₋₄ alkyl, C₁₋₄ haloalkyl, and C₃₋₄ cycloalkyl.

8. The compound or pharmaceutically acceptable salt of any one of claims 1 to 5 wherein R¹ is pyrimidinyl optionally substituted with 1, 2, or 3 substituents each independently selected from halogen, C₁₋₄ alkyl, C₁₋₄ haloalkyl, and C₃₋₄ cycloalkyl.

9. The compound or pharmaceutically acceptable salt of any one of claims 1 to 8 wherein each of R² and R³ is independently H, F, or C₁₋₄ alkyl.

10. The compound or pharmaceutically acceptable salt of any one of claims 1 to 9 wherein R⁷ is methyl.

11. The compound or pharmaceutically acceptable salt of any one of claims 1 to 10 wherein each of Y¹ and Y² is independently CR⁴; or wherein one of Y¹ and Y² is N, and the other of Y¹ and Y² is CR⁴; or wherein Y¹ is N and Y² is N.

12. The compound or pharmaceutically acceptable salt of any one of claims 1 to 11 wherein each R⁴ is independently H, halogen, C₁₋₂ alkyl, C₁₋₂ haloalkyl, C₁₋₂ alkoxy, or C₁₋₂ haloalkoxy.

13. A compound of claim 1 selected from:
(2*R*)-2-(5-chloro-2-methoxypyridin-4-yl)-1-[(3*S*)-3-{[6-methyl-5-(2-methyl-2*H*-tetrazol-5-yl)pyridin-2-yl]amino}pyrrolidin-1-yl]propan-1-one;
(2*R*)-2-(5-fluoro-2-methoxypyridin-4-yl)-1-[(3*S*)-3-({6-methyl-5-[1-methyl-5-(trifluoromethyl)-1*H*-1,2,4-triazol-3-yl]pyridin-2-yl}amino)pyrrolidin-1-yl]propan-1-one;
(2*R*)-1-[(3*S*)-3-({5-[5-(1,1-difluoroethyl)-1-methyl-1*H*-1,2,4-triazol-3-yl]-6-methylpyridin-2-yl}amino)pyrrolidin-1-yl]-2-(5-fluoro-2-methoxypyridin-4-yl)propan-1-one;
(2*R*)-2-(5-fluoro-2-methoxypyridin-4-yl)-1-[(3*S*)-3-{[6-methyl-5-(2-methyl-2*H*-tetrazol-5-yl)pyridin-2-yl]amino}pyrrolidin-1-yl]propan-1-one;
(2*R*)-2-(5-chloro-2-methoxypyridin-4-yl)-1-[(3*S*)-3-{[6-methyl-5-(pyrimidin-2-yl)pyridin-2-yl]amino}pyrrolidin-1-yl]propan-1-one;
(2*R*)-1-[(3*S*)-3-({5-[5-(difluoromethyl)-1-methyl-1*H*-1,2,4-triazol-3-yl]-6-methylpyridin-2-yl}amino)pyrrolidin-1-yl]-2-(5-fluoro-2-methoxypyridin-4-yl)propan-1-one;
(2*R*)-1-[(3*S*)-3-({5-[5-(difluoromethyl)-1-(²H₃)methyl-1*H*-1,2,4-triazol-3-yl]-6-methylpyridin-2-yl}amino)pyrrolidin-1-yl]-2-(5-fluoro-2-methoxypyridin-4-yl)propan-1-one;
2-(5-chloro-2-methoxypyridin-4-yl)-1-[(3*S*)-3-({5-[5-(difluoromethyl)-1-methyl-1*H*-1,2,4-triazol-3-yl]-6-methylpyridin-2-yl}amino)pyrrolidin-1-yl]propan-1-one, DIAST-2;
2-(5-chloro-2-methoxypyridin-4-yl)-1-[(3*S*)-3-({6-methyl-5-[1-methyl-5-(trifluoromethyl)-1*H-*1,2,4-triazol-3-yl]pyridin-2-yl}amino)pyrrolidin-1-yl]propan-1-one, DIAST-2; and
(2*R*)-2-(5-fluoro-2-methoxypyridin-4-yl)-1-[(3*S*)-3-{[6-methyl-5-([1,2,4]triazolo[1,5-*a*]pyridin-2-yl)pyridin-2-yl]amino}pyrrolidin-1-yl]propan-1-one, or pharmaceutically acceptable salt thereof.

14. A pharmaceutical composition comprising a therapeutically effective amount of a compound or pharmaceutically acceptable salt of any one of claims 1 to 13 and a pharmaceutically acceptable carrier.

15. A compound or pharmaceutically acceptable salt of any one of claims 1 to 13 for use in treating a condition, disease, or disorder selected from selected from cachexia; anorexia or anorexia nervosa; nausea; emesis; weight loss; failure to thrive; sarcopenia; muscle wasting; muscle weakness; frailty; osteoporosis; bone disorders; pain; neuropathic pain; anxiety; depression; hypertension; malnutrition; obesity; sexual dysfunction; and inflammatory disease.

16. A method for antagonizing a melanocortin-4 receptor (MC4R) comprising contacting the MC4R with a compound or pharmaceutically acceptable salt of any one of claims 1 to 13; with the proviso that the method is not a method for treatment of the human or animal body by therapy or a diagnostic method practiced on the human or animal body.

## Patentansprüche

1. Verbindung der Formel I: 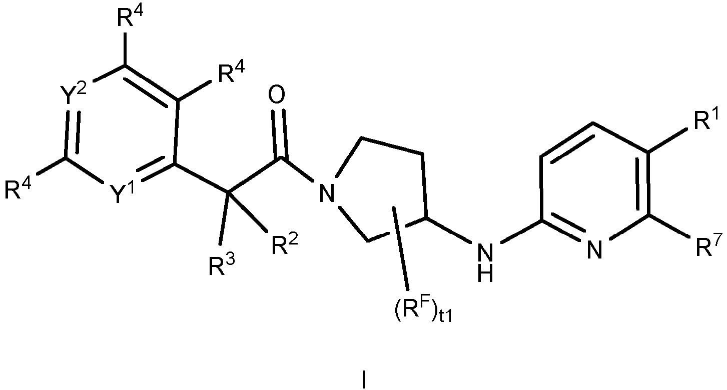 oder ein pharmazeutisch annehmbares Salz davon, wobei:
R¹ 1,2,4-Triazolyl, Tetrazolyl, 1,2,4-Oxadiazolyl, Imidazolyl, 6,7-Dihydro-[1,2,4]triazolo[5,1-b][1,3]oxazinyl, [1,2,4]Triazolo[1,5-a]pyridinyl-, Pyrazolyl oder Pyrimidinyl ist, von denen jedes gegebenenfalls mit 1, 2 oder 3 Substituenten substituiert ist, die jeweils unabhängig aus Halogen, C₁₋₄-Alkyl, C₁₋₄-Halogenalkyl und C₃₋₄-Cycloalkyl ausgewählt sind;
jedes von R² und R³ unabhängig H, Halogen, C₁₋₄-Alkyl oder C₁₋₄-Halogenalkyl ist;
oder R² und R³ zusammen mit dem Kohlenstoffatom, an das sie gebunden sind, ein C₃₋₄-Cycloalkyl bilden, das gegebenenfalls mit 1, 2, 3, 4 oder 5 Substituenten substituiert ist, die jeweils unabhängig aus Halogen ausgewählt sind;
jedes von Y¹ und Y² unabhängig CR⁴ oder N ist;
jedes R⁴ unabhängig H, Halogen, C₁₋₄-Alkyl, C₁₋₄-Halogenalkyl, C₁₋₄-Alkoxy oder C₁₋₄-Halogenalkoxy ist;
jedes R^{F} unabhängig Halogen, -OH, C₁₋₄-Alkyl, C₁₋₄-Halogenalkyl, C₁₋₄-Alkoxy, C₁₋₄-Halogenalkoxy, C₃₋₄-Cycloalkyl oder (C₃₋₄-Cycloalkyl)-C₁₋₄-alkyl- ist;
oder wenn zwei R^{F}-Gruppen an demselben Kohlenstoffatom des Pyrrolidinrings der Formel I substituiert sind, sie zusammen mit dem Kohlenstoffatom, an das sie gebunden sind, gegebenenfalls ein C₃₋₆-Cycloalkyl bilden, das gegebenenfalls mit 1, 2, 3, 4 oder 5 Substituenten substituiert ist, die jeweils unabhängig aus Halogen, -OH, C₁₋₄-Alkyl, C₁₋₄-Halogenalkyl, C₁₋₄-Alkoxy und C₁₋₄-Halogenalkoxy ausgewählt sind;
R⁷ Methyl oder Ethyl ist; und
t1 0, 1, 2, 3 oder 4 ist.

2. Verbindung nach Anspruch 1, wobei die Verbindung eine Verbindung der Formel I-a ist: 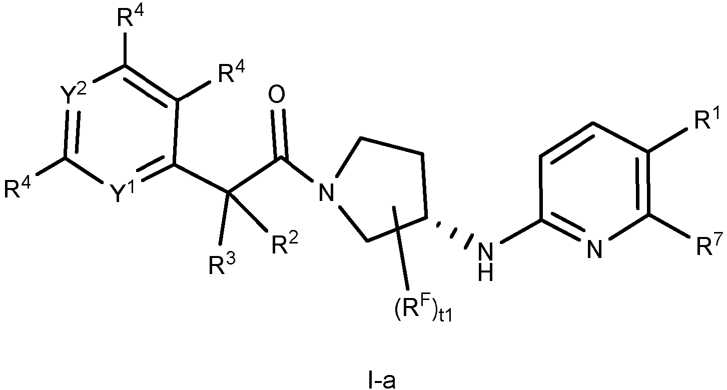 oder ein pharmazeutisch annehmbares Salz davon.

3. Verbindung nach Anspruch 1 oder 2, wobei die Verbindung eine Verbindung der Formel I-b ist: 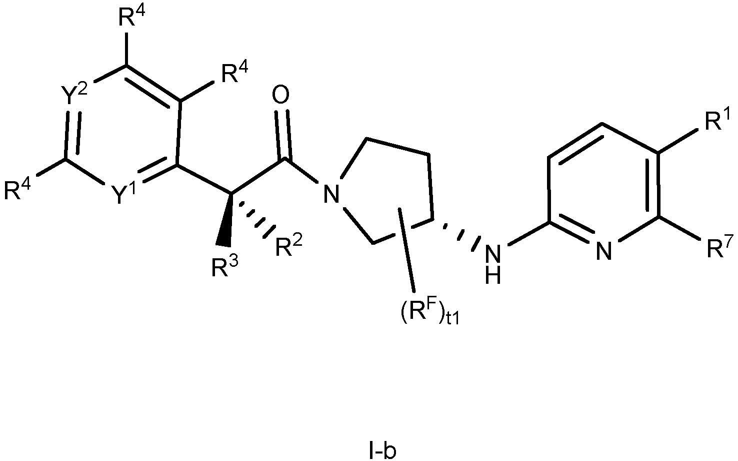 oder ein pharmazeutisch annehmbares Salz davon.

4. Verbindung nach Anspruch 1, wobei die Verbindung eine Verbindung der Formel II ist: 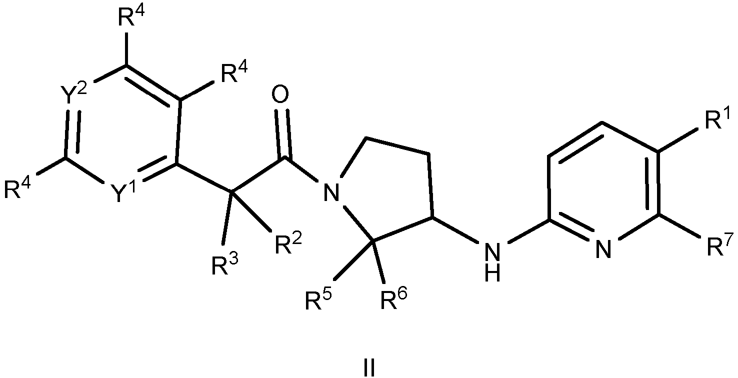
oder ein pharmazeutisch annehmbares Salz davon, wobei:
jedes von R⁵ und R⁶ unabhängig H, Methyl oder Ethyl ist;
oder R⁵ und R⁶ zusammen mit dem Kohlenstoffatom, an das sie gebunden sind, ein C₃₋₆-Cycloalkyl bilden, das gegebenenfalls mit 1, 2, 3, 4 oder 5 Substituenten substituiert ist, die jeweils unabhängig aus Halogen, -OH, C₁₋₄-Alkyl, C₁₋₄-Halogenalkyl, C₁₋₄-Alkoxy und C₁₋₄-Halogenalkoxy ausgewählt sind.

5. Verbindung oder pharmazeutisch annehmbares Salz nach Anspruch 1 oder 4, wobei die Verbindung eine Verbindung der Formel III ist: 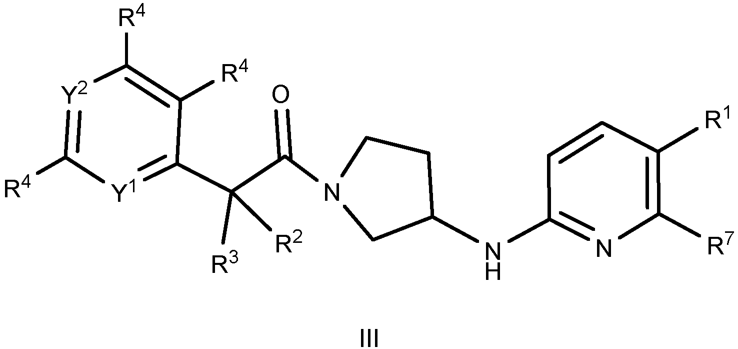 oder ein pharmazeutisch annehmbares Salz davon.

6. Verbindung oder pharmazeutisch annehmbares Salz nach einem der Ansprüche 1 bis 5, wobei R¹ 1,2,4-Triazolyl, Tetrazolyl, 1,2,4-Oxadiazolyl oder Imidazolyl ist, von denen jedes gegebenenfalls mit 1, 2 oder 3 Substituenten substituiert ist, die jeweils unabhängig aus Halogen, C₁₋₄-Alkyl, C₁₋₄-Halogenalkyl und C₃₋₄-CyCloalkyl ausgewählt sind.

7. Verbindung oder pharmazeutisch annehmbares Salz nach einem der Ansprüche 1 bis 5, wobei R¹ 6,7-Dihydro-[1,2,4]triazolo[5,1-b][1,3]oxazinyl oder [1,2,4]Triazolo[1,5-a]pyridinyl- ist, von denen jedes gegebenenfalls mit 1, 2 oder 3 Substituenten substituiert ist, die jeweils unabhängig aus Halogen, C₁₋₄-Alkyl, C₁₋₄-Halogenalkyl und C₃₋₄-Cycloalkyl ausgewählt sind.

8. Verbindung oder pharmazeutisch annehmbares Salz nach einem der Ansprüche 1 bis 5, wobei R¹ Pyrimidinyl ist, das gegebenenfalls mit 1, 2 oder 3 Substituenten substituiert ist, die jeweils unabhängig aus Halogen, C₁₋₄-Alkyl, C₁₋₄-Halogenalkyl und C₃₋₄-Cycloalkyl ausgewählt sind.

9. Verbindung oder pharmazeutisch annehmbares Salz nach einem der Ansprüche 1 bis 8, wobei jedes von R² und R³ unabhängig H, F oder C₁₋₄-Alkyl ist.

10. Verbindung oder pharmazeutisch annehmbares Salz nach einem der Ansprüche 1 bis 9, wobei R⁷ Methyl ist.

11. Verbindung oder pharmazeutisch annehmbares Salz nach einem der Ansprüche 1 bis 10, wobei jedes von Y¹ und Y² unabhängig CR⁴ ist; oder wobei eines von Y¹ und Y² N ist und das andere von Y¹ und Y² CR⁴ ist; oder wobei Y¹ N ist und Y² N ist.

12. Verbindung oder pharmazeutisch annehmbares Salz nach einem der Ansprüche 1 bis 11, wobei jedes R⁴ unabhängig H, Halogen, C₁₋₂-Alkyl, C₁₋₂-Halogenalkyl, C₁₋₂-Alkoxy oder C₁₋₂-Halogenalkoxy ist.

13. Verbindung nach Anspruch 1, ausgewählt aus:
(2*R*)-2-(5-Chlor-2-methoxypyridin-4-yl)-1-[(3*S*)*-*3-{[6-methyl-5-(2-methyl-2*H-*tetrazol-5-yl)pyridin-2-yl]amino}pyrrolidin-1-yl]propan-1-on;
(2*R*)-2-(5-Fluor-2-methoxypyridin-4-yl)-1-[(3*S*)-3-({6-methyl-5-[1-methyl-5-(trifluormethyl)-1*H*-1,2,4-triazol-3-yl]pyridin-2-yl}amino)pyrrolidin-1-yl]propan-1-on;
(2*R*)-1-[(3*S*)-3-({5-[5-(1,1-Difluorethyl)-1-methyl-1*H*-1,2,4-triazol-3-yl]-6-methylpyridin-2-yl}amino)pyrrolidin-1-yl]-2-(5-fluor-2-methoxypyridin-4-yl)propan-1-on;
(2*R*)-2-(5-Fluor-2-methoxypyridin-4-yl)-1-[(3*S*)-3-{[6-methyl-5-(2-methyl-2*H-*tetrazol-5-yl)pyridin-2-yl]amino}pyrrolidin-1-yl]propan-1-on;
(2*R*)-2-(5-Chlor-2-methoxypyridin-4-yl)-1-[(3*S*)-3-{[6-methyl-5-(pyrimidin-2-yl)pyridin-2-yl]amino}pyrrolidin-1-yl]propan-1-on;
(2*R*)-1-[(3*S*)-3-({5-[5-(Difluormethyl)-1-methyl-1*H*-1,2,4-triazol-3-yl]-6-methylpyridin-2-yl}amino)pyrrolidin-1-yl]-2-(5-fluor-2-methoxypyridin-4-yl)propan-1-on;
(2*R*)-1-[(3*S*)-3-({5-[5-(Difluormethyl)-1-(²H₃)methyl-1*H*-1,2,4-triazol-3-yl]-6-methylpyridin-2-yl}amino)pyrrolidin-1-yl]-2-(5-fluor-2-methoxypyridin-4-yl)propan-1-on;
2-(5-Chlor-2-methoxypyridin-4-yl)-1-[(3*S*)-3-({5-[5-(difluormethyl)-1-methyl-1*H*-1,2,4-triazol-3-yl]-6-methylpyridin-2-yl}amino)pyrrolidin-1-yl]propan-1-on, DIAST-2;
2-(5-Chlor-2-methoxypyridin-4-yl)-1-[(3*S*)-3-({6-methyl-5-[1-methyl-5-(trifluormethyl)-1*H*-1,2,4-triazol-3-yl]pyridin-2-yl}amino)pyrrolidin-1-yl]propan-1-on, DIAST-2; und
(2*R*)-2-(5-Fluor-2-methoxypyridin-4-yl)-1-[(3*S*)-3-{[6-methyl-5-([1,2,4]triazolo[1,5-*a*]pyridin-2-yl)pyridin-2-yl]amino}pyrrolidin-1-yl]propan-1-on,
oder ein pharmazeutisch annehmbares Salz davon.

14. Pharmazeutische Zusammensetzung, umfassend eine therapeutisch wirksame Menge einer Verbindung oder eines pharmazeutisch annehmbaren Salzes nach einem der Ansprüche 1 bis 13 und einen pharmazeutisch annehmbaren Träger.

15. Verbindung oder pharmazeutisch annehmbares Salz nach einem der Ansprüche 1 bis 13 zur Verwendung bei der Behandlung eines Zustands, einer Erkrankung oder Störung, ausgewählt aus Kachexie; Anorexie oder Anorexia nervosa; Übelkeit; Erbrechen; Gewichtsverlust; Gedeihstörung; Sarkopenie; Muskelschwund; Muskelschwäche; Gebrechlichkeit; Osteoporose; Knochenstörungen; Schmerz; neuropathischem Schmerz; Angst; Depression; Bluthochdruck; Mangelernährung; Fettleibigkeit; sexueller Dysfunktion und entzündlicher Erkrankung.

16. Verfahren zur Antagonisierung eines Melanocortin-4-Rezeptors (MC4R), umfassend das Inkontaktbringen des MC4R mit einer Verbindung oder einem pharmazeutisch annehmbaren Salz nach einem der Ansprüche 1 bis 13; mit der Maßgabe, dass das Verfahren kein Verfahren zur Behandlung des menschlichen oder tierischen Körpers durch Therapie oder ein diagnostisches Verfahren ist, das am menschlichen oder tierischen Körper durchgeführt wird.

## Revendications

1. Composé de Formule I : 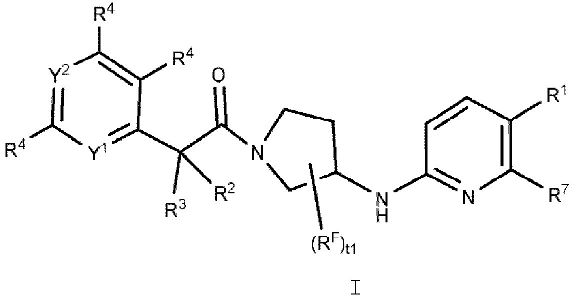 ou sel pharmaceutiquement acceptable de celui-ci, dans lequel :
R¹ est 1,2,4-triazolyle, tétrazolyle, 1,2,4-oxadiazolyle, imidazolyle, 6,7-dihydro-[1,2,4]triazolo[5,1-b][1,3]oxazinyle, [1,2,4]triazolo[1,5-a]pyridinyl-, pyrazolyle ou pyrimidinyle, chacun étant optionnellement substitué par 1, 2 ou 3 substituants chacun indépendamment sélectionnés parmi halogène, alkyle en C₁₋₄, haloalkyle en C₁₋₄ et cycloalkyle en C₃₋₄ ;
chacun de R² et R³ est indépendamment H, halogène, alkyle en C₁₋₄ ou haloalkyle en C₁₋₄ ;
ou R² et R³ avec l'atome de carbone auquel ils sont liés forment un cycloalkyle en C₃₋₄ optionnellement substitué par 1, 2, 3, 4 ou 5 substituants chacun indépendamment sélectionnés parmi halogène ;
chacun de Y¹ et Y² est indépendamment CR⁴ ou N ;
chaque R⁴ est indépendamment H, halogène, alkyle en C₁₋₄, haloalkyle en C₁₋₄, alcoxy en C₁₋₄ ou haloalcoxy en C₁₋₄ ;
chaque R^{F} est indépendamment halogène, -OH, alkyle en C₁₋₄, haloalkyle en C₁₋₄, alcoxy en C₁₋₄, haloalcoxy en C₁₋₄, cycloalkyle en C₃₋₄ ou (cycloalkyle en C₃₋₄)-alkyle en C₁₋₄ ;
ou lorsque deux groupes R^{F} sont substitués sur le même atome de carbone du cycle pyrrolidine de Formule I, ils forment optionnellement avec l'atome de carbone auquel ils sont liés un cycloalkyle en C₃₋₆ optionnellement substitué par 1, 2, 3, 4 ou 5 substituants chacun indépendamment sélectionnés parmi halogène, -OH, alkyle en C₁₋₄, haloalkyle en C₁₋₄, alcoxy en C₁₋₄ et haloalcoxy en C₁₋₄ ;
R⁷ est méthyle ou éthyle ; et
t1 vaut 0, 1, 2, 3 ou 4.

2. Composé selon la revendication 1, dans lequel le composé est un composé de Formule I-a : 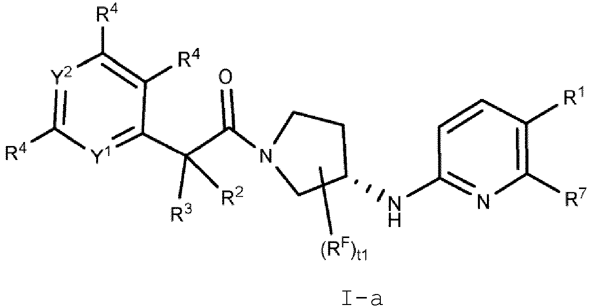 ou un sel pharmaceutiquement acceptable de celui-ci.

3. Composé selon la revendication 1 ou 2, dans lequel le composé est un composé de Formule I-b : 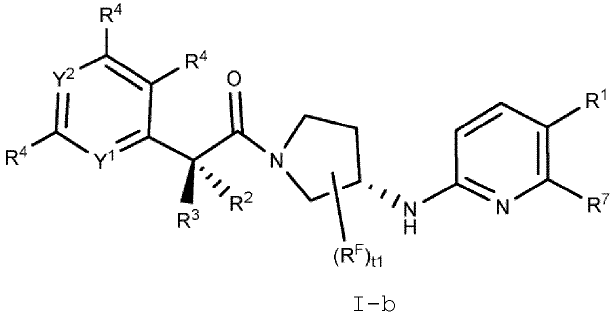 ou un sel pharmaceutiquement acceptable de celui-ci.

4. Composé selon la revendication 1, dans lequel le composé est un composé de Formule II : 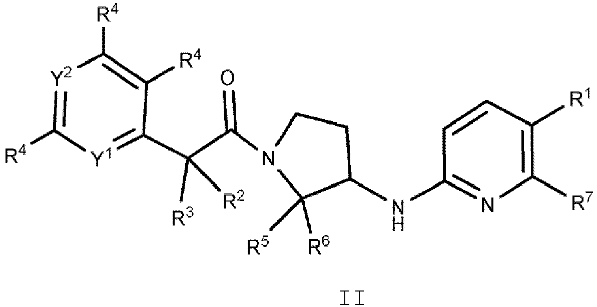 ou un sel pharmaceutiquement acceptable de celui-ci, dans lequel :
chacun de R⁵ et R⁶ est indépendamment H, méthyle ou éthyle ;
ou R⁵ et R⁶ avec l'atome de carbone auquel ils sont liés forment un cycloalkyle en C₃₋₆ optionnellement substitué par 1, 2, 3, 4 ou 5 substituants chacun indépendamment sélectionnés parmi halogène, -OH, alkyle en C₁₋₄, haloalkyle en C₁₋₄, alcoxy en C₁₋₄ et haloalcoxy en C₁₋₄.

5. Composé ou sel pharmaceutiquement acceptable selon la revendication 1 ou 4, dans lequel le composé est un composé de Formule III : 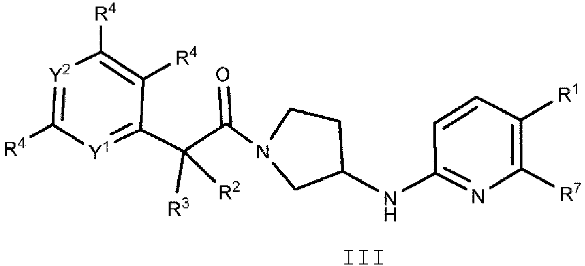 ou un sel pharmaceutiquement acceptable de celui-ci.

6. Composé ou sel pharmaceutiquement acceptable selon l'une des revendications 1 à 5 dans lequel R¹ est 1,2,4-triazolyle, tétrazolyle, 1,2,4-oxadiazolyle ou imidazolyle, chacun étant optionnellement substitué 1, 2 ou 3 substituants chacun indépendamment sélectionnés parmi halogène, alkyle en C₁₋₄, haloalkyle en C₁₋₄ et cycloalkyle en C₃₋₄.

7. Composé ou sel pharmaceutiquement acceptable selon l'une des revendications 1 à 5 dans lequel R¹ est 6,7-dihydro-[1,2,4]triazolo[5,1-b][1,3]oxazinyle ou [1,2,4]triazolo[1,5-a]pyridinyl-, chacun étant optionnellement substitué par 1, 2 ou 3 substituants chacun indépendamment sélectionnés parmi halogène, alkyle en C₁₋₄, haloalkyle en C₁₋₄ et cycloalkyle en C₃₋₄.

8. Composé ou sel pharmaceutiquement acceptable selon l'une des revendications 1 à 5, dans lequel R¹ est pyrimidinyle optionnellement substitué par 1, 2 ou 3 substituants chacun indépendamment sélectionnés parmi halogène, alkyle en C₁₋₄, haloalkyle en C₁₋₄ et cycloalkyle en C₃₋₄.

9. Composé ou sel pharmaceutiquement acceptable selon l'une des revendications 1 à 8, dans lequel chacun de R² et R³ est indépendamment H, F ou alkyle en C₁₋₄.

10. Composé ou sel pharmaceutiquement acceptable selon l'une des revendications 1 à 9, dans lequel R⁷ est méthyle.

11. Composé ou sel pharmaceutiquement acceptable selon l'une des revendications 1 à 10, dans lequel chacun de Y¹ et Y² est indépendamment CR⁴ ; ou dans lequel l'un de Y¹ et Y² est N, et l'autre de Y¹ et Y² est CR⁴ ; ou dans lequel Y¹ est N et Y² est N.

12. Composé ou sel pharmaceutiquement acceptable selon l'une des revendications 1 à 11, dans lequel chaque R⁴ est indépendamment H, halogène, alkyle en C₁₋₂, haloalkyle en C₁₋₂, alcoxy en C₁₋₂ ou haloalcoxy en C₁₋₂.

13. Composé selon la revendication 1, sélectionné parmi :
(2*R*)-2-(5-chloro-2-méthoxypyridin-4-yl)-1-[(3*S*)-3-{[6-méthyl-5-(2-méthyl-2*H*-tétrazol-5-yl)pyridin-2-yl]amino}pyrrolidin-1-yl]propan-1-one ;
(2*R*)-2-(5-fluoro-2-méthoxypyridin-4-yl)-1-[(3*S*)-3-({6-méthyl-5-[1-méthyl-5-(trifluorométhyl)-1*H*-1,2,4-triazol-3-yl]pyridin-2-yl}amino)pyrrolidin-1-yl]propan-1-one ;
(2*R*)-1-[(3*S*)-3-({5-[5-(1,1-difluoroéthyl)-1-méthyl-1*H*-1,2,4-triazol-3-yl]-6-méthylpyridin-2-yl}amino)pyrrolidin-1-yl]-2-(5-fluoro-2-méthoxypyridin-4-yl)propan-1-one ;
(2*R*)-2-(5-fluoro-2-méthoxypyridin-4-yl)-1-[(3*S*)-3-{[6-méthyl-5-(2-méthyl-2*H*-tétrazol-5-yl)pyridin-2-yl]amino}pyrrolidin-1-yl]propan-1-one ;
(2*R*)-2-(5-chloro-2-méthoxypyridin-4-yl)-1-[(3*S*)-3-{[6-méthyl-5-(pyrimidin-2-yl)pyridin-2-yl]amino}pyrrolidin-1-yl]propan-1-one ;
(2*R*)-1-[(3*S*)-3-({5-[5-(difluorométhyl)-1-méthyl-1*H*-1,2,4-triazol-3-yl]-6-méthylpyridin-2-yl}amino)pyrrolidin-1-yl]-2-(5-fluoro-2-méthoxypyridin-4-yl)propan-1-one ;
(2*R*)-1-[(3*S*)-3-({5-[5-(difluorométhyl)-1-(²H₃)méthyl-1*H*-1,2,4-triazol-3-yl]-6-méthylpyridin-2-yl}amino)pyrrolidin-1-yl]-2-(5-fluoro-2-méthoxypyridin-4-yl)propan-1-one ;
2-(5-chloro-2-méthoxypyridin-4-yl)-1-[(3*S*)-3-({5-[5-(difluorométhyl)-1-méthyl-1*H*-1,2,4-triazol-3-yl]-6-méthylpyridin-2-yl}amino)pyrrolidin-1-yl]propan-1-one, DIAST-2 ;
2-(5-chloro-2-méthoxypyridin-4-yl)-1-[(3*S*)-3-({6-méthyl-5-[1-méthyl-5-(trifluorométhyl)-1*H*-1,2,4-triazol-3-yl]pyridin-2-yl}amino)pyrrolidin-1-yl]propan-1-one, DIAST-2 ; et
(2*R*)-2-(5-fluoro-2-méthoxypyridin-4-yl)-1-[(3*S*)-3-{[6-méthyl-5-([1,2,4]triazolo[1,5-*a*]pyridin-2-yl)pyridin-2-yl]amino}pyrrolidin-1-yl]propan-1-one, ou un sel pharmaceutiquement acceptable de ceux-ci.

14. Composition pharmaceutique comprenant une quantité thérapeutiquement efficace d'un composé ou d'un sel pharmaceutiquement acceptable selon l'une des revendications 1 à 13 et un support pharmaceutiquement acceptable.

15. Composé ou sel pharmaceutiquement acceptable selon l'une des revendications 1 à 13 pour utilisation dans le traitement d'une affection, d'une maladie ou d'un trouble sélectionné(e) parmi cachexie ; anorexie ou anorexie nerveuse ; nausée ; vomissement ; perte de poids ; retard de croissance ; sarcopénie ; perte musculaire ; faiblesse musculaire ; fragilité ; ostéoporose ; troubles osseux ; douleur ; douleur neuropathique ; anxiété ; dépression ; hypertension ; malnutrition ; obésité ; dysfonctionnement sexuel ; et maladie inflammatoire.

16. Procédé d'antagonisation d'un récepteur de mélanocortine-4 (MC4R) comprenant la mise en contact du MC4R avec un composé ou un sel pharmaceutiquement acceptable selon l'une des revendications 1 à 13 ; à condition que le procédé ne soit pas un procédé de traitement du corps humain ou animal par thérapie ou un procédé de diagnostic pratiqué sur le corps humain ou animal.
